# EUROPÄISCHE PATENTSCHRIFT

(11) **EP 1 392 662 B1**
(45) Veröffentlichungstag und Bekanntmachung des Hinweises auf die Patenterteilung: **07.01.2009**
(21) Anmeldenummer: 02738100.3
(22) Anmeldetag: 23.05.2002
(51) Int. Cl.: C07D 239/48, A61K 31/505, A61K 31/506, A61P 35/00, C07D 239/47, C07D 239/34, C07D 239/42, C07D 405/12, C07D 401/12, C07D 403/12, C07D 409/12, C07D 417/12

(54) **CDK INHIBITORISCHE PYRIMIDINE, DEREN HERSTELLUNG UND VERWENDUNG ALS ARZNEIMITTEL**
CDK INHIBITING PYRIMIDINES, PRODUCTION THEREOF AND THEIR USE AS MEDICAMENTS
PYRIMIDINE INHIBITRICE DE LA CDK, SA PRODUCTION ET SON UTILISATION COMME MEDICAMENT

(30) Priorität: 29.05.2001 DE 10127581; 11.03.2002 DE 10212098
(43) Veröffentlichungstag der Anmeldung: 03.03.2004
(73) Patentinhaber: Bayer Schering Pharma Aktiengesellschaft, 13353 Berlin (DE)
(72) Erfinder: BRUMBY, Thomas, 12163 Berlin (DE); JAUTELAT, Rolf, 10439 Berlin (DE); PRIEN, Olaf, 10711 Berlin (DE); SCHÄFER, Martina, 13187 Berlin (DE); SIEMEISTER, Gerhard, 13503 Berlin (DE); LÜCKING, Ulrich, 11115 Berlin (DE); HUWE, Christoph, 13005 Berlin (DE)
(86) Internationale Anmeldenummer: PCT/EP2002/005669
(87) Internationale Veröffentlichungsnummer: WO 2002/096888

(56) Entgegenhaltungen:
- EP-A- 0 224 339
- EP-A- 0 310 550
- WO-A-00/39101
- WO-A-00/53595
- WO-A-01/14375
- WO-A-02/04429
- WO-A-98/33798
- WO-A-99/50251
- DE-A- 4 029 650
- JP-A- 3 127 790
- DING, SHENG ET AL: "A Combinatorial Scaffold Approach toward Kinase-Directed Heterocycle Libraries" JOURNAL OF THE AMERICAN CHEMICAL SOCIETY (2002), 124(8), 1594-1596, XP002210160
- DATABASE CHEMCATS [Online] CHEMICAL ABSTRACTS SERVICE, COLUMBUS, OHIO, US; 21. Januar 2002 (2002-01-21) retrieved from STN XP002210161 & "Ambinter Exploratory Library" , AMBINTER , F-75016 PARIS
- DATABASE CHEMCATS [Online] CHEMICAL ABSTRACTS SERVICES, COLUMBUS, OHIO, US; retrieved from STN XP002210162 & "Oak Samples Product List" 8. Oktober 2001 (2001-10-08) , OAK SAMPLES LTD. , 03680 KIEV-142, UKRAINE
- DATABASE CROSSFIRE BEILSTEIN [Online] Beilstein Institut zur Förderung der Chemischen Wissenschaften, Frankfurt am Main, DE; Database accession no. BRN 249340, 265505 XP002210163 & NAITO, I. ET AL.: CHEM. PHARM. BULL., Bd. 6, 1958, Seiten 338-341,
- BOSCHELLI D H ET AL: "SYNTHESIS AND TYROSINE KINASE INHIBITORY ACTIVITY OF A SERIES OF 2-AMINO-8-H-PYRIDOÄ2,3-DÜPYRIMIDINES: IDENTIFICATION OF POTENT, SELECTIVE PLATELET-DERIVED GROWTH FACTOR RECEPTOR TYROSINE KINASE INHIBITORS" JOURNAL OF MEDICINAL CHEMISTRY, AMERICAN CHEMICAL SOCIETY. WASHINGTON, US, Bd. 41, Nr. 22, 1998, Seiten 4365-4377, XP002191993 ISSN: 0022-2623

## Beschreibung

Die vorliegende Erfindung betrifft Pyrimidinderivate, deren Herstellung sowie deren Verwendung als Medikament zur Behandlung verschiedener Erkrankungen.

Die CDKs (cyclin-dependent kinase) ist eine Enzymfamilie, die eine wichtige Rolle bei der Regulation des Zellzyklus spielt und somit ein besonders interessantes Ziel für die Entwicklung kleiner inhibitorischer Moleküle ist. Selektive Inhibitoren der CDKs können zur Behandlung von Krebs oder anderen Erkrankungen, die Störungen der Zellproliferation zur Ursache haben, verwendet werden.

Pyrimidine und Analoga sind bereits als Wirkstoffe beschrieben wie beispielsweise die 2-Anilino-Pyrimidine als Fungizide (DE 4029650) oder substituierte Pyrimidinderivate zur Behandlung von neurologischen oder neurodegenerativen Erkrankungen (WO 99/19305). Als CDK-Inhibitoren werden unterschiedlichste Pyrimidinderivate beschrieben, beispielsweise Bis(anilino)-pyrimidinderivate (WO 00/12486) oder 2-Amino-4-substituierte Pyrimidine (WO 01/ 14375), in der im Vergleich zu den Strukturen der vorliegenden Erfindung ein unterschiedliches Substitutionsmuster für Position 4 des Pyrimidins (-X- R²) offenbart wird, welches einen Imidazo[1,2a]pyrid-3-yl- oder Pyrazolo[2,3a]pyrid-3-ylring darstellen und dieser direkt an den Pyrimidinring gebunden sein muss. Purine werden in der WO 99/02162 offenbart und 5-Cyano-Pyrimidine in der WO 02/04429. WO 00/12486 (Pyrido [2,3] pyrimidines und 4-Aminopyrimidine) und WO 98/33798 offenbaren ebenfalls Anilinopyrimidine, wobei die Position 5 des Pyrimidins im Vergleich zu den Strukturen der vorliegenden Erfindung verschieden substituiert ist. N-Heterocyclyringsubstituenten in Position 4 des Pyrimidins offenbart WO 00/53595, in der zusätzlich als Anilinsubstituenten Reste der Formel (la) vorgesehen sind, die nicht -SO₂NR³R⁴ oder -SO₂R⁷ sein können. Bicyclische Heteroaryle werden in WO 99/50251 offenbart. Die Strukturen aus WO 00/39101 weisen im Vergleich zu den Strukturen der vorliegenden Erfindung ein unterschiedliches Substitutionsmuster am Anilin auf und Arylamino- oder ein Heteroarylaminoreste in Position 4 des Pyrimidins. Annellierte Pyrimidine werden in WO 99/50251 offenbart.

Als nächstliegender Stand der Technik wird die WO 02/04429 angesehen, deren Strukturen sich im Wesentlichen durch eine Cyanogruppe in Position 5 des Pyrimidins von den Strukturen der vorliegenden Efindung unterscheiden.

Die Aufgabe der vorliegenden Erfindung ist es Verbindungen bereitzustellen, die bessere Eigenschaften als die bereits bekannten Inhibitoren haben. Die hier beschriebenen Substanzen sind besser wirksam, da sie bereits im nanomolaren Bereich inhibieren und so von anderen bereits bekannten CDK-Inhibitoren wie z.B. Olomoucin und Roscovitin zu unterscheiden sind.

Es wurde nun gefunden, dass Verbindungen der allgemeinen Formel 1 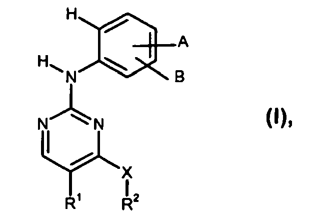 in der
- R¹: Halogen
- R²: für -CH(CH₃)-(CH₂)ₙ-R⁵, -CH-(CH₂OH)₂, -(CH₂)ₙR⁷ -CH(C₃H₇)-(CH₂)ₙ-R⁵, -CH(C₂H₅)-(CH₂)ₙ-R⁵, -CH₂-CN, -CH(CH₃)COCH₃, -CH(CH₃)-C(OH)(CH₃)₂, -CH(CH(OH)CH₃)OCH₃, -CH(C₂H₅)CO-R⁵, C₂-C₄-Alkinyl, -(CH₂)ₙ-COR⁵, -(CH₂)ₙ-CO-C₁-C₆-Alkyl, -(CH₂)ₙ-C(OH)(CH₃)-Phenyl, -CH(CH₃)-C(CH₃)-R⁵, -CH(CH₃)-C(CH₃)(C₂H₅)-R⁵,-CH(OCH₃)-CH₂-R⁵, -CH₂-CH(OH)-R⁵, -CH(OCH₃)-CHR⁵-CH₃, -CH(CH₃)-CH(OH)-CH₂-CH=CH₂, -CH(C₂H₅)-CH(OH)-(CH₂)ₙ-CH₃, -CH(CH₃)-CH(OH)-(CH₂)ₙ-CH₃, -CH(CH₃)-CH(OH)-CH(CH₃)₂, (CH₂OAC)₂, -(CH₂)ₙ-R⁶, -(CH₂)ₙ-(CF₂)ₙ-CF₃, -CH(CH₂)n-R⁵)₂, -CH(CH₃)-CO-NH₂, -CH(CH₂OH)-Phenyl, -CH(CH₂OH)-CH(OH)-(CH₂)ₙR⁵, -CH(CH₂OH) CH(OH)-Phenyl, -CH(CH₂OH)-C₂H₄-R⁵, -(CH₂)ₙ-C≡C-C(CH₃)=CH-COR⁵, -CH(Ph)-(CH₂)ₙ-R⁵, -(CH₂)ₙ-COR⁵,-(CH₂)ₙPO₃(R⁵)₂, -(CH₂)ₙ-COR⁵, -CH((CH₂)ₙOR⁵)CO-R⁵,-(CH₂)ₙCONHCH((CH₂)ₙR⁵)₂, -(CH₂)ₙNH-COR⁵,-CH(CH₂)ₙR⁵-(CH₂)ₙC₃-C₁₀-Cycloalkyl, -(CH₂)ₙ-C₃-C₁₀-Cycloalkyl, C₃-C₁₀-Cycloalkyl, gegebenenfalls ein- oder mehrfach, gleich oder verschieden mit Hydroxy, C₁-C₆-Alkyl oder der Gruppe -COONH(CH₂)ₙCH₃ oder -NR³R⁴ substituiertes C₁-C₆-Alkyl, C₃-C₁₀-Cycloalkyl, -(CH₂)ₙ-O-(CH₂)n-R⁵, -(CH2)ₙ-NR³R⁴, -CH(C₃H₇)-(CH₂)ₙ-OC(O)-(CH₂)ₙ-CH₃, -(CH₂)ₙ-R⁵, -C(CH₃)₂-(CH₂)ₙ-R⁵, -C(CH₂)ₙ(CH₃)-(CH₂)ₙR⁵, -C(CH₂)ₙ-(CH₂)ₙR⁵, -CH(t-Butyl)-(CH₂)ₙ-R⁵, -CCH₃(C₃H₇)-(CH₂)ₙR⁵, -CH(C₃H₇)-(CH₂)ₙ-R⁵, -CH(C₃H₇)-COR⁵, -CH(C₃H₇)-(CH₂)ₙ-OC(O)-NH-Ph, -CH((CH₂)ₙ(C₃H₇))-(CH₂)ₙR⁵, -CH(C₃H₇)-(CH₂)ₙ-OC(O)-NH-Ph(OR⁵)₃, -NR³R⁴, -NH-(CH₂)ₙ-NR³R⁴, R⁵-(CH₂)ₙ-C*H-CH(R⁵)(CH₂)ₙ-R⁵, -(CH₂)ₙ-CO-NH-(CH₂)ₙ-CO-R⁵, -OC(O)NH-C₁-C₆-Alkyl oder -(CH₂)ₙ-CO-NH-(CH₂)ₙ-CH-((CH₂)ₙR⁵)₂,
oder für C₃-C₁₀-Cycloalkyl steht, welches mit der Gruppe 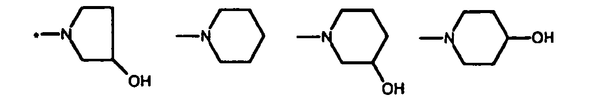 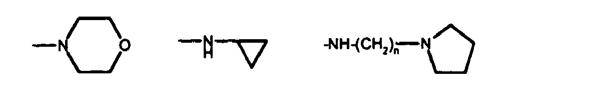 substituiert ist, oder für die Gruppe 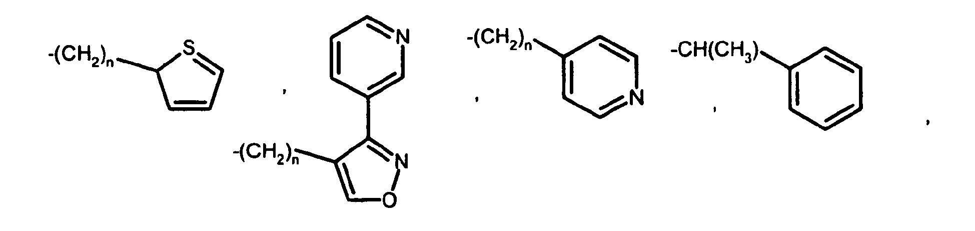 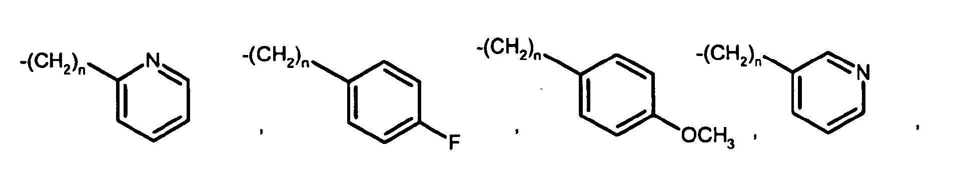 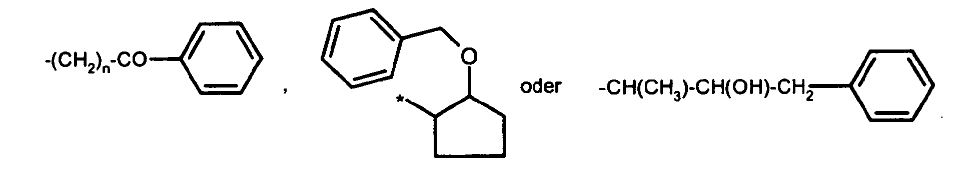 oder 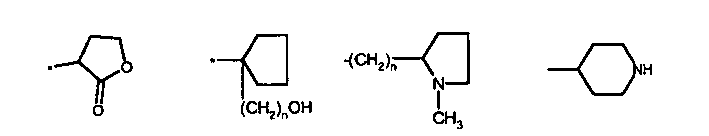 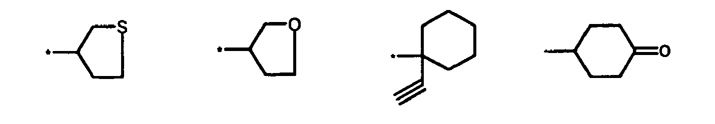 steht,
- X: für Sauerstoff oder für die Gruppe -NH- oder -N(C₁-C₃-Alkyl) steht, oder
- R²: für die Gruppe 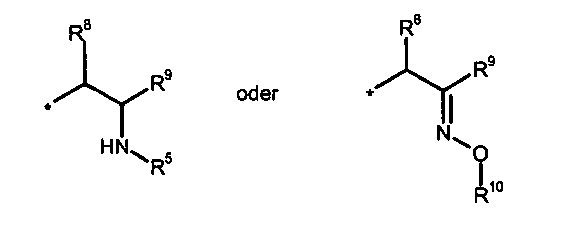 steht, oder
- X und R²: gemeinsam eine Gruppe 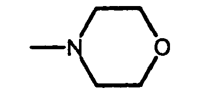 bilden,
- A: für die Gruppe -SO₂R⁷, SO₂-C₂H₄-OH, -SO₂CF₃ oder -SO₂-NR³R⁴ steht,
- B: für Wasserstoff, Hydroxy oder C₁-C₃-Alkyl steht
oder
- A und B: gemeinsam eine Gruppe 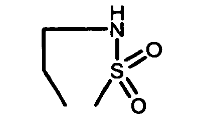 bilden können,
- R³ und R⁴: jeweils unabhängig voneinander für Wasserstoff, Phenyl, Benzyloxy, C₁-C₁₂-Alkyl, C₁-C₆-Alkoxy, C₂-C₄-Alkenyloxy, C₃-C₆-Cycloalkyl, Hydroxy, Hydroxy-C₁-C₆-alkyl, Dihydroxy-C₁-C₆-alkyl, Heteroaryl, Heterocyclo-C₃-C₁₀-alkyl, Heteroaryl-C₁-C₃-alkyl,
gegebenenfalls mit Cyano substituiertes C₃-C₆-Cycloalkyl-C₁-C₃-alkyl, oder für
gegebenenfalls ein- oder mehrfach, gleich oder verschieden mit Phenyl, Pyridyl, Phenyloxy, C₃-C₆-Cycloalkyl, C₁-C₆-Alkyl oder C₁-C₆-Alkoxy substituiertes C₁-C₆-Alkyl steht, wobei das Phenyl selbst ein oder mehrfach, gleich oder verschieden mit Halogen, Trifluormethyl, C₁-C₆-Alkyl, C₁-C₆-Alkoxy oder mit der Gruppe -SO₂NR³R⁴ substituiert sein kann,
oder für die Gruppe -(CH₂)ₙNR³R⁴, -CNHNH₂ oder - NR³R⁴ oder für 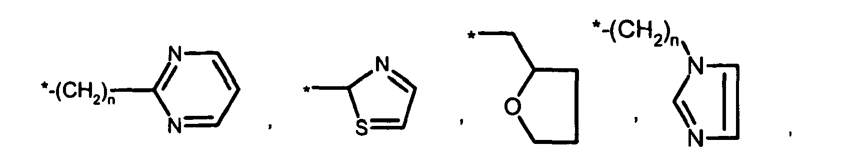 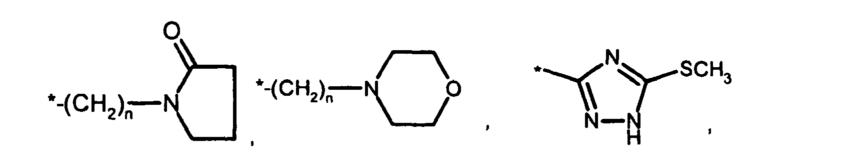 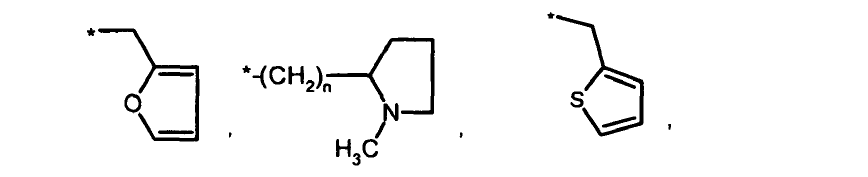 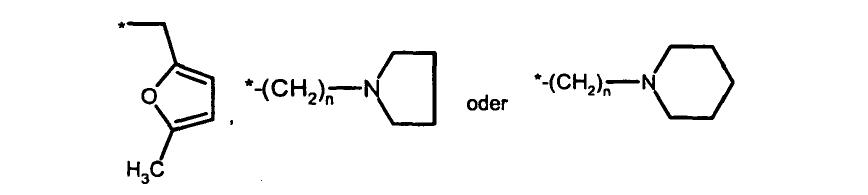 stehen, welche gegebenenfalls mit C₁-C₆-Alkyl substituiert sein können,
- R⁵: für Hydroxy, Phenyl, C₁-C₆-Alkyl, C₃-C₆-Cycloalkyl, Benzoxy, C₁-C₆-Alkylthio oder C₁-C₆-Alkoxy steht,
- R⁶: für die Gruppe 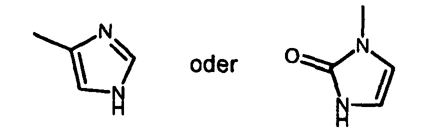 steht,
- R⁷: für Halogen, Hydroxy, Phenyl, C₁-C₆-Alkyl, -(CH₂)ₙOH, -NR³R⁴ oder die Gruppe 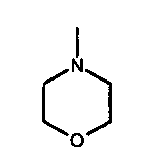 steht,
- R⁸, R⁹ und R¹⁰: für Wasserstoff, Hydroxy, C₁-C₆-Alkyl oder für die Gruppe -(CH₂)ₙ-COOH stehen, und
- n: für 0 - 6 stehen, bedeuten,
sowie deren Diastereoisomeren, Enantiomeren und Salze.

Unter Alkyl ist jeweils ein geradkettiger oder verzweigter Alkylrest, wie beispielsweise Methyl, Ethyl, Propyl, Isopropyl, Butyl, Isobutyl, sek. Butyl, tert. Butyl, Pentyl, Isopentyl, Hexyl, Heptyl, Octyl, Nonyl und Decyl, zu verstehen.

Unter Alkoxy ist jeweils ein geradkettiger oder verzweigter Alkoxyrest, wie beispielsweise Methyloxy, Ethyloxy, Propyloxy, lsopropyloxy, Butyloxy, Isobutyloxy, sek. Butyloxy, tert.-Butyloxy, Pentyloxy, Isopentyloxy oder Hexyloxy zu verstehen.

Unter Alkylthio ist jeweils ein geradkettiger oder verzweigter Alkylthiorest, wie beispielsweise Methylthio, Ethylthio, Propylthio, lsopropylthio, Butylthio, Isobutylthio, sek. Butylthio, tert.-Butylthio, Pentylthio, Isopentylthio oder Hexylthio zu verstehen.

Unter Cycloalkyl sind im allgemeinen monocyclische Alkylringe wie Cyclopropyl, Cyclobutyl, Cyclopentyl, Cyclohexyl, Cycloheptyl, Cyclooctyl, Cyclononyl oder Cyclodecyl, aber auch bicyclische Ringe oder tricyclische Ringe wie zum Beispiel Norbomyl, Adamantanyl, etc. zu verstehen.

Unter den Ringsystemen, bei denen gegebenenfalls ein- oder mehrere mögliche Doppelbindungen im Ring enthalten sein können, sind zum Beispiel Cycloalkenyle wie Cyclopropenyl, Cyclobutenyl, Cyclopentenyl, Cyclohexenyl, Cycloheptenyl zu verstehen, wobei die Anknüpfung sowohl an der Doppelbindung wie auch an den Einfachbindungen erfolgen kann.

Falls A und B, R³ und R⁴, X und R² ,jeweils unabhängig voneinander, gemeinsam einen C₃-C₁₀-Cycloalkyl-Ring bilden, der gegebenenfalls durch ein-oder mehrere Heteroatome wie Stickstoff-Atome, Sauerstoff-Atome und/ oder Schwefel-Atome unterbrochen sein kann, und/ oder durch ein oder mehrere =C=O Gruppen im Ring unterbrochen sein kann, und/ oder gegebenenfalls ein oder mehrere mögliche Doppelbindungen im Ring enthalten sein können, sind aber auch die unter Heteroarylrest bzw. Heterocycloalkyl und Heterocycloalkenyl genannten Definitionen zu verstehen.

Unter Halogen ist jeweils Fluor, Chlor, Brom oder Jod zu verstehen.

Die Alkenyl-Substituenten sind jeweils geradkettig oder verzweigt, wobei beispielsweise folgenden Reste gemeint sind: Vinyl, Propen-1-yl, Propen-2-yl, But-1-en-1-yl, But-1-en-2-yl, But-2-en-1-yl, But-2-en-2-yl, 2-Methyl-prop-2-en-1-yl, 2-Methyl-prop-1-en-1-yl, But-1-en-3-yl, Ethinyl, Prop-1-in-1-yl, But-1-in-1-yl, But-2-in-1-yl, But-3-en-1-yl, Allyl.

Unter Alkinyl ist jeweils ein geradkettiger oder verzweigter Alkinyl-Rest zu verstehen, der 2 - 6, bevorzugt 2 - 4 C-Atome enthält. Beispielsweise seien die folgenden Reste genannt: Acetylen, Propin-1-yl, Propin-3-yl, But-1-in-1-yl, But-1-in-4-yl, But-2-in-1-yl, But-1-in-3-yl, etc.

Der Arylrest umfaßt jeweils 3 -12 Kohlenstoffatome und kann jeweils benzokondensiert sein.
Beispielsweise seien genannt: Cyclopropenyl, Cyclopentadienyl, Phenyl, Tropyl, Cyclooctadienyl, Indenyl, Naphthyl, Azulenyl, Biphenyl, Fluorenyl, Anthracenyl etc.

Der Heteroarylrest umfaßt jeweils 3 - 16 Ringatome und kann anstelle des Kohlenstoffs ein- oder mehrere, gleiche oder verschiedene Heteroatome, wie Sauerstoff, Stickstoff oder Schwefel im Ring enthalten, und kann mono-, bi- oder tricyclisch sein, und kann zusätzlich jeweils benzokondensiert sein.

Beispielsweise seien genannt:
Thienyl, Furanyl, Pyrrolyl, Oxazolyl, Thiazolyl, Imidazolyl, Pyrazolyl, Isoxazolyl, Isothiazolyl, Oxadiazolyl, Triazolyl, Thiadiazolyl, etc. und Benzoderivate davon, wie z. B. Benzofuranyl, Benzothienyl, Benzoxazolyl, Benzimidazolyl, Indazolyl, Indolyl, lsoindolyl, etc.; oder Pyridyl, Pyridazinyl, Pyrimidinyl, Pyrazinyl, Triazinyl, etc. und Benzoderivate davon, wie z. B. Chinolyl, lsochinolyl, etc.; oder Azocinyl, Indolizinyl, Purinyl, etc. und Benzoderivate davon; oder Chinolinyl, Isochinolinyl, Cinnolinyl, Phthalazinyl, Chinazolinyl, Chinoxalinyl, Naphthyridinyl, Pteridinyl, Carbazolyl, Acridinyl, Phenazinyl, Phenothiazinyl, Phenoxazinyl, Xanthenyl, Oxepinyl, etc.
Heterocycloalkyl steht für einen 3 -12 Kohlenstoffatome umfassenden Alkylring, der anstelle des Kohlenstoffes ein oder mehrere, gleich oder verschiedene Heteroatome, wie z. B. Sauerstoff, Schwefel oder Stickstoff enthält.
   Als Heterocycloalkyle seien z. B. genannt: Oxiranyl, Oxethanyl, Aziridinyl, Azetidinyl, Tetrahydrofuranyl, Pyrrolidinyl, Dioxolanyl, Imidazolidinyl, Pyrazolidinyl, Dioxanyl, Piperidinyl, Morpholinyl, Dithianyl, Thiomorpholinyl, Piperazinyl, Trithianyl, Chinuclidinyl etc.
Heterocycloalkenyl steht für einen 3 - 12 Kohlenstoffatome umfassenden Alkylring, der anstelle des Kohlenstoffes ein oder mehrere, gleich oder verschiedene Heteroatome, wie z. B. Sauerstoff, Schwefel oder Stickstoff enthält, und der teilgesättigt ist.
   Als Heterocycloalkenyle seien z. B. genannt: Pyran, Thiin, Dihydroazet, etc.

Ist eine saure Funktion enthalten, sind als Salze die physiologisch verträglichen Salze organischer und anorganischer Basen geeignet, wie beispielsweise die gut löslichen Alkali- und Erdalkalisalze sowie N-Methyl-glukamin, Dimethylglukamin, Ethyl-glukamin, Lysin, 1,6-Hexadiamin, Ethanolamin, Glukosamin, Sarkosin, Serinol, Tris-hydroxy-methyl-amino-methan, Aminopropandiol, Sovak-Base, 1-Amino-2,3,4-butantriol.

Ist eine basische Funktion enthalten, sind die physiologisch verträglichen Salze organischer und anorganischer Säuren geeignet wie Salzsäure, Schwefelsäure, Phosphorsäure, Zitronensäure, Weinsäure u.a.

Besonders wirksam sind folgende Verbindungen
5-Bromo-N2-[4-(2-diethylamino-ethanesulfonyl)-phenyl]-N4-prop-2-ynyl-pyrimidine-2,4-diamine,
N2-(3-Benzenesulfonyl-phenyl)-5-bromo-N4-prop-2ynyl-pyrimidine-2,4-diamine, 5-Bromo-N2-[4-(morpholine-4-sulfonyl)-phenyl]-N4-prop-2-ynyl-pyrimidine-2,4-diamine,
4-[5-Fluoro-4-((R)-1-hydroxymethyl-2-methyl-propylamino)-pyrimidin-2-ylamino]-benzenesulfonamide,
4-[4-((R)-1-Hydroxymethyl-2-methyl-propylamino)-5-iodo-pyrimidin-2-ylamino]-benzenesulfonamide,
4-(5-Fluoro-4-prop-2-ynylamino-pyrimidin-2-ylamino)-benzenesulfonamide,
4-{5-Bromo-4-[2-(2-oxo-2,3-dihydro-imidazol-1-yl)-ethylamino]-pyrimidin-2-ylamino}-benzenesulfonamide,
4-[5-Bromo-4-(2,2,3,3,3-pentafluoro-propoxy)-pyrimidin-2-ylamino]-benzenesulfonamide,
4-[[5-Bromo-4-(1,3-bisacetoxy-2-propyloxy)-2-pyrimidinyl]amino]-benzolsulfonamide,
4-[5-Bromo-4-(2-hydroxy-1-hydroxymethyl-ethoxy)-pyrimidin-2-ylamino]-benzenesulfonamide,
(S)-2-[5-Bromo-2-(4-sulfamoyl-phenylamino)-pyrimidin-4-ylamino]-propionamide,
5-Bromo-N4-prop-2-ynyl-N2-(3-trifluoromethanesulfonyl-phenyl)-pyrimidine-2,4-diamine,
3-(5-Bromo-4-prop-2-ynylamino-pyrimidin-2-ylamino)-benzenesulfonamide,
3-(5-Bromo-4-prop-2-ynylamino-pyrimidin-2-ylamino)-N-butyl-benzenesulfonamide,
2-[3-(5-Bromo-4-prop-2-ynylamino-pyrimidin-2-ylamino)-benzenesulfonyl]-ethanol,
4-(5-Bromo-4-prop-2-ynylamino-pyrimidin-2-ylamino)-benzenesulfonamide,
4-(5-Chloro-4-prop-2-ynylamino-pyrimidin-2-ylamino)-benzenesulfonamide,
2-[5-Bromo-2-(4-sulfamoyl-phenylamino)-pyrimidin-4-ylamino]-butyric acid methyl ester,
4-[5-Bromo-4-((R)-1-hydroxymethyl-2-methyl-propylamino)-pyrimidin-2-yl amino]-benzenesulfonamide,
2-{5-Bromo-2-[3-(2-hydroxy-ethanesulfonyl)-phenylamino]-pyrimidin-4-ylamino}-propane-1,3-diol,
4-[5-Bromo-4-(2-hydroxy-1-hydroxymethyl-ethylamino)-pyrimidin-2-ylamino]-benzenesulfonamide,
4-[5-Bromo-4-(cyclopropylmethyl-amino)-pyrimidin-2-ylamino]-benzenesulfonamide,
4-[5-Bromo-4-((R)-1-hydroxymethyl-2-methyl-propylamino)-pyrimidin-2-yl amino]-N-(2-hydroxy-ethyl)-benzenesulfonamide,
4-[5-Bromo-4-((S)-2-methoxy-1-methyl-ethylamino)-pyrimidin-2-ylamino]-benzenesulfonamide,
2-{3-[5-Bromo-4-((S)-2-methoxy-1-methyl-ethylamino)-pyrimidin-2-ylamino]-benzenesulfonyl}-ethanol,
2-{3-[5-Bromo-4-(2-morpholin-4-yl-ethylamino)-pyrimidin-2-ylamino]-benzenesulfonyll-ethanol,
4-[5-Bromo-4-(2-morpholin-4-yl-ethylamino)-pyrimidin-2-ylamino]-benzenesulfonamide,
4-[5-Bromo-4-(4,4,4-trifluoro-butoxy)-pyrimidin-2-ylamino]-benzenesulfonamide,
4-[5-Bromo-4-(2-ethoxy-1-ethoxymethyl-ethoxy)-pyrimidin-2-ylamino]-benzenesulfonamide,
4-[5-Bromo-2-(4-sulfamoyl-phenylamino)-pyrimidin-4-ylamino]-butyric acid,
4-[5-Bromo-4-((R)-1-hydroxymethyl-2-methyl-propylamino)-pyrimid in-2-yl amino]-N-(2-hydroxy-ethyl)-benzenesulfonamide,
(R)-2-{5-Bromo-2-[3-(2-hydroxy-ethanesulfonyl)-phenylamino]-pyrimidin-4-ylamino}-3-methyl-butan-1-ol,
4-[5-Bromo-4-((S)-1-hydroxymethyl-2-methyl-propylamino)-pyrimidin-2-yl amino]-benzenesulfonamide,
4-[5-Bromo-4-((R)-2-hydroxy-1-phenyl-ethylamino)-pyrimidin-2-ylamino]-benzenesulfonamide,
4-[5-Bromo-4-((S)-2-methoxy-1-methyl-ethylamino)-pyrimidin-2-ylamino]-N-methyl-benzenesulfonamide,
4-[5-Bromo-4-((S)-2-methoxy-1-methyl-ethylamino)-pyrimidin-2-ylamino]-N,N-dimethyl-benzenesulfonamide,
5-[5-Bromo-2-(4-sulfamoyl-phenylamino)-pyrimidin-4-ylamino]-pentanoic acid benzyl ester,
6-[5-Bromo-2-(4-sulfamoyl-phenylamino)-pyrimidin-4-ylamino]-hexanoic acid methyl ester,
4-(5-lodo-4-prop-2-ynylamino-pyrimidin-2-ylamino)-benzenesulfonamide
4-[5-Bromo-4-((S)-1-hydroxymethyl-3-methylsulfanyl-propylamino)-pyrimidin-2-ylamino]-benzenesulfonamide,
4-[5-Bromo-4-((1 S,2S)-2-hydroxy-1-hydroxymethyl-2-phenyl-ethylamino)-pyrimidin-2-ylamino]-benzenesulfonamide,
4-[5-Bromo-4-((R)-2-oxo-tetrahydro-furan-3-ylamino)-pyrimidin-2-ylamino]-benzenesulfonamide,
4-[5-Bromo-4-((S)-1-hydroxymethyl-2,2-dimethyl-propylamino)-pyrimidin-2-ylamino]-benzenesulfonamide,
4-{3-[5-Bromo-2-(4-sulfamoyl-phenylamino)-pyrimidin-4-ylamino]-propionylamino}-butyric acid methyl ester,
6-[5-Bromo-2-(4-sulfamoyl-phenylamino)-pyrimidin-4-ylamino]-hexanoic acid,
4-(5-Bromo-4-cyclohexylamino-pyrimidin-2-ylamino)-benzenesulfonamide,
4-{6-[5-Bromo-2-(4-sulfamoyl-phenylamino)-pyrimidin-4-ylamino]-hexanoylamino}-butyric acid methyl ester,
6-[5-Bromo-2-(4-sulfamoyl-phenylamino)-pyrimidin-4-ylamino]-hexanoic acid methyl ester,
4-[5-Bromo-4-((1R,2S)-2,3-dihydroxy-1-hydroxymethyl-propylamino)-pyrimidin-2-ylamino]-benzenesulfonamide,
4-[5-Bromo-4-((R)-2-cyclohexyl-1-hydroxymethyl-ethylamino)-pyrimidin-2-ylamino]-benzenesulfonamide,
6-[5-Bromo-2-(4-sulfamoyl-phenylamino)-pyrimidin-4-ylamino]-hexanoic acid (2-hydroxy-1-hydroxymethyl-ethyl)-amide,
4-[5-Bromo-4-((S)-2-hydroxy-1-methyl-ethylamino)-pyrimidin-2-ylamino]-benzenesulfonamide,
4-[5-Bromo-4-(4-hydroxy-cyclohexylamino)-pyrimidin-2-ylamino]-benzenesulfonamide,
4-{4-[(Adamantan-1-ylmethyl)-amino]-5-bromo-pyrimidin-2-ylamino}-benzenesulfonamide,
4-[5-Bromo-4-((R)-1-hydroxymethyl-2-methyl-propylamino)-pyrimidin-2-yl amino]-N-methyl-benzenesulfonamide,
(R)-2-[5-Bromo-2-(4-sulfamoyl-phenylamino)-pyrimidin-4-ylamino]-3-methyl-butyric acid,
4-(5-Bromo-4-cyclohexylamino-pyrimidin-2-ylamino)-N-(2-hydroxy-ethyl)-benzenesulfonamide,
4-[5-Bromo-4-(2,2,3,3,4,4,4-heptafluoro-butylamino)-pyrimidin-2-ylamino]-N-(2-hydroxy-ethyl)-benzenesulfonamide,
4-[5-Bromo-4-((S)-2-methoxy-1-methyl-ethylamino)-pyrimidin-2-ylamino]-N-(2-hydroxy-ethyl)-benzenesulfonamide,
Phenyl-carbamic acid (R)-2-[5-bromo-2-(4-sulfamoyl-phenylamino)-pyrimidin-4-ylamino]-3-methyl-butyl ester,
(R)-2-{5-Bromo-2-[4-(2-hydroxy-ethylsulfamoyl)-phenylamino]-pyrimidin-4-ylamino}-3-methyl-butyric acid,
(R)-2-{5-Bromo-2-[4-(2-hydroxy-ethylsulfamoyl)-phenylamino]-pyrimidin-4-ylamino}-3-methyl-butyric acid methyl ester,
4-(5-Chloro-4-prop-2-ynylamino-pyrimidin-2-ylamino)-N-(2-hydroxy-ethyl)-benzenesulfonamide,
4-[5-Bromo-4-(2-hydroxy-1,1-dimethyl-ethylamino)-pyrimidin-2-ylamino]-benzenesulfonamide,
4-[5-Bromo-4-((S)-1-hydroxymethyl-3-methyl-butylamino)-pyrimidin-2-ylamino]-benzenesulfonamide,
4-[5-Bromo-4-(tetrahydro-pyran-4-yloxy)-pyrimidin-2-ylamino]-benzenesulfonamide,
4-[5-Bromo-4-((1 S,2S)-2-hydroxy-cyclohexylamino)-pyrimidin-2-ylamino]-benzenesulfonamide,
Butyl-carbamic acid 4-[5-bromo-2-(4-sulfamoyl-phenylamino)-pyrimidin-4-ylamino]-cyclohexyl ester,
(R)-2-[5-Bromo-2-(4-methanesulfonyl-phenylamino)-pyrimidin-4-ylamino]-3-methyl-butan-1-ol,
(R)-2-{5-Bromo-2-[4-(morpholine-4-sulfonyl)-phenylamino]-pyrimidin-4-ylamino}-3-methyl-butan-1-ol,
4-[5-Bromo-4-(4-dimethylamino-cyclohexylamino)-pyrimidin-2-ylamino]-benzenesulfonamide,
4-[5-Bromo-4-(4-dimethylamino-cyclohexylamino)-pyrimidin-2-ylamino]-benzenesulfonamide,
4-[5-Bromo-4-(4-piperidin-1-yl-cyclohexylamino)-pyrimidin-2-ylamino]-benzenesulfonamide,
4-[5-Bromo-4-((R)-1-hydroxymethyl-2-methyl-propylamino)-pyrimidin-2-yl amino]-3-hydroxy-benzenesulfonamide,
4-[5-Bromo-4-(4-cyclopropylamino-cyclohexylamino)-pyrimidin-2-ylamino] -benzenesulfonamide,
(R)-2-[5-Bromo-2-(3-methanesulfonyl-phenylamino)-pyrimidin-4-ylamino]-3-methyl-butan-1-ol,
4-{5-Bromo-4-[2-(2-ethoxy-ethoxy)-ethoxy]-pyrimidin-2-ylamino}-benzenesulfonamide,
4-{5-Bromo-4-[4-(2-dimethylamino-ethylamino)-cyclohexylamino]-pyrimidin-2-ylamino}-benzenesulfonamide,
4-{5-Bromo-4-[4-(2-pyrrolidin-1-yl-ethylamino)-cyclohexylamino]-pyrimidin-2-ylamino}-benzenesulfonamide,
4-{5-Bromo-4-[4-(4-hydroxy-piperidin-1-yl)-cyclohexylamino]-pyrimidin-2-ylamino}-benzenesulfonamide,
4-{5-Bromo-2-[4-(2-hydroxy-ethylsulfamoyl)-phenylamino]-pyrimidin-4-ylamino}-butyric acid methyl ester,
4-{5-Bromo-4-[4-(3-hydroxy-pyrrolidin-1-yl)-cyclohexylamino]-pyrimidin-2-ylamino}-benzenesulfonamide,
4-{5-Bromo-4-[4-(3-hydroxy-pyrrolidin-1-yl)-cyclohexylamino]-pyrimidin-2-ylamino}-benzenesulfonamide,
4-[5-Bromo-4-((R)-1-hydroxymethyl-3-methyl-butylamino)-pyrimidin-2-ylamino]-benzenesulfonamide,
4-{5-Bromo-4-[2-(1-methyl-pyrrolidin-2-yl)-ethylamino]-pyrimidin-2-ylamino}-benzenesulfonamide,
4-[5-Bromo-4-(piperidin-4-yloxy)-pyrimidin-2-ylamino]-benzenesulfonamide,
4-[5-Bromo-4-(3-dimethylamino-propylamino)-pyrimidin-2-ylamino]-benzenesulfonamide,
Acetic acid (R)-2-[5-bromo-2-(4-sulfamoyl-phenylamino)-pyrimidin-4-ylamino]-3-methyl-butyl ester,
4-[5-Bromo-4-(4-hydroxy-cyclohexylamino)-pyrimidin-2-ylamino]-N-(2-hydroxyethyl)-benzenesulfonamide,
(3,4,5-Trimethoxy-phenyl)-carbamicacid (R)-2-[5-bromo-2-(4-sulfamoyl-phenylamino)-pyrimidin-4-ylamino]-3-methyl-butyl ester,
4-(5-Bromo-4-cycloheptylamino-pyrimidin-2-ylamino)-benzenesulfonamide,
4-[5-Bromo-4-((R)-1-hydroxymethyl-2,2-dimethyl-propylamino)-pyrimidin-2-ylamino]-benzenesulfonamide,
4-[5-Bromo-4-(4-oxo-cyclohexylamino)-pyrimidin-2-ylamino]-benzenesulfonamide,
(E)-6-{5-Chloro-2-[4-(2-hydroxy-ethylsulfamoyl)-phenylamino]-pyrimidin-4-ylamino}-3-methyl-hex-2-en-4-ynoic acid methyl ester,
4-[5-Bromo-4-(1-hydroxymethyl-cyclopentylamino)-pyrimidin-2-ylamino]-benzenesulfonamide,
4-[4-(Bicyclo[2.2.1]hept-2-ylamino)-5-bromo-pyrimidin-2-ylamino]-benzenesulfonamide,
4-[5-Bromo-4-(4-morpholin-4-yl-cyclohexylamino)-pyrimidin-2-ylamino]-benzenesulfonamide,
4-[5-Bromo-4-(4-morpholin-4-yl-cyclohexylamino)-pyrimidin-2-ylamino]-benzenesulfonamide,
4-[5-Bromo-4-((R)-2-hydroxy-1-methyl-ethylamino)-pyrimidin-2-ylamino]-benzenesulfonamide,
4-({5-Bromo-4-[(R)-(2-hydroxy-1-methylethyl)amino]pyrimidin-2-yl}amino)benzenesulfonamide,
4-[5-Bromo-4-(2,2,3,3,3-pentafluoro-propylamino)-pyrimidin-2-ylamino]-benzenesulfonamide,
4-[5-Bromo-4-(4-hydroxy-butylamino)-pyrimidin-2-ylamino]-benzenesulfonamide,
4-[5-Bromo-4-(1-ethynyl-cyclohexylamino)-pyrimidin-2-ylamino]-benzenesulfonamide,
4-[5-Bromo-4-(3-hydroxy-1-phenyl-propylamino)-pyrimidin-2-ylamino]-benzenesulfonamide,
(+)-4-[5-Bromo-4-(2-methyl-cyclohexylamino)-pyrimidin-2-ylamino]-benzenesulfonamide,
(-)-4-[5-Bromo-4-(2-methyl-cyclohexylamino)-pyrimidin-2-ylamino]-benzenesulfonamide,
4-{5-Bromo-4-[4-(2-hydroxy-1-hydroxymethyl-ethylamino)-cyclohexylamino]-pyrimidin-2-ylamino}-benzenesulfonamide,
4-{5-Bromo-4-[4-(2-hydroxy-1-hydroxymethyl-ethylamino)-cyclohexylamino]-pyrimidin-2-ylamino}-benzenesulfonamide,
(R)-2-[5-Bromo-2-(4-trifluoromethanesulfonyl-phenylamino)-pyrimidin-4-ylamino]-3-methyl-butan-1-ol,
4-[5-Bromo-4-(4-cyclopropylamino-cyclohexylamino)-pyrimidin-2-ylamino] -benzenesulfonamide,
4-[5-Bromo-4-(4-cyclopropylamino-cyclohexylamino)-pyrimidin-2-ylamino] -benzenesulfonamide,
4-[5-Bromo-4-(tetrahydro-thiophen-3-ylamino)-pyrimidin-2-ylamino]-benzenesulfonamide,
2-[5-Bromo-2-(4-sulfamoyl-phenylamino)-pyrimidin-4-ylamino]-3-hydroxy-propionic acid methyl ester,
4-[5-Bromo-4-((R)-1-hydroxymethyl-2-methyl-propylamino)-pyrimidin-2-yl amino]-N-pyrimidin-2-yl-benzenesulfonamide,
4-[5-Bromo-4-((R)-1-hydroxymethyl-2-methyl-propylamino)-pyrimidin-2-yl amino]-N-thiazol-2-yl-benzenesulfonamide,
4-[5-Bromo-4-(4-hydroxy-butylamino)-pyrimidin-2-ylamino]-N-methyl-benzenesulfonamide,
4-{5-Bromo-4-[2-(3H-imidazol-4-yl)-ethylamino]-pyrimidin-2-ylamino}-benzenesulfonamide,
4-[5-Bromo-4-(1-ethyl-1-hydroxymethyl-propylamino)-pyrimidin-2-ylamino]-benzenesulfonamide,
4-[5-Bromo-4-(1-hydroxymethyl-1,2-dimethyl-propylamino)-pyrimidin-2-ylamino]-benzenesulfonamide,
4-[5-Bromo-4-(4-methyl-cyclohexylamino)-pyrimidin-2-ylamino]-benzenesulfonamide,
4-{5-Bromo-4-[(S)-(tetrahydro-furan-3-yl)amino]-pyrimidin-2-ylamino}-benzenesulfonamide,
4-[5-Bromo-4-(cyclohexylmethyl-amino)-pyrimidin-2-ylamino]-benzenesulfonamide,
3-[5-Bromo-2-(4-sulfamoyl-phenylamino)-pyrimidin-4-ylamino]-propionic acid,
4-[5-Bromo-2-(4-sulfamoyl-phenylamino)-pyrimidin-4-ylamino]-butyric acid ethyl ester,
{3-[5-Bromo-2-(4-sulfamoyl-phenylamino)-pyrimidin-4-ylamino]-propyl}-phosphonic acid diethyl ester,
4-{5-Bromo-4-[(3-pyridin-3-yl-isoxazol-4-ylmethyl)-amino]-pyrimidin-2-ylamino}-benzenesulfonamide,
{[5-Bromo-2-(4-sulfamoyl-phenylamino)-pyrimidin-4-ylamino]-methyl}-phosphonic acid dimethyl ester,
{[5-Bromo-2-(4-sulfamoyl-phenylamino)-pyrimidin-4-ylamino]-methyl}-phosphonic acid monomethyl ester,
4-{5-Bromo-4-[(pyridin-4-ylmethyl)-amino]-pyrimidin-2-ylamino}-benzenesulfonamide,
4-{5-Bromo-4-[(thiophen-2-ylmethyl)-amino]-pyrimidin-2-ylamino}-benzenesulfonamide,
4-{5-Bromo-4-[(pyridin-2-ylmethyl)-amino]-pyrimidin-2-ylamino}-benzenesulfonamide,
4-[5-Bromo-4-(4-fluoro-benzylamino)-pyrirmidin-2-ylamino]-benzenesulfonamide,
4-[5-Bromo-4-(4-methoxy-benzylamino)-pyrimidin-2-ylamino]-benzenesulfonamide,
{3-[5-Bromo-2-(4-sulfamoyl-phenylamino)-pyrimidin-4-ylamino]-propyl}-phosphonic acid,
4-[5-Bromo-4-(2-hydroxy-ethylamino)-pyrimidin-2-ylamino]-benzenesulfonamide,
4-[5-Bromo-4-(1-phenyl-ethylamino)-pyrimidin-2-ylamino]-benzenesulfonamide,
N-Allyloxy-4-[5-bromo-4-((R)-1-hydroxymethyl-2-methyl-propylamino)-pyrimidin-2-ylamino]-benzenesulfonamide,
3-[5-Bromo-4-((R)-1-hydroxymethyl-2-methyl-propylamino)-pyrimidin-2-yl amino]-N-(5-hydroxy-pentyl)-benzenesulfonamide,
3-[5-Bromo-4-((R)-1-hydroxymethyl-2-methyl-propylamino)-pyrimidin-2-yl amino]-N-(2-hydroxy-1-hydroxymethyl-ethyl)-benzenesulfonamide,
3-[5-Bromo-4-((R)-1-hydroxymethyl-2-methyl-propylamino)-pyrimidin-2-ylamino]-N-(3-hydroxy-propyl)-benzenesulfonamide,
N-Benzyloxy-4-[5-bromo-4-((R)-1-hydroxymethyl-2-methyl-propylamino)-pyrimidin-2-ylamino]-benzenesulfonamide,
4-[5-Bromo-4-((R)-1-hydroxymethyl-2-methyl-propylamino)-pyrimidin-2-ylamino]-N-(2-hydroxy-butyl)-benzenesulfonamide,
4-[5-Bromo-4-((R)-1-hydroxymethyl-2-methyl-propylamino)-pyrimidin-2-yl amino]-N-cyclopropylmethyl-benzenesulfonamide,
4-[5-Bromo-4-((R)-1-hydroxymethyl-2-methyl-propylamino)-pyrimidin-2-ylamino]-N-(4-cyano-cyclohexylmethyl)-benzenesulfonamide,
4-[5-Bromo-4-((R)-1-hydroxymethyl-2-methyl-propylamino)-pyrimidin-2-ylamino]-N-(5-hydroxy-pentyl)-benzenesulfonamide,
4-[5-Bromo-4-((R)-1-hydroxymethyl-2-methyl-propylamino)-pyrimidin-2-ylamino]-N-(tetrahydro-furan-2-ylmethyl)-benzenesulfonamide,
4-[5-Bromo-4-((S)-1-hydroxymethyl-2-methyl-propylamino)-pyrimidin-2-ylamino]-N-hydroxy-benzenesulfonamide,
4-[5-Bromo-4-((S)-1-hydroxymethyl-propylamino)-pyrimidin-2-ylamino]-benzenesulfonamide,
4-[5-Bromo-4-((R)-1-hydroxymethyl-2-methyl-propylamino)-pyrimidin-2-ylamino]-N-(2-hydroxy-1-hydroxymethyl-ethyl)-benzenesulfonamide,
4-[5-Bromo-4-((R)-1-hydroxymethyl-2-methyl-propylamino)-pyrimidin-2-ylamino]-N-(3-hydroxy-propyl)-benzenesulfonamide,
4-[5-Bromo-4-((R)-1-hydroxymethyl-2-methyl-propylamino)-pyrimidin-2-ylamino]-N-hydroxy-benzenesulfonamide,
5-[5-Bromo-4-((R)-1-hydroxymethyl-2-methyl-propylamino)-pyrimidin-2-ylamino]-2-methyl-benzenesulfonamide,
3-[5-Bromo-4-((R)-1-hydroxymethyl-2-methyl-propylamino)-pyrimidin-2-ylamino]-N-ethoxy-benzenesulfonamide,
N-Allyloxy-3-[5-bromo-4-((R)-1-hydroxymethyl-2-methyl-propylamino)-pyrimidin-2-ylamino]-benzenesulfonamide,
4-(5-Bromo-4-morpholin-4-yl-pyrimidin-2-ylamino)-benzenesulfonamide,
7-[(5-Bromo-4-{[(R)-2-hydroxy-1-methylethyl]amino}pyrimidin-2-yl)amino]-3,4-dihydro-2H-1,2-benzothiazine 1,1-dioxide ,
4-[5-Bromo-4-(2-hydroxy-2-phenyl-propylamino)-pyrimidin-2-ylamino]-benzenesulfonamide,
4-[5-Bromo-4-(2-hydroxy-1,2-dimethyl-propylamino)-pyrimidin-2-ylamino]-benzenesulfonamide,
4-[5-Bromo-4-(2-hydroxy-1,2-dimethyl-butylamino)-pyrimidin-2-ylamino]-benzenesulfonamide,
4-[5-Bromo-4-(2-oxo-2-phenyl-ethylamino)-pyrimidin-2-ylamino]-benzenesulfonamide,
4-[5-Bromo-4-(2-hydroxy-propylamino)-pyrimidin-2-ylamino]-benzenesulfonamide,
4-[5-Bromo-4-(2-hydroxy-1-methyl-propylamino)-pyrimidin-2-ylamino]-benzenesulfonamide,
4-(5-Bromo-4-sec-butylamino-pyrimidin-2-ylamino)-benzenesulfonamide,
4-[5-Bromo-4-(2-hydroxy-1-methyl-butylamino)-pyrimidin-2-ylamino]-benzenesulfonamide,
4-[5-Bromo-4-(3,3-dimethyl-2-oxo-butylamino)-pyrimidin-2-ylamino]-benzenesulfonamide,
4-[5-Bromo-4-(2-hydroxy-1-methyl-3-phenyl-propylamino)-pyrimidin-2-ylamino]-benzenesulfonamide,
4-[5-Bromo-4-(2-hydroxy-butylamino)-pyrimidin-2-ylamino]-benzenesulfonamide,
4-[5-Bromo-4-(2-hydroxy-1-methyl-pent-4-enylamino)-pyrimidin-2-ylamino]-benzenesulfonamide,
4-[5-Bromo-4-(2-hydroxy-1-methyl-pentylamino)-pyrimidin-2-ylamino]-benzenesulfonamide,
4-[5-Bromo-4-(1-methyl-2-oxo-propylamino)-pyrimidin-2-ylamino]-benzenesulfonamide,
4-[5-Bromo-4-(2-hydroxy-1,3-dimethyl-butylamino)-pyrimidin-2-ylamino]-benzenesulfonamide,
4-[4-((1 R,2R)-2-Benzyloxy-cyclopentylamino)-5-bromo-pyrimidin-2-ylamino]-benzenesulfonamide,
4-[4-((1 S,2S)-2-Benzyloxy-cyclopentylamino)-5-bromo-pyrimidin-2-ylamino]-benzenesulfonamide,
4-[5-Bromo-4-(1-ethyl-2-hydroxy-propylamino)-pyrimidin-2-ylamino]-benzenesulfonamide,
4-[5-Bromo-4-(1-ethyl-2-hydroxy-butylamino)-pyrimidin-2-ylamino]-benzenesulfonamide,
4-[5-Bromo-4-(2-hydroxy-2-phenyl-ethylamino)-pyrimidin-2-ylamino]-benzenesulfonamide,
4-[5-Bromo-4-(2-hydroxy-3,3-dimethyl-butylamino)-pyrimidin-2-ylamino]-benzenesulfonamide,
4-[5-Bromo-4-(2-hydroxyimino-1-methyl-propylamino)-pyrimidin-2-ylamino]-benzenesulfonamide,
4-(5-Bromo-4-{2-[(E)-methoxyimino]-1-methyl-propylamino}-pyrimidin-2-ylamino)-benzenesulfonamide,
4-(5-Bromo-4-{2-[(E)-tert-butoxyimino]-1-methyl-propylamino}-pyrimidin-2-ylamino)-benzenesulfonamide,
[2-[5-Bromo-2-(4-sulfamoyl-phenylamino)-pyrimidin-4-ylamino]-1-methyl-prop-(E)-ylideneaminooxy]-acetic acid,
4-[5-Bromo-4-(2-cyclopropylamino-1-methyl-propylamino)-pymidin-2-ylamino]-benzenesulfonamide,
4-[5-Bromo-4-(2-hydroxy-1-methoxymethyl-ethylamino)-pyrimidin-2-ylamino]-benzenesulfonamide,
4-[5-Bromo-4-(2-hydroxy-1-methoxymethyl-propylamino)-pyrimidin-2-ylamino]-benzenesulfonamide,
5-[5-Bromo-4-((R)-1-hydroxymethyl-propylamino)-pyrimidin-2-ylamino]-N-cyclohexylmethyl-2-methyl-benzenesulfonamide,
5-[5-Bromo-4-((R)-1-hydroxymethyl-propylamino)-pyrimidin-2-ylamino]-2-methyl-N-(4-phenyl-butyl)-benzenesulfonamide,
5-[5-Bromo-4-((R)-1-hydroxymethyl-propylamino)-pyrimidin-2-ylamino]-N-(3,3-diphenyl-propyl)-2-methyl-benzenesulfonamide,
5-[5-Bromo-4-((R)-1-hydroxymethyl-propylamino)-pyrimidin-2-ylamino]-N-(2-dimethylamino-ethyl)-2-methyl-benzenesulfonamide,
5-[5-Bromo-4-((R)-1-hydroxymethyl-propylamino)-pyrimidin-2-ylamino]-N-3-imidazol-1-yl-propyl)-2-methyl-benzenesulfonamide,
5-[5-Bromo-4-((R)-1-hydroxymethyl-propylamino)-pyrimidin-2-ylamino]-2-methyl-N-(3-trifluoromethyl-benzyl)-benzenesulfonamide,
5-[5-Bromo-4-((R)-1-hydroxymethyl-propylamino)-pyrimidin-2-ylamino]-N-heptyl-2-methyl-benzenesulfonamide,
5-[5-Bromo-4-((R)-1-hydroxymethyl-propylamino)-pyrimidin-2-ylamino]-2-methyl-N-octyl-benzenesulfonamide,
5-[5-Bromo-4-((R)-1-hydroxymethyl-propylamino)-pyrimidin-2-ylamino]-2-methyl-N-nonyl-benzenesulfonamide,
5-[5-Bromo-4-((R)-1-hydroxymethyl-propylamino)-pyrimidin-2-ylamino]-N-decyl-2-methyl-benzenesulfonamide,
5-[5-Bromo-4-((R)-1-hydroxymethyl-propylamino)-pyrimidin-2-ylamino]-N-furan-2-ylmethyl-2-methyl-benzenesulfonamide,
5-[5-Bromo-4-((R)-1-hydroxymethyl-propylamino)-pyrimidin-2-ylamino]-2-methyl-N-(4-trifluoromethyl-benzyl)-benzenesulfonamide,
5-[5-Bromo-4-((R)-1-hydroxymethyl-propylamino)-pyrimidin-2-ylamino]-2-methyl-N-(3-phenyl-propyl)-benzenesulfonamide,
5-[5-Bromo-4-((R)-1-hydroxymethyl-propylamino)-pyrimidin-2-ylamino]-2-methyl-N-[3-(2-oxo-pyrrolidin-1-yl)-propyl]-benzenesulfonamide,
5-[5-Bromo-4-((R)-1-hydroxymethyl-propylamino)-pyrimidin-2-ylamino]-2-methyl-N-(3-methyl-butyl)-benzenesulfonamide,
5-[5-Bromo-4-((R)-1-hydroxymethyl-propylamino)-pyrimidin-2-ylamino]-N-hexyl-2-methyl-benzenesulfonamide,
5-[5-Bromo-4-((R)-hydroxymethyl-propylamino)-pyrimidin-2-ylamino]-N-(3-dimethylamino-propyl)-2-methyl-benzenesulfonamide,
5-[5-Bromo-4-((R)-1-hydroxymethyl-propylamino)-pyrimidin-2-ylamino]-N-(2-diisopropylamino-ethyl)-2-methyl-benzenesulfonamide,
5-[5-Bromo-4-((R)-1-hydroxymethyl-propylamino)-pyrimidin-2-ylamino]-N-cyclopropyl-2-methyl-benzenesulfonamide,
5-[5-Bromo-4-((R)-1-hydroxymethyl-propylamino)-pyrimidin-2-ylamino]-N-(4-cyano-cyclohexylmethyl)-2-methyl-benzenesulfonamide,
5-[5-Bromo-4-((R)-1-hydroxymethyl-propylamino)-pyrimidin-2-ylamino]-2-methyl-N-phenethyl-benzenesulfonamide,
5-[5-Bromo-4-((R)-1-hydroxymethyl-propylamino)-pyrimidin-2-ylamino]-N-[2-(4-chloro-phenyl)-ethyl]-2-methyl-benzenesulfonamide,
5-[5-Bromo-4-((R)-1-hydroxymethyl-propylamino)-pyrimidin-2-ylamino]-2-methyl-N-(5-methyl-furan-2-ylmethyl)-benzenesulfonamide,
5-[5-Bromo-4-((R)-1-hydroxymethyl-propylamino)-pyrimidin-2-ylamino]-2-methyl-N-(2-morpholin-4-yl-ethyl)-benzenesulfonamide,
5-[5-Bromo-4-((R)-1-hydroxymethyl-propylamino)-pyrimidin-2-ylamino]-N-(2-methoxy-ethyl)-2-methyl-benzenesulfonamide,
5-[5-Bromo-4-((R)-1-hydroxymethyl-propylamino)-pyrimidin-2-ylamino]-2-methyl-N-thiophen-2-ylmethyl-benzenesulfonamide,
5-[5-Bromo-4-((R)-1-hydroxymethyl-propylamino)-pyrimidin-2-ylamino]-N-[2-(4-methoxy-phenyl)-ethyl]-2-methyl-benzenesulfonamide,
5-[5-Bromo-4-((R)-1-hydroxymethyl-propylamino)-pyrimidin-2-ylamino]-2-methyl-N-(2-pyrrolidin-1-yl-ethyl)-benzenesulfonamide,
5-[5-Bromo-4-((R)-1-hydroxymethyl-propylamino)-pyrimidin-2-ylamino]-2-methyl-N-(2-piperidin-1-yl-ethyl)-benzenesulfonamide,
5-[5-Bromo-4-((R)-1-hydroxymethyl-propylamino)-pyrimidin-2-ylamino]-N-(3-fluoro-benzyl)-2-methyl-benzenesulfonamide,
5-[5-Bromo-4-((R)-1-hydroxymethyl-propylamino)-pyrimidin-2-ylamino]-2-methyl-N-[2-(1-methyl-pyrrolidin-2-yl)-ethyl]-benzenesulfonamide,
5-[5-Bromo-4-((R)-1-hydroxymethyl-propylamino)-pyrimidin-2-ylamino]-2-methyl-N-pyridin-3-ylmethyl-benzenesulfonamide,
5-[5-Bromo-4-((R)-1-hydroxymethyl-propylamino)-pyrimidin-2-ylamino]-N-(4-fluoro-benzyl)-2-methyl-benzenesulfonamide,
5-[5-Bromo-4-((R)-1-hydroxymethyl-propylamino)-pyrimidin-2-ylamino]-2-methyl-N-pyridin-4-ylmethyl-benzenesulfonamide,
5-[5-Bromo-4-((R)-1-hydroxymethyl-propylamino)-pyrimidin-2-ylamino]-2-methyl-N-(2-phenoxy-ethyl)-benzenesulfonamide,
5-[5-Bromo-4-((R)-1-hydroxymethyl-propylamino)-pyrimidin-2-ylamino]-N-(2-methoxy-benzyl)-2-methyl-benzenesulfonamide,
5-[5-Bromo-4-((R)-1-hydroxymethyl-propylamino)-pyrimidin-2-ylamino]-2-methyl-N-[2-(4-sulfamoyl-phenyl)-ethyl]-benzenesulfonamide,
5-[5-Bromo-4-((R)-1-hydroxymethyl-propylamino)-pyrimidin-2-ylamino]-2-methyl-N-(4-methyl-benzyl)-benzenesulfonamide,
5-[5-Bromo-4-((R)-2-hydroxy-1-methyl-ethylamino)-pyrimidin-2-ylamino]-N-cyclohexylmethyl-2-methyl-benzenesulfonamide,
5-[5-Bromo-4-((R)-2-hydroxy-1-methyl-ethylamino)-pyrimidin-2-ylamino]-2-methyl-N-(4-phenyl-butyl)-benzenesulfonamide,
5-[5-Bromo-4-((R)-2-hydroxy-1-methyl-ethylamino)-pyrimidin-2-ylamino]-N-(2-dimethylamino-ethyl)-2-methyl-benzenesulfonamide,
5-[5-Bromo-4-((R)-2-hydroxy-1-methyl-ethylamino)-pyrimidin-2-ylamino]-N-(3-imidazol-1-yl-propyl)-2-methyl-benzenesulfonamide,
5-[5-Bromo-4-((R)-2-hydroxy-1-methyl-ethylamino)-pyrimidin-2-ylamino]-2-methyl-N-(3-trifluoromethyl-benzyl)-benzenesulfonamide,
5-[5-Bromo-4-((R)-2-hydroxy-1-methyl-ethylamino)-pyrimidin-2-ylamino]-N-furan-2-ylmethyl-2-methyl-benzenesulfonamide,
5-[5-Bromo-4-((R)-2-hydroxy-1-methyl-ethylamino)-pyrimidin-2-ylamino]-2-methyl-N-(4-trifluoromethyl-benzyl)-benzenesulfonamide,
5-[5-Bromo-4-((R)-2-hydroxy-1-methyl-ethylamino)-pyrimidin-2-ylamino]-2-methyl-N-(3-phenyl-propyl)-benzenesulfonamide,
5-[5-Bromo-4-((R)-2-hydroxy-1-methyl-ethylamino)-pyrimidin-2-ylamino]-2-methyl-N-[3-(2-oxo-pyrrolid in-1-yl)-propyl]-benzenesulfonamide,
5-[5-Bromo-4-((R)-2-hydroxy-1-methyl-ethylamino)-pyrimidin-2-ylamino]-2-methyl-N-(3-methyl-butyl)-benzenesulfonamide,
5-[5-Bromo-4-((R)-2-hydroxy-1-methyl-ethylamino)-pyrimidin-2-ylamino]-N-hexyl-2-methyl-benzenesulfonamide,
5-[5-Bromo-4-((R)-2-hydroxy-1-methyl-ethylamino)-pyrimidin-2-ylamino]-N-(3-dimethylamino-propyl)-2-methyl-benzenesulfonamide,
5-[5-Bromo-4-((R)-2-hydroxy-1-methyl-ethylamino)-pyrimidin-2-ylamino]-N-(2-diisopropylamino-ethyl)-2-methyl-benzenesulfonamide,
5-[5-Bromo-4-((R)-2-hydroxy-1-methyl-ethylamino)-pyrimidin-2-ylamino]-N-cyclopropyl-2-methyl-benzenesulfonamide,
5-[5-Bromo-4-((R)-2-hydroxy-1-methyl-ethylamino)-pyrimidin-2-ylamino]-N-(4-cyano-cyclohexylmethyl)-2-methyl-benzenesulfonamide,
5-[5-Bromo-4-((R)-2-hydroxy-1-methyl-ethylamino)-pyrimidin-2-ylamino]-2-methyl-N-phenethyl-benzenesulfonamide,
5-[5-Bromo-4-((R)-2-hydroxy-1-methyl-ethylamino)-pyrimidin-2-ylamino]-N-[2-(4-chloro-phenyl)-ethyl]-2-methyl-benzenesulfonamide,
5-[5-Bromo-4-((R)-2-hydroxy-1-methyl-ethylamino)-pyrimidin-2-ylamino]-2-methyl-N-(5-methyl-furan-2-ylmethyl)-benzenesulfonamide,
5-[5-Bromo-4-((R)-2-hydroxy-1-methyl-ethylamino)-pyrimidin-2-ylamino]-2-methyl-N-(2-morpholin-4-yl-ethyl)-benzenesulfonamide,
5-[5-Bromo-4-((R)-2-hydroxy-1-methyl-ethylamino)-pyrimidin-2-ylamino]-N-(2-methoxy-ethyl)-2-methyl-benzenesulfonamide,
5-[5-Bromo-4-((R)-2-hydroxy-1-methyl-ethylamino)-pyrimidin-2-ylamino]-2-methyl-N-thiophen-2-ylmethyl-benzenesulfonamide,
5-[5-Bromo-4-((R)-2-hydroxy-1-methyl-ethylamino)-pyrimidin-2-ylamino]-N-[2-(4-methoxy-phenyl)-ethyl]-2-methyl-benzenesulfonamide,
5-[5-Bromo-4-((R)-2-hydroxy-1-methyl-ethylamino)-pyimidin-2-ylamino]-2-methyl-N-(2-pyrrolidin-1-yl-ethyl)-benzenesulfonamide,
5-[5-Bromo-4-((R)-2-hydroxy-1-methyl-ethylamino)-pyrimidin-2-ylamino]-2-methyl-N-(2-piperidin-1-yl-ethyl)-benzenesulfonamide,
5-[5-Bromo-4-((R)-2-hydroxy-1-methyl-ethylamino)-pyrimidin-2-ylamino]-N-(3-fluoro-benzyl)-2-methyl-benzenesulfonamide,
5-[5-Bromo-4-((R)-2-hydroxy-1-methyl-ethylamino)-pyrimidin-2-ylamino]-2-methyl-N-[2-(1-methyl-pyrrolidin-2-yl)-ethyl]-benzenesulfonamide,
5-[5-Bromo-4-((R)-2-hydroxy-1-methyl-ethylamino)-pyrimidin-2-ylamino]-2-methyl-N-pyridin-3-ylmethyl-benzenesulfonamide,
5-[5-Bromo-4-((R)-2-hydroxy-1-methyl-ethylamino)-pyrimidin-2-ylamino]-N-(4-fluoro-benzyl)-2-methyl-benzenesulfonamide,
5-[5-Bromo-4-((R)-2-hydroxy-1-methyl-ethylamino)-pyrimidin-2-ylamino]-2-methyl-N-pyridin-4-ylmethyl-benzenesulfonamide,
5-[5-Bromo-4-((R)-2-hydroxy-1-methyl-ethylamino)-pyrimidin-2-ylamino]-2-methyl-N-(2-phenoxy-ethyl)-benzenesulfonamide,
5-[5-Bromo-4-((R)-2-hydroxy-1-methyl-ethylamino)-pyrimidin-2-ylamino]-N-(2-methoxy-benzyl)-2-methyl-benzenesulfonamide,
5-[5-Bromo-4-((R)-2-hydroxy-1-methyl-ethylamino)-pyrimidin-2-ylamino]-2-methyl-N-[2-(4-sulfamoyl-phenyl)-ethyl]-benzenesulfonamide,
5-[5-Bromo-4-((R)-2-hydroxy-1-methyl-ethylamino)-pyrimidin-2-ylamino]-2-methyl-N-(4-methyl-benzyl)-benzenesulfonamide,
3-[5-Bromo-4-((R)-1-hydroxymethyl-propylamino)-pyrimidin-2-ylamino]-N-cyclohexylmethyl-benzenesulfonamide,
3-[5-Bromo-4-((R)-1-hydroxymethyl-propylamino)-pyrimidin-2-ylamino]-N-(3-imidazol-1-yl-propyl)-benzenesulfonamide,
3-[5-Bromo-4-((R)-1-hydroxymethyl-propylamino)-pyrimidin-2-ylamino]-N-(3-trifluoromethyl-benzyl)-benzenesulfonamide,
3-[5-Bromo-4-((R)-1-hydroxymethyl-propylamino)-pyrimidin-2-ylamino]-N-furan-2-ylmethyl-benzenesulfonamide,
3-[5-Bromo-4-((R)-1-hydroxymethyl-propylamino)-pyrimidin-2-ylamino]-N-(4-trifluoromethyl-benzyl)-benzenesulfonamide,
3-[5-Bromo-4-((R)-1-hydroxymethyl-propylamino)-pyrimidin-2-ylamino]-N-(3-phenyl-propyl)-benzenesulfonamide,
3-[5-Bromo-4-((R)-1-hydroxymethyl-propylamino)-pyrimidin-2-ylamino]-N-[3-(2-oxo-pyrrolidin-1-yl)-propyl]-benzenesulfonamide,
3-[5-Bromo-4-((R)-1-hydroxymethyl-propylamino)-pyrimidin-2-ylamino]-N-(3-methyl-butyl)-benzenesulfonamide,
3-[5-Bromo-4-((R)-1-hydroxymethyl-propylamino)-pyrimidin-2-ylamino]-N-(3-dimethylamino-propyl)-benzenesulfonamide,
3-[5-Bromo-4-((R)-1-hydroxymethyl-propylamino)-pyrimidin-2-ylamino]-N-(2-diisopropylamino-ethyl)-benzenesulfonamide,
3-[5-Bromo-4-((R)-1-hydroxymethyl-propylamino)-pyrimidin-2-ylamino]-N-(4-cyano-cyclohexylmethyl)-benzenesulfonamide,
3-[5-Bromo-4-((R)-1-hydroxymethyl-propylamino)-pyrimidin-2-ylamino]-N-phenethyl-benzenesulfonamide,
3-[5-Bromo-4-((R)-1-hydroxymethyl-propylamino)-pyrimidin-2-ylamino]-N-[2-(4-chloro-phenyl)-ethyl]-benzenesulfonamide,
3-[5-Bromo-4-((R)-1-hydroxymethyl-propylamino)-pyrimidin-2-ylamino]-N-(5-methyl-furan-2-ylmethyl)-benzenesulfonamide,
3-[5-Bromo-4-((R)-1-hydroxymethyl-propylamino)-pyrimidin-2-ylamino]-N-(2-morpholin-4-yl-ethyl)-benzenesulfonamide,
3-[5-Bromo-4-((R)-1-hydroxymethyl-propylamino)-pyrimidin-2-ylamino]-N-(2-methoxy-ethyl)-benzenesulfonamide,
3-[5-Bromo-4-((R)-1-hydroxymethyl-propylaminorpyrimidin-2-ylamino]-N-thiophen-2-ylmethyl-benzenesulfonamide,
3-[5-Bromo-4-((R)-1-hydroxymethyl-propylamino)-pyrimidin-2-ylamino]-N-(2-pyrrolidin-1-yl-ethyl)-benzenesulfonamide,
3-[5-Bromo-4-((R)-1-hydroxymethyl-propylamino)-pyrimidin-2-ylamino]-N-(2-piperidin-1-yl-ethyl)-benzenesulfonamide,
3-[5-Bromo-4-((R)-1-hydroxymethyl-propylamino)-pyrimidin-2-ylamino]-N-(3-fluoro-benzyl)-benzenesulfonamide,
3-[5-Bromo-4-((R)-1-hydroxymethyl-propylamino)-pyrimidin-2-ylamino]-N-[2-(1-methyl-pyrrolidin-2-yl)-ethyl]-benzenesulfonamide,
3-[5-Bromo-4-((R)-1-hydroxymethyl-propylamino)-pyrimidin-2-ylamino]-N-pyridin-3-ylmethyl-benzenesulfonamide,
3-[5-Bromo-4-((R)-1-hydroxymethyl-propylamino)-pyrimidin-2-ylamino]-N-(4-fluoro-benzyl)-benzenesulfonamide,
3-[5-Bromo-4-((R)-1-hydroxymethyl-propylamino)-pyrimidin-2-ylamino]-N-pyridin-4-ylmethyl-benzenesulfonamide,
3-[5-Bromo-4-((R)-1-hydroxymethyl-propylamino)-pyrimidin-2-ylamino]-N-(2-phenoxy-ethyl)-benzenesulfonamide,
3-[5-Bromo-4-((R)-1-hydroxymethyl-propylamino)-pyrimidin-2-ylamino]-N-(2-methoxy-benzyl)-benzenesulfonamide,
3-[5-Bromo-4-((R)-1-hydroxymethyl-propylamino)-pyrimidin-2-ylamino]-N-(4-methyl-benzyl)-benzenesulfonamide,
3-[5-Bromo-4-((R)-1-hydroxymethyl-propylamino)-pyrimidin-2-ylamino]-N-(5-methylsulfanyl-1H-[1,2,4]triazol-3-yl)-benzenesulfonamide,
3-[5-Bromo-4-((R)-2-hydroxy-1-methyl-ethylamino)-pyrimidin-2-ylamino]-N-(2-dimethylamino-ethyl)-benzenesulfonamide,
3-[5-Bromo-4-((R)-2-hydroxy-1-methyl-ethylamino)-pyrimidin-2-ylamino]-N-(3-imidazol-1-yl-propyl)-benzenesulfonamide,
3-[5-Bromo-4-((R)-2-hydroxy-1-methyl-ethylamino)-pyrimidin-2-ylamino]-N-(3-trifluoromethyl-benzyl)-benzenesulfonamide,
3-[5-Bromo-4-((R)-2-hydroxy-1-methyl-ethylamino)-pyrimidin-2-ylamino]-N-furan-2-ylmethyl-benzenesulfonamide,
3-[5-Bromo-4-((R)-2-hydroxy-1-methyl-ethylamino)-pyrimidin-2-ylamino]-N-(4-trifluoromethyl-benzyl)-benzenesulfonamide,
3-[5-Bromo-4-((R)-2-hydroxy-1-methyl-ethylamino)-pyrimidin-2-ylamino]-N-[3-(2-oxo-pyrrolidin-1-yl)-propyl]-benzenesulfonamide,
3-[5-Bromo-4-((R)-2-hydroxy-1-methyl-ethylamino)-pyrimidin-2-ylamino]-N-(3-methyl-butyl)-benzenesulfonamide,
3-[5-Bromo-4-((R)-2-hydroxy-1-methyl-ethylamino)-pyrimidin-2-ylamino]-N-(3-dimethylamino-propyl)-benzenesulfonamide,
3-[5-Bromo-4-((R)-2-hydroxy-1-methyl-ethylamino)-pyrimidin-2-ylamino]-N-(2-diisopropylamino-ethyl)-benzenesulfonamide,
3-[5-Bromo-4-((R)-2-hydroxy-1-methyl-ethylamino)-pyrimidin-2-ylamino]-N-(4-cyano-cyclohexylmethyl)-benzenesulfonamide,
3-[5-Bromo-4-((R)-2-hydroxy-1-methyl-ethylamino)-pyrimidin-2-ylamino]-N-phenethyl-benzenesulfonamide,
3-[5-Bromo-4-((R)-2-hydroxy-1-methyl-ethylamino)-pyrimidin-2-ylamino]-N-[2-(4-chloro-phenyl)-ethyl]-benzenesulfonamide,
3-[5-Bromo-4-((R)-2-hydroxy-1-methyl-ethylamino)-pyrimidin-2-ylamino]-N-(5-methyl-furan-2-ylmethyl)-benzenesulfonamide,
3-[5-Bromo-4-((R)-2-hydroxy-1-methyl-ethylamino)-pyrimidin-2-ylamino]-N-(2-morpholin-4-yl-ethyl)-benzenesulfonamide,
3-[5-Bromo-4-((R)-2-hydroxy-1-methyl-ethylamino)-pyrimidin-2-ylamino]-N-(2-methoxy-ethyl)-benzenesulfonamide,
3-[5-Bromo-4-((R)-2-hydroxy-1-methyl-ethylamino)-pyrimidin-2-ylamino]-N-thiophen-2-ylmethyl-benzenesulfonamide,
3-[5-Bromo-4-((R)-2-hydroxy-1-methyl-ethylamino)-pyrimidin-2-ylamino]-N-(2-pyrrolidin-1-yl-ethyl)-benzenesulfonamide,
3-[5-Bromo-4-((R)-2-hydroxy-1-methyl-ethylamino)-pyrimidin-2-ylamino]-N-(2-piperidin-1-yl-ethyl)-benzenesulfonamide,
3-[5-Bromo-4-((R)-2-hydroxy-1-methyl-ethylamino)-pyrimidin-2-ylamino]-N-(3-fluoro-benzyl)-benzenesulfonamide,
3-[5-Bromo-4-((R)-2-hydroxy-1-methyl-ethylamino)-pyrimidin-2-ylamino]-N-[2-(1-methyl-pyrrolidin-2-yl)-ethyl]-benzenesulfonamide,
3-[5-Bromo-4-((R)-2-hydroxy-1-methyl-ethylamino)-pyrimidin-2-ylamino]-N-pyridin-3-ylmethyl-benzenesulfonamide,
3-[5-Bromo-4-((R)-2-hydroxy-1-methyl-ethylamino)-pyrimidin-2-ylamino]-N-(4-fluoro-benzyl)-benzenesulfonamide,
3-[5-Bromo-4-((R)-2-hydroxy-1-methyl-ethylamino)-pyrimidin-2-ylamino]-N-pyridin-4-ylmethyl-benzenesulfonamide,
3-[5-Bromo-4-((R)-2-hydroxy-1-methyl-ethylamino)-pyrimidin-2-ylamino]-N-(2-phenoxy-ethyl)-benzenesulfonamide,
3-[5-Bromo-4-((R)-2-hydroxy-1-methyl-ethylamino)-pyrimidin-2-ylamino]-N-(2-methoxy-benzyl)-benzenesulfonamide,
3-[5-Bromo-4-((R)-2-hydroxy-1-methyl-ethylamino)-pyrimidin-2-ylamino]-N-(4-methyl-benzyl)-benzenesulfonamide,
3-[5-Bromo-4-((R)-2-hydroxy-1-methyl-ethylamino)-pyrimidin-2-ylamino]-N-(5-methylsulfanyl-1 H-[1,2,4]triazol-3-yl)-benzenesulfonamide,
4-[5-Bromo-4-((R)-1-hydroxymethyl-propylamino)-pyrimidin-2-ylamino]-benzenesulfonamide,
3-[5-Bromo-4-((R)-2-hydroxy-1-methyl-ethylamino)-pyrimidin-2-ylamino]-benzenesulfonyl fluoride,
5-[5-Bromo-4-((R)-2-hydroxy-1-methyl-ethylamino)-pyrimidin-2-ylamino]-2-methyl-benzenesulfonyl fluoride,
3-[5-Bromo-4-((R)-1-hydroxymethyl-propylamino)-pyrimidin-2-ylamino]-benzenesulfonyl fluoride,
5-[5-Bromo-4-((R)-1-hydroxymethyl-propylamino)-pyrimidin-2-ylamino]-2-methyl-benzenesulfonyl fluoride,
3-[5-Bromo-4-((S)-1-hydroxymethyl-2-methyl-propylamino)-pyrimidin-2-ylamino]-benzenesulfonyl fluoride,
3-[5-Bromo-4-((S)-1-hydroxymethyl-2-methyl-propylamino)-pyrimidin-2-ylamino]-benzenesulfonyl fluoride,
4-[5-Bromo-4-((R)-2-hydroxy-1-methyl-ethylamino)-pyrimidin-2-ylamino]-benzenesulfonyl fluoride,
4-[5-Bromo-4-((R)-1-hydroxymethyl-propylamino)-pyrimidin-2-ylamino]-benzenesulfonyl fluoride,
4-[5-Bromo-4-((S)-1-hydroxymethyl-2-methyl-propylamino)-pyrimidin-2-ylamino]-benzenesulfonyl fluoride,
4-[5-Bromo-4-((R)-2-hydroxy-1-methyl-ethylamino)-pyrimidin-2-ylamino]-N-cyclohexylmethyl-benzenesulfonamide,
4-[5-Bromo-4-((R)-2-hydroxy-1-methyl-ethylamino)-pyrimidin-2-ylamino]-N-(4-phenyl-butyl)-benzenesulfonamide,
4-[5-Bromo-4-((R)-2-hydroxy-1-methyl-ethylamino)-pyrimidin-2-ylamino]-N-(2-dimethylamino-ethyl)-benzenesulfonamide,
4-[5-Bromo-4-((R)-2-hydroxy-1-methyl-ethylamino)-pyrimidin-2-ylamino]-N-(3-imidazol-1-yl-propyl)-benzenesulfonamide,
4-[5-Bromo-4-((R)-2-hydroxy-1-methyl-ethylamino)-pyrimidin-2-ylamino]-N-(3-trifluoromethyl-benzyl)-benzenesulfonamide,
4-[5-Bromo-4-((R)-2-hydroxy-1-methyl-ethylamino)-pyrimidin-2-ylamino]-N-(4-trifluoromethyl-benzyl)-benzenesulfonamide,
4-[5-Bromo-4-((R)-2-hydroxy-1-methyl-ethylamino)-pyrimidin-2-ylamino]-N-(3-phenyl-propyl)-benzenesulfonamide,
4-[5-Bromo-4-((R)-2-hydroxy-1-methyl-ethylamino)-pyrimidin-2-ylamino]-N-[3-(2-oxo-pyrrolidin-1-yl)-propyl]-benzenesulfonamide,
4-[5-Bromo-4-((R)-2-hydroxy-1-methyl-ethylamino)-pyrimidin-2-ylamino]-N-(3-methyl-butyl)-benzenesulfonamide,
4-[5-Bromo-4-((R)-2-hydroxy-1-methyl-ethylamino)-pyrimidin-2-ylamino]-N-(2-diisopropylamino-ethyl)-benzenesulfonamide,
4-[5-Bromo-4-((R)-2-hydroxy-1-methyl-ethylamino)-pyrimidin-2-ylamino]-N-cyclopropyl-benzenesulfonamide,
4-[5-Bromo-4-((R)-2-hydroxy-1-methyl-ethylamino)-pyrimidin-2-ylamino]-N-(4-cyano-cyclohexylmethyl)-benzenesulfonamide,
4-[5-Bromo-4-((R)-2-hydroxy-1-methyl-ethylamino)-pyrimidin-2-ylamino]-N-phenethyl-benzenesulfonamide,
4-[5-Bromo-4-((R)-2-hydroxy-1-methyl-ethylamino)-pyrimidin-2-ylamino]-N-[2-(4-chloro-phenyl)-ethyl]-benzenesulfonamide,
4-[5-Bromo-4-((R)-2-hydroxy-1-methyl-ethylamino)-pyrimidin-2-ylamino]-N-(5-methyl-furan-2-ylmethyl)-benzenesulfonamide,
4-[5-Bromo-4-((R)-2-hydroxy-1-methyl-ethylamino)-pyrimidin-2-ylamino]-N-(2-morpholin-4-yl-ethyl)-benzenesulfonamide,
4-[5-Bromo-4-((R)-2-hydroxy-1-methyl-ethylamino)-pyrimidin-2-ylamino]-N-(2-methoxy-ethyl)-benzenesulfonamide,
4-[5-Bromo-4-((R)-2-hydroxy-1-methyl-ethylamino)-pyrimidin-2-ylamino]-N-thiophen-2-ylmethyl-benzenesulfonamide,
4-[5-Bromo-4-((R)-2-hydroxy-1-methyl-ethylamino)-pyrimidin-2-ylamino]-N-[2-(4-methoxy-phenyl)-ethyl]-benzenesulfonamide,
4-[5-Bromo-4-((R)-2-hydroxy-1-methyl-ethylamino)-pyrimidin-2-ylamino]-N-(2-pyrrolidin-1-yl-ethyl)-benzenesulfonamide,
4-[5-Bromo-4-((R)-2-hydroxy-1-methyl-ethylamino)-pyrimidin-2-ylamino]-N-(2-piperidin-1-yl-ethyl)-benzenesulfonamide,
4-[5-Bromo-4-((R)-2-hydroxy-1-methyl-ethylamino)pyrimidin-2-ylamino]-N-(3-fluoro-benzyl)-benzenesulfonamide,
4-[5-Bromo-4-((R)-2-hydroxy-1-methyl-ethylamino)-pyrimidin-2-ylamino]-N-[2-(1-methyl-pyrrolidin-2-yl)-ethyl]-benzenesulfonamide,
4-[5-Bromo-4-((R)-2-hydroxy-1-methyl-ethylamino)-pyrimidin-2-ylamino]-N-pyridin-3-ylmethyl-benzenesulfonamide,
4-[5-Bromo-4-((R)-2-hydroxy-1-methyl-ethylamino)-pyrimidin-2-ylamino]-N-(4-fluoro-benzyl)-benzenesulfonamide,
4-[5-Bromo-4-((R)-2-hydroxy-1-methyl-ethylamino)-pyrimidin-2-ylamino]-N-pyridin-4-ylmethyl-benzenesulfonamide,
4-[5-Bromo-4-((R)-2-hydroxy-1-methyl-ethylamino)-pyrimidin-2-ylamino]-N-(2-phenoxy-ethyl)-benzenesulfonamide,
4-[5-Bromo-4-((R)-2-hydroxy-1-methyl-ethylamino)-pyrimidin-2-ylamino]-N-(2-methoxy-benzyl)-benzenesulfonamide,
4-[5-Bromo-4-((R)-2-hydroxy-1-methyl-ethylamino)-pyrimidin-2-ylamino]-N-(4-methyl-benzyl)-benzenesulfonamide,
4-[5-Bromo-4-((R)-2-hydroxy-1-methyl-ethylamino)-pyrimidin-2-ylamino]-N-(5-methylsulfanyl-1 H-[1,2,4]triazol-3-yl)-benzenesulfonamide,
4-[5-Bromo-4-((R)-1-hydroxymethyl-propylamino)-pyrimidin-2-ylamino]-N-cyclohexylmethyl-benzenesulfonamide,
4-[5-Bromo-4-((R)-1-hydroxymethyl-propylamino)-pyrimidin-2-ylamino]-N-(2-dimethylamino-ethyl)-benzenesulfonamide,
4-[5-Bromo-4-((R)-1-hydroxymethyl-propytamino)-pyrimidin-2-ylamino]-N-(3-imidazol-1-yl-propyl)-benzenesulfonamide,
4-[5-Bromo-4-((R)-1-hydroxymethyl-propylamino)-pyrimidin-2-ylamino]-N-(3-trifluoromethyl-benzyl)-benzenesulfonamide,
4-[5-Bromo-4-((R)-1-hydroxymethyl-propylamino)-pyrimidin-2-ylamino]-N-furan-2-ylmethyl-benzenesulfonamide,
4-[5-Bromo-4-((R)-1-hydroxymethyl-propylamino)-pyrimidin-2-ylamino]-N-(4-trifluoromethyl-benzyl)-benzenesulfonamide,
4-[5-Bromo-4-((R)-1-hydroxymethyl-propylamino)-pyrimidin-2-ylamino]-N-(3-phenyl-propyl)-benzenesulfonamide,
4-[5-Bromo-4-((R)-1-hydroxymethyl-propylamino)-pyrimidin-2-ylamino]-N-[3-(2-oxo-pyrrolidin-1-yl)-propyl]-benzenesulfonamide,
4-[5-Bromo-4-((R)-1-hydroxymethyl-propylamino)-pyrimidin-2-ylamino]-N-(3-methyl-butyl)-benzenesulfonamide,
4-[5-Bromo-4-((R)-1-hydroxymethyl-propylamino)-pyrimidin-2-ylamino]-N-(3-dimethylamino-propyl)-benzenesulfonamide,
4-[5-Bromo-4-((R)-1-hydroxymethyl-propylamino)-pyrimidin-2-ylamino]-N-(2-diisopropylamino-ethyl)-benzenesulfonamide,
4-[5-Bromo-4-((R)-1-hydroxymethyl-propylamino)-pyrimidin-2-ylamino]-N-cyclopropyl-benzenesulfonamide,
4-[5-Bromo-4-((R)-1-hydroxymethyl-propylamino)-pyrimidin-2-ylamino]-N-(4-cyano-cyclohexylmethyl)benzenesulfonamide,
4-[5-Bromo-4-((R)-1-hydroxymethyl-propylamino)-pyrimidin-2-ylamino]-N-phenethyl-benzenesulfonamide,
4-[5-Bromo-4-((R)-1-hydroxymethyl-propylamino)-pyrimidin-2-ylamino]-N-(3,4-dimethoxy-benzyl)-benzenesulfonamide,
4-[5-Bromo-4-((R)-1-hydroxymethyl-propylamino)-pyrimidin-2-ylamino]-N-[2-(4-chloro-phenyl)-ethyl]-benzenesulfonamide,
4-[5-Bromo-4-((R)-1-hydroxymethyl-propylamino)-pyrimidin-2-ylamino]-N-(5-methyl-furan-2-ylmethyl)-benzenesulfonamide,
4-[5-Bromo-4-((R)-1-hydroxymethyl-propylamino)-pyrimidin-2-ylamino]-N-(2-morpholin-4-yl-ethyl)-benzenesulfonamide,
4-[5-Bromo-4-((R)-1-hydroxymethyl-propylamino)-pyrimidin-2-ylamino]-N-(2-methoxy-ethyl)-benzenesulfonamide,
4-[5-Bromo-4-((R)-1-hydroxymethyl-propylamino)-pyrimidin-2-ylamino]-N-thiophen-2-ylmethyl-benzenesulfonamide,
4-[5-Bromo-4-((R)-1-hydroxymethyl-propylamino)-pyrimidin-2-ylamino]-N-[2-(4-methoxy-phenyl)-ethyl]-benzenesulfonamide,
4-[5-Bromo-4-((R)-1-hydroxymethyl-propylamino)-pyrimidin-2-ylamino]-N-(2-pyrrolidin-1-yl-ethyl)-benzenesulfonamide,
4-[5-Bromo-4-((R)-1-hydroxymethyl-propylamino)-pyrimidin-2-ylamino]-N-(2-piperidin-1-yl-ethyl)-benzenesulfonamide,
4-[5-Bromo-4-((R)-1-hydroxymethyl-propylamino)-pyrimidin-2-ylamino]-N-(3-fluoro-benzyl)-benzenesulfonamide,
4-[5-Bromo-4-((R)-1-hydroxymethyl-propylamino)-pyrimidin-2-ylamino]-N-[2-(1-methyl-pyrrolidin-2-yl)-ethyl]-benzenesulfonamide,
4-[5-Bromo-4-((R)-1-hydroxymethyl-propylamino)-pyrimidin-2-ylamino]-N-pyridin-3-ylmethyl-benzenesulfonamide,
4-[5-Bromo-4-((R)-1-hydroxymethyl-propylamino)-pyrimidin-2-ylamino]-N-(4-fluoro-benzyl)-benzenesulfonamide,
4-[5-Bromo-4-((R)-1-hydroxymethyl-propylamino)-pyrimidin-2-ylamino]-N-pyridin-4-ylmethyl-benzenesulfonamide,
4-[5-Bromo-4-((R)-1-hydroxymethyl-propylamino)-pyrimidin-2-ylamino]-N-(2-phenoxy-ethyl)-benzenesulfonamide,
4-[5-Bromo-4-((R)-1-hydroxymethyl-propylamino)-pyrimidin-2-ylamino]-N-(2-methoxy-benzyl)-benzenesulfonamide,
4-[5-Bromo-4-((R)-1-hydroxymethyl-propylamino)-pyrimidin-2-ylamino]-N-(4-methyl-benzyl)-benzenesulfonamide or
4-[5-Bromo-4-((R)-1-hydroxymethyl-propylamino)-pyrimidin-2-ylamino]-N-(5-methylsulfanyl-1H-[1,2,4]triazol-3-yl)-benzenesulfonamide

Die erfindungsgemäßen Verbindungen inhibieren im wesentlichen Zyklinabhängige Kinasen, worauf auch deren Wirkung zum Beispiel gegen Krebs, wie solide Tumoren und Leukämie, Autoimmunerkrankungen wie Psoriasis, Alopezie, und Multiple Sklerose, Chemotherapeutika-induzierte Alopezie und Mukositis, kardiovaskuläre Erkrankungen, wie Stenosen, Arteriosklerosen und Restenosen, infektiöse Erkrankungen, wie z. B. durch unizelluläre Parasiten, wie Trypanosoma, Toxoplasma oder Plasmodium, oder durch Pilze hervorgerufen, nephrologische Erkrankungen, wie z. B. Glomerulonephritis, chronische neurodegenerative Erkrankungen, wie Huntington's Erkrankung, amyotrophe Lateralsklerose, Parkinsonsche Erkrankung, AIDS Dementia und Alzheimer'sche Erkrankung, akute neurodegenerative Erkrankungen, wie Ischämien des Gehirns und Neurotraumata, virale Infektionen, wie z. B. Cytomegalus-Infektionen, Herpes, Hepatitis B und C, und HIV Erkrankungen basiert.

Der eukaryote Zellteilungszyklus stellt die Duplikation des Genoms und seine Verteilung auf die Tochterzellen sicher, indem er eine koordinierte und regulierte Abfolge von Ereignissen durchläuft. Der Zellzyklus wird in vier aufeinanderfolgende Phasen eingeteilt: die G1 Phase repräsentiert die Zeit vor der DNA-Replikation, in der die Zelle wächst und für externe Stimuli empfänglich ist. In der S Phase repliziert die Zelle ihre DNA, und in der G2 Phase bereitet sie sich auf den Eintritt in die Mitose vor. In der Mitose (M Phase) wird die replizierte DNA getrennt und die Zellteilung vollzogen.

Die Zyklin-abhängigen Kinasen (CDKs), eine Familie von Serin/Threonin-Kinasen, deren Mitglieder die Bindung eines Zyklins (Cyc) als regulatorische Untereinheit zu ihrer Aktivierung benötigen, treiben die Zelle durch den Zellzyklus. Unterschiedliche CDK/Cyc Paare sind in den verschiedenen Phasen des Zellzyklus aktiv. Für die grundlegende Funktion des Zellzyklus bedeutende CDK/Cyc Paare sind beispiels-weise CDK4(6)/CycD, CDK2/CycE, CDK2/CycA, CDK1/CycA und CDK1/CycB. Einige Mitglieder der CDK-Enzymfamilie haben eine regulatorische Funktion indem sie die Aktivität der vorgenannten Zellzyklus-CDKs beeinflussen, während anderen Mitgliedern der CDK-Enzymfamlie noch keine bestimmte Funktion zugeordnet werden konnte. Eine von diesen, CDK5, zeichnet sich dadurch aus, daß sie eine atypische, von den Zyklinen abweichende, regulatorische Untereinheit besitzt (p35), und ihre Aktivität im Gehirn am höchsten ist.

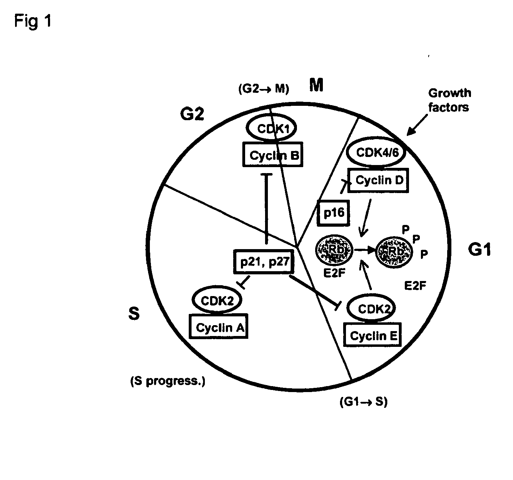

Der Eintritt in den Zellzyklus und das Durchlaufen des "Restriction Points", der die Unabhängigkeit einer Zelle von weiteren Wachstumssignalen für den Abschluß der begonnenen Zellteilung markiert, werden durch die Aktivität der CDK4(6)/CycD und CDK2/CycE Komplexe kontrolliert. Das wesentliche Substrat dieser CDK-Komplexe ist das Retinoblastoma-Protein (Rb), das Produkt des Retinoblastoma Tumorsuppressor Gens. Rb ist ein transkriptionelles Ko-Repressor Protein. Neben anderen noch weitgehend unverstandenen Mechanismen, bindet und inaktiviert Rb Transkriptionsfaktoren vom E2F-Typ, und bildet transkriptionelle Repressorkomplexe mit Histon-Deacetylasen (HDAC) (Zhang H.S. et al. (2000). Exit from G1 and S phase of the cell cycle is regulated by repressor complexes containing HDAC-Rb-hSWI/SNF and RbhSWI/SNF. Cell 101, 79-89). Durch die Phosphorylierung des Rb durch CDKs werden gebundene E2F Transkriptionsfaktoren freigesetzt und führen zu transkriptioneller Aktivierung von Genen, deren Produkte für die DNA Synthese und die Progression durch die S-Phase benötigt werden. Zusätzlich bewirkt die Rb-Phosphorylierung die Auflösung der Rb-HDAC Komplexe, wodurch weitere Gene aktiviert werden. Die Phosphorylierung von Rb durch CDK's ist mit dem Überschreiten des "Restriction Points" gleichzusetzen. Für die Progression durch die S-Phase und deren Abschluß ist die Aktivität der CDK2/CycE und CDK2/CycA Komplexe notwendig, z. B. wird die Aktivität der Transkriptionsfaktoren vom E2F-Typ mittels Phosphorylierung durch CDK2/CycA abgeschaltet sobald die Zellen in die S-Phase eingetreten sind. Nach vollständiger Replikation der DNA steuert die CDK1 im Komplex mit CycA oder CycB den Eintritt und das Durchlaufen der Phasen G2 und M (Abb. 1).

Entsprechend der außerordentlichen Bedeutung des Zellteilungszyklus ist das Durchlaufen des Zyklus streng reguliert und kontrolliert. Die Enzyme, die für die Progression durch den Zyklus notwendig sind, müssen zu dem richtigen Zeitpunkt aktiviert werden, und auch wieder abgeschaltet werden sobald die entsprechende Phase durchlaufen ist. Entsprechende Kontrollpunkte ("Checkpoints") arretieren die Progression durch den Zellzyklus falls DNA-Schäden detektiert werden, oder die DNA-Replikation, oder der Aufbau des Spindelapparates noch nicht beendet ist.
Die Aktivität der CDKs wird durch verschiedene Mechanismen, wie Synthese und Degradation der Zykline, Komplexierung der CDKs mit den entsprechenden Zyklinen, Phosphorylierung und Dephosphorylierung regulatorischer Threonin-und Tyrosin-Reste, und die Bindung natürlicher inhibitorischer Proteine, direkt kontrolliert. Während die Proteinmenge der CDKs in einer proliferierenden Zelle relativ konstant ist, oszilliert die Menge der einzelnen Zykline mit dem Durchlaufen des Zyklus. So wird zum Beispiel die Expression von CycD während der frühen G1 Phase durch Wachstumsfaktoren stimuliert, und die Expression von CycE wird nach Überschreiten des "Restriction Points" durch die Aktivierung der Transkriptionsfaktoren vom E2F-Typ induziert. Die Zykline selbst werden durch Ubiquitin-vermittelte Proteolyse abgebaut. Aktivierende und inaktivierende Phosphorylierungen regulieren die Aktivität der CDK's, zum Beispiel phosphorylieren CDK-aktivierende Kinasen (CAKs) Thr160/161 der CDK1, wohingegen die Familie der Wee1/Myt1 Kinasen CDK1 durch Phosphorylierung von Thr14 und Tyr15 inaktivieren. Diese inaktivierenden Phosphorylierungen können durch cdc25 Phosphatasen wieder aufgehoben werden. Sehr bedeutsam ist die Regulation der Aktivität der CDK/Cyc-Komplexe durch zwei Familien natürlicher CDK Inhibitorproteine (CKls), den Proteinprodukten der p21 Genfamilie (p21, p27, p57) und der p16 Genfamilie (p15, p16, p18, p19). Mitglieder der p21 Familie binden an Zyklin-Komplexe der CDKs 1,2,4,6, inhibieren aber nur Komplexe die CDK1 oder CDK2 enthalten. Mitglieder der p16 Familie sind spezifische Inhibitoren der CDK4- und CDK6-Komplexe.

Oberhalb dieser komplexen direkten Regulation der Aktivität der CDKs liegt die Ebene der Kontrollpunkt-Regulation. Kontrollpunkte erlauben der Zelle das geordnete Ablaufen der einzelnen Phasen während des Zellzykluses zu verfolgen. Die wichtigsten Kontrollpunkte liegen am Übergang von G1 nach S und von G2 nach M. Der G1-Kontrollpunkt stellt sicher, daß die Zelle keine DNA-Synthese beginnt falls sie nicht entsprechend ernährt ist, mit anderen Zellen oder dem Substrat korrekt interagiert, und ihre DNA intakt ist. Der G2/M Kontrollpunkt stellt die vollständige Replikation der DNA und den Aufbau der mitotischen Spindel sicher, bevor die Zelle in die Mitose eintritt. Der G1 Kontrollpunkt wird von dem Genprodukt des p53 Tumorsuppressorgens aktiviert. p53 wird nach Detektion von Veränderungen im Metabolismus oder der genomischen Integrität der Zelle aktiviert und kann entweder einen Stopp der Zellzyklusprogression oder Apoptose auslösen. Dabei spielt die transkriptionelle Aktivierung der Expression des CDK Inhibitorproteins p21 durch p53 eine entscheidende Rolle. Ein zweiter Zweig des G1 Kontrollpunktes umfaßt die Aktivierung der ATM und Chk1 Kinasen nach DNA-Schädigung durch UV-Licht oder ionisierende Strahlung und schließlich die Phosphorylierung und den nachfolgenden proteolytischen Abbau der cdc25A Phosphatase (Mailand N. et al. (2000). Rapid destruction of human cdc25A in response to DNA damage. Science 288, 1425-1429). Daraus resultiert eine Arretierung des Zellzykluses, da die inhibitorische Phosphorylierung der CDKs nicht entfernt wird. Nach Aktivierung des G2/M Kontrollpunktes durch Schädigung der DNA sind beide Mechanismen in ähnlicher Weise daran beteiligt, die Progression durch den Zellzyklus zu stoppen.

Der Verlust der Regulation des Zellzyklusses und der Verlust der Funktion der Kontrollpunkte sind Charakteristika von Tumorzellen. Der CDK-Rb-Signalweg ist in über 90% humaner Tumorzellen von Mutationen betroffen. Diese Mutationen, die schließlich zur inaktivierenden Phosphorylierung des RB führen, schließen die Überexpression von D- und E-Zyklinen durch Genamplifikation oder chromosomale Translokationen, inaktivierende Mutationen oder Deletionen von CDK-Inhibitoren des p16-Typs, sowie erhöhten (p27) oder verminderten (CycD) Proteinabbau ein. Die zweite Gruppe von Genen, die durch Mutationen in Tumorzellen getroffen sind, kodiert für Komponenten der Kontrollpunkte. So ist p53, das essentiell für die G1 und G2/M Kontrollpunkte ist, das am häufigsten mutierte Gen in humanen Tumoren (ca. 50%). In Tumorzellen, die p53 ohne Mutation exprimieren, wird es häufig aufgrund einer stark erhöhten Proteindegradation inaktiviert. In ähnlicher Weise sind die Gene anderer für die Funktion der Kontrollpunkte notwendiger Proteine von Mutationen betroffen, zum Beispiel ATM (inaktivierende Mutationen) oder cdc25 Phosphatasen (Überexpression).

Überzeugende experimentelle Daten deuten darauf hin, daß CDK2/Cyc-Komplexe eine entscheidende Position während der Zellzyklusprogression einnehmen: (1) Sowohl dominant-negative Formen der CDK2, wie die transkriptionelle Repression der CDK2 Expression durch anti-sense Oligonukleotide bewirken einen Stopp der Zellzyklusprogression. (2) Die Inaktivierung des CycA Gens in Mäusen ist letal. (3) Die Störung der Funktion des CDK2/CycA Komplexes in Zellen mittels zell-permeabler Peptide führte zur Tumorzell-selektiven Apoptose (Chen Y.N.P. et al. (1999). Selective killing of transformed cells by cyclin/cyclin-dependent kinase 2 antagonists. Proc. Natl. Acad. Sci. USA 96, 4325-4329).

Veränderungen der Zellzykluskontrolle spielen nicht nur bei Krebserkrankungen ein Rolle. Der Zellzyklus wird durch eine Reihe von Viren, sowohl durch transformierende, wie durch nicht-transformierende, aktiviert um die Vermehrung der Viren in der Wirtszelle zu ermöglichen. Der fälschliche Eintritt in den Zellzyklus von normalerweise post-mitotischen Zellen wird mit verschiedenen neurodegenerativen Erkrankungen in Zusammenhang gebracht. Die Mechanismen der Zellzyklusregulation, ihrer Veränderungen in Krankheiten und eine Vielzahl von Ansätzen zur Entwicklung von Inhibitoren der Zellzyklusprogression und speziell der CDKs wurden bereits in mehreren Publikationen ausführlich zusammenfassend beschrieben (Sielecki T.M. et al. (2000). Cyclin-dependent kinase inhibitors: useful targets in cell cycle regulation. J. Med. Chem. 43, 1-18; Fry D.W. & Garrett M.D. (2000). Inhibitors of cyclin-dependent kinases as therapeutic agents for the treatment of cancer. Curr. Opin. Oncol. Endo. Metab. Invest. Drugs 2, 40-59; Rosiania G.R. & Chang Y.T. (2000). Targeting hyperproliferative disorders with cyclin dependent kinase inhibitors. Exp. Opin. Ther. Patents 10, 215-230; Meijer L. et al. (1999). Properties and potential applications of chemical inhibitors of cyclin-dependent kinases. Pharmacol. Ther. 82, 279-284; Senderowicz A.M. & Sausville E.A. (2000). Preclinical and clinical development of cyclin-dependent kinase modulators. J. Natl. Cancer Inst. 92, 376-387).

Zur Verwendung der erfindungsgemäßen Verbindungen als Arzneimittel werden diese in die Form eines pharmazeutischen Präparats gebracht, das neben dem Wirkstoff für die enterale oder parenterale Applikation geeignete pharmazeutische, organische oder anorganische inerte Trägermaterialien, wie zum Beispiel, Wasser, Gelatine, Gummi arabicum, Milchzucker, Stärke, Magnesiumstearat, Talk, pflanzliche Öle, Polyalkylenglykole usw. enthält. Die pharmazeutischen Präparate können in fester Form, zum Beispiel als Tabletten, Dragees, Suppositorien, Kapseln oder in flüssiger Form, zum Beispiel als Lösungen, Suspensionen oder Emulsionen vorliegen. Gegebenenfalls enthalten sie darüber hinaus Hilfsstoffe, wie Konservierungs-, Stabilisierungs-, Netzmittel oder Emulgatoren; Salze zur Veränderung des osmotischen Drucks oder Puffer. Diese pharmazeutischen Präparate sind ebenfalls Gegenstand der vorliegenden Erfindung.

Für die parenterale Anwendung sind insbesondere Injektionslösungen oder Suspensionen, insbesondere wäßrige Lösungen der aktiven Verbindungen in polyhydroxyethoxyliertem Rizinusöl, geeignet.

Als Trägersysteme können auch grenzflächenaktive Hilfsstoffe wie Salze der Gallensäuren oder tierische oder pflanzliche Phospholipide, aber auch Mischungen davon sowie Liposomen oder deren Bestandteile verwendet werden.

Für die orale Anwendung sind insbesondere Tabletten, Dragees oder Kapseln mit Talkum und/oder Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff beigefügt ist.

Die enteralen, parenteralen und oralen Applikationen sind ebenfalls Gegenstand der vorliegenden Erfindung.

Die Dosierung der Wirkstoffe kann je nach Verabfolgungsweg, Alter und Gewicht des Patienten, Art und Schwere der zu behandelnden Erkrankung und ähnlichen Faktoren variieren. Die tägliche Dosis beträgt 0,5-1000 mg, vorzugsweise 50-200 mg, wobei die Dosis als einmal zu verabreichende Einzeldosis oder unterteilt in 2 oder mehreren Tagesdosen gegeben werden kann.
Ebenfalls Gegenstand der vorliegenden Erfindung ist die Verwendung der Verbindungen der allgemeinen Formel 1, zur Herstellung eines Arzneimittels zur Behandlung von Krebs, Autoimmunerkrankungen, kardiovaskulären Erkrankungen, Chemotherapeutika-induzierter Alopezie und Mukositis, infektiösen Erkrankungen, nephrologischen Erkrankungen, chronischen und akuten neurodegenerativen Erkrankungen und viralen Infektionen, wobei unter Krebs solide Tumoren und Leukämie, unter Autoimmunerkrankungen Psoriasis, Alopezie und Multiple Sklerose, unter kardiovaskulären Erkrankungen Stenosen, Arteriosklerosen und Restenosen, unter infektiösen Erkrankungen durch unizelluläre Parasiten hervorgerufene Erkrankungen, unter nephrologischen Erkrankungen Glomerulonephritis, unter chronisch neurodegenerativen Erkrankungen Huntington's Erkrankung, amyotrophe Lateralsklerose, Parkinsonsche Erkrankung, AIDS Dementia und Alzheimer'sche Erkrankung, unter akut neurodegenerativen Erkrankungen Ischämien des Gehirns und Neurotraumata, und unter viralen Infektionen Cytomegalus-Infektionen, Herpes, Hepatitis B oder C, und HIV Erkrankungen zu verstehen sind.

Ebenfalls Gegenstand der vorliegenden Erfindung sind Arzneimittel zur Behandlung der oben aufgeführten Erkrankungen, die mindestens eine Verbindung gemäß der allgemeinen Formel I enthalten, sowie Arzneimittel mit geeigneten Formulierungs- und Trägerstoffen.

Die erfindungsgemäßen Verbindungen der allgemeinen Formel I sind unter anderem hervorragende Inhibitoren der Zyklin-abhängigen Kinasen, wie CDK1, CDK2, CDK3, CDK4, CDK5, CDK6, CDK7, CDK8 und CDK9, sowie der Glycogen-Synthase-Kinase (GSK-3β).

Soweit die Herstellung der Ausgangsverbindungen nicht beschrieben wird, sind diese bekannt oder analog zu bekannten Verbindungen oder hier beschriebenen Verfahren herstellbar. Es ist ebenfalls möglich, alle hier beschriebenen Umsetzungen in Parallel-Reaktoren oder mittels kombinatorischer Arbeitstechniken durchzuführen.
Die Isomerengemische können nach üblichen Methoden wie beispielsweise Kristallisation, Chromatographie oder Salzbildung in die Enantiomeren bzw. E/Z-Isomeren aufgetrennt werden.
Die Herstellung der Salze erfolgt in üblicher Weise, indem man eine Lösung der Verbindung der Formel I mit der äquivalenten Menge oder einem Überschuß einer Base oder Säure, die gegebenenfalls in Lösung ist, versetzt und den Niederschlag abtrennt oder in üblicher Weise die Lösung aufarbeitet.

### Herstellung der erfindungsgemäßen Verbindungen

Die nachfolgenden Beispiele erläutern die Herstellung der erfindungsgemäßen Verbindungen, ohne den Umfang der beanspruchten Verbindungen auf diese Beispiele zu beschränken.
Die erfindungsgemäßen Verbindungen der allgemeinen Formel I lassen sich nach folgenden allgemeinen Verfahrenschemata herstellen:

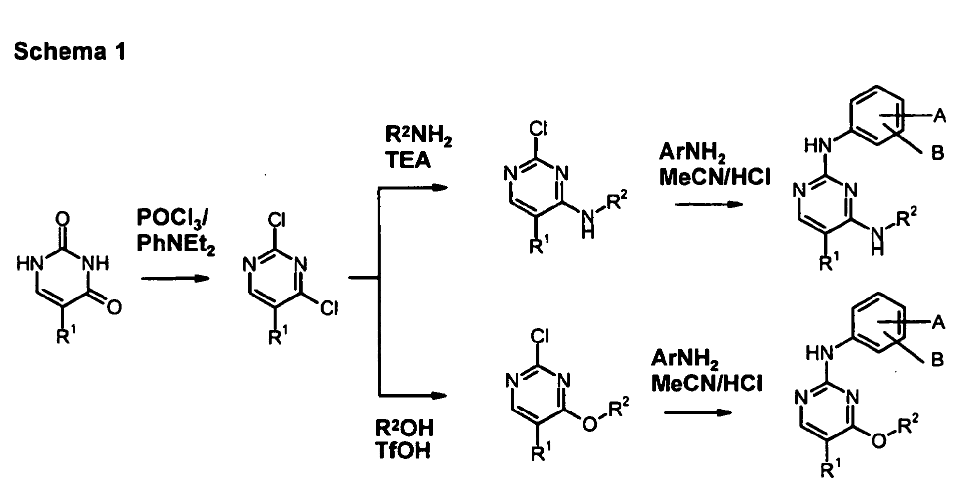

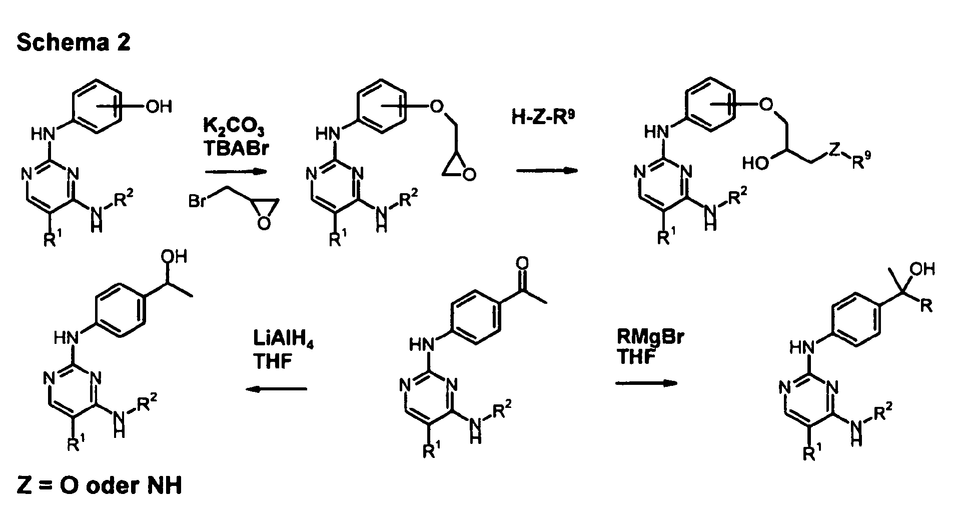

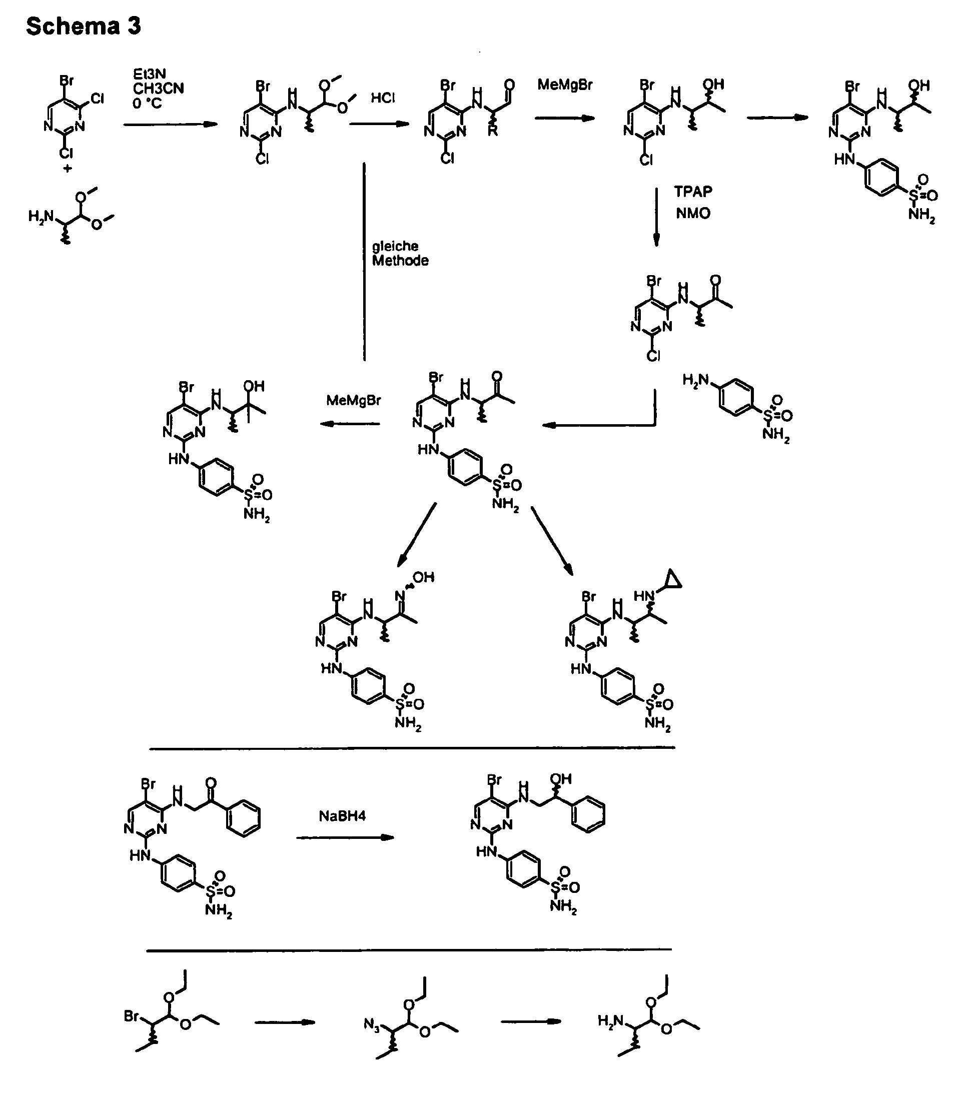

### Beispiel 1

### Herstellung von 5-Brom-N2-(4-difluorormethylthiophenyl)-N4-2-propynyl-2,4-pyrimidindiamin (erfolgt nach Verfahrensschema 1) (Verbindung 23).

245 mg (1 mmol) 2-Chlor-4-2-propynylaminopyrimidin werden in 2 ml Acetonitril gelöst und eine Suspension von 4-(Difluormethylthio)-anilinhydrochlorid [hergestellt aus 352 mg (2 mmol) 4-(Difluormethylthio)-Anilin, 1 ml Acetonitril and 0,5 ml wässrige HCl (4M in Dioxan)] wird bei Raumtemperatur hinzugegeben. Anschließend wird das Reaktionsgemisch über Nacht unter N₂-Atmosphäre am Rückfluss erhitzt. Nach Abkühlung wird das Gemisch filtriert, die verbleibende feste Phase wird mit H₂O gewaschen und getrocknet. Eine Ausbeute von 328 mg (85%) des Produktes ist zu erwarten.

| | | | |
|---|---|---|---|
| 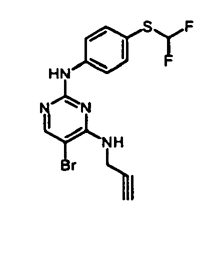 | 6H | 8.25 (s,1 H) | Ausbeute: |
| | 2C H | 7.86 (d,2H) 7.51 (d,2H) | 85% |
| | | 7.38 | |
| | 4C | (t,56.8Hz,1H) 4.18 (m,2H) | Schmelzpunkt: >235°C |
| | H | 3.16 (sb,1H) | |
| | | 10.24 (sb,1H) | |
| | NH | 8.17 (sb,1 H) | |

### Beispiel 2

### Herstellung von 5-Brom-N-(3-(oxiranylmethoxy)phenyl)-2-(2-propynyloxy)-2-pyrimidinamin (Verbindung 51) und erfolgt nach Verfahrensschema 2.

1,55 g (4.9 mmol) der Verbindung 20 werden in 5,5 ml Epibromhydrin gelöst, 1,38 g K₂CO₃ und 65 mg Tetrabutylammoniumbromid werden dazu gegeben. Das Reaktionsgemisch wird unter Stickstoffatmosphäre bei 100°C 1 Stunde gerührt. Nach Zugabe von Ethylacetat wird das resultierende Präzipitat gesammelt und vom Ethanol umkristallisiert. Die Produktausbeute beträgt 1,15 g (62%) als weißes Pulver.

| | | | |
|---|---|---|---|
| 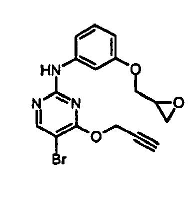 | 6H | 8.45 (s,1 H) | |
| | 2CH | 7.47 (s,1 H) | |
| | | 7.32 (d,1 H) | Ausbeute: 62% |
| | | 7.20 (t,1H) | |
| | | 6.40 (d,1 H) | Schmelzpunkt: 173°C |
| | | 4.32 (dd,1 H) | |
| | | 3.82 (dd,1 H) | |
| | | 3.3-3.4(m,1 H) | |
| | | 2.87 (t,1 H) | |
| | | 2.72 (dd,1 H) | |
| | 4CH | 5.13 (d,2H) | |
| | | 3.67 (t,1 H) | |
| | NH | 9.84 (sb,1H) | |

Die Substanz 40 wird analog zu Beispiel 2 hergestellt

| | | | |
|---|---|---|---|
| 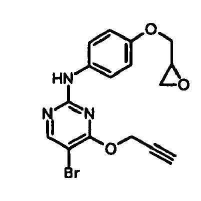 | 6-H | 8.36 (s,1H) | Chromatographie: |
| | 2CH | 7.60 (d,1H) | H/EA 1:3 0.5%TEA |
| | | 6.91 (d,1H) | |
| | | 4.28 (dd,1 H) | |
| | | 3.79 (dd,1H) | Ausbeute: 38% |
| | | 3.31 (m,1H) | |
| | | 2.84 (dd, 1 H) | Schmelzpunkt: 140-141°C |
| | | 2.70 (dd,1 H) | |
| | 4CH | 5.07 (d,12H) | |
| | | 3.65 (t,1 H) | |
| | NH | 9.65 (sb,1 H) | |
| | OH | | |

### Beispiel 3

### Herstellung 1-(4-((5-Brom-4-(2-propynyloxy)-pyrimidin-2-yl)-amino)phenoxy)-3-(4-phenylpiperazin-1-yl)-2-propanol (Verbindung 41).

Zu einer Lösung von 19 mg (0.05 mM) der Substanz 51 in N,N'-Dimethylpropylhamstoff (DMPU) werden 0.2 ml einer 0.5 M 4-Phenylpiperazin-Lösung in DMPU gegeben. Das Reaktionsgemisch wird für 18 Stunden bei einer Temperatur von 80°C gehalten. Nach dem Abkühlen werden 3,5 ml tertiärer Butylmethylether hinzugegeben und die organische Phase wird 5 mal mit 1,5 ml H₂O extrahiert und anschließend im Vakuum evaporiert. Der verbleibende Rest wird an 1,7g (15 µM) Lichrosphere Si60 (Gradient: Dichloromethan / Hexan 1:1 zu DCM und dann Dichloromethan / Methanol 99:1 bis 93:7) chromatographiert. Eine Produktausbeute von 17 mg (64%) wird erreicht.

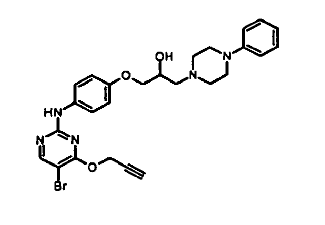

In analoger Verfahrensweise werden auch die folgenden Verbindungen hergestellt:

| Nr. | Struktur | Nr. | Struktur | Nr. | Struktur |
|---|---|---|---|---|---|
| 96 | 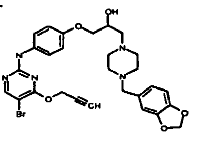 | 97 | 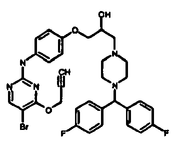 | 98 | 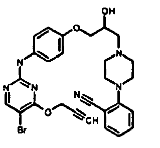 |
| 99 | 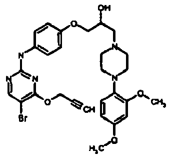 | 100 | 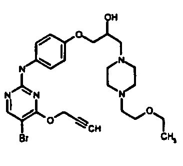 | 101 | 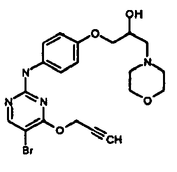 |
| 102 | 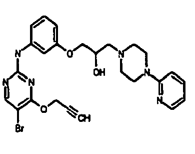 | 103 | 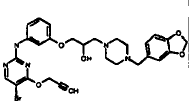 | 104 | 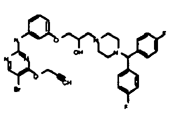 |
| 105 | 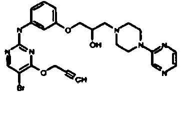 | 106 | 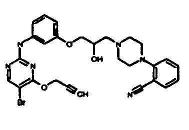 | 107 | 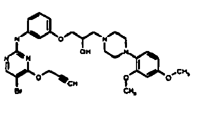 |
| 108 | 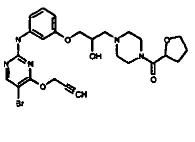 | 109 | 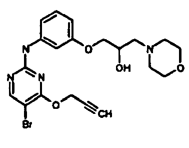 | 110 | 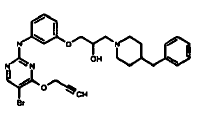 |
| 111 | 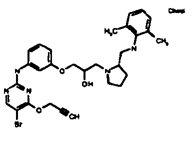 | 112 | 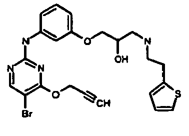 | 113 | 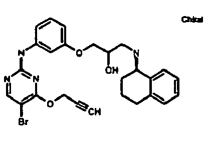 |
| 114 | 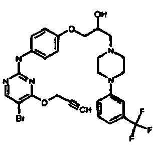 | 115 | 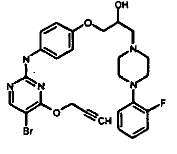 | 116 | 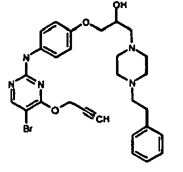 |
| 117 | 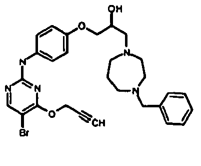 | 118 | 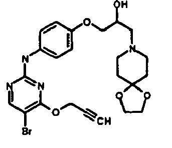 | 119 | 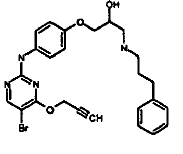 |

Folgende Verbindungen wurden in analoger Verfahrensweise zu den beschriebenen Beispielen hergestellt.

| Nr | Struktur | Name |
|---|---|---|
| 28 | 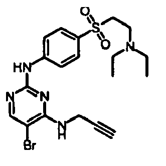 | 5-Brom-N2-(4-(2-Diethylaminoethylsulfonyl)phenyl)-N4-2-propynyl-2,4-pyrimidindiamin |
| 30 | 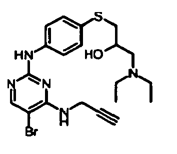 | 1-(4-[5-Brom-4-(2-propynylamino)-2-pyrimidinyl]amino-phenylthio)-3-(diethylamino)-2-propanol |
| 32 | 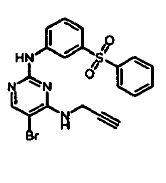 | 5-Brom-N2-(3-phenylsulfonylphenyl)-N4-2-propynyl-2,4-pyrimidindiamin |
| 33 | 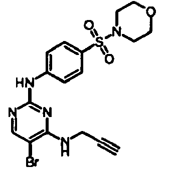 | N-[4-[[5-Brom-4-(2-propynylamino)-2-pyrimidinyl]amino]-benzolsulfonyl]morpholin |
| 41 | 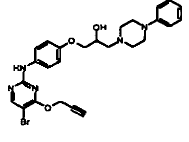 | 1-(4-((5-Brom-4-(2-propynyloxy)-pryrimidin-2-yl)-amino)phenoxy)-3-(4-phenylpiperazin-1-yl)-2-propanol |
| 57 | 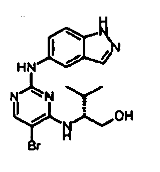 | N-[5-Brom-4-((2R)-1-hydroxy-4-methyl-2-butylamino)-2-pyrimidinyl]-indazol-5-amin |
| 58 | 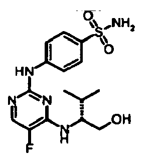 | 4-[[5-Fluor-4-((2R)-1-hydroxy-3-methyl-2-butylamino)-2-pyrimidinyl]amino]-benzolsulfonamid |
| 59 | 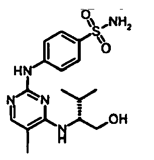 | 4-[[5-lod-4-((2R)-1-hydroxy-3-methyl-2-butylamino)-2-pyrimidinyl]amino]-benzolsulfonamid |
| 62 | 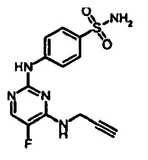 | 4-[[5-Fluor-4-(2-propynylamino)-2-pyrimidinyl]amino]-benzolsulfonamid |
| 65 | 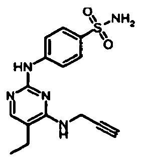 | 4-[[5-Ethyl-4-(2-propynylamino)-2-pyrimidinyl]amino]-benzolsulfonamid |
| 66 | 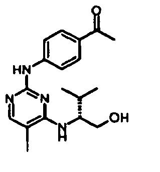 | 1-[4-[(5-lod-4-((2R)-1-hydroxy-3-methyl-2-butylamino)-2-pyrimidinyl)amino]phenyl]-ethanon |
| 68 | 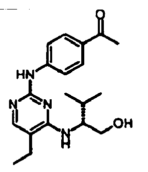 | 1-[4-[(5-Ethyl-4-((2R)-1-hydroxy-3-methyl-2-butylamino)-2-pyrimidinyl)amino]phenyl]-ethanon |
| 72 | 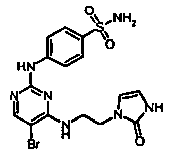 | 4-[[5-Brom-4-(2-(2-oxo-imidazolin-1-yl)ethylamin)-2-pyrimidinyl]amino]-benzolsulfonamid |
| 73 | 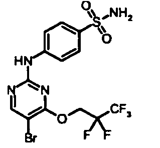 | 4-[[5-Brom-4-(2,2,3,3,3-pentafluorpropyloxy)-2-pyrimidinyl]amino]-benzolsulfonamid |
| 75 | 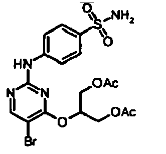 | 4-[[5-Brom-4-(1,3-bisacetoxy-2-propyloxy)-2-pyrimidinyl]amino]-benzolsulfonamid |
| 76 | 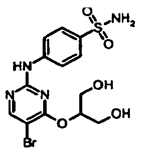 | 4-[[5-Brom-4-(1,3-dihydroxy-2-propyloxy)-2-pyrimidinyl]amino]-benzolsulfonamid |
| 79 | 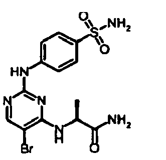 | N□-(5-Brom-2-(4-sulfamoylphenyl)amino-pyrimidin-4-yl)-L-alanineamid |
| 83 | 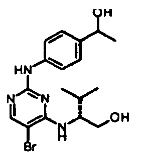 | 1-[4-[(5-Brom-4-(2-propynylamino)-2-pyrimidinyl)amino]phenyl]-ethanol |

Folgende Verbindungen wurden analog zu den beschriebenen Syntheseverfahren gemäß Schema 1 oder 2 hergestellt:
Alle NMR-Spektren werden in angegebenem Lösungsmittel gemessen oder in DMSO.

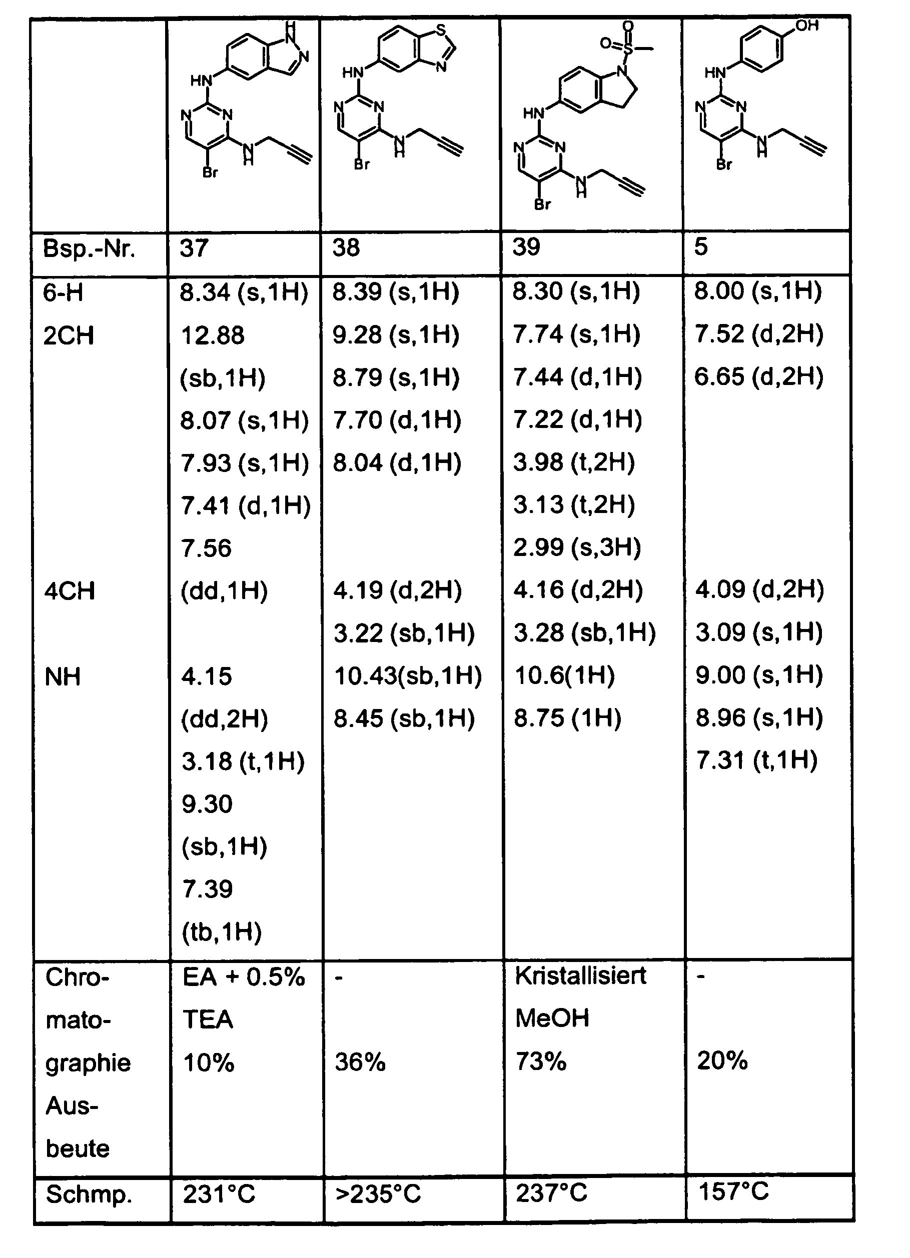

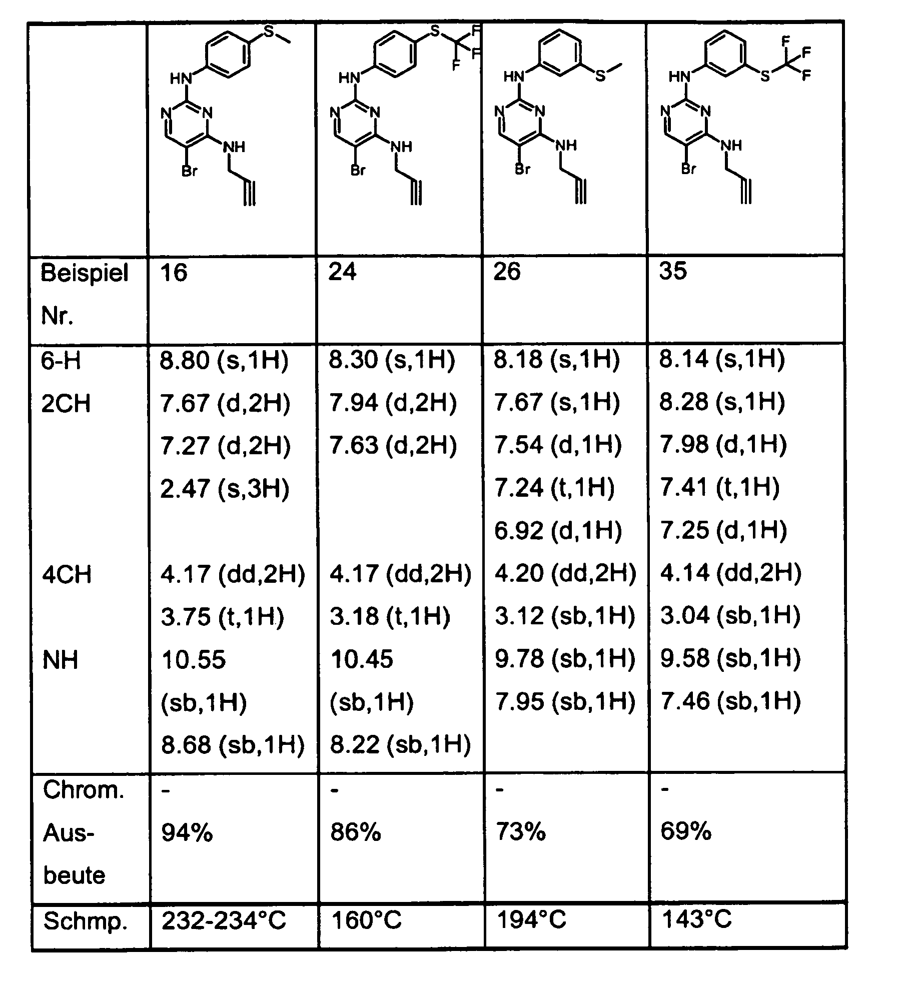

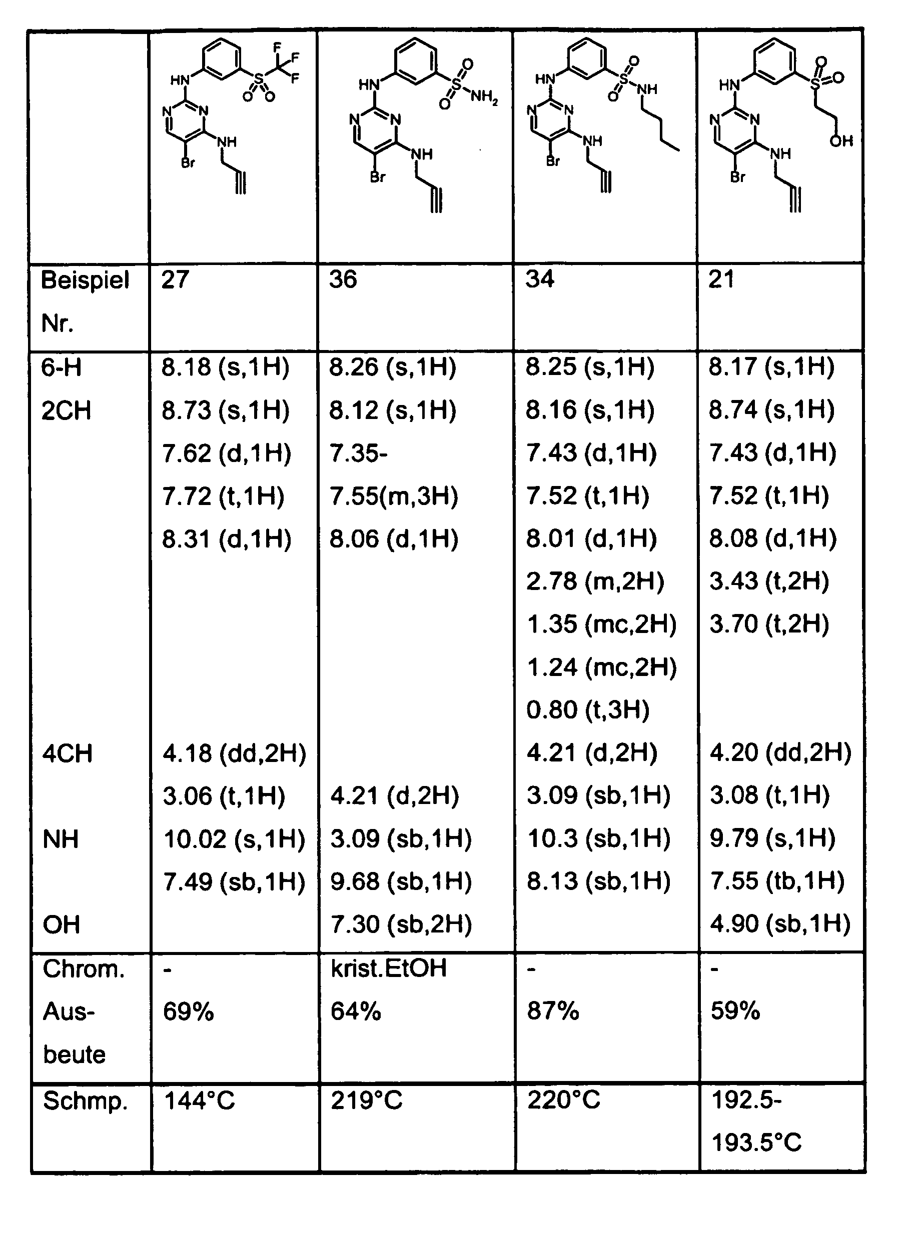

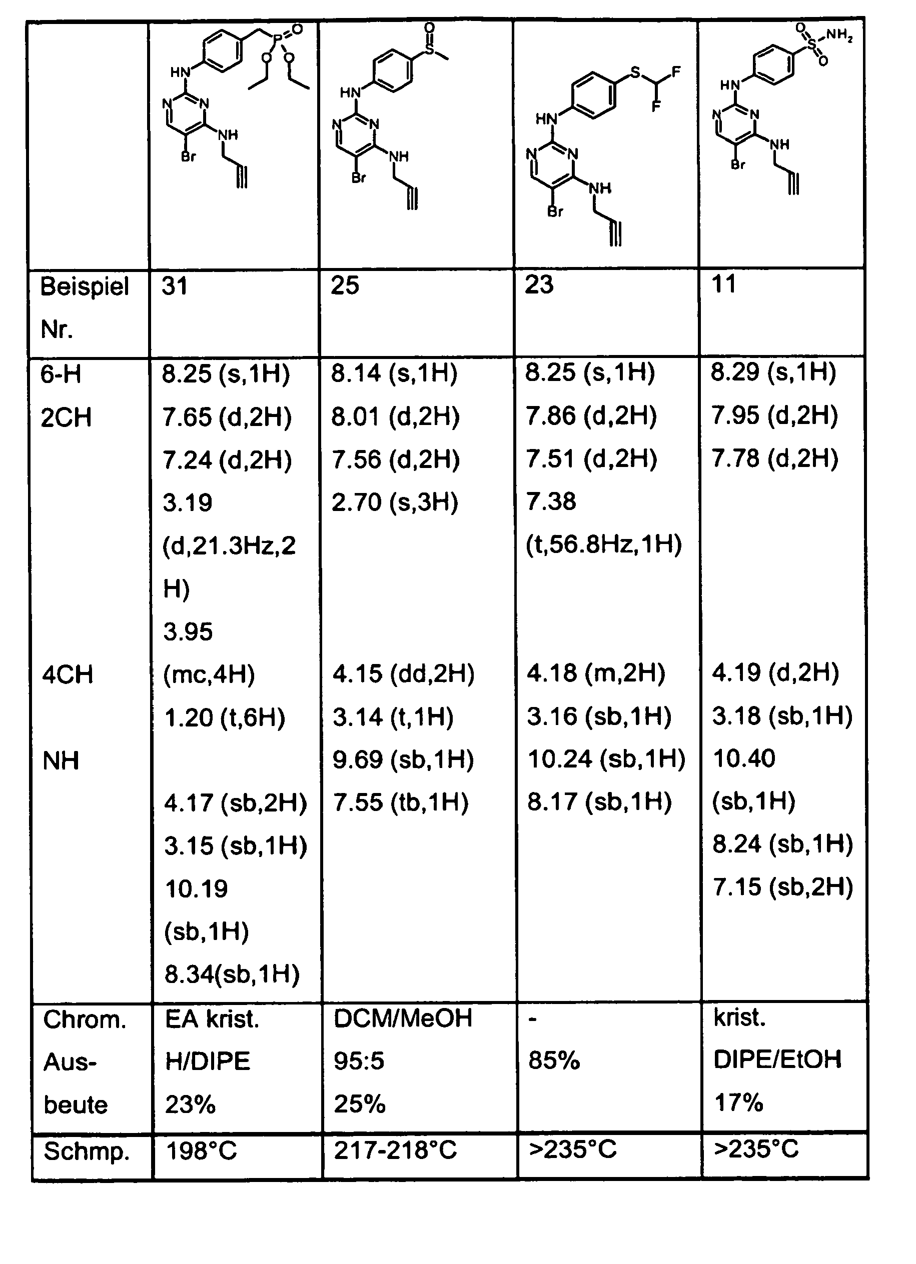

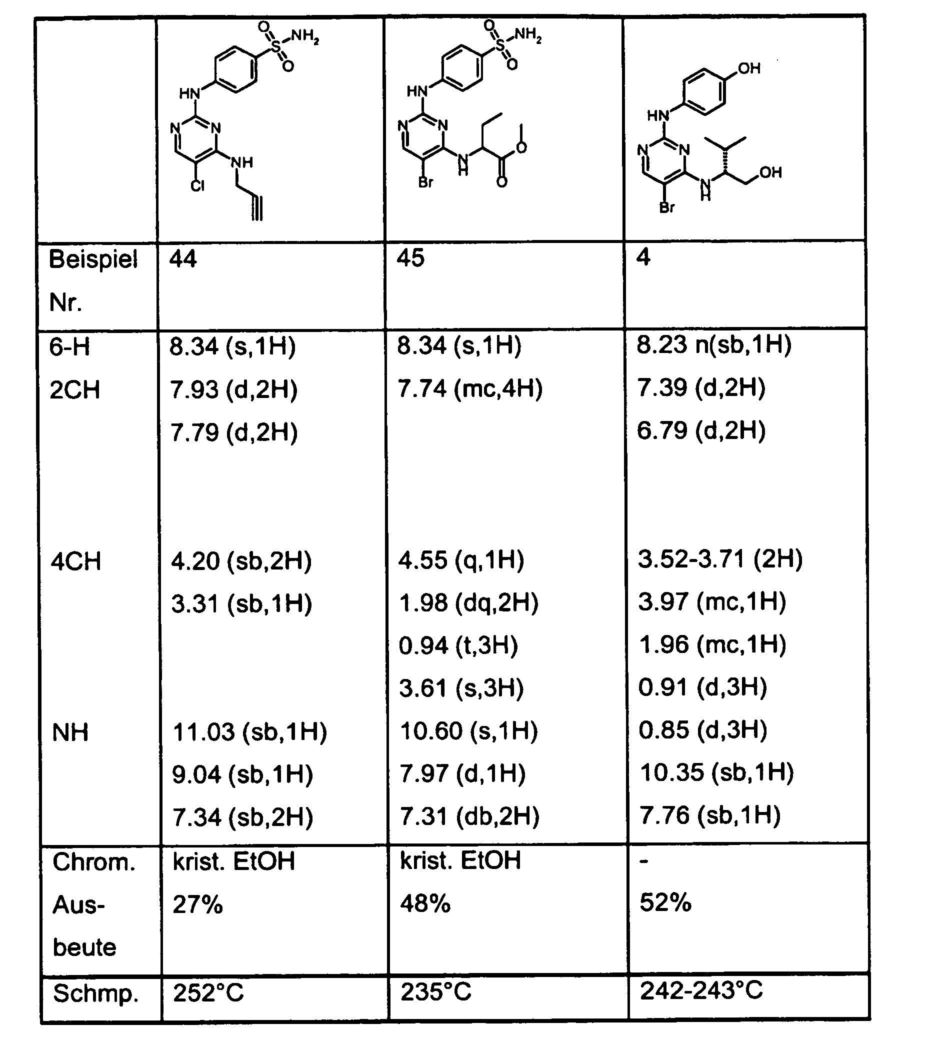

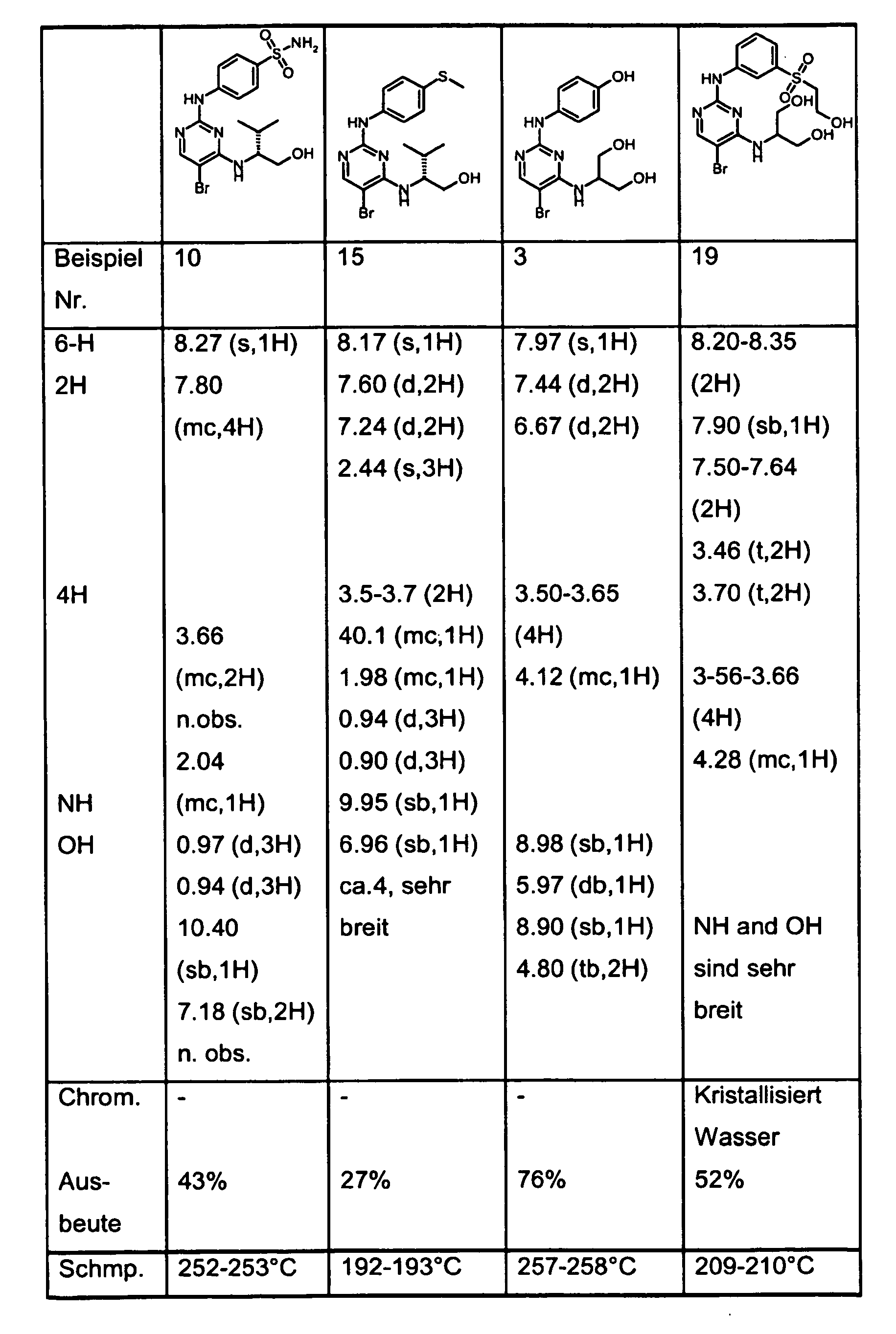

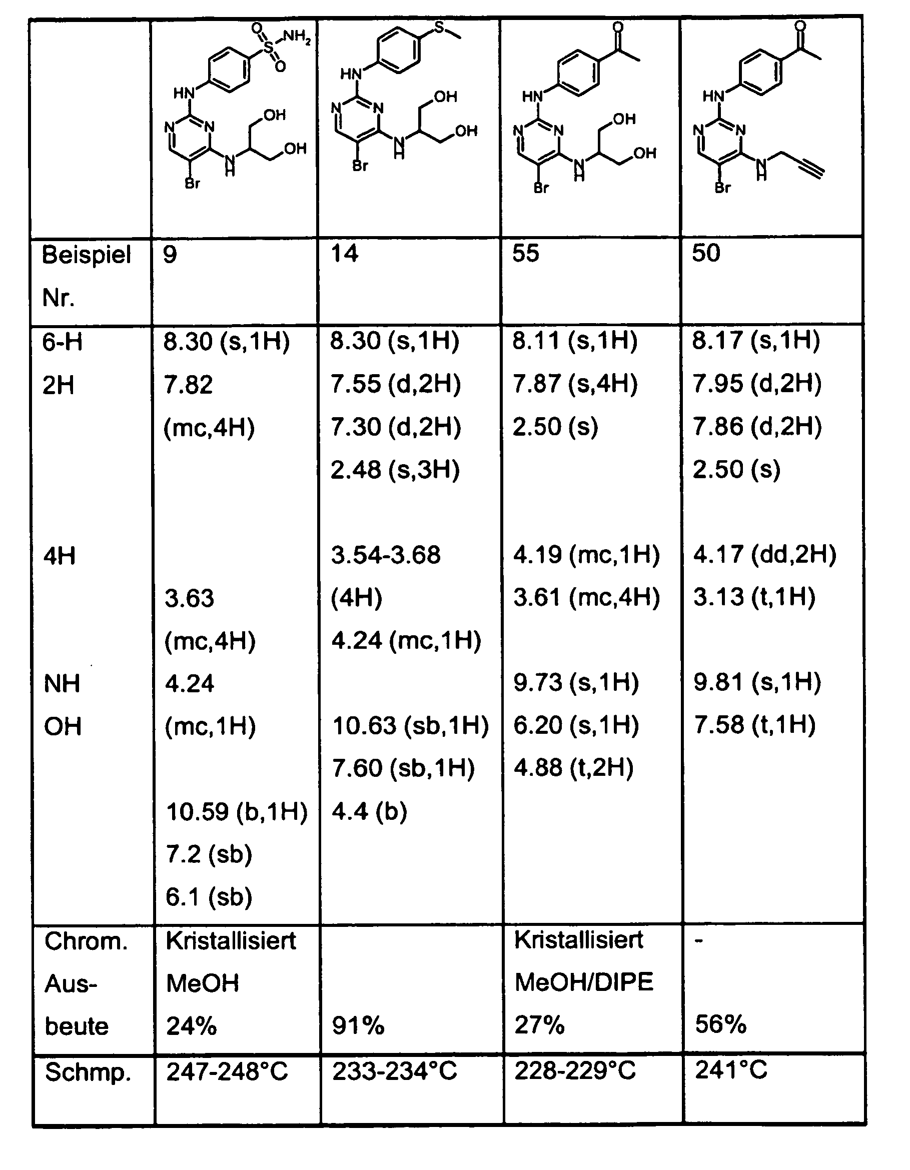

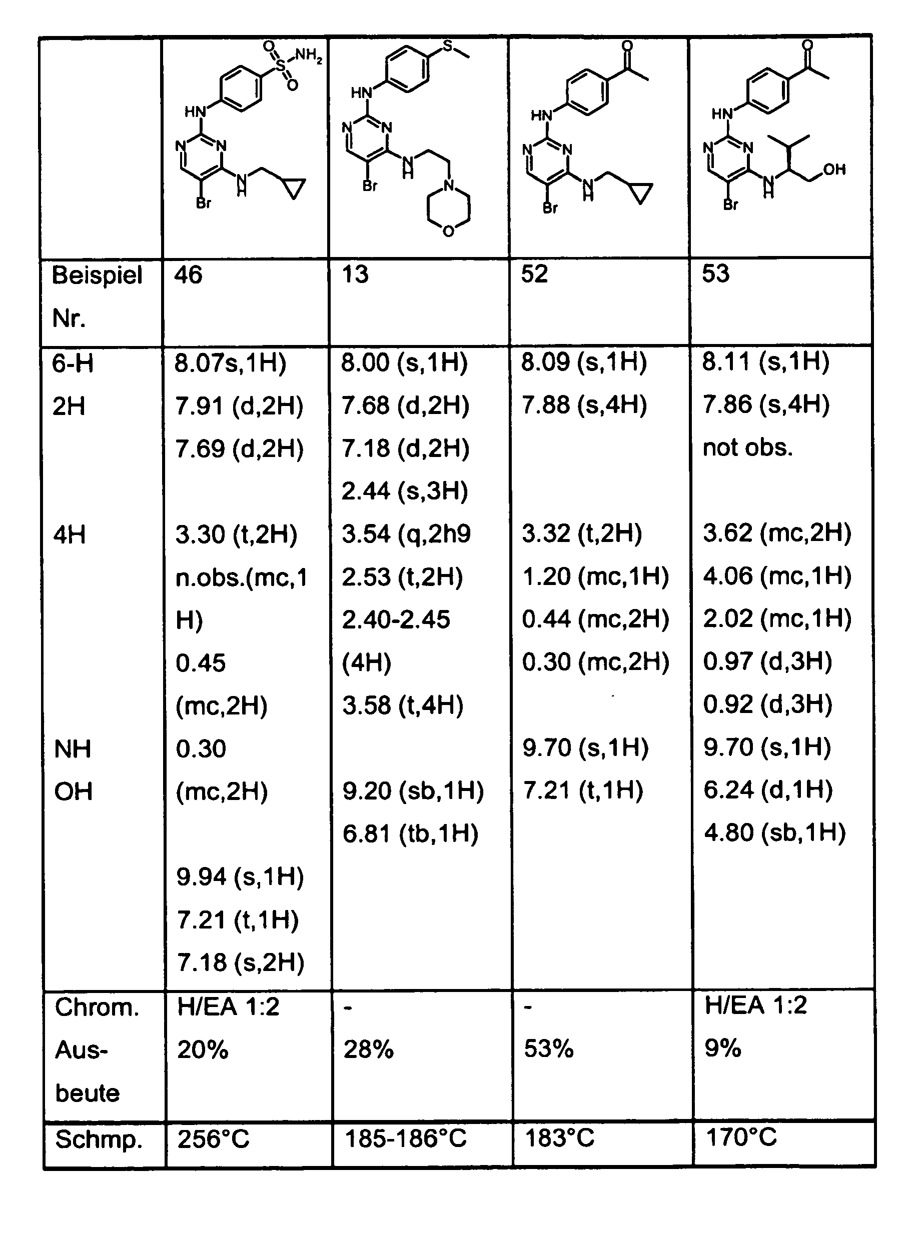

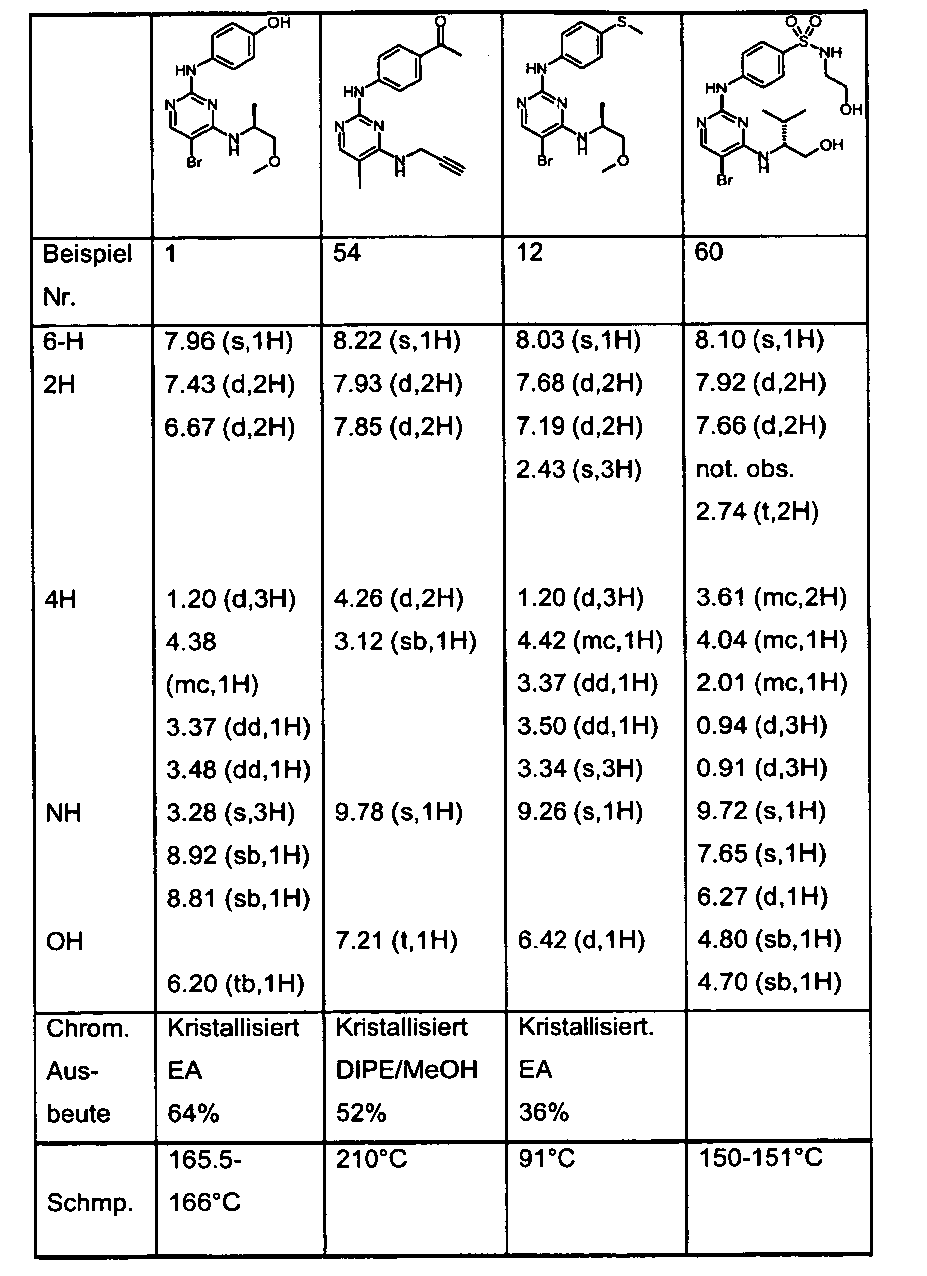

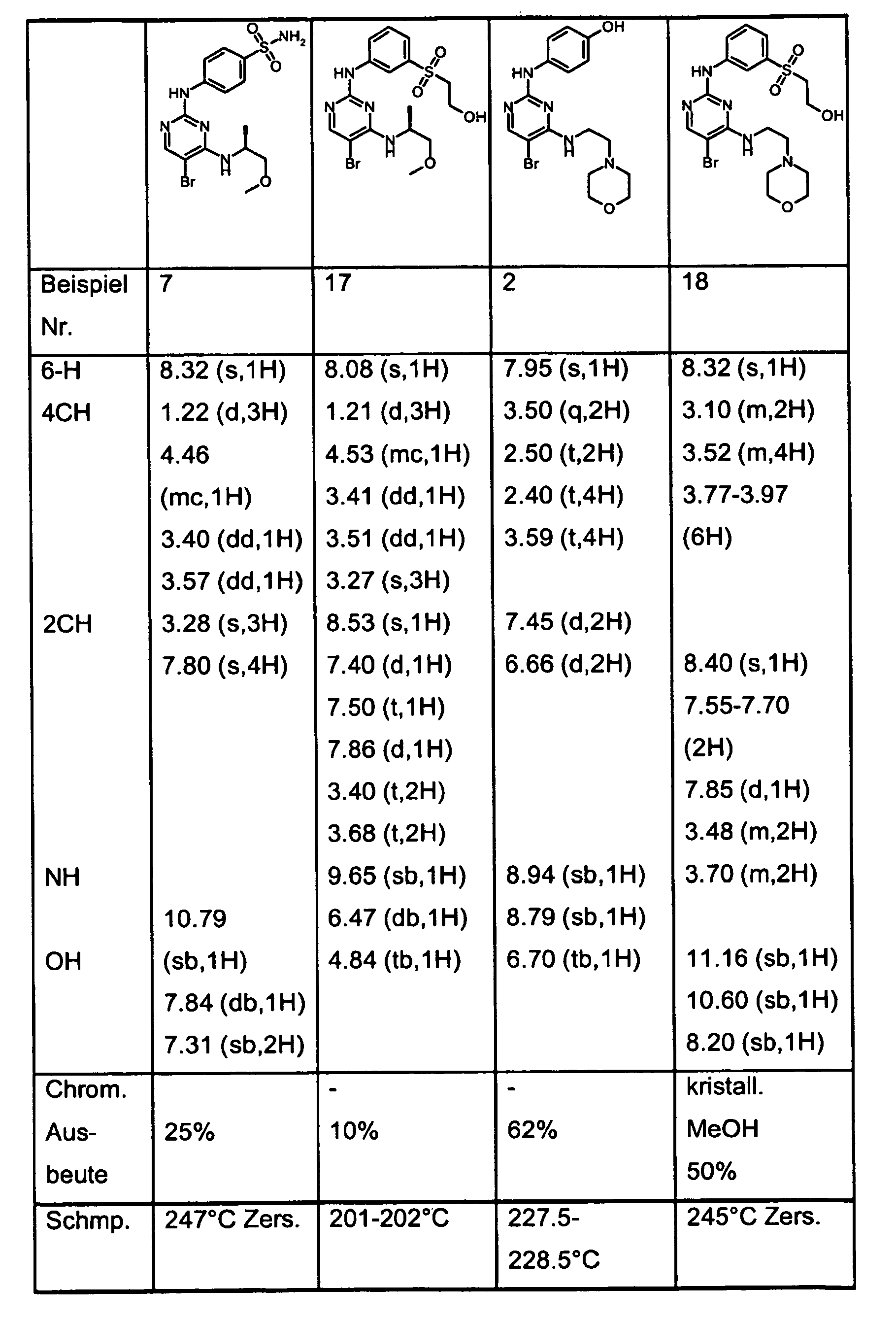

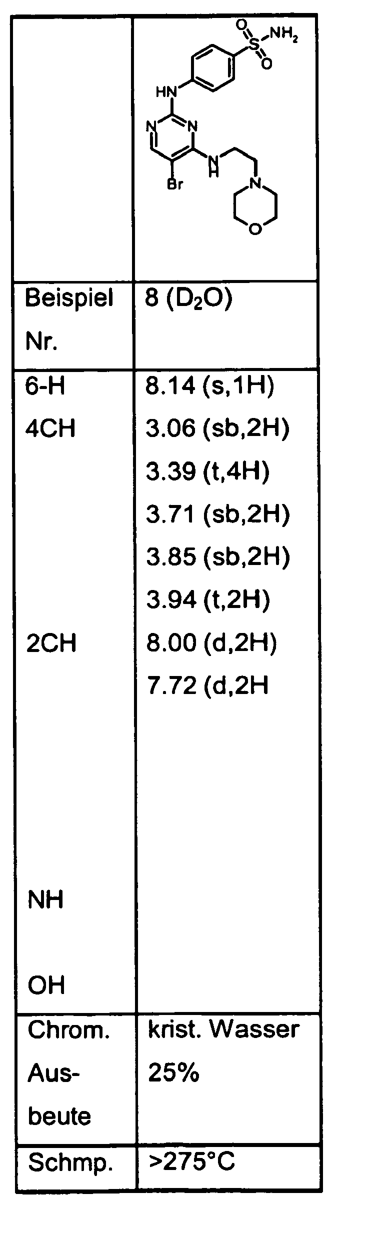

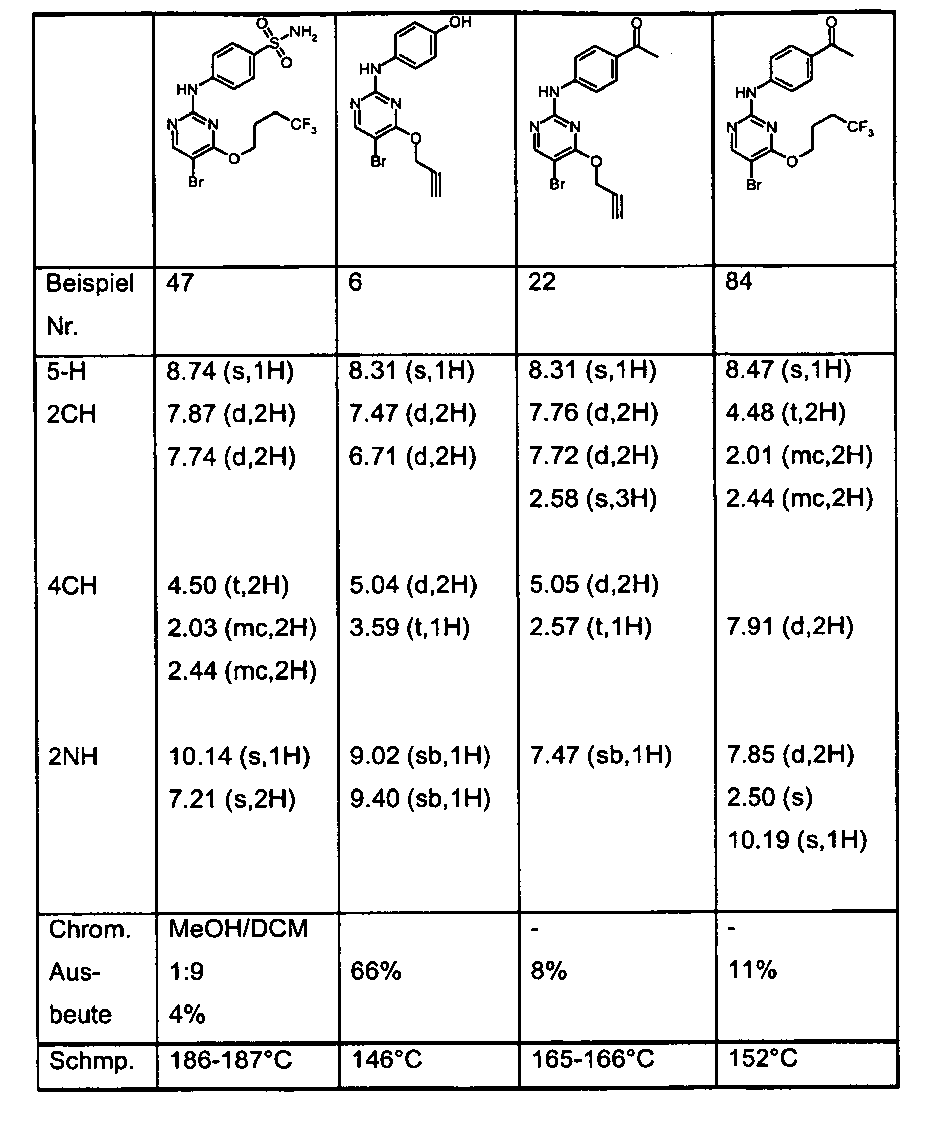

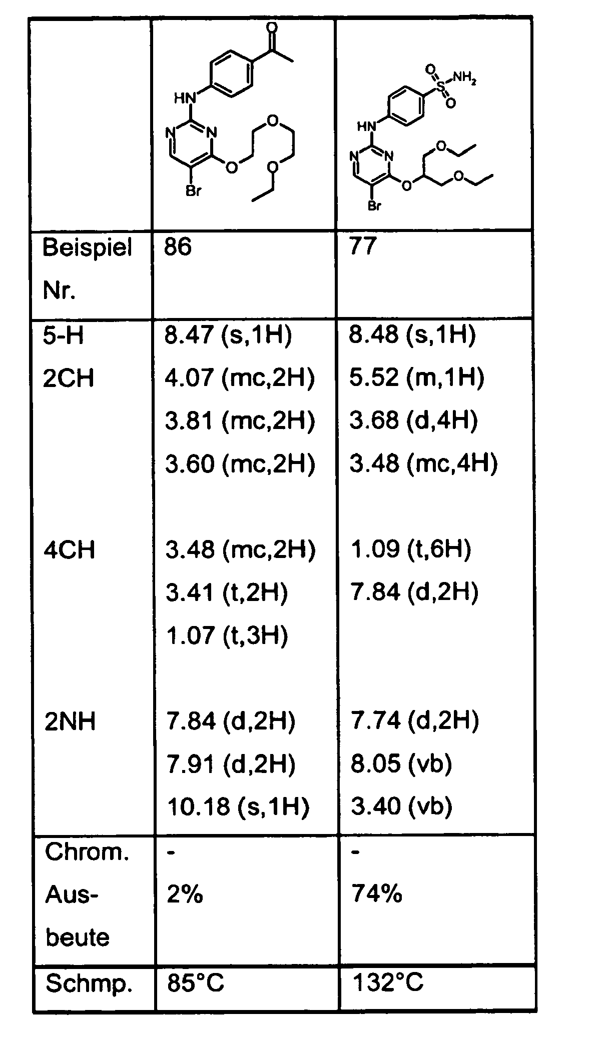

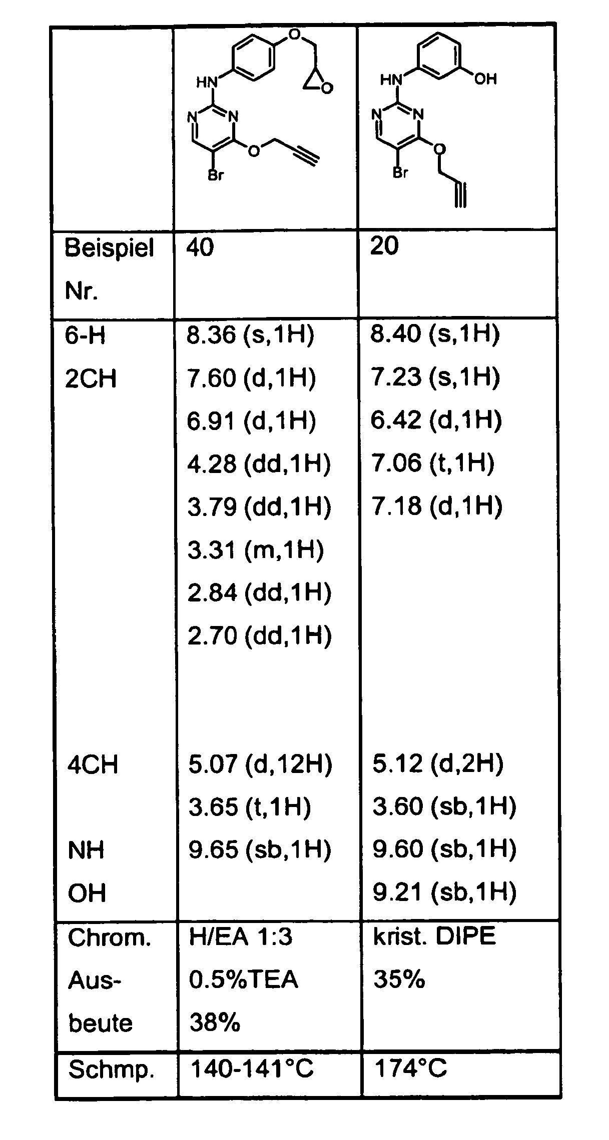

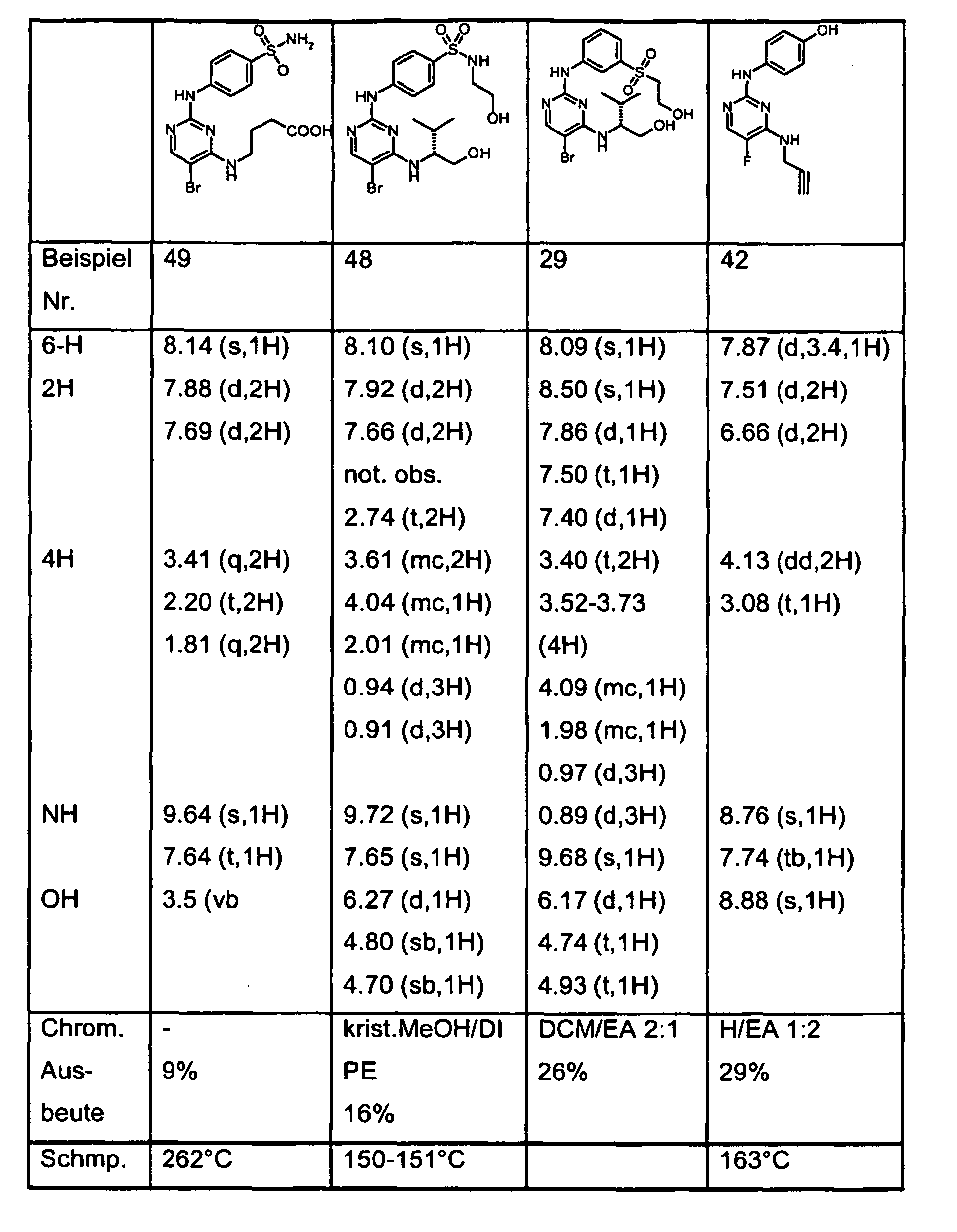

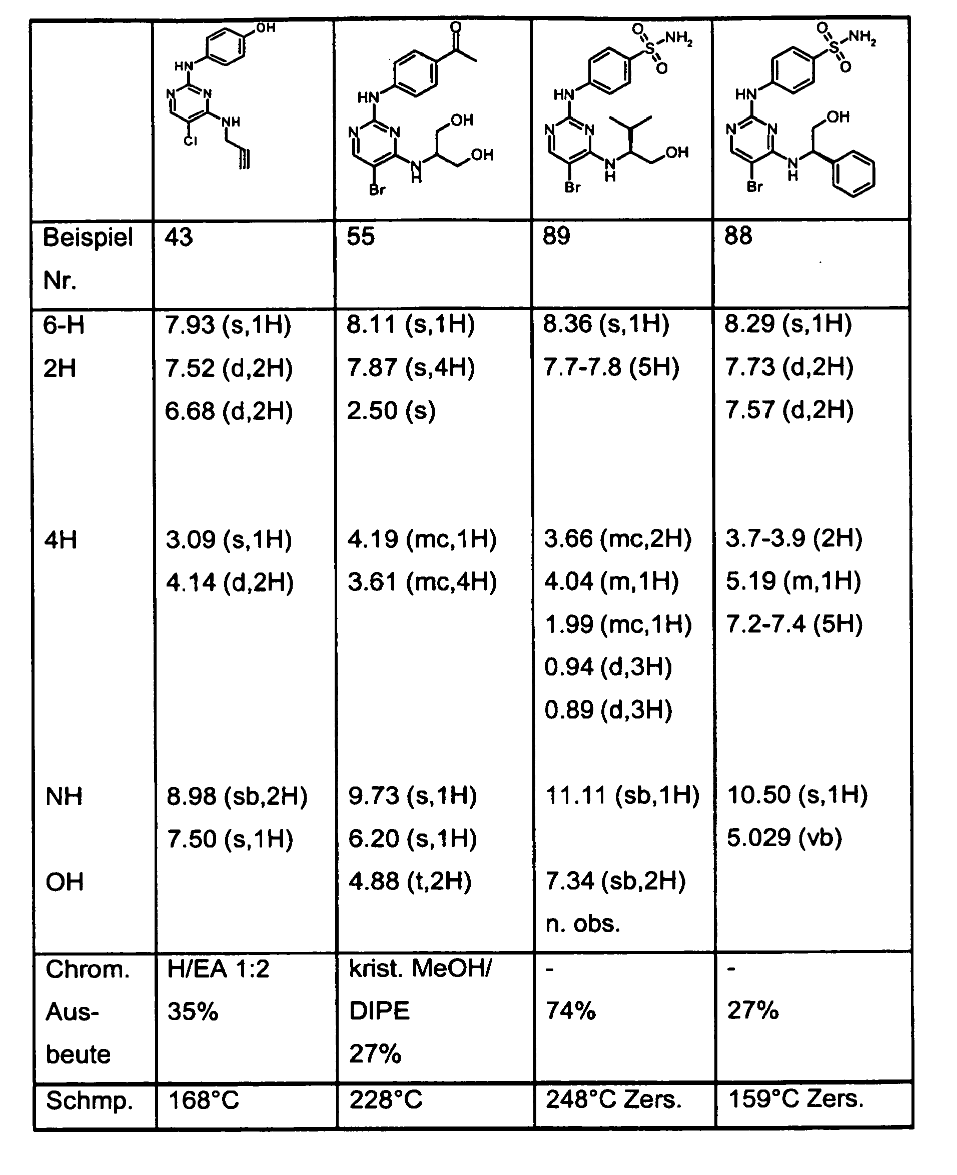

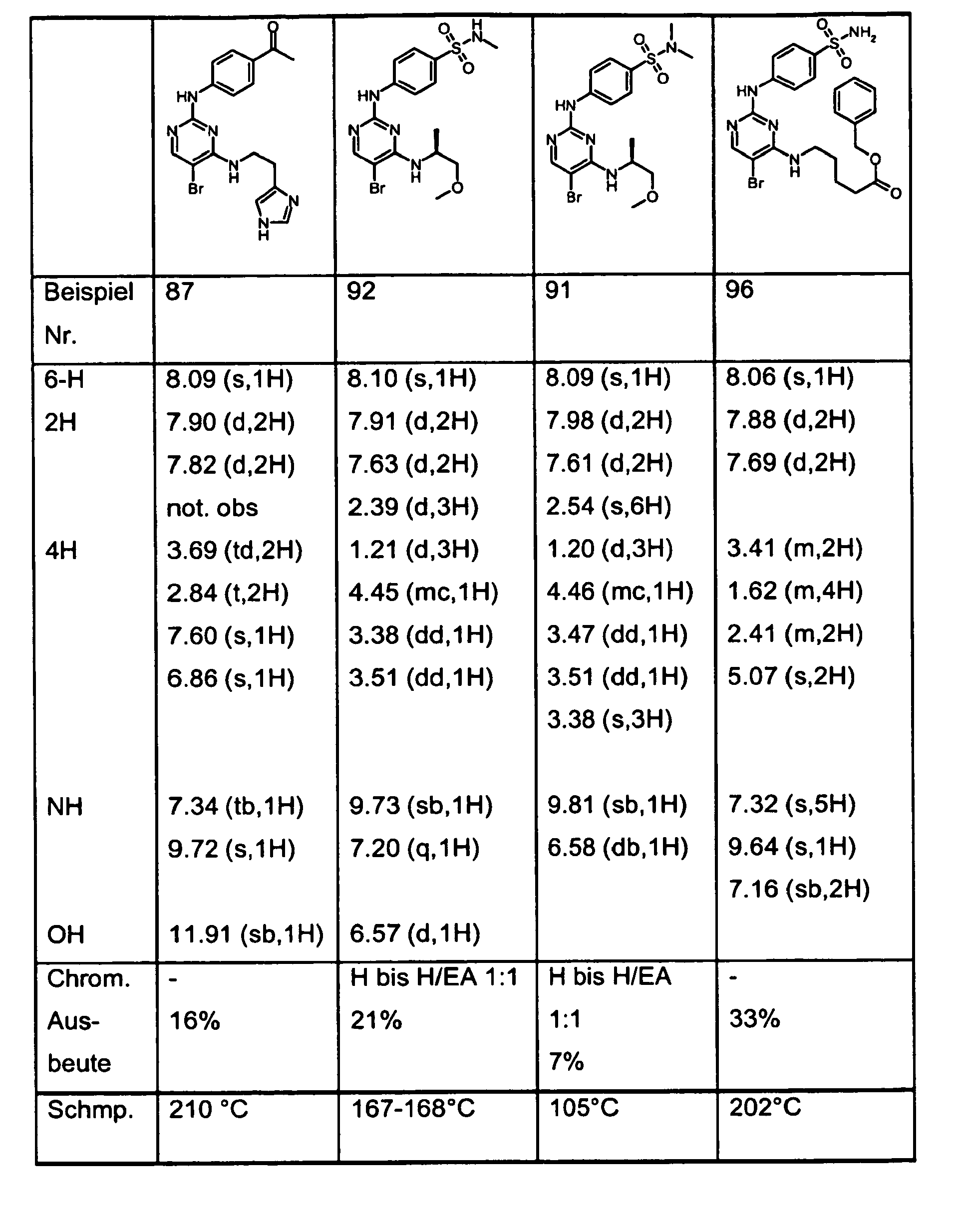

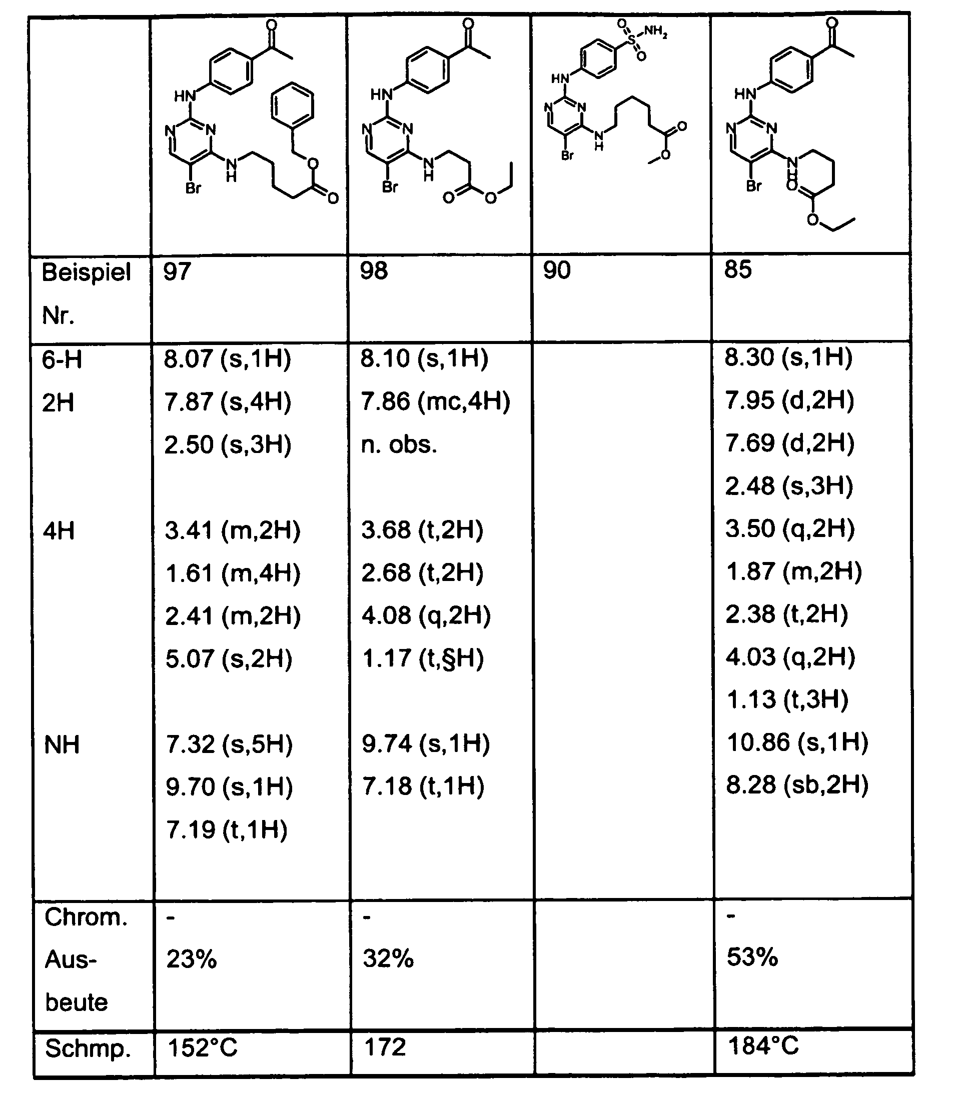

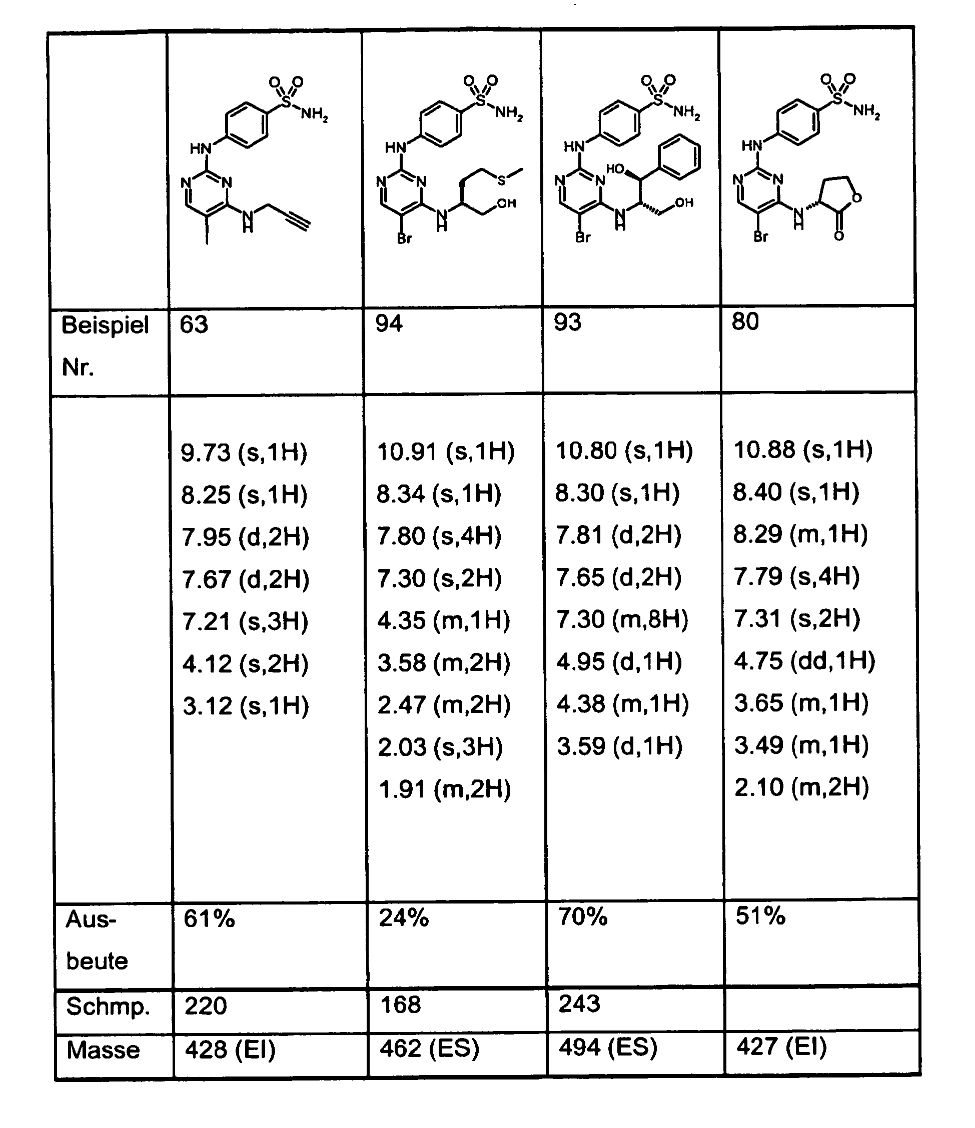

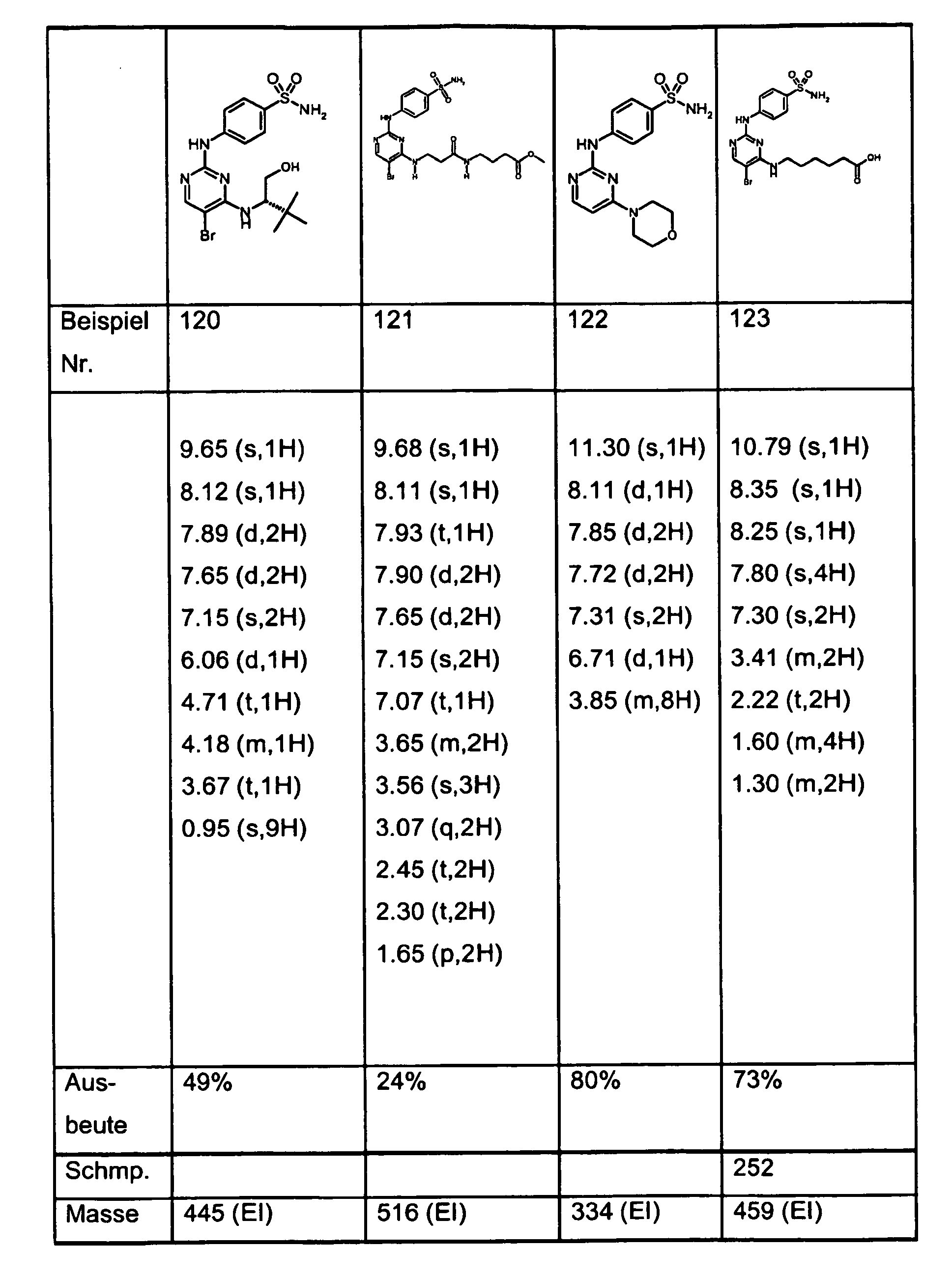

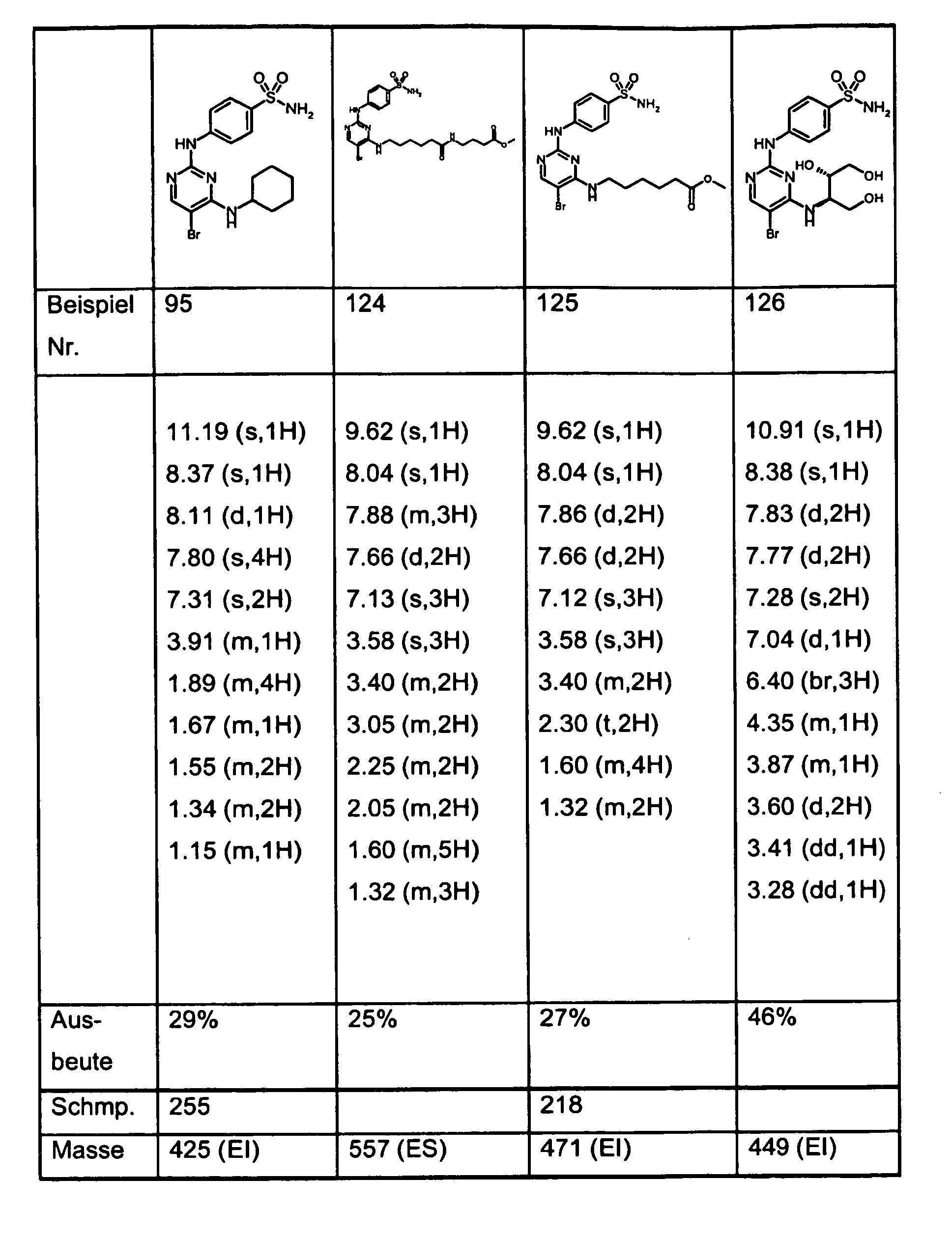

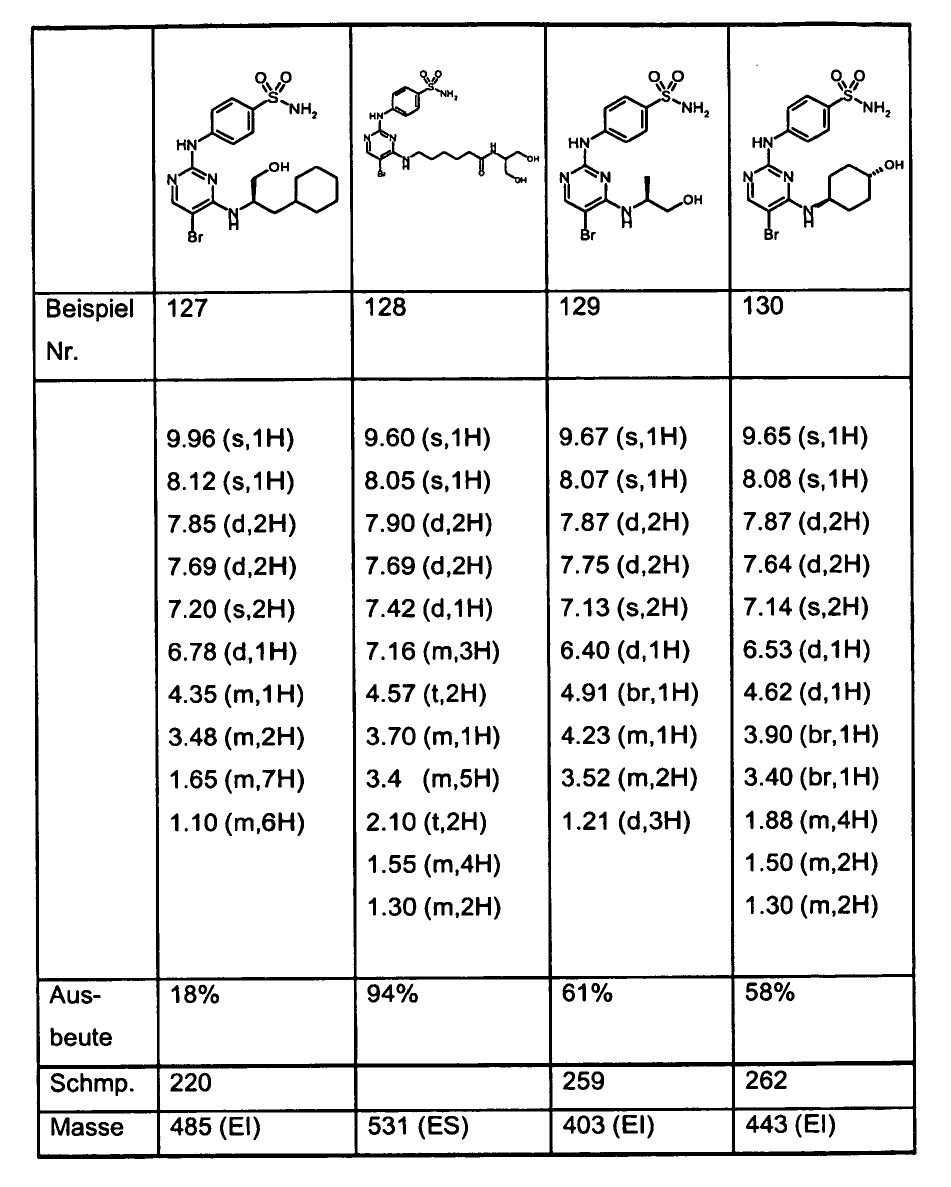

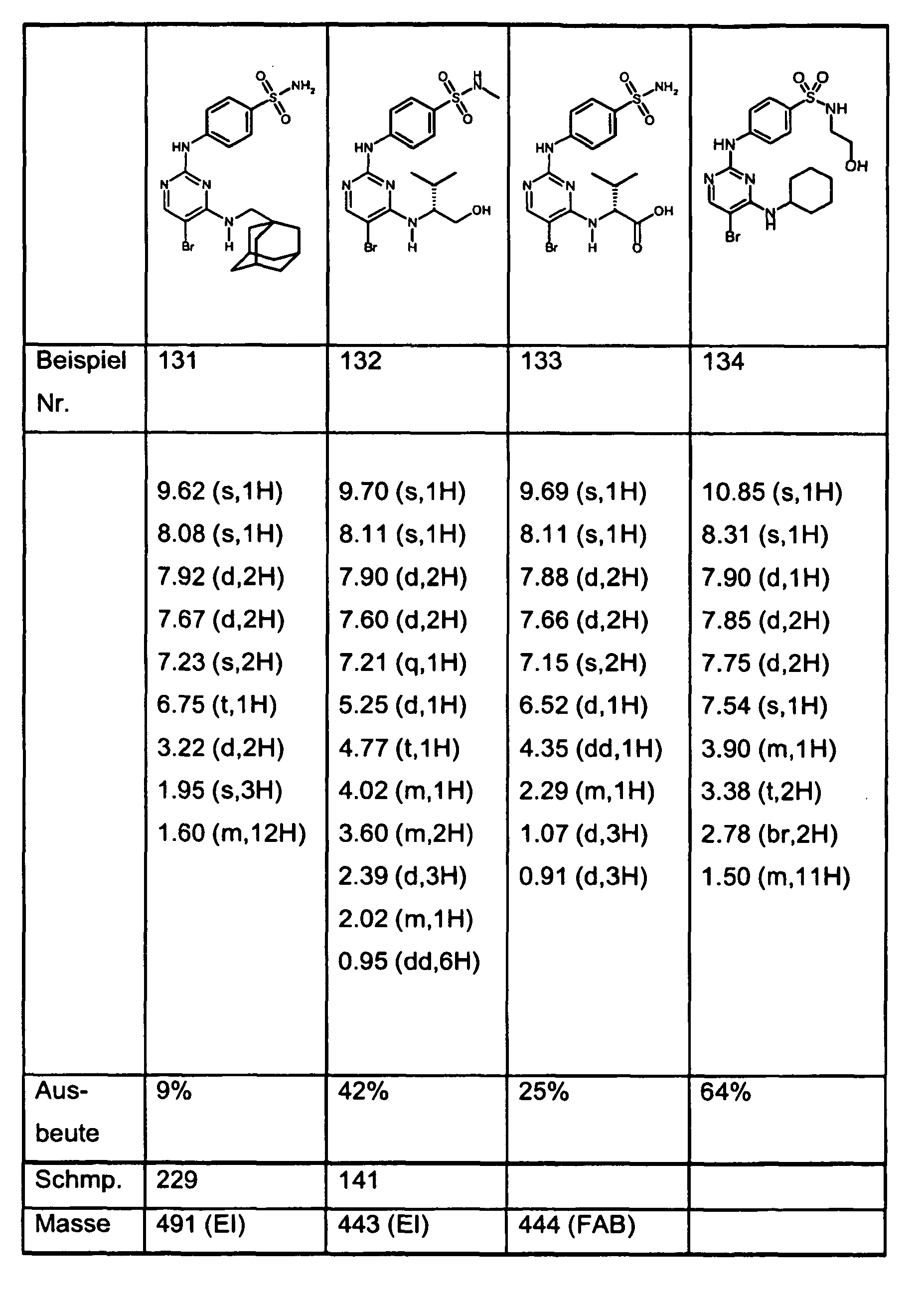

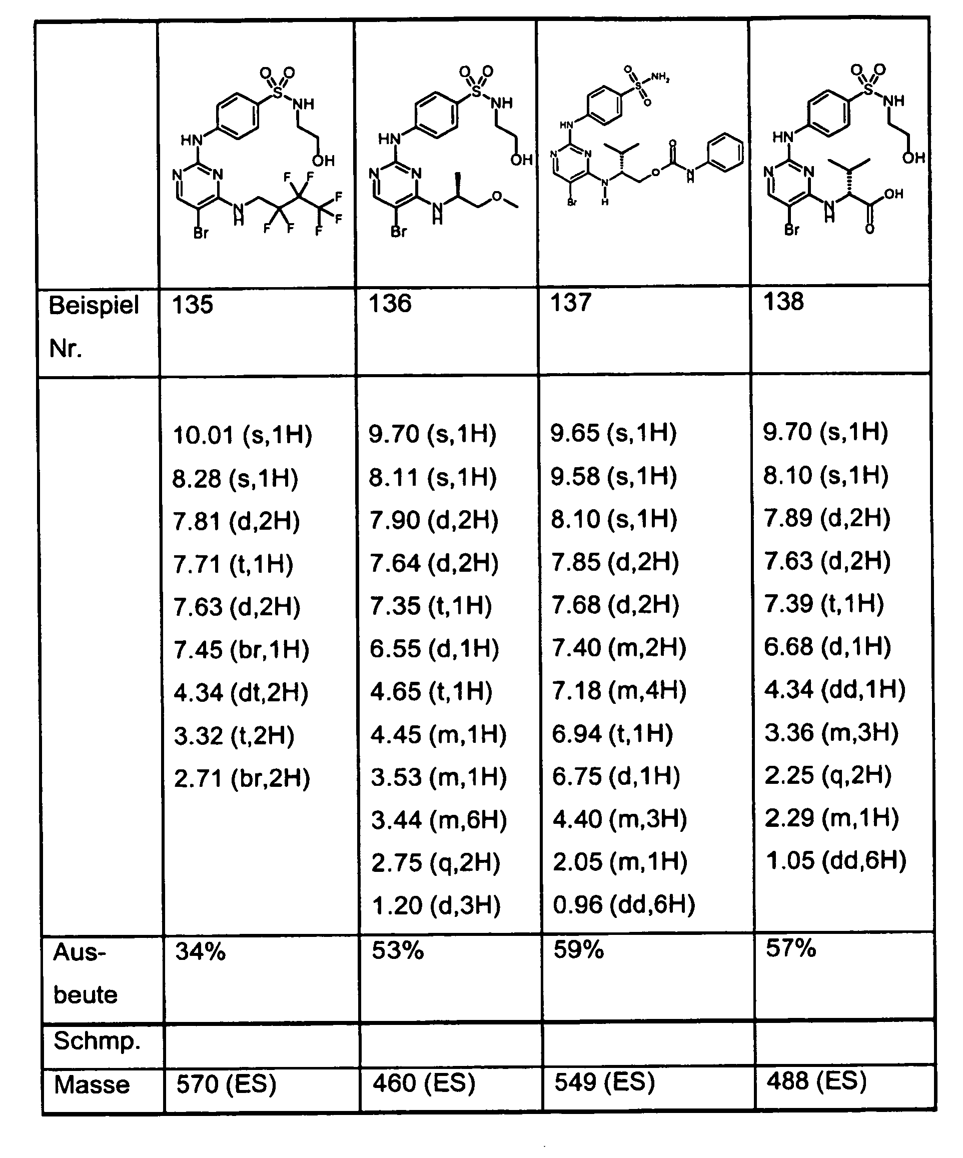

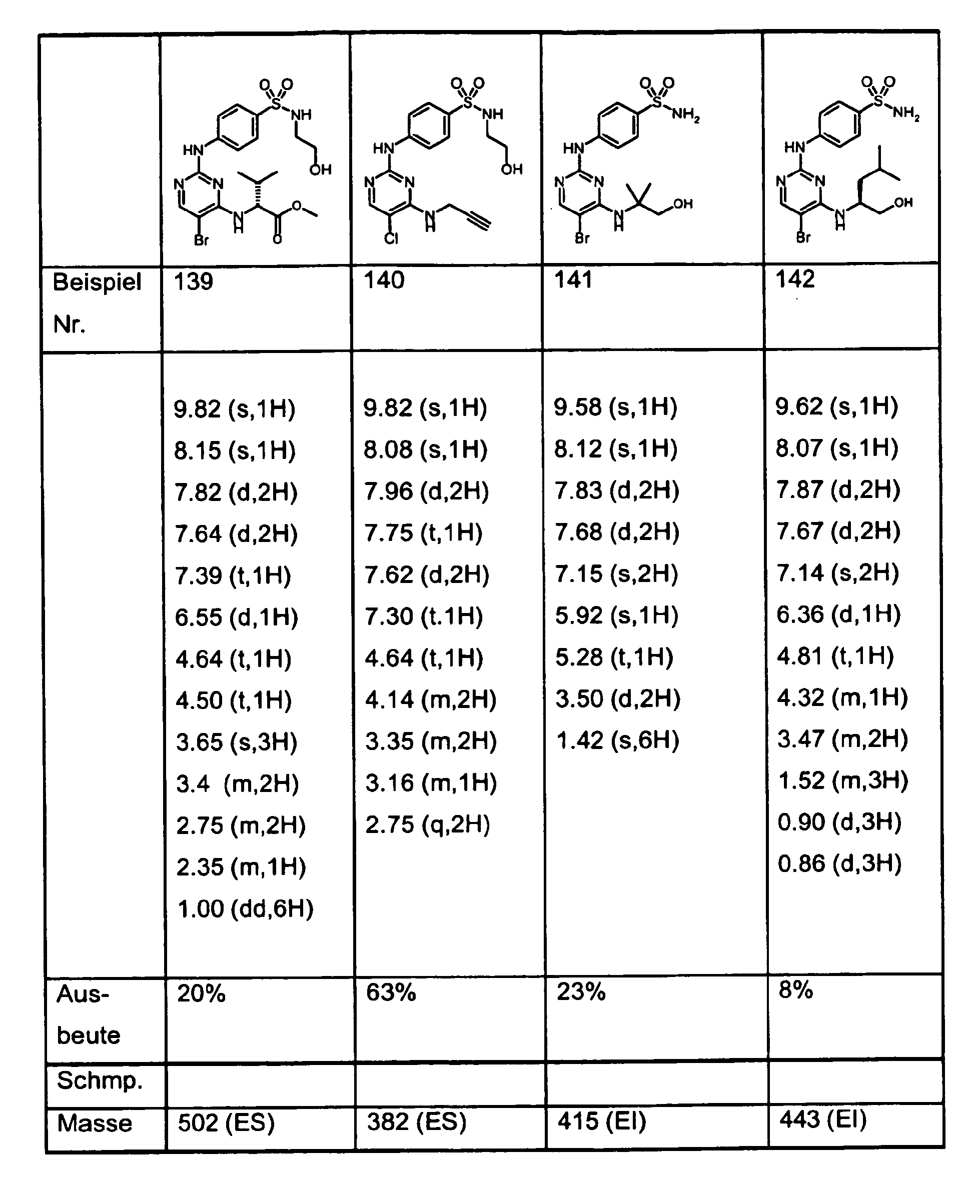

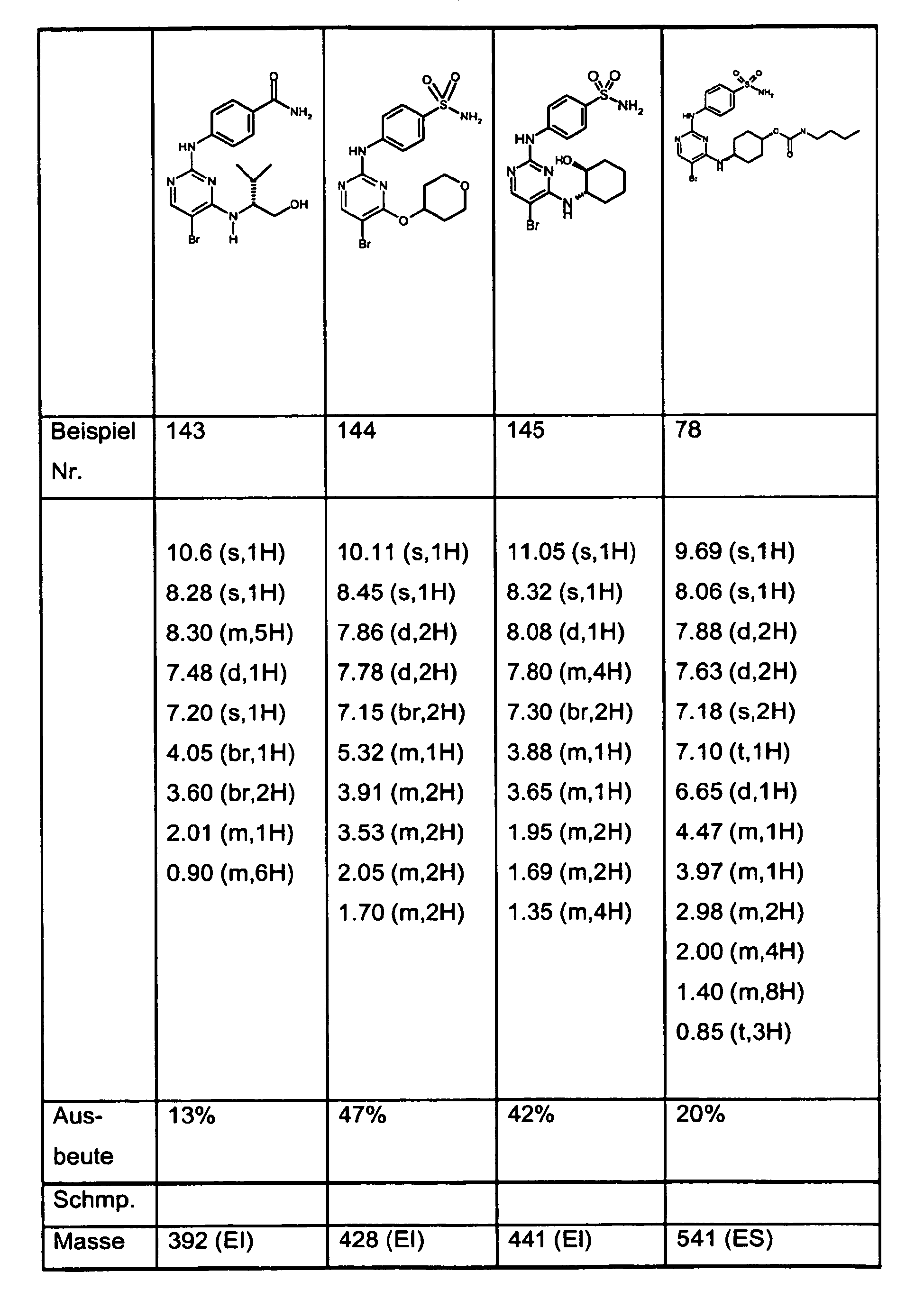

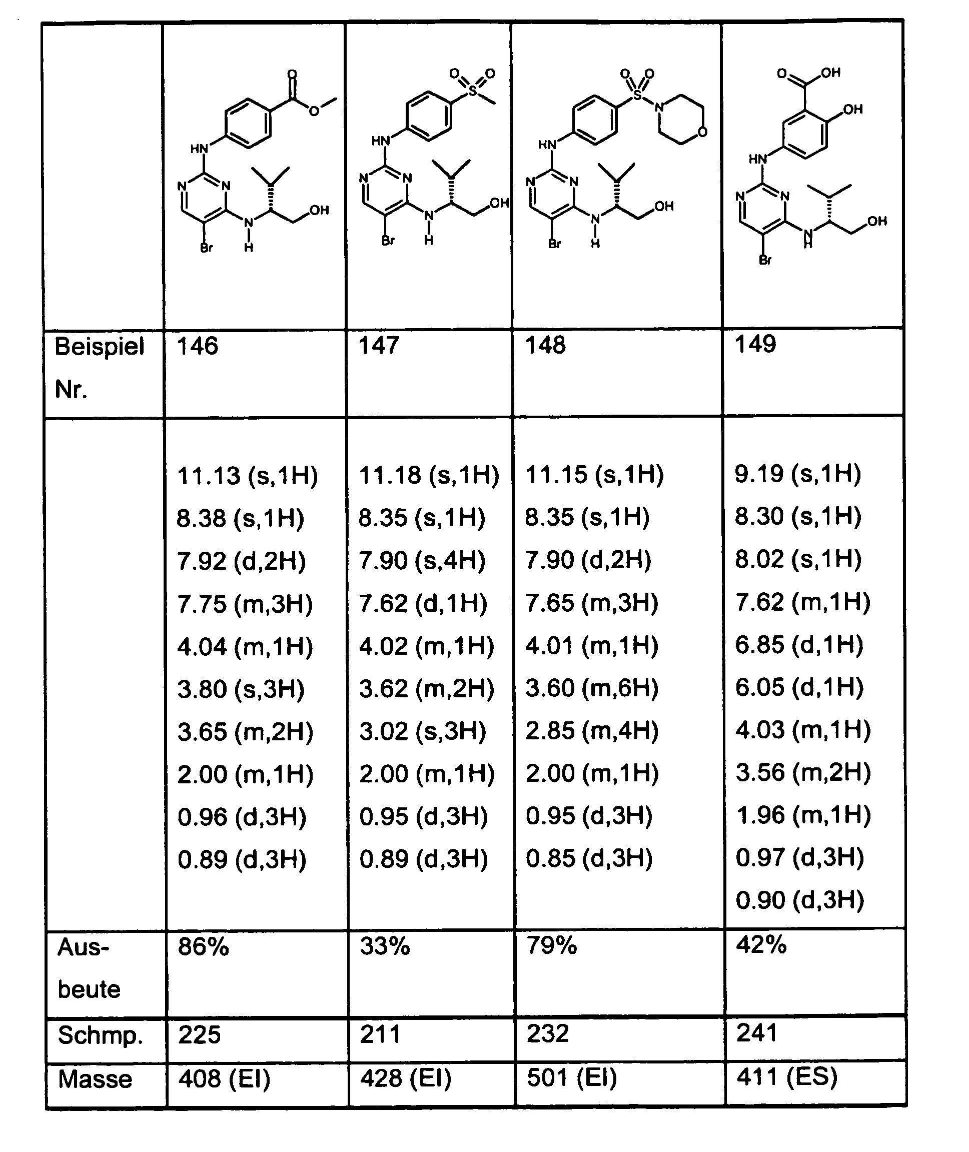

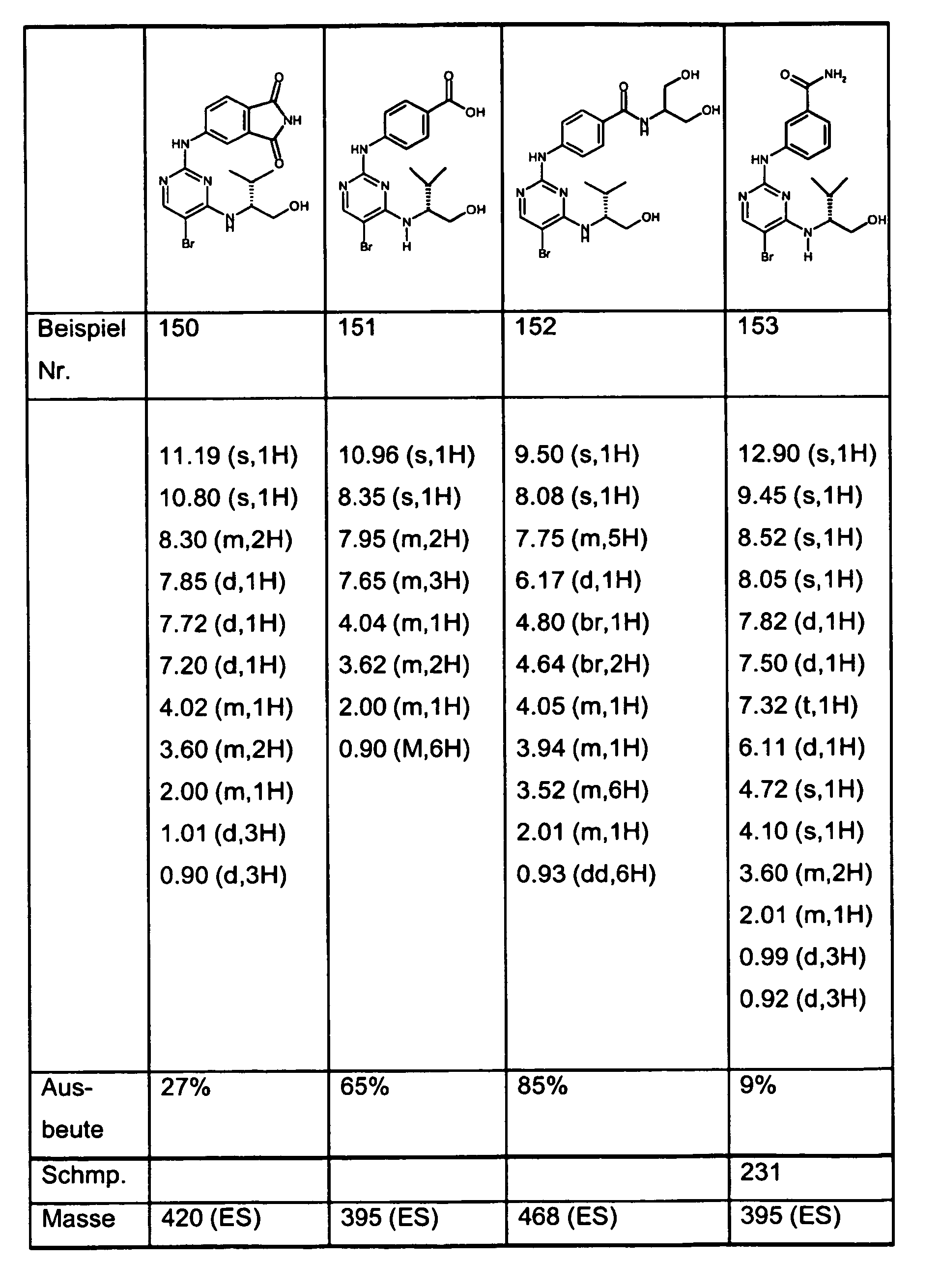

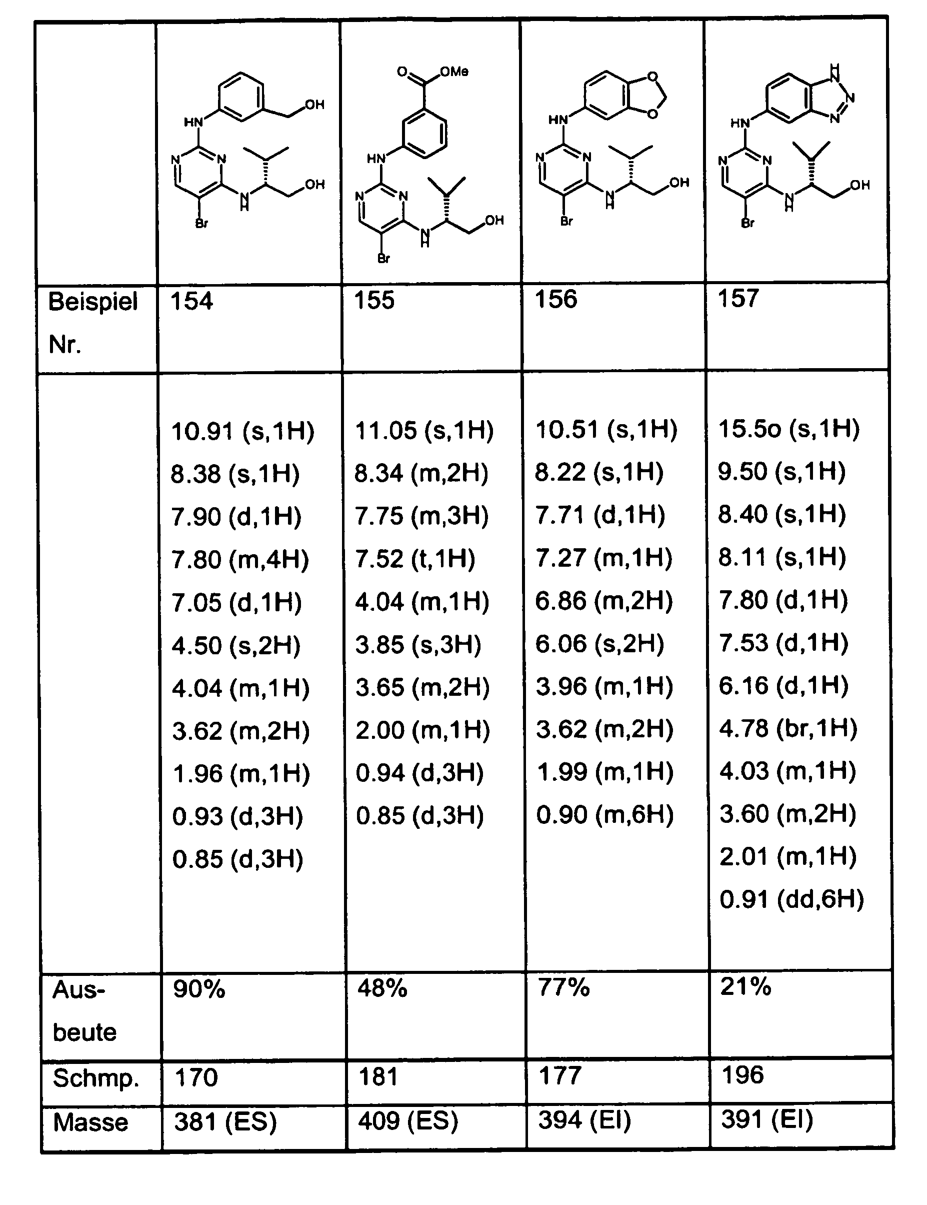

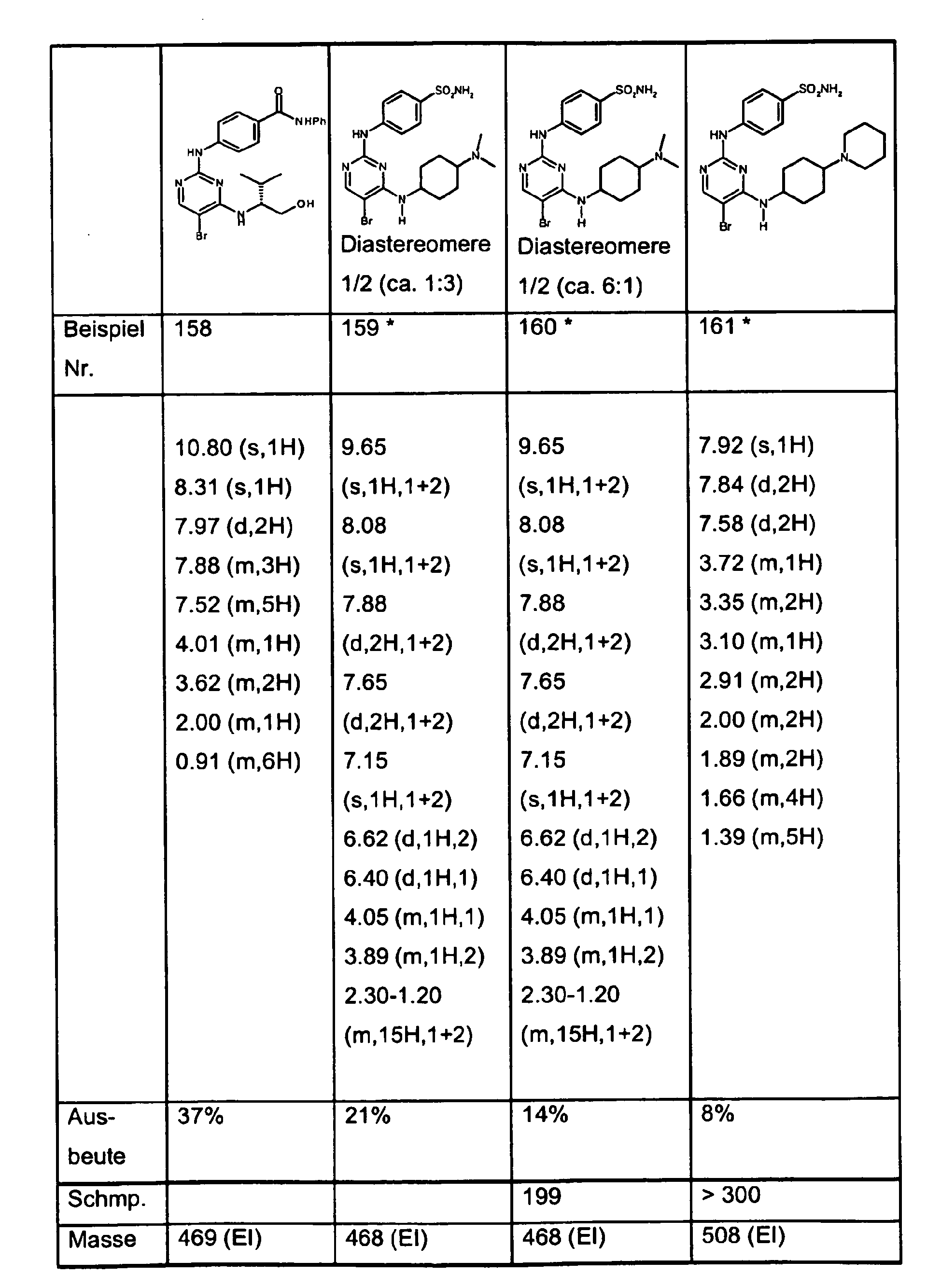

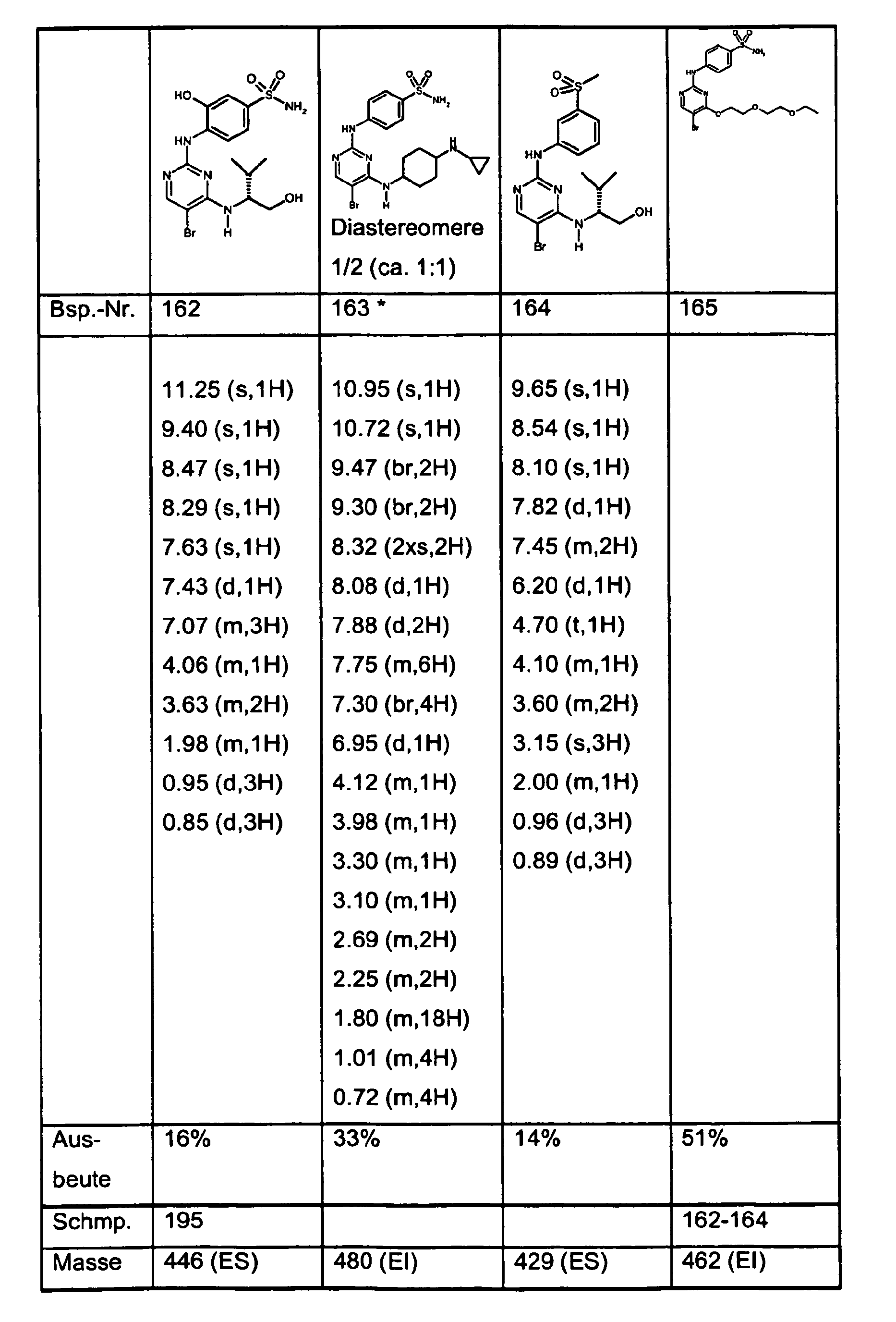

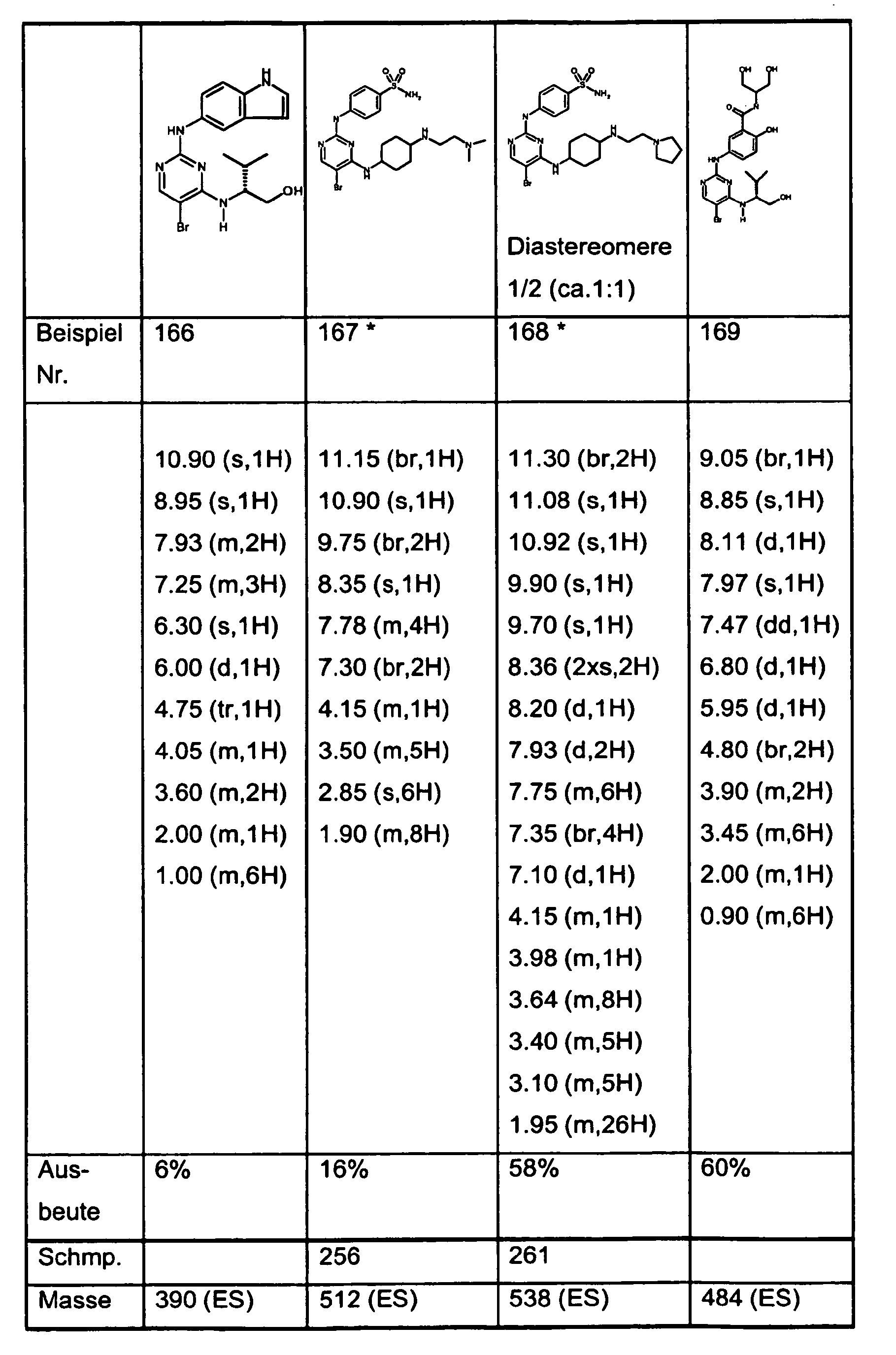

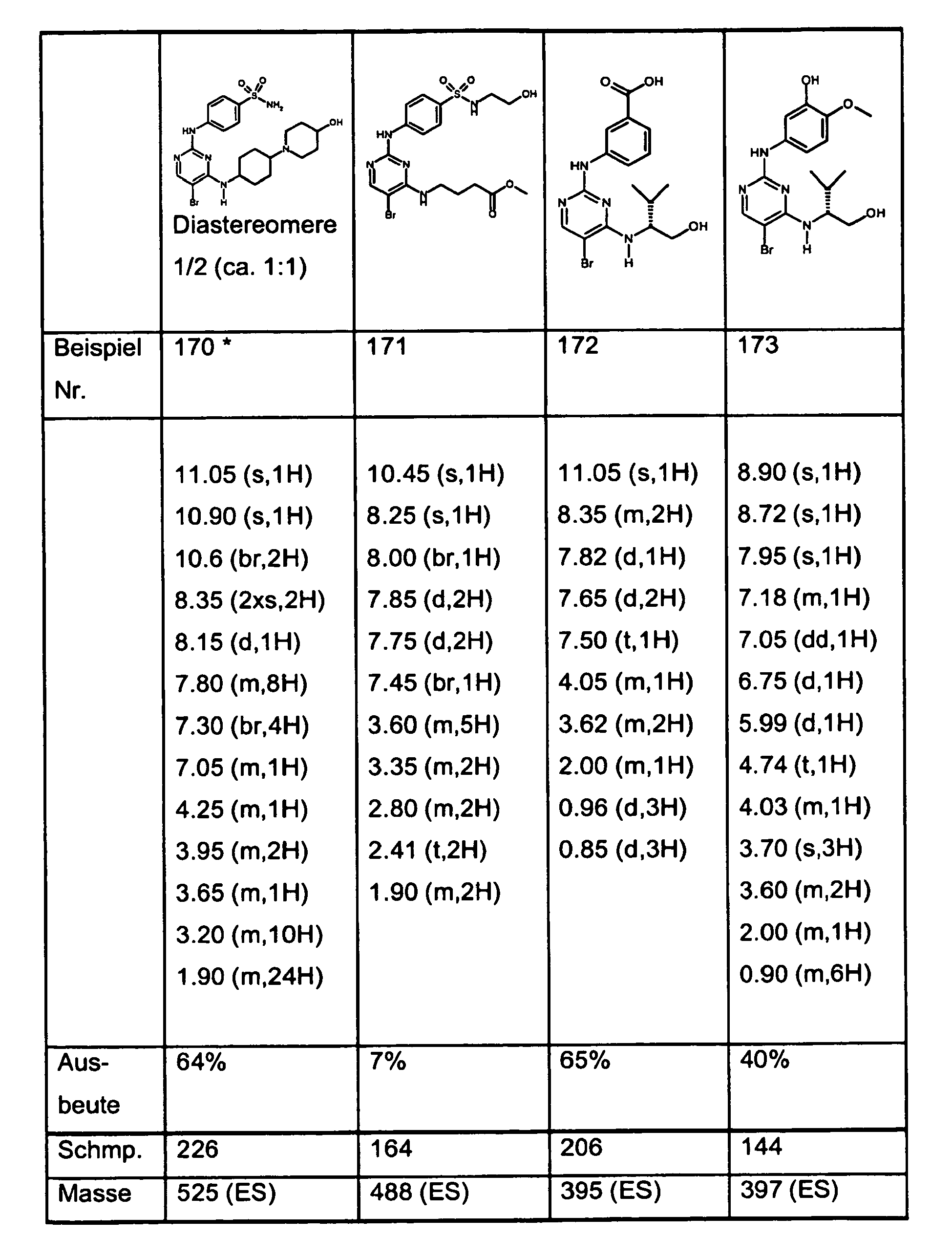

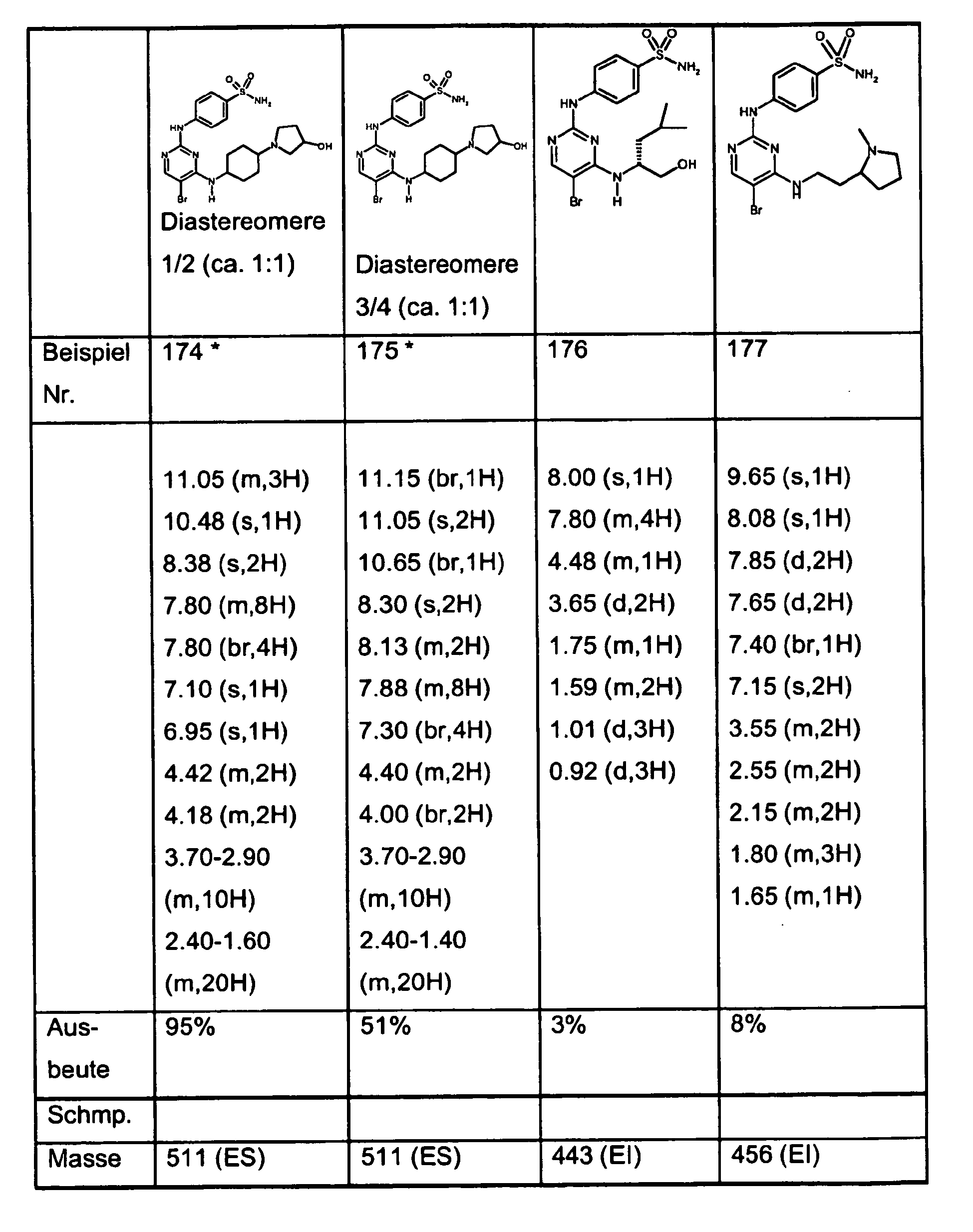

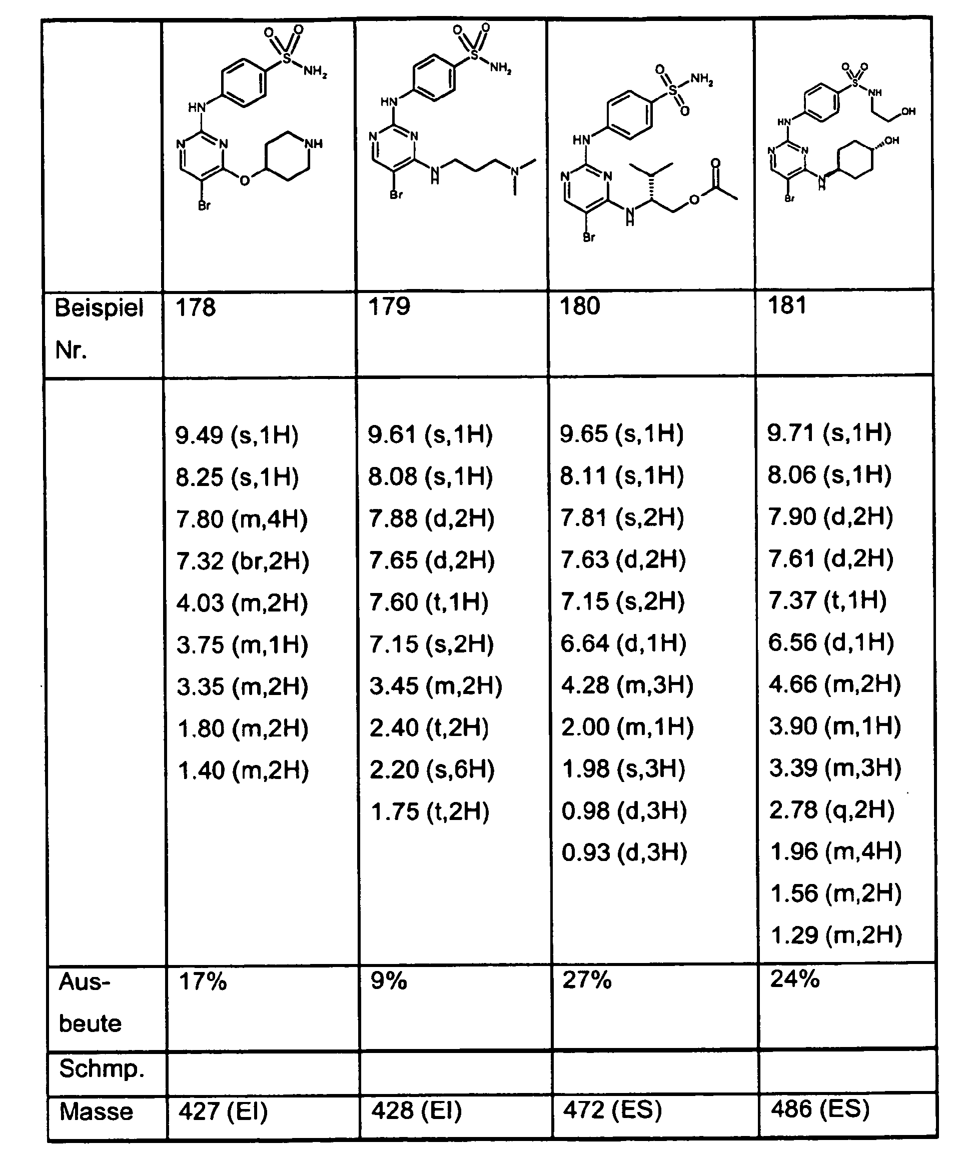

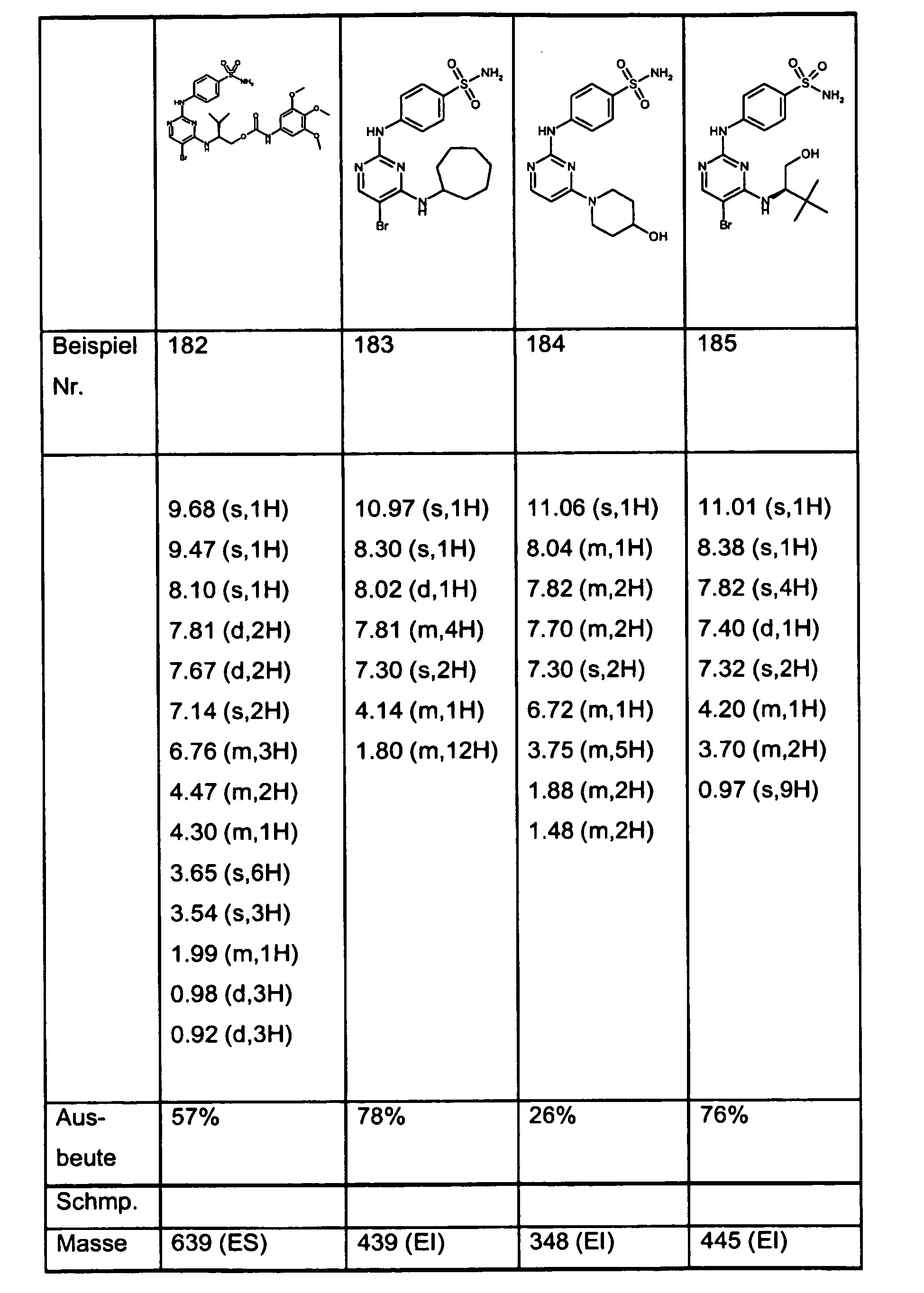

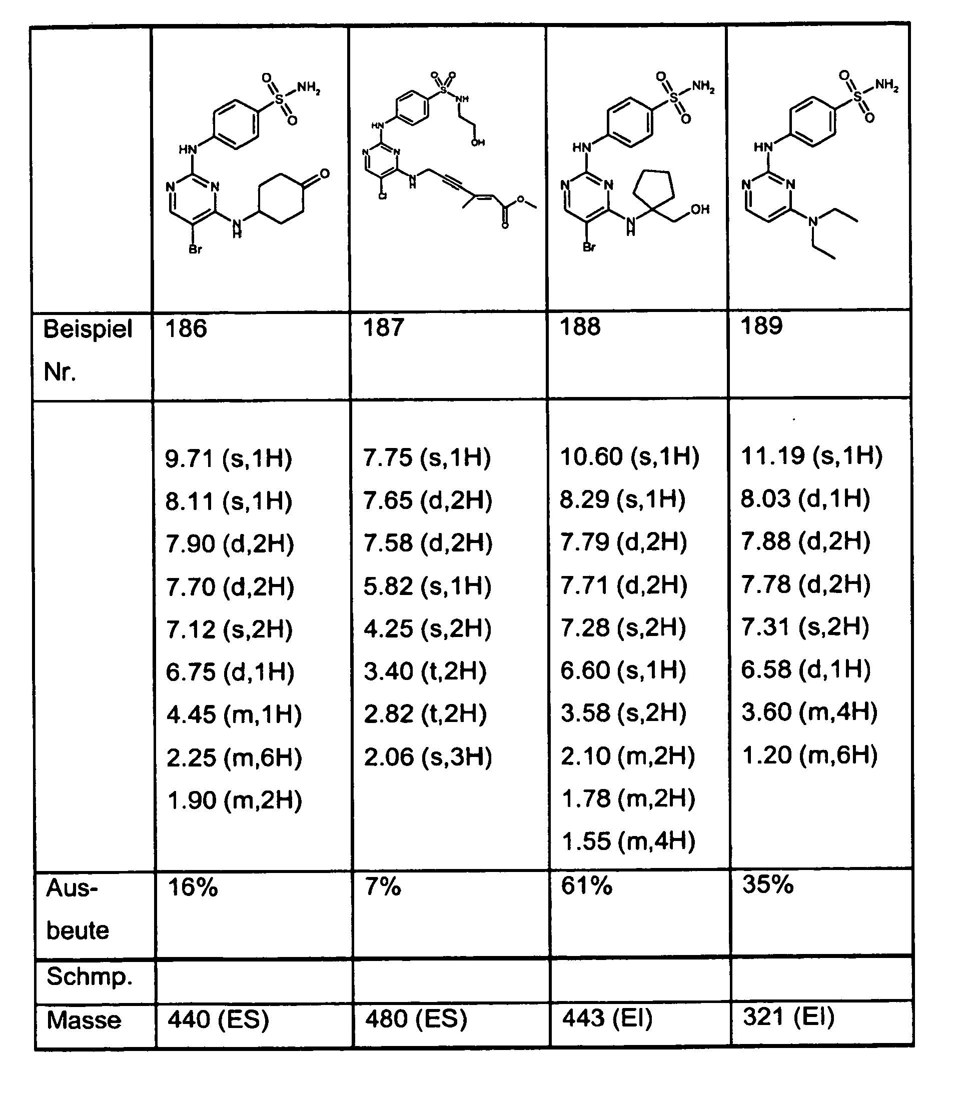

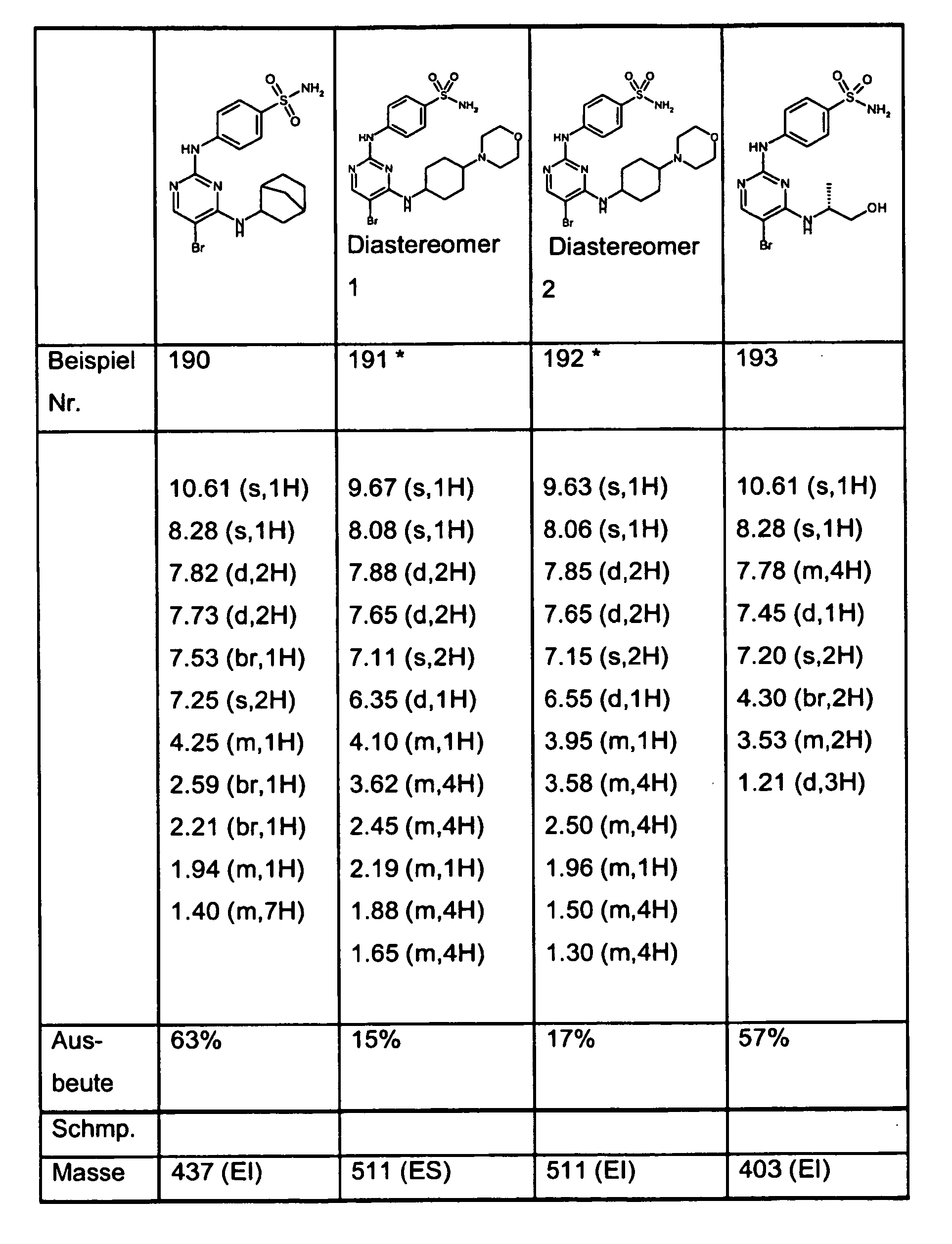

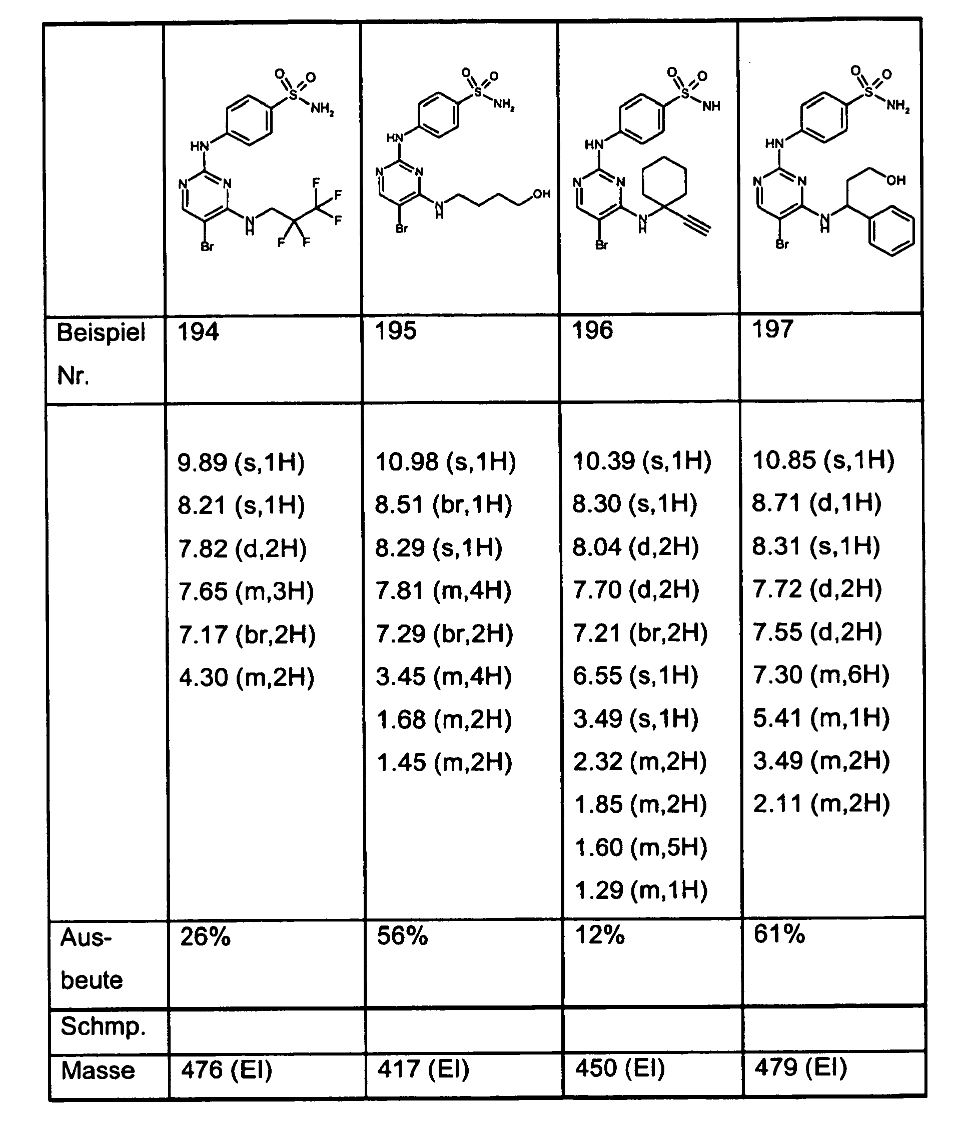

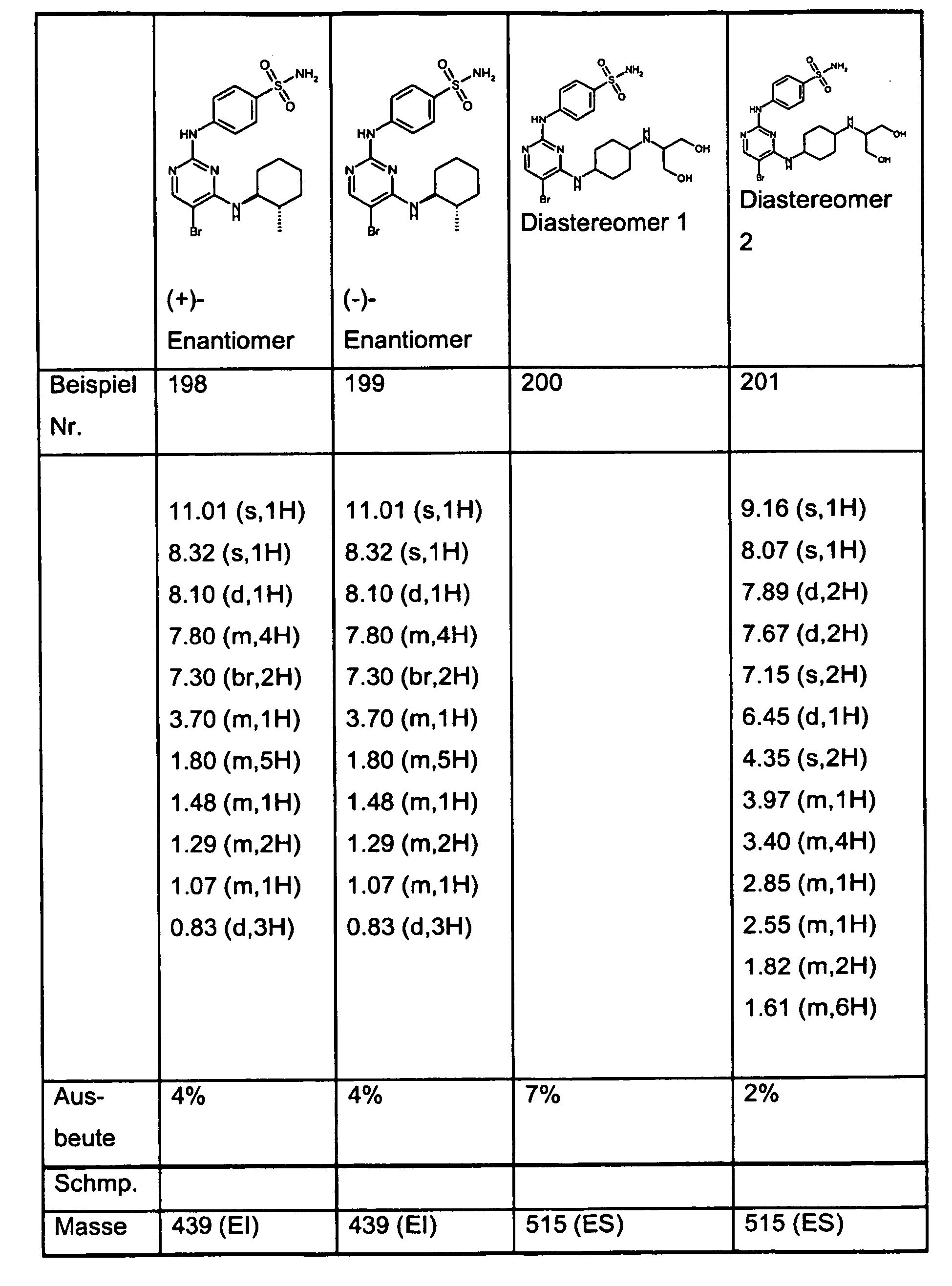

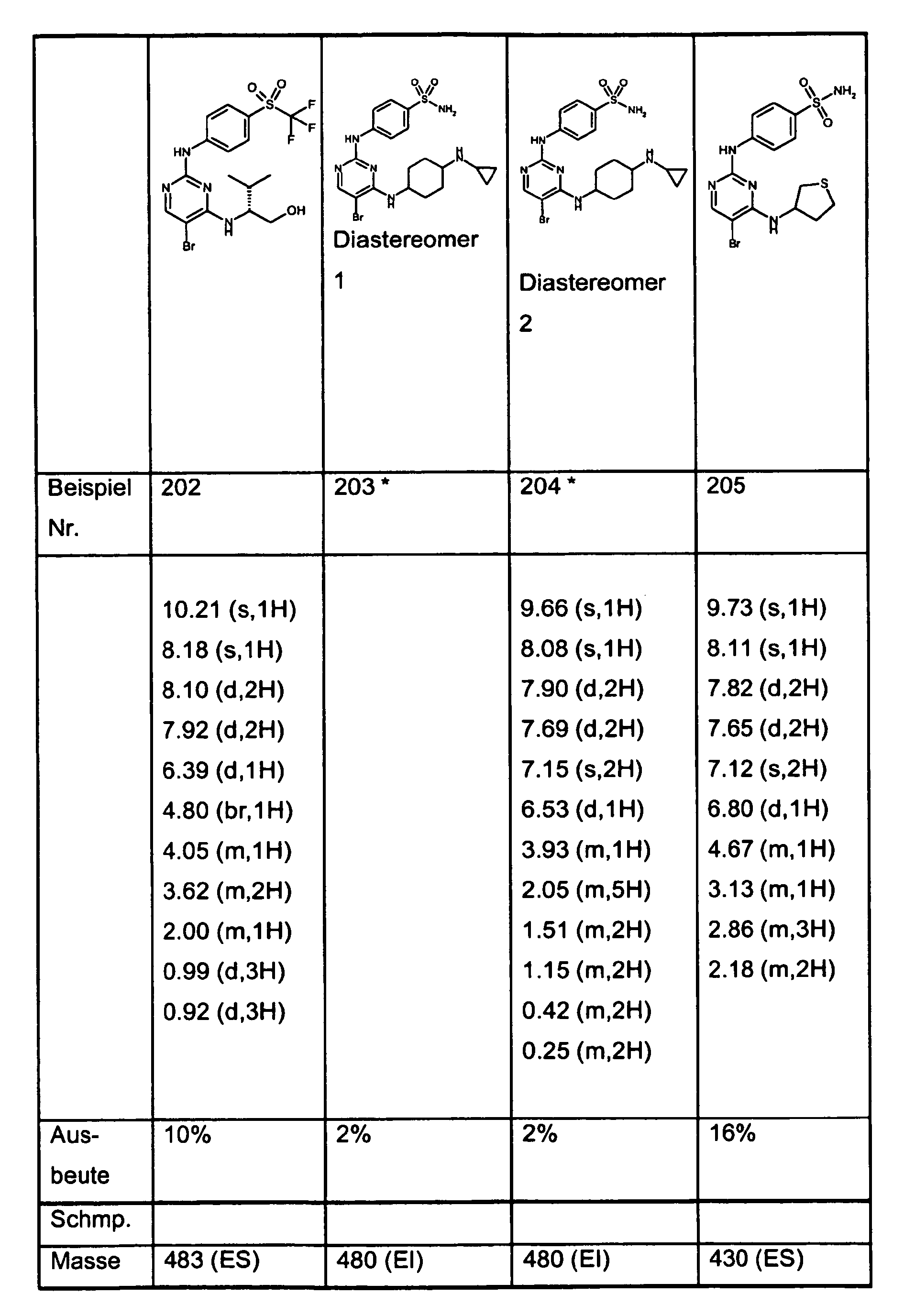

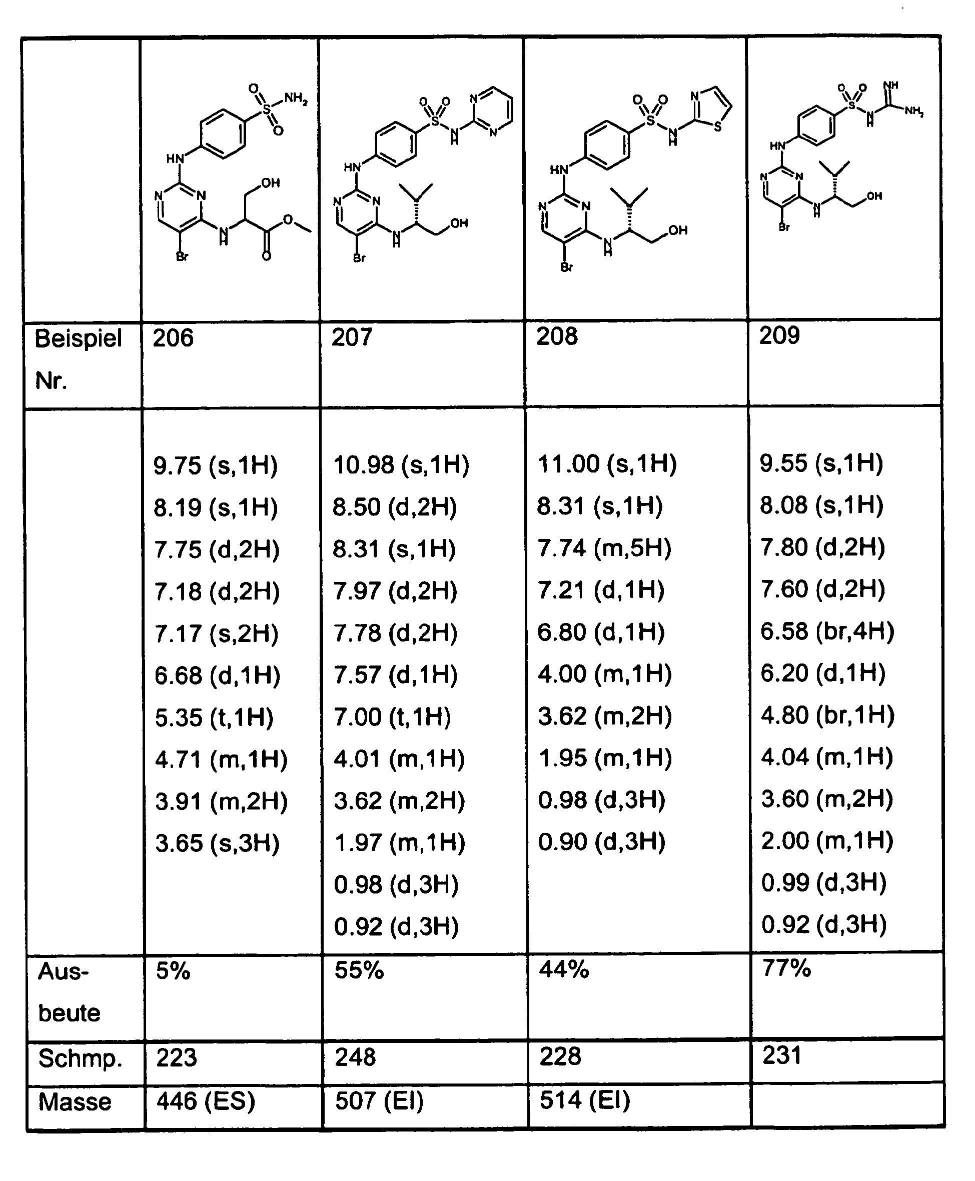

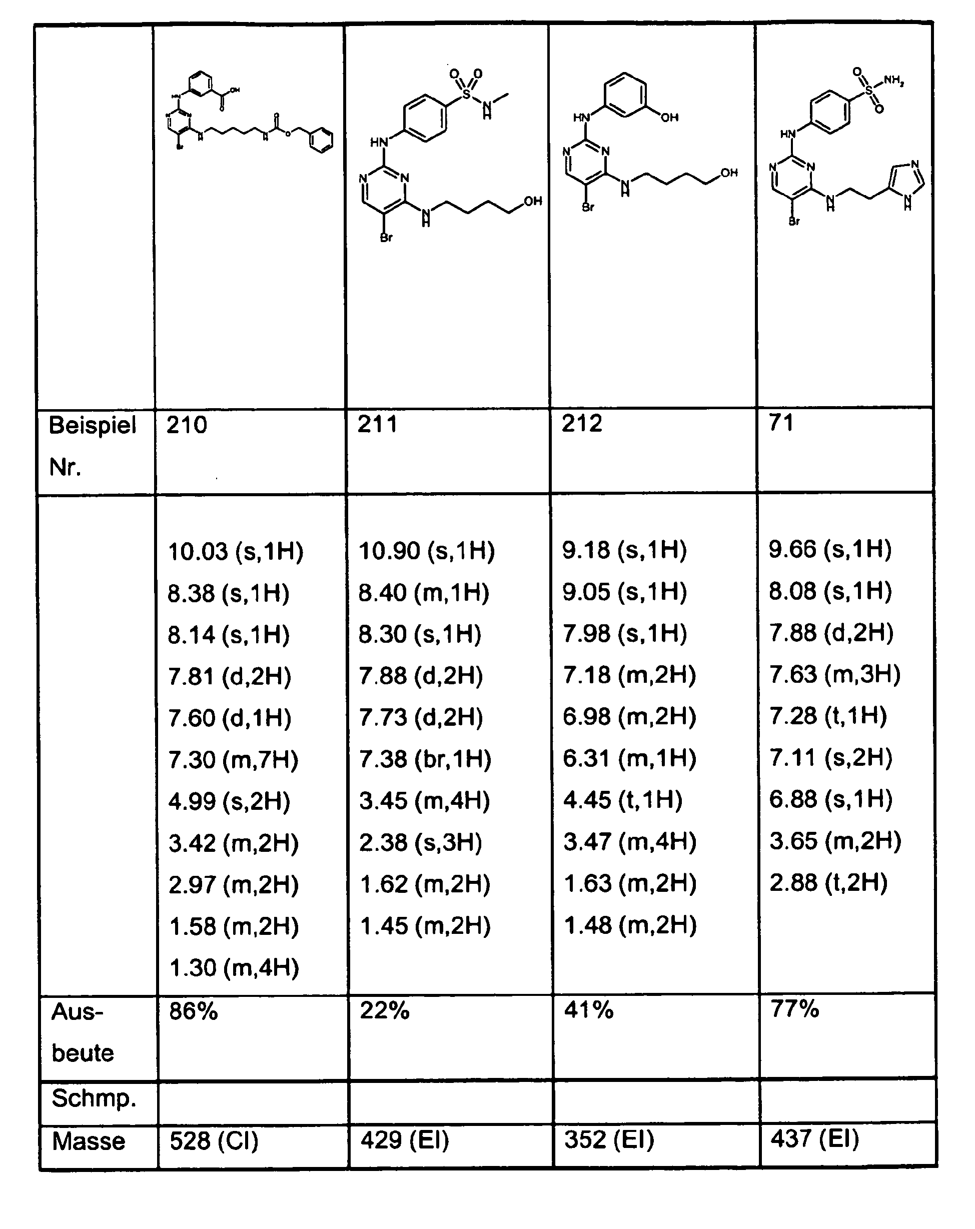

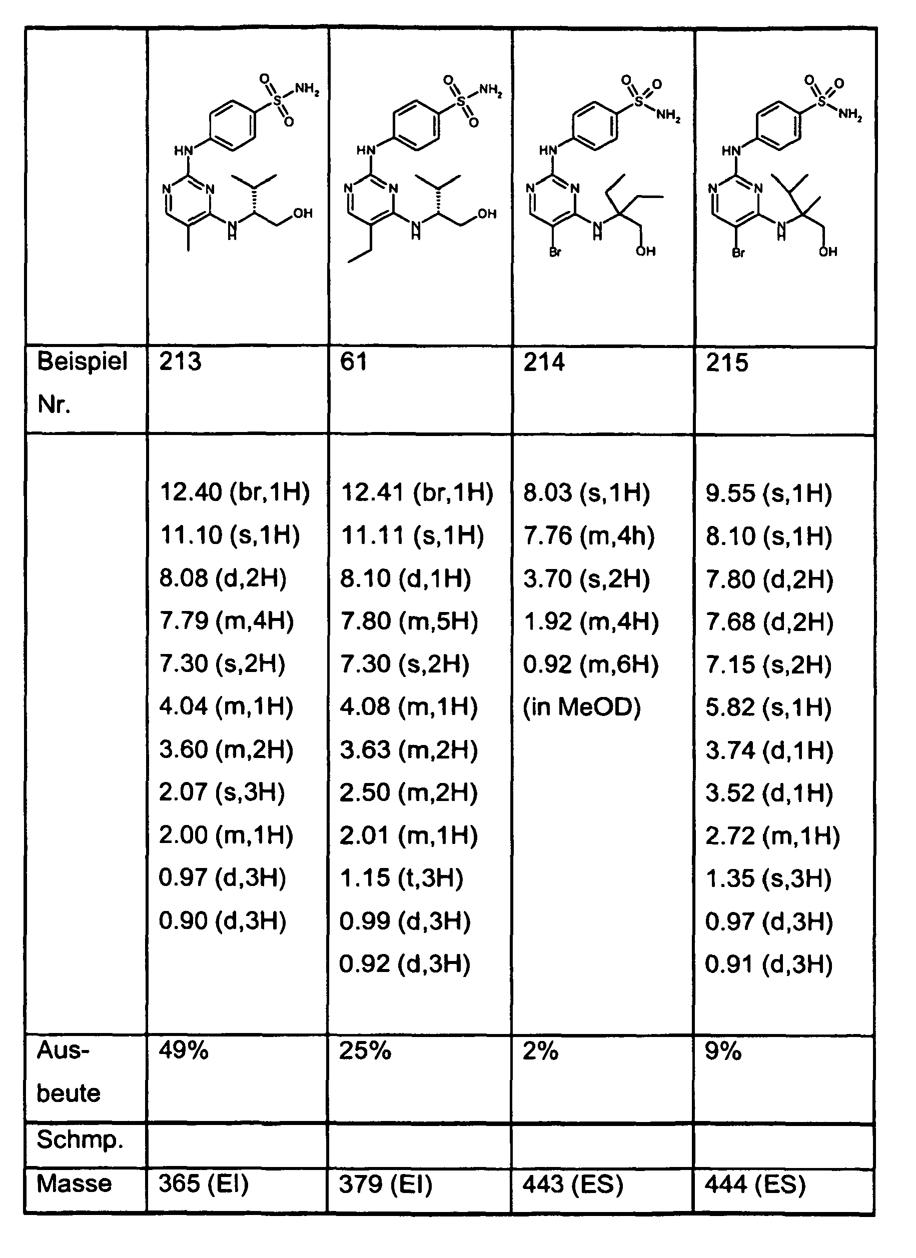

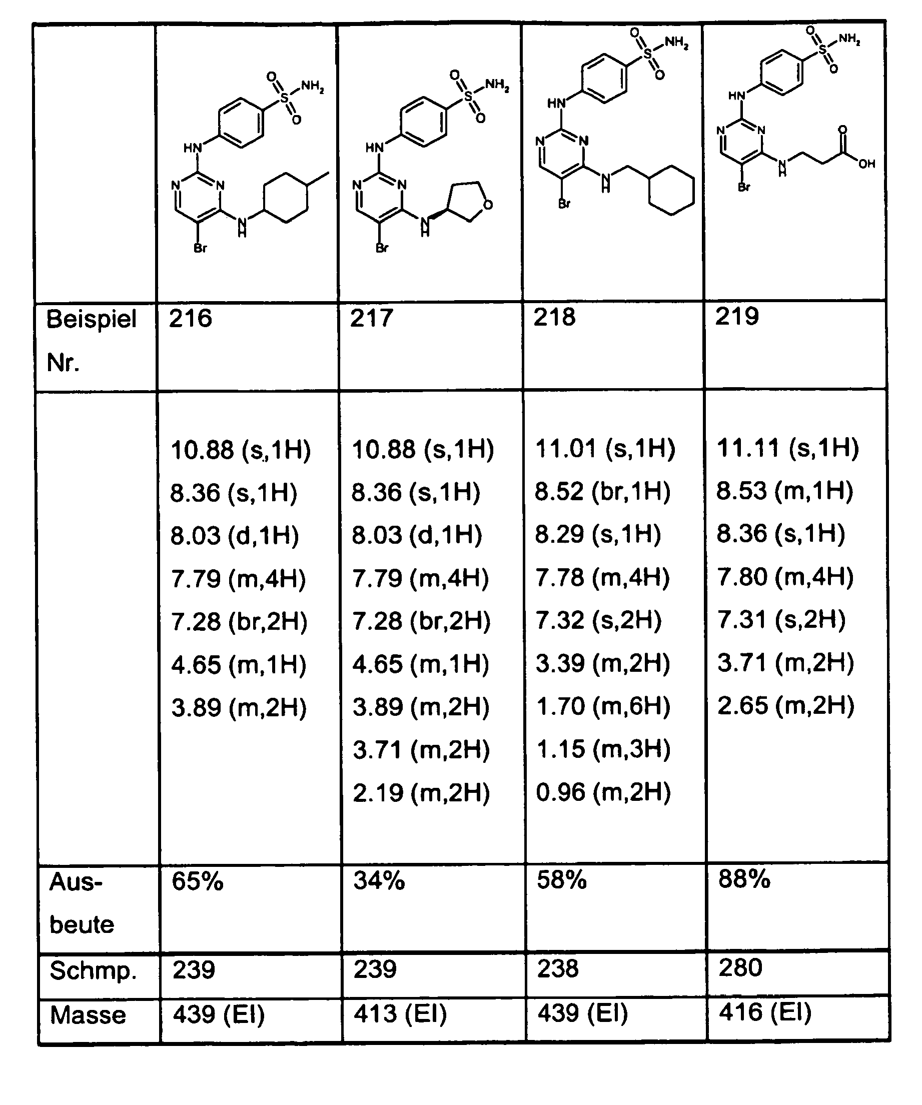

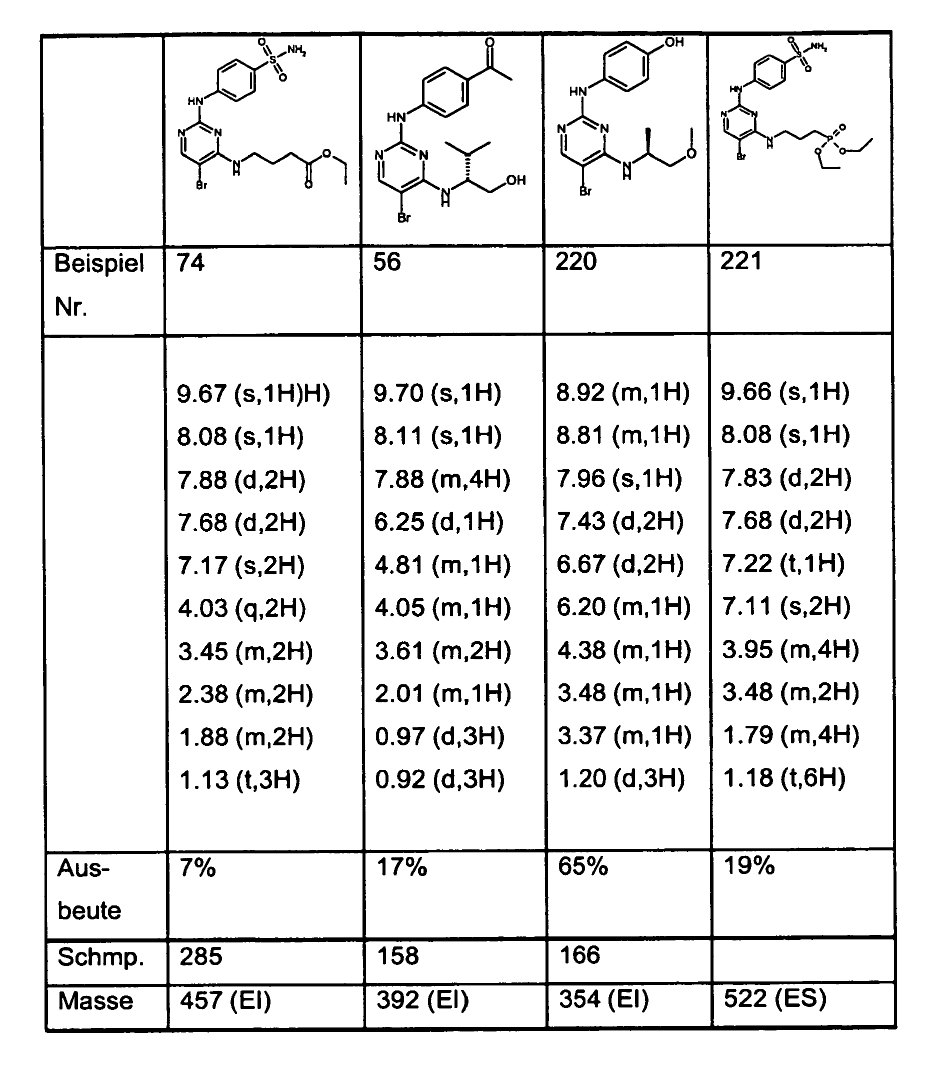

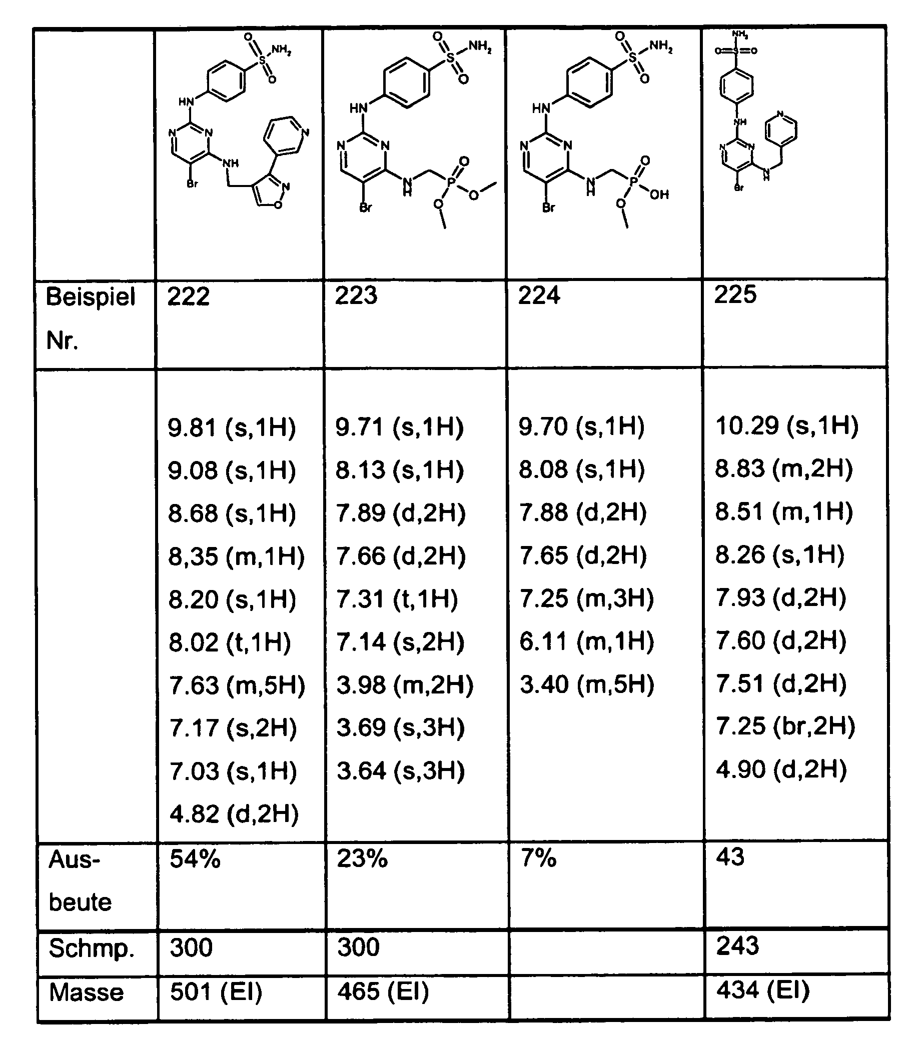

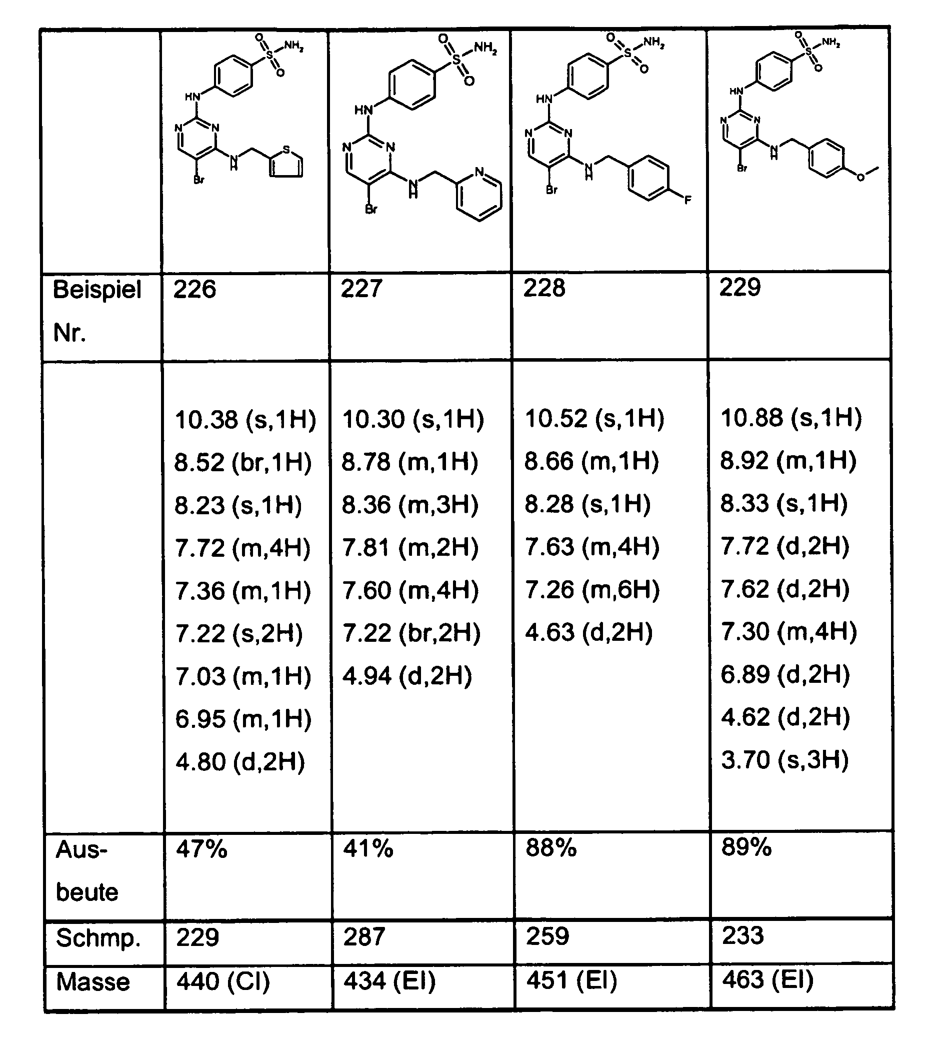

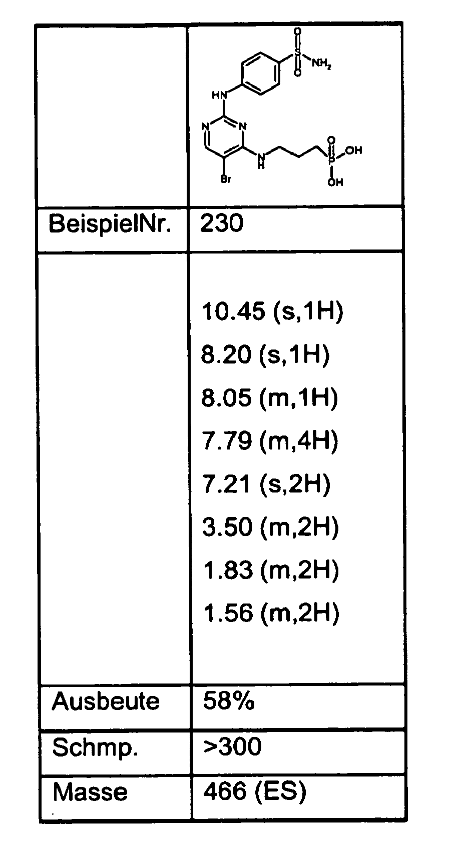

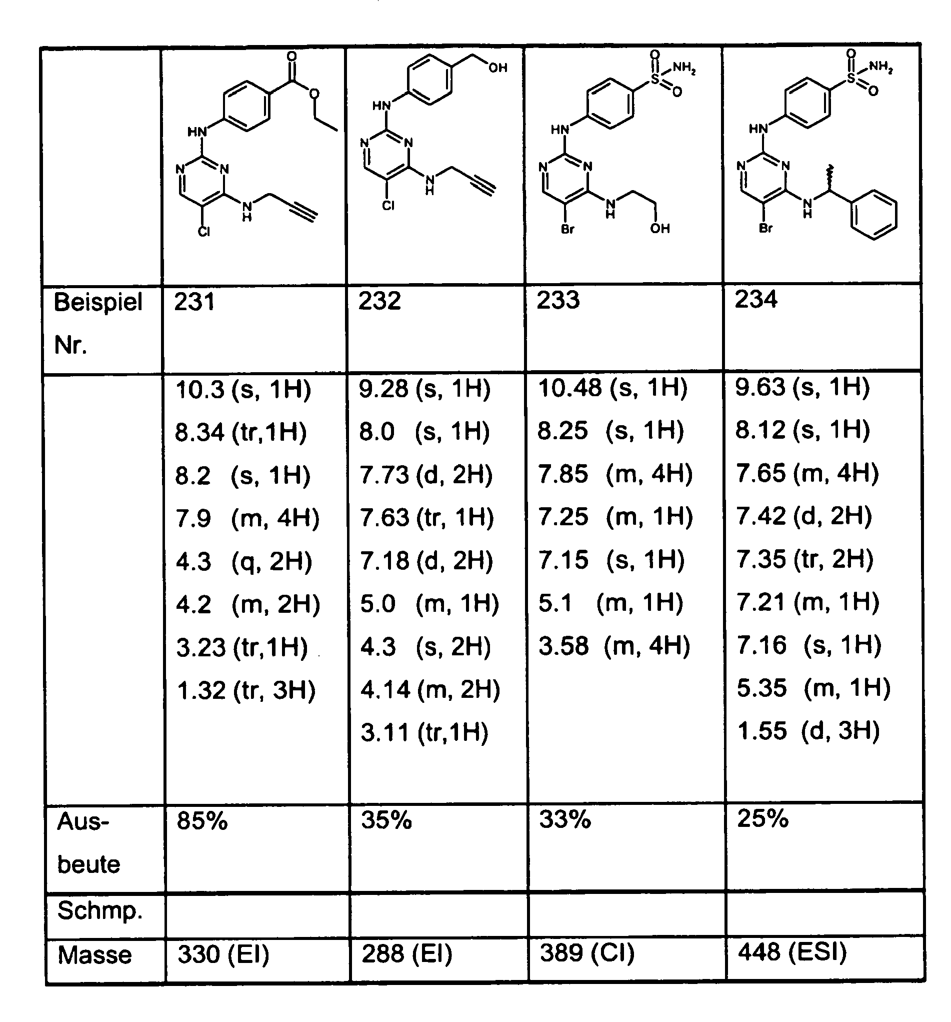

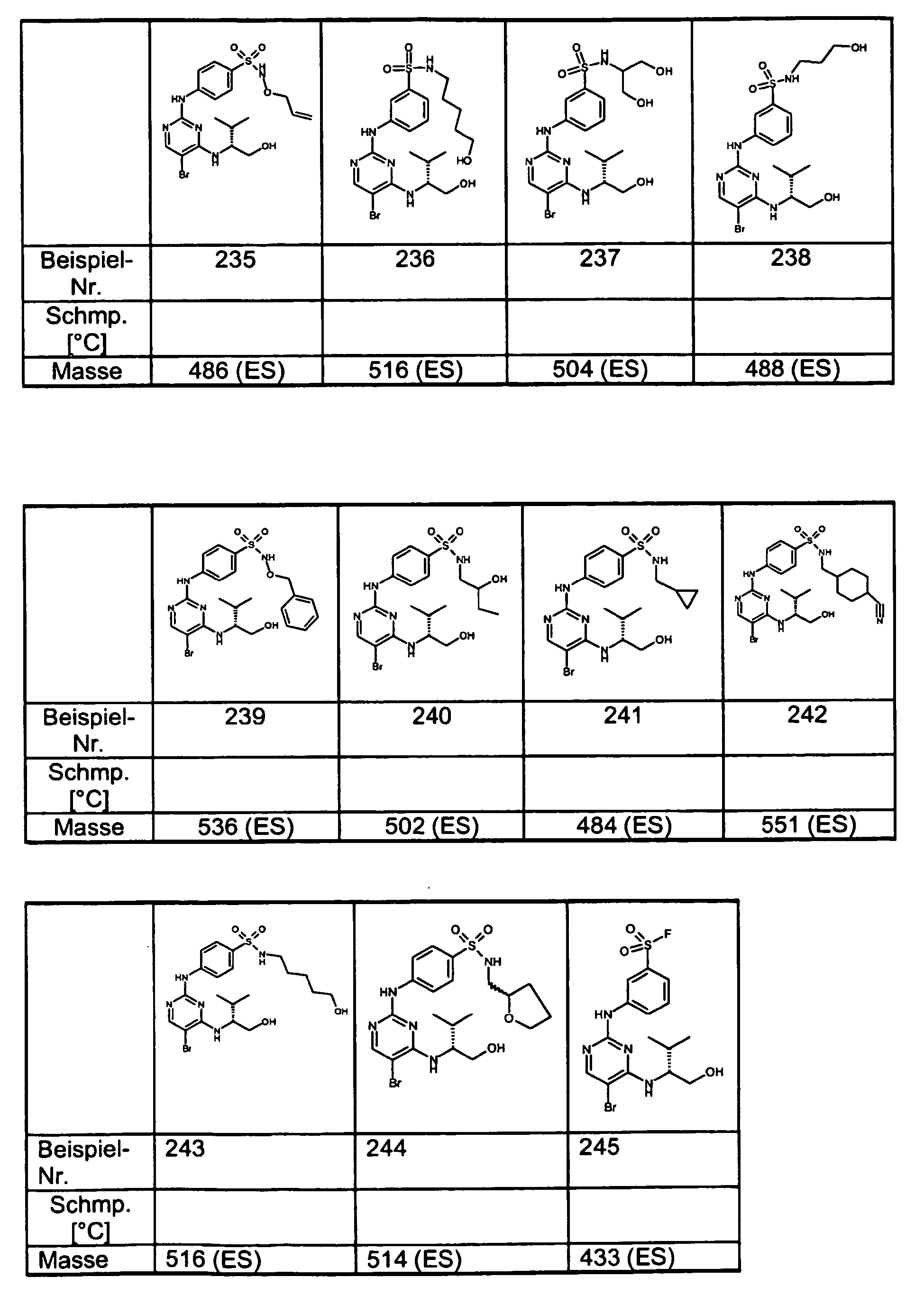

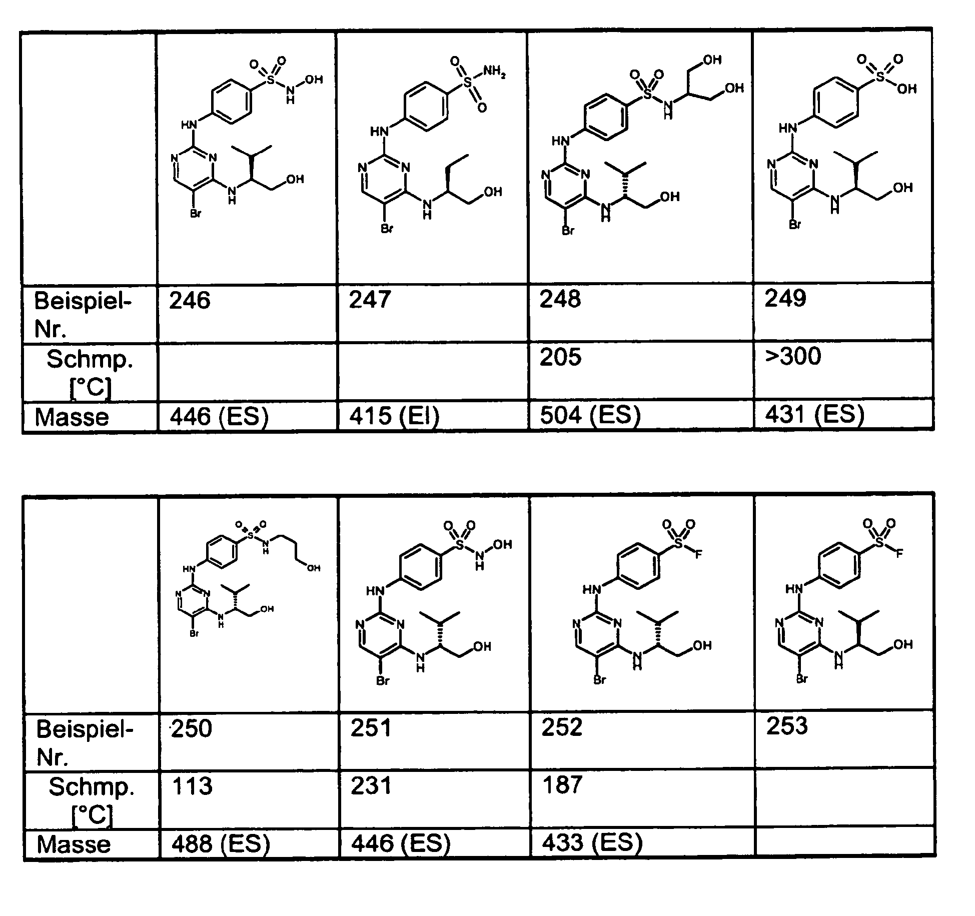

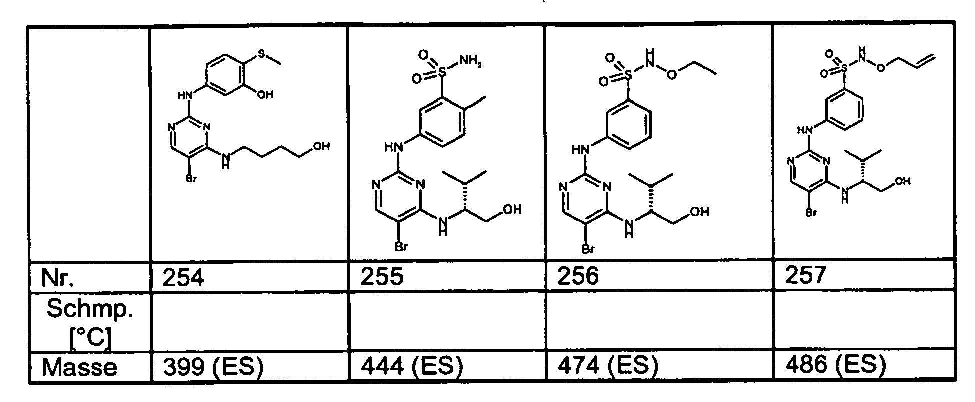

Die mit *) gekennzeichneten Verbindungen Nr. 159, 160, 161, 163, 167, 168, 170, 174, 175, 191, 192, 203 und 204 können über die unter Beispiel Nr. 295 beschriebene Verfahrensvariante hergestellt werden.

### Beispiel 258

### Herstellung von 4-(5-Brom-4-morpholin-4-yl-pyrimidin-2-ylamino)-phenylsulfonamid

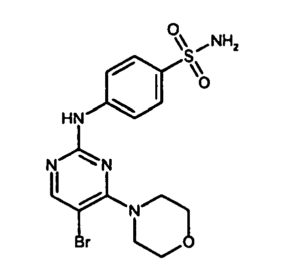

202 mg (0.60 mmol) der Verbindung Beispiel Nr. 122 werden mit 1 ml Wasser sowie 0.2 g (1.2 mmol) Brom versetzt und bei Raumtemperatur gerührt. Nach 24 Stunden werden erneut 0.2 g (1.2 mmol) Brom zugegeben und weitere 24 Stunden bei Raumtemperatur gerührt. Das Lösungsmittel wird mittels Unterdruck evaporiert und der verbleibende Rückstand chromatographisch (Flashmaster II, DCM / MeOH 7:3) gereinigt. Man erhält 17 mg (0.04 mmol, 7%) des Produktes als weissen Feststoff.

| | | | | |
|---|---|---|---|---|
| | 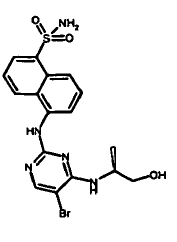 | 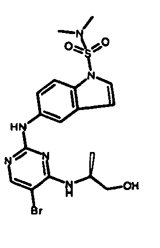 | 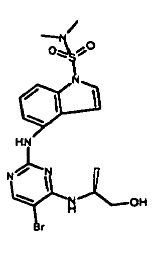 | 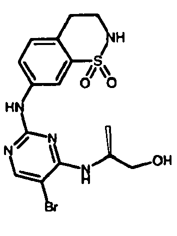 |
| Beispiel-Nr. | 259 | 260 | 261 | 262 |
| Schmp. [°C] | | 205-207 | 202-203 | |
| Masse | MS (ES) 452, 454 (M+ H, 100 %) | | | 428 (ES) |

| **Beispiel- Nr.** | **Verbindung** | **ESI-MS** |
|---|---|---|
| 263 | 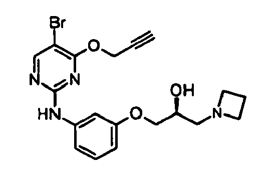 | 434 |
| 264 | 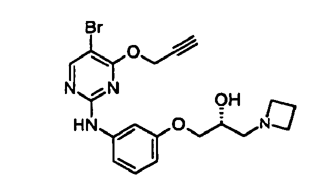 | 434 |
| 265 | 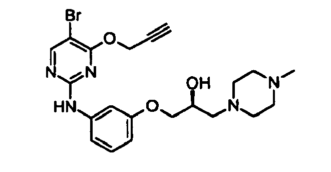 | 477 |
| 266 | 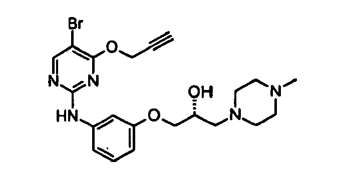 | 477 |
| 267 | 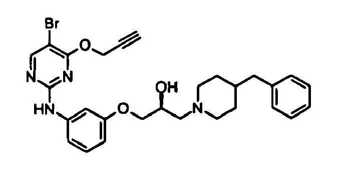 | 552 |
| 268 | 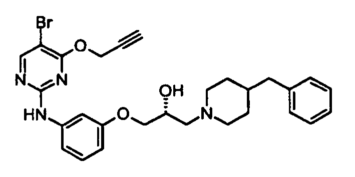 | 552 |

Analog der unter Beispiel 6.0 (s. Herstellung der Zwischenprodukte, Seite 186) beschriebenen Verfahrensweise zur Herstellung der Zwischenprodukte wurden auch folgende Verbindungen hergestellt:

| **Beispiel- Nr.** | **269** | **270** | **271** |
|---|---|---|---|
| | 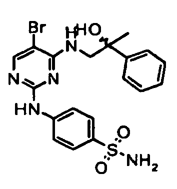 | 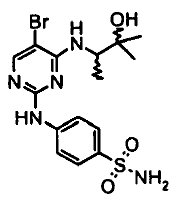 | 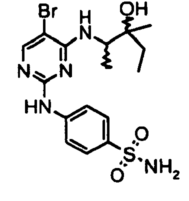 |
| Ausbeute | 47% | 90% | |
| Masse | ESI : | ESI : | ESI : |
| | MH⁺ 480 | MH⁺ 432 | MH⁺ 446 (18%) |
| | (100%) | (100%) | |
| | 478 (97%) | 430 (94%) | |
| | 115 (30%) | 157 (43%) | |

Analog zu Herstellungsbeispiel 1 wurden auch die folgenden Verbindungen hergestellt:

| | | | | |
|---|---|---|---|---|
| | 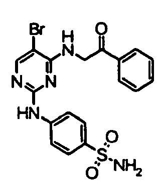 | 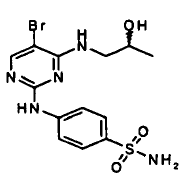 | 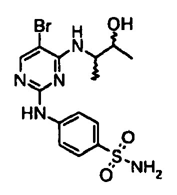 | 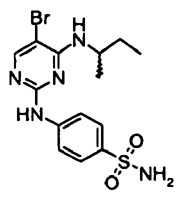 |
| Beispiel -Nr. | 272 | 273 | 274 | 275 |
| Ausbeute | 61% | 44% | 42% | 68% |
| Masse | EI : | EI : | ESI : | EI : |
| | M⁺ 463 (4%) | M⁺ 403 (24%) | MH⁺ 418 | M⁺ 401 (33%) |
| | 277 (8%) | 358 (100%) | 100% | 372 (100%) |
| | 105 (100%) | 277 (52%) | 416 (94%) 346 (8%) | 344 (38%) |
| | 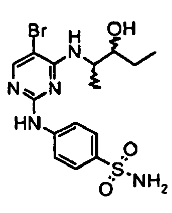 | 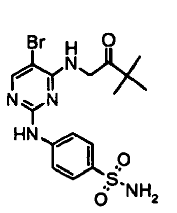 | 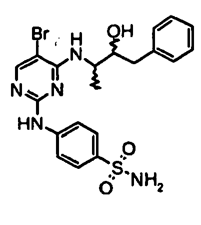 | 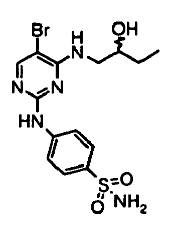 |
| Beispiel -Nr. | 276 | 277 | 278 | 279 |
| Ausbeute | 81% | 58% | ∼20% | 30% |
| Masse | EI : | ESI : | ESI : | ESI : |
| | M⁺ 431 (5%) | MH⁺ 444 | MH+ 494 | MH+ 418 |
| | 372 (100%) | (100%) | (75%) | (100%) |
| | 291 (46%) | 442 (97%) | 346 (18%) | 416 (97%) |
| | | 115 (20%) | 214 (55%) | 310 (27%) |
| | 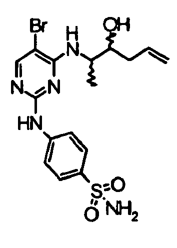 | 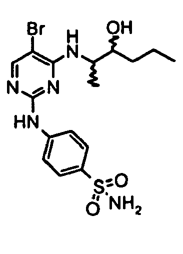 | 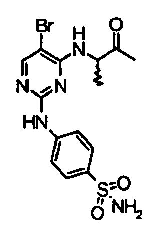 | 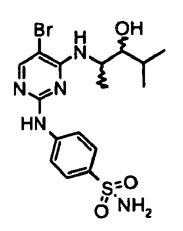 |
| Beispiel -Nr. | 280 | 281 | 282 | 283 |
| Ausbeute | 55% | 43% | ∼18% | 35% |
| Masse | ESI : | ESI : | ESI : | ESI : |
| | MH⁺ 444 | MH⁺ 446 | MH⁺ 416 | MH⁺ 446 |
| | (100%) | (100%) | (100%) | (100%) |
| | 442 (97%) | 444 (95%) | 414 (96%) | 444 (90%) |
| | 214 (12%) | 346 (5%) | 317 (4%) | |
| | 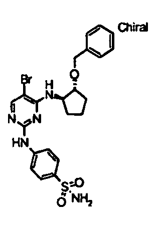 | 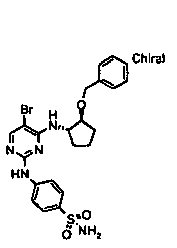 | 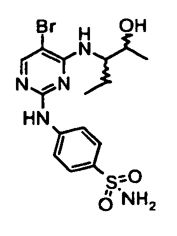 | 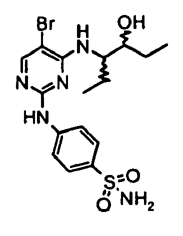 |
| Beispiel -Nr. | 284 | 285 | 286 | 287 |
| Ausbeute | 51% | 46% | 47% | 61% |
| Masse | ESI : | ESI : | ESI . | ESI . |
| | MH⁺ 520 | MH⁺ 520 | MH⁺ 432 | MH⁺ 446 |
| | (100%) | (100%) | (100%) | (100%) |
| | 518 (97%) | 518 (97%) | 430 (95%) | 444 (93%) |
| | 115 (27%) | 115 (23%) | 346 (5%) | 115 (13%) |

Gemäß nachfolgender Herstellungsvariante werden auch die folgen Verbindungen synthetisiert:

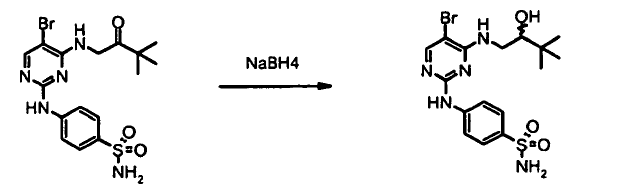

30 mg (0,0678 mMol) der Verbindung Nr. 277 werden in 1 ml Methanol/Tetrahydrofuran 1:1 gelöst. Nach Zugabe von ∼10 mg Natriumborhydrid wird 2 Stunden nachgerührt. Dann wird unter Kühlung mit -3-4 Tropfen Eisessig gequencht und eingeengt. Nachfolgend wird das Rohprodukt

| | | |
|---|---|---|
| | 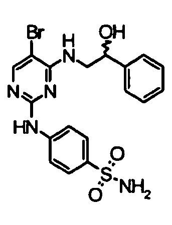 | 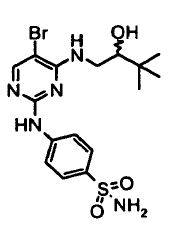 |
| Beispiel -Nr. | 288 | 289 |
| Ausbeute | 52% | 70% |
| Masse | EI: | ESI : |
| | M⁺ 465 (5%) | MH⁺ 446 |
| | 358 (40%) | (100%) |
| | 207 (31%) | 444 (93%) |
| | | 117 (20%) |

### Beispiel 290

### Herstellung der Oximether-Pyrimidin-Verbindungen der allgemeinen Formel I

Die Herstellung der Oximether erfolgt nach dem folgenden allgemeinen Reaktionsschema:

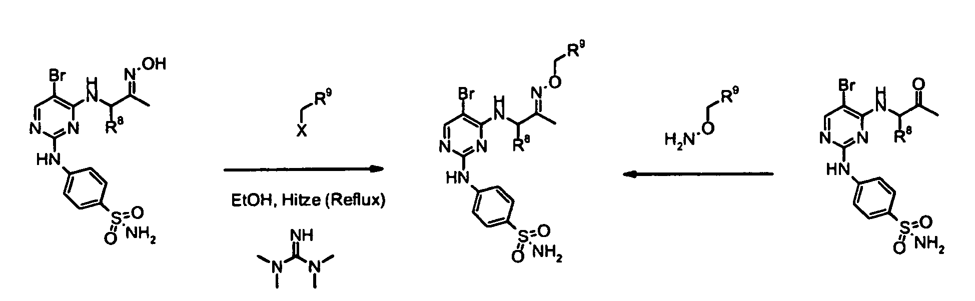

R⁸ und R⁹ haben die in der allgemeinen Formel I angegebenen Bedeutungen.

### Herstellung von Beispiel 290

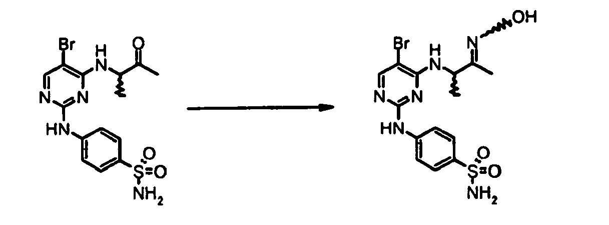

50 mg (0,12 mMol) der Verbindung Nr. 282, 34 mg Hydroxylammoniumchlorid und 150 mg pulversiertes KOH werden 2 Stunden in 2 ml Ethanol unter Rückfluß gekocht. Danach wird auf Eiswasser gegossen und mit Eisessig angesäuert, 3 mal mit Dichlormethan/Isopropanol 4:1 extrahiert , getrocknet mit Magnesiumsulfat und eingeengt. Der Rückstand wird mit Acetonitril aufgeschlemmt, absaugt und bei 60 °C getrocknet. Ausbeute: 28 mg (54% der Theorie) gewünschte Verbindung.

### Masse

ESI :
MH⁺ 429 (29%)
371 (61 %)
289 (91%)

In analoger Verfahrensweise wurden auch folgende Verbindungen hergestellt:

| | | | |
|---|---|---|---|
| | 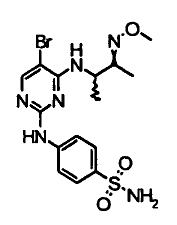 | 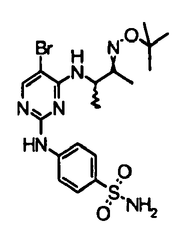 | 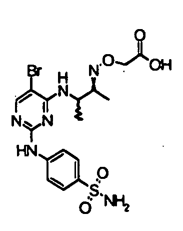 |
| Besispiel-Nr. | 291 | 292 | 293 |
| Ausbeute | 34% | 36% | 40% |
| Masse | ESI : | ESI : | ESI : |
| | MH+ 443 (95%) | MH+ 485 (92%) | MH⁺ 487 (91%) |
| | 445 (99%) | 487 (99%) | 489 (89%) |
| | 373 (32%) | | 373 (32%) |

### Beispiel 294

### Reduktive Aminierung

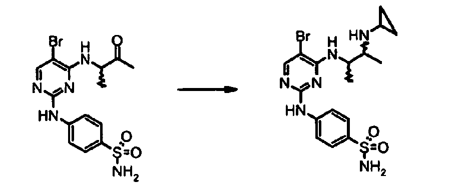

50 mg (0,12 mMol) der Verbindung Nr. 282 und 7,5 mg (0,132 mMol) Cyclopropylamin werden in 2 ml 1,2-Dichlorethan gelöst. Nach Zugabe von 9,1 mg ( 0,144 mMol) Natriumcyanoborhydrid lässt man 12 Stunden nachrühren. Dann wird mit Dichlormethan/ Isopropanol 4:1 verdünnt, 2 mal mit Wasser gewaschen, getrocknet mit Magnesiumsulfat und einengt. Der Rückstand wird über Kieselgel mit Dichlormethan/ Methanol 95:5 chromatographiert. Ausbeute : 18 mg (33 % der Theorie) gewünschte Verbindung.

| | |
|---|---|
| | 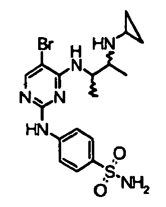 |
| Ausbeute | 33% |
| Masse | ESI : |
| | MH⁺ 457 |
| | (98%) |
| | 455 (93%) |
| | 249 (55%) |

In analoger Verfahrensweise werden auch die Verbindungen Nr. 159, 160, 161, 163, 167, 168, 170, 174, 175, 191, 192, 203 und 204 hergestellt.

### Beispiel 295 und 296

In analoger Verfahrensweise zu Beispiel 1 werden auch folgende zwei Verbindungen hergestellt:

| | | |
|---|---|---|
| | 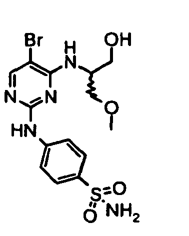 | 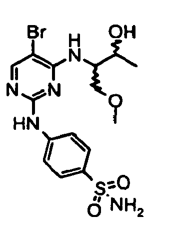 |
| Beispiel | 295 | 296 |
| Ausbeute | 46 % | 47 % |
| Masse | ESI : | ESI : |
| | MH⁺ 432 (30%) | MH⁺ 446 (45%) |
| | 434 (31%) | 448 (49%) |
| | 123 (100%) | 123 (90%) |

### Herstellung der Sulfonamide der allgemeinen Formel I

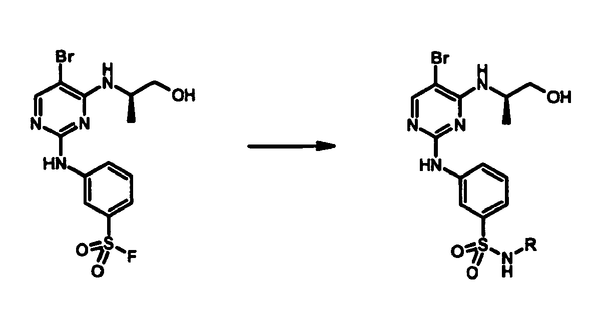

0.2 mmol Sulfonsäurefluorid werden im Reaktor eines Synthesizers vorgelegt, Man gibt 1.0 ml Solvens, vorzugsweise 2-Butanol hinzu. Nacheinander werden über eine Pipette 0.2 ml (0.2 mmol) von DMAP - gelöst in einem Solvens, beispielsweise DMSO oder 2-Butanol - und 0.2 mL (0.2 mmol) des Amins, gelöst in 2-Butanol, hinzugegeben. Die Reaktionsmischung wird anschliessend für 20 Stunden bei 80°C gerührt. Nach beendeteter Reaktion wird das Rohprodukt abpipettiert und der Reaktor mit 1.0 mL THF nachgewaschen. Die Lösung des Rohproduktes wird dann eingeengt und mittels HPLC gereinigt.

Es wurden die nachfolgenden Verbindungen hergestellt:

| **Beispiel- Nr.** | **Verbindung** | **Molgewicht** | **ESI-MS** |
|---|---|---|---|
| 297 | 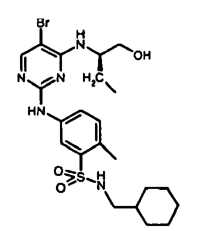 | 526,4968 | 526/528 |
| 298 | 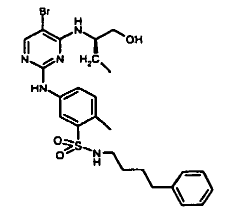 | 562,5298 | 562/564 |
| 299 | 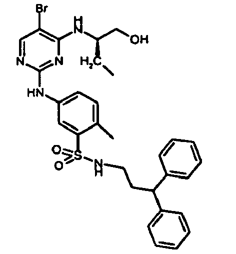 | 624,6006 | 624/626 |
| 300 | 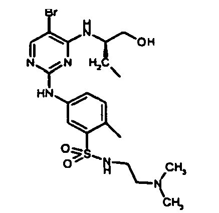 | 501,4471 | 501/503 |
| 301 | 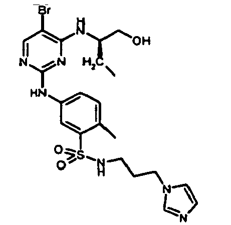 | 538,4682 | 538/540 |
| 302 | 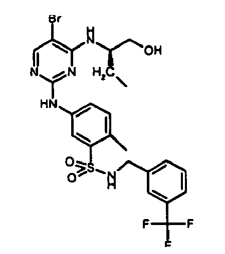 | 588,4465 | 588/590 |
| 303 | 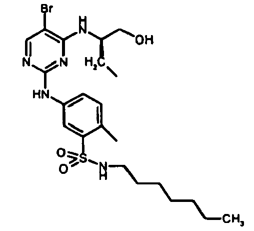 | 528,5126 | 528/530 |
| 304 | 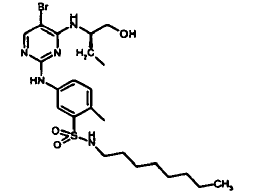 | 542,5394 | 542/544 |
| 305 | 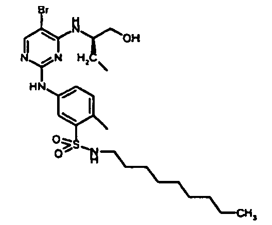 | 556,5662 | 556/558 |
| 306 | 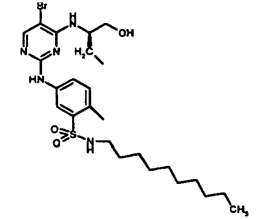 | 570,593 | 570/572 |
| 307 | 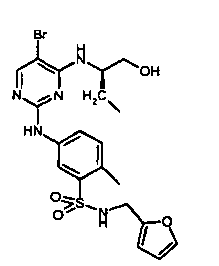 | 510,4106 | 510/512 |
| 308 | 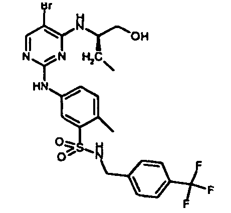 | 588,4465 | 588/590 |
| 309 | 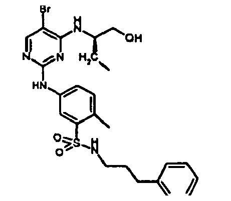 | 548,503 | 548/550 |
| 310 | 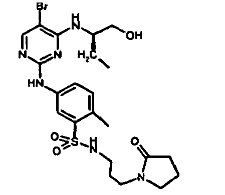 | 555,4949 | 555/557 |
| 311 | 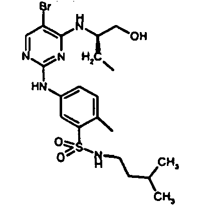 | 500,459 | 500/502 |
| 312 | 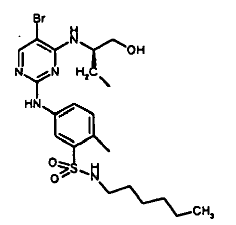 | 514,4858 | 514/516 |
| 313 | 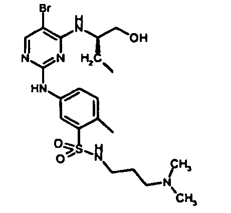 | 515,4739 | 515/517 |
| 314 | 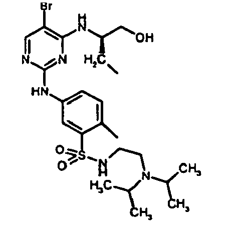 | 557,5543 | 557/559 |
| 315 | 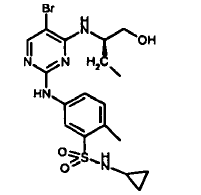 | 470,3896 | 470/472 |
| 316 | 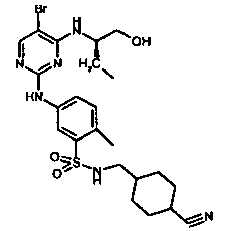 | 551,5069 | 551/553 |
| 317 | 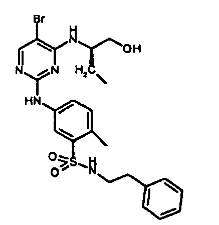 | 534,4762 | 534/536 |
| 318 | 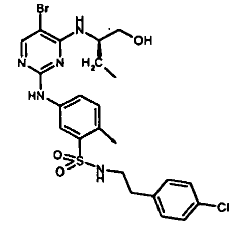 | 568,9213 | 568/570 |
| 319 | 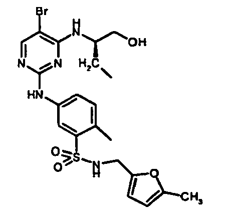 | 524,4374 | 524/526 |
| 320 | 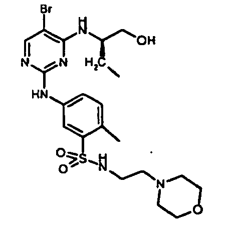 | 543,4839 | 543/545 |
| 321 | 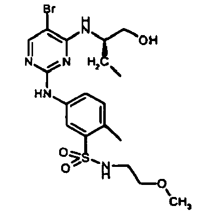 | 488,4044 | 488/490 |
| 322 | 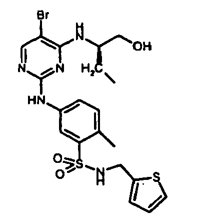 | 526,4776 | 526/528 |
| 323 | 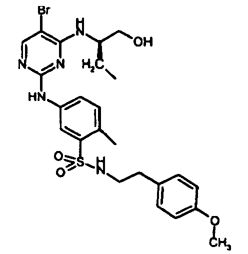 | 564,502 | 564/566 |
| 324 | 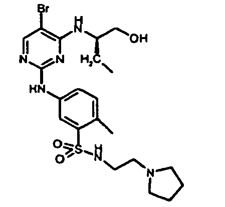 | 527,4849 | 527/529 |
| 325 | 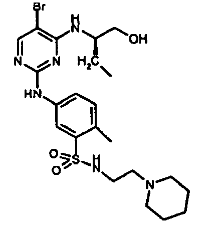 | 541,5117 | 541/543 |
| 326 | 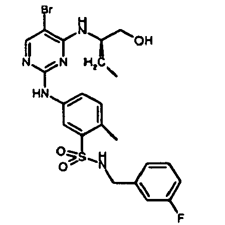 | 538,4395 | 538/540 |
| 327 | 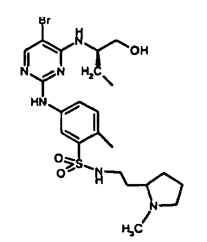 | 541,5117 | 541/543 |
| 328 | 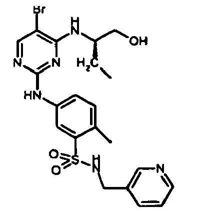 | 521,4375 | 521/523 |
| 329 | 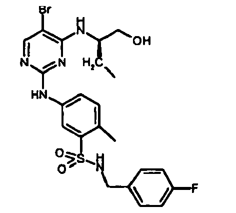 | 538,4395 | 538/540 |
| 330 | 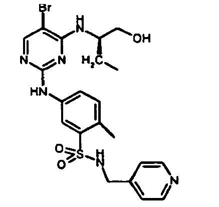 | 521,4375 | 521/523 |
| 331 | 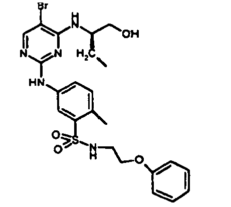 | 550,4752 | 550/552 |
| 332 | 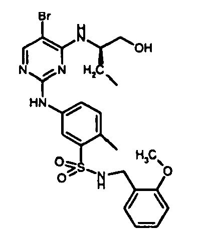 | 550,4752 | 550/552 |
| 333 | 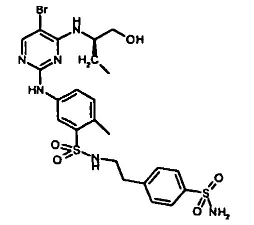 | 613,5551 | 613/615 |
| 334 | 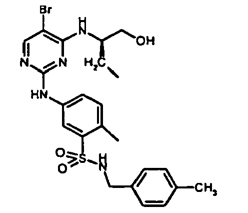 | 534,4762 | 534/536 |
| 335 | 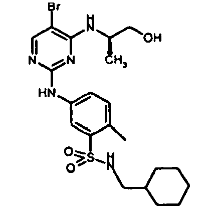 | 512,47 | 512/514 |
| 336 | 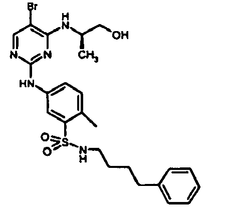 | 548,503 | 548/550 |
| 337 | 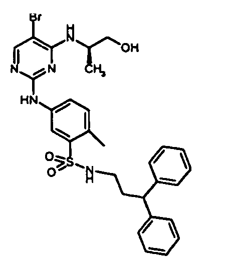 | 610,5738 | 610/612 |
| 338 | 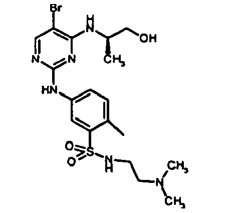 | 487,4203 | 487/489 |
| 339 | 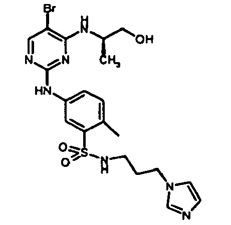 | 524,4414 | 524/526 |
| 340 | 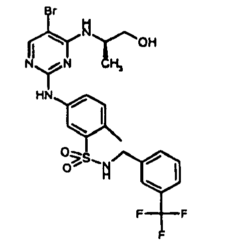 | 574,4197 | 574/576 |
| 341 | 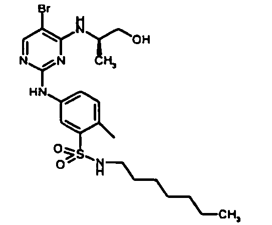 | 514,4858 | 516/514 |
| 342 | 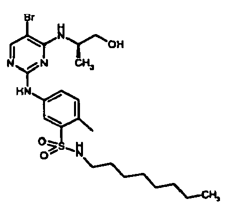 | 528,5126 | 528/530 |
| 343 | 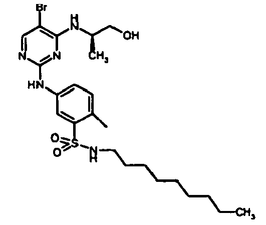 | 542,5394 | 542/544 |
| 344 | 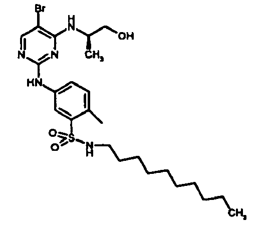 | 556,5662 | 556/558 |
| 345 | 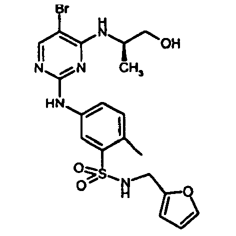 | 496,3838 | 496/498 |
| 346 | 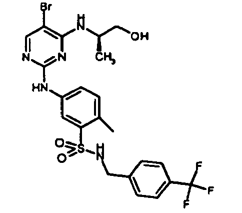 | 574,4197 | 574/576 |
| 347 | 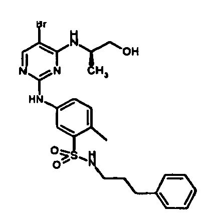 | 534,4762 | 534/536 |
| 348 | 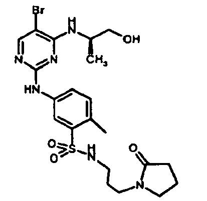 | 541,4681 | 541/543 |
| 349 | 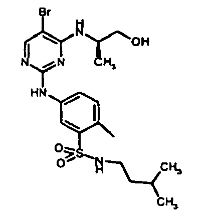 | 486,4322 | 486/488 |
| 350 | 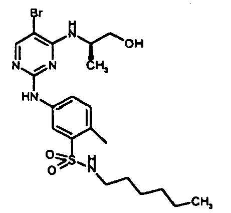 | 500,459 | 500/502 |
| 351 | 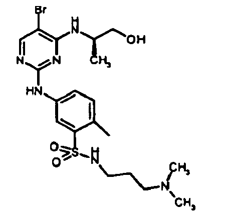 | 501,4471 | 501/503 |
| 352 | 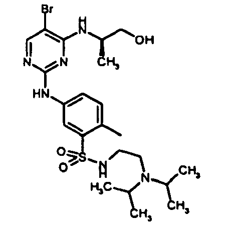 | 543,5275 | 543/545 |
| 353 | 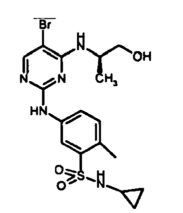 | 456,3628 | 456/458 |
| 354 | 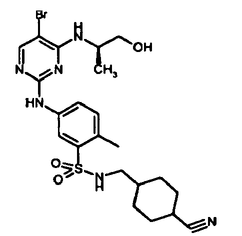 | 537,4801 | 537/539 |
| 355 | 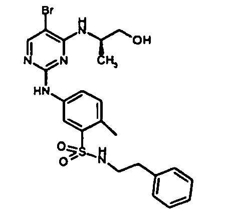 | 520,4494 | 520/522 |
| 356 | 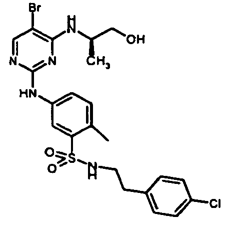 | 554,8945 | 554/556 |
| 357 | 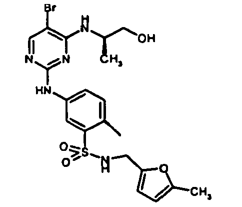 | 510,4106 | 510/512 |
| 358 | 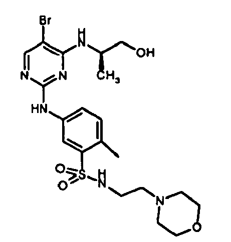 | 529,4571 | 529/531 |
| 359 | 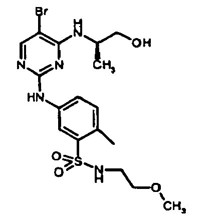 | 474,3776 | 474/476 |
| 360 | 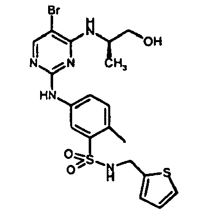 | 512,4508 | 541/514 |
| 361 | 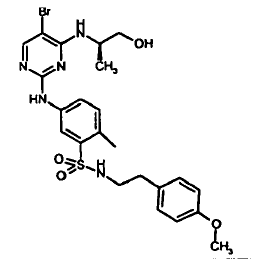 | 550,4752 | 550/552 |
| 362 | 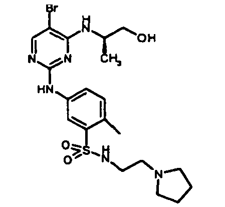 | 513,4581 | 513/515 |
| 363 | 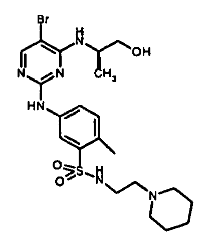 | 527,4849 | 527/529 |
| 364 | 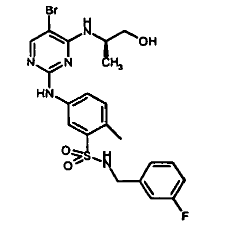 | 524,4127 | 524/526 |
| 365 | 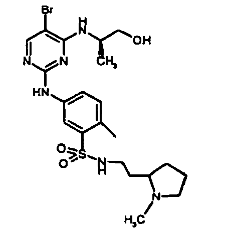 | 527,4849 | 527/529 |
| 366 | 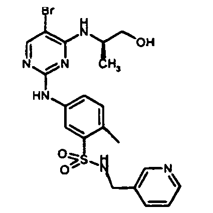 | 507,4107 | 507/509 |
| 367 | 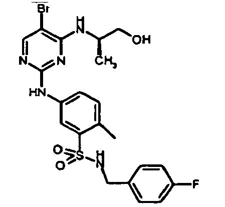 | 524,4127 | 524/526 |
| 368 | 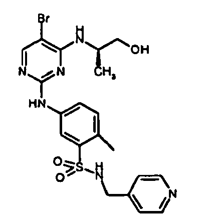 | 507,4107 | 507/509 |
| 369 | 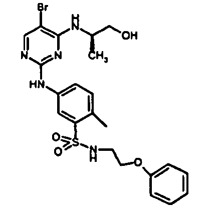 | 536,4484 | 536/538 |
| 370 | 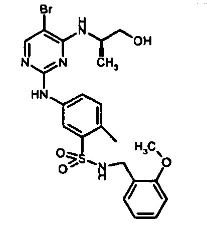 | 536,4484 | 536/538 |
| 371 | 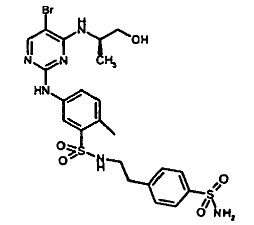 | 599,5283 | 599/601 |
| 372 | 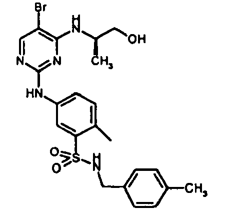 | 520,4494 | 520/522 |
| 373 | 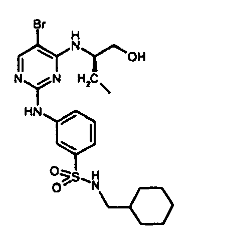 | 512,47 | 512/514 |
| 374 | 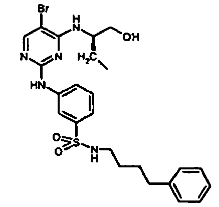 | 548,503 | 548/550 |
| 375 | 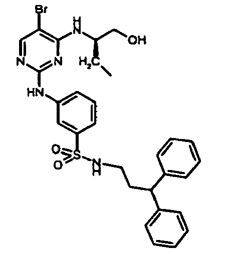 | 610,5738 | 610/612 |
| 376 | 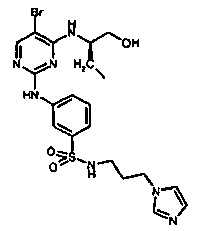 | 524,4414 | 524/526 |
| 377 | 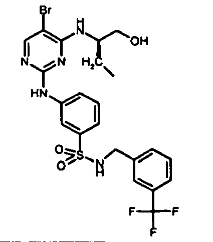 | 574,4197 | 574/576 |
| 378 | 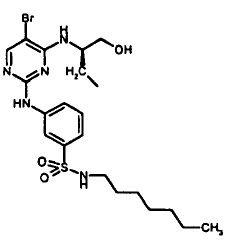 | 514,4858 | 514/516 |
| 379 | 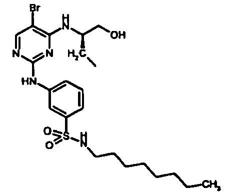 | 528,5126 | 528/530 |
| 380 | 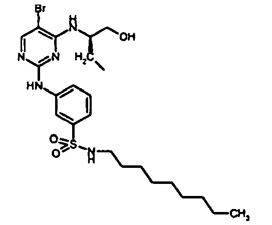 | 542,5394 | 542/544 |
| 381 | 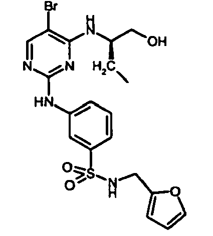 | 496,3838 | 496/498 |
| 382 | 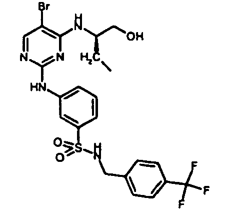 | 574,4197 | 574/576 |
| 383 | 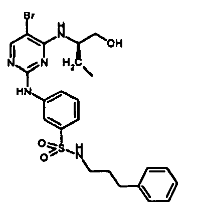 | 534,4762 | 534/536 |
| 384 | 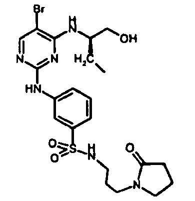 | 541,4681 | 541/543 |
| 385 | 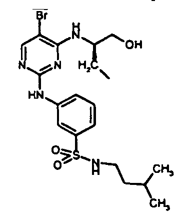 | 486,4322 | 486/488 |
| 386 | 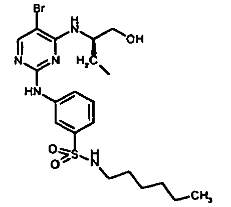 | 500,459 | 500/502 |
| 387 | 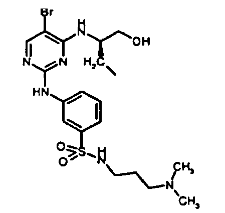 | 501,4471 | 501/503 |
| 388 | 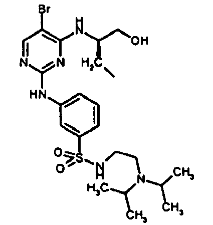 | 543,5275 | 543/545 |
| 389 | 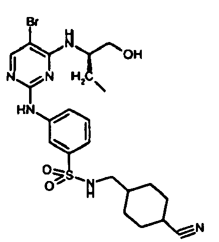 | 537,4801 | 537/539 |
| 390 | 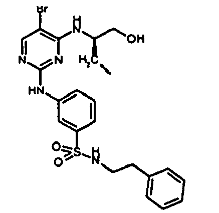 | 520,4494 | 520/522 |
| 391 | 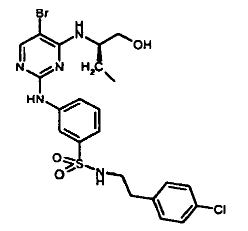 | 554,8945 | 554/556 |
| 392 | 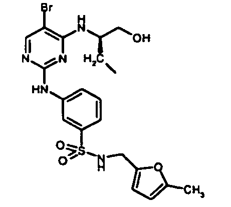 | 510,4106 | 510/512 |
| 393 | 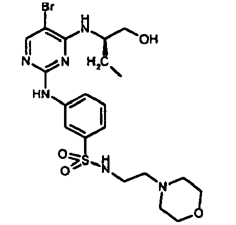 | 529,4571 | 529/531 |
| 394 | 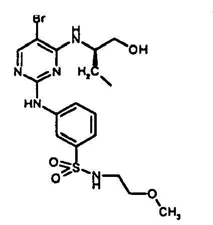 | 474,3776 | 474/476 |
| 395 | 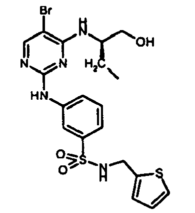 | 512,4508 | 512/514 |
| 396 | 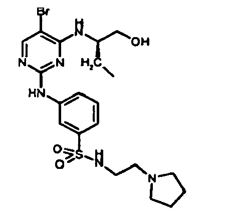 | 513,4581 | 513/515 |
| 397 | 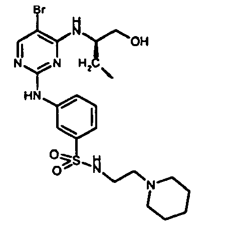 | 527,4849 | 527/529 |
| 398 | 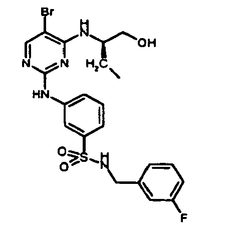 | 524,4127 | 524/526 |
| 399 | 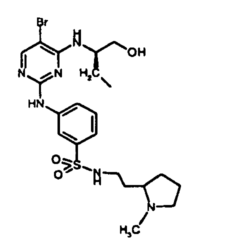 | 527,4849 | 527/529 |
| 400 | 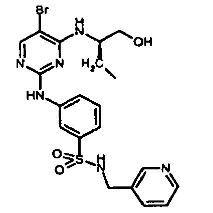 | 507,4107 | 507/509 |
| 401 | 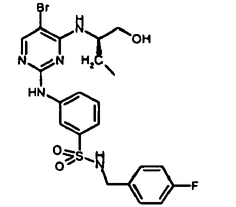 | 524,4127 | 524/526 |
| 402 | 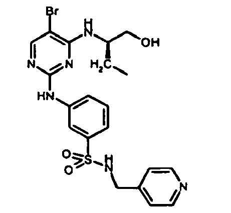 | 507,4107 | 507/509 |
| 403 | 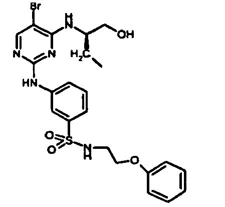 | 536,4484 | 526/538 |
| 404 | 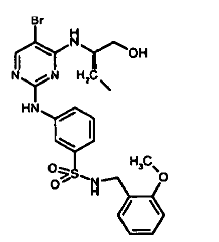 | 536,4484 | 536/538 |
| 405 | 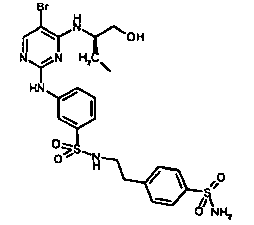 | 599,5283 | 599/601 |
| 406 | 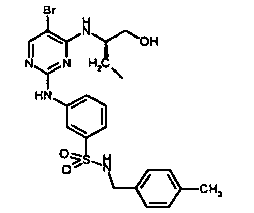 | 520,4494 | 520/522 |
| 407 | 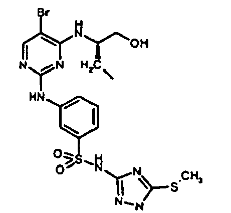 | 529,4419 | 529/531 |
| 408 | 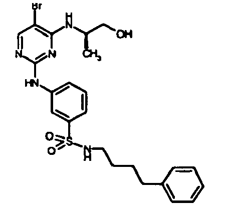 | 534,4762 | 534/536 |
| 409 | 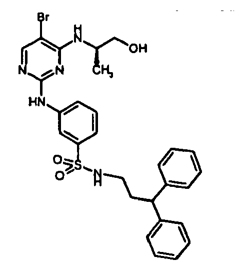 | 596,547 | 596/598 |
| 410 | 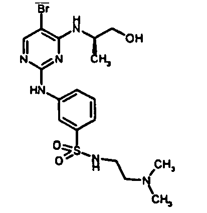 | 473,3935 | 473/475 |
| 411 | 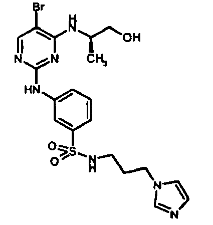 | 510,4146 | 510/512 |
| 412 | 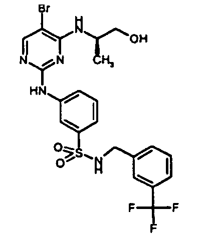 | 560,3929 | 560/562 |
| 413 | 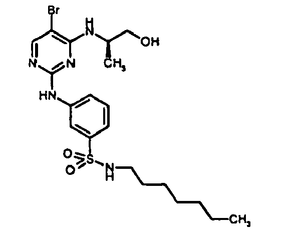 | 500,459 | 500/502 |
| 414 | 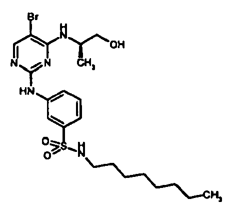 | 514,4858 | 514/516 |
| 415 | 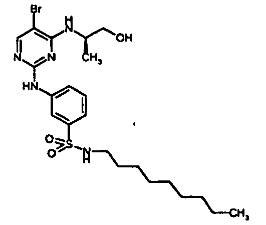 | 528,5126 | 528/530 |
| 416 | 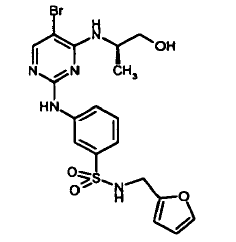 | 482,357 | 482/484 |
| 417 | 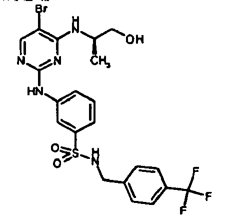 | 560,3929 | 560/562 |
| 418 | 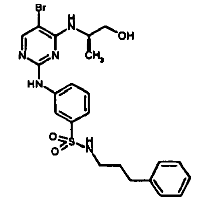 | 520,4494 | 520/522 |
| 419 | 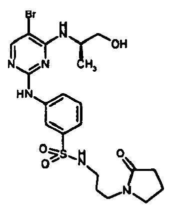 | 527,4413 | 527/529 |
| 420 | 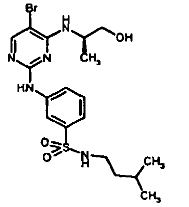 | 472,4054 | 472/474 |
| 421 | 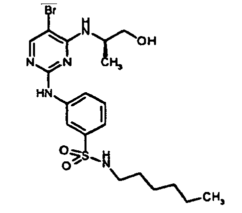 | 486,4322 | 486/488 |
| 422 | 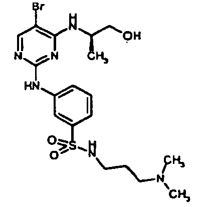 | 487,4203 | 487/489 |
| 423 | 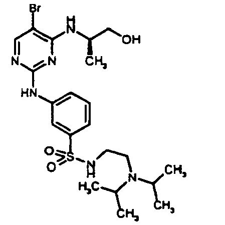 | 529,5007 | 529/531 |
| 424 | 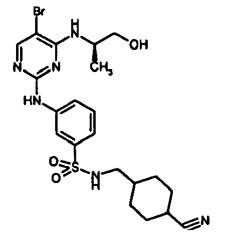 | 523,4532 | 523/525 |
| 425 | 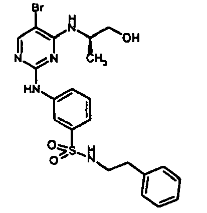 | 506,4226 | 506/508 |
| 426 | 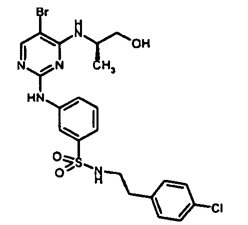 | 540,8677 | 540/542 |
| 427 | 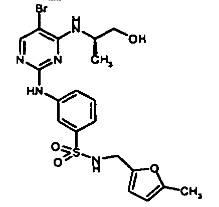 | 496,3838 | 496/498 |
| 428 | 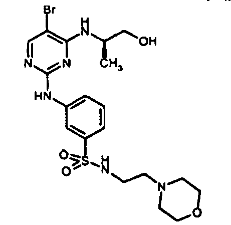 | 515,4303 | 515/517 |
| 429 | 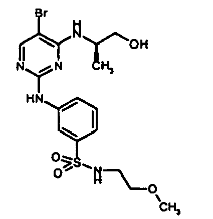 | 460,3508 | 460/462 |
| 430 | 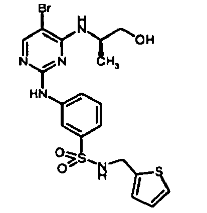 | 498,424 | 498/500 |
| 431 | 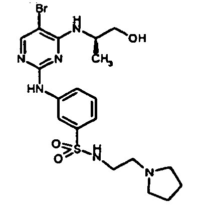 | 499,4313 | 499/501 |
| 432 | 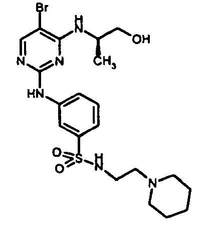 | 513,4581 | 513/515 |
| 433 | 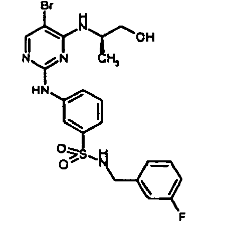 | 510,3859 | 510/512 |
| 434 | 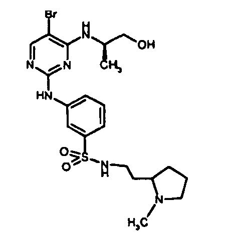 | 513,4581 | 513/515 |
| 435 | 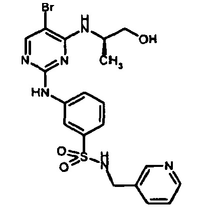 | 493,3839 | 493/495 |
| 436 | 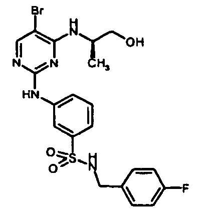 | 510,3859 | 510/512 |
| 437 | 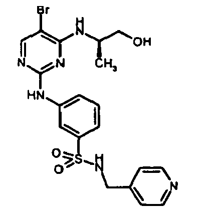 | 493,3839 | 493/495 |
| 438 | 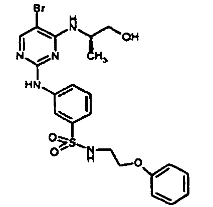 | 522,4216 | 522/524 |
| 439 | 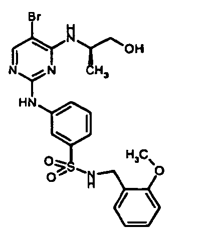 | 522,4216 | 522/524 |
| 440 | 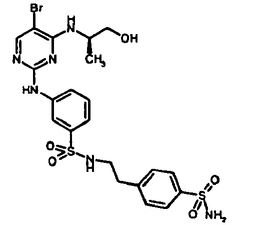 | 585,5015 | 585/587 |
| 441 | 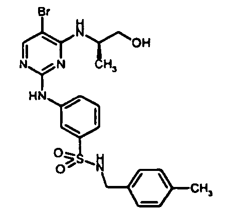 | 506,4226 | 506/508 |
| 442 | 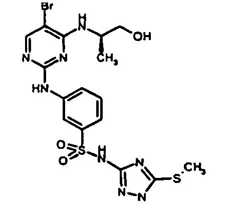 | 515,4151 | 515/517 |
| 443 *) | 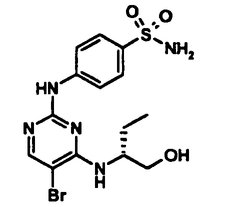 | 416,30 | 416/418 |

| | | | |
|---|---|---|---|
| *) hergestellt nach dem unter Sulfonamide beschriebenen Verfahren | | | |

### Herstellung der Pyrimidin-Sulfonylfluoride der allgemeinen Formel I

Die Herstellung der Pyrimidin-Sulfonsäurefluoride erfolgt analog zur Herstellung der Sulfonsäureamide.

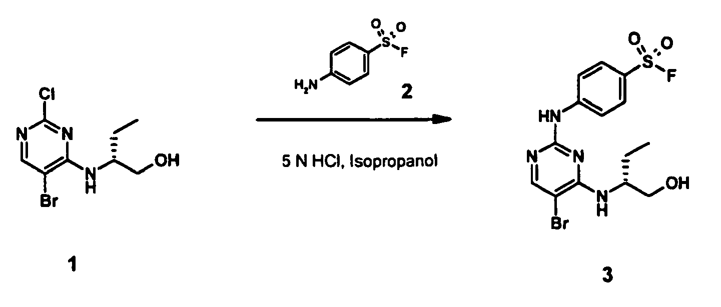

| **Beispiel-Nr.** | **Verbindung** | **Molgewicht** | **Schmelzpunkt [°C] und ESI-MS** |
|---|---|---|---|
| 444 | 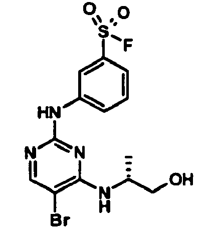 | 405,25 | 217-220 |
| | | | 405/407 |
| 445 | 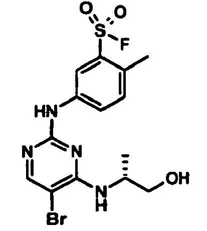 | 419,27 | 196-202 |
| | | | 419/421 |
| 446 | 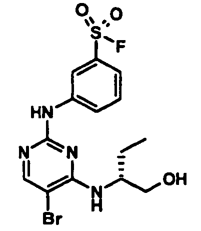 | 419,27 | 165-196 |
| | | | 419/421 |
| 447 | 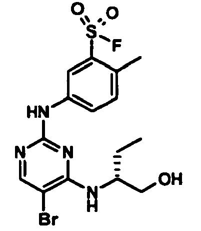 | 433,30 | 198-204 |
| | | | 433/435 |
| 448 | 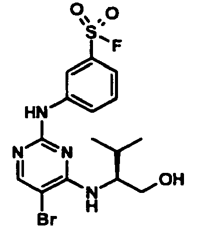 | 433,30 | 144-149 |
| | | | 433/435 |
| 449 | 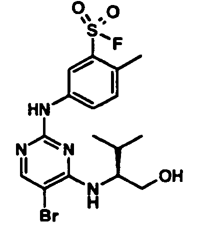 | 447,33 | 219-222 |
| | | | 447/449 |
| 450 | 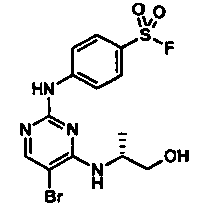 | 405,25 | 170-173 |
| | | | 405/407 |
| 451 | 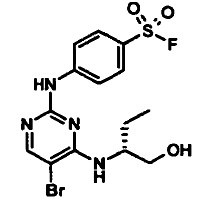 | 419,27 | 226-228 |
| | | | 419/421 |
| 452 | 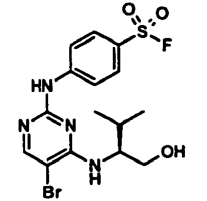 | 433,30 | 433/435 |
| 453 | 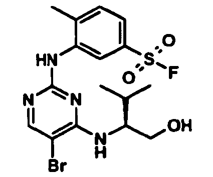 | 447,33 | 447/449 |
| 454 | 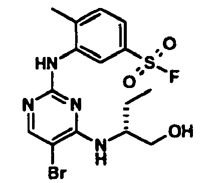 | 433,30 | 433/435 |
| 455 | 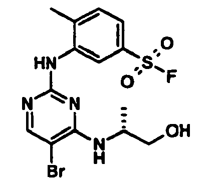 | 419,27 | 419/421 |
| 456 | 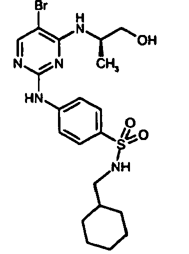 | 498,4432 | 498/500 |
| 457 | 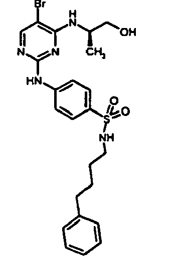 | 534,4762 | 534/536 |
| 458 | 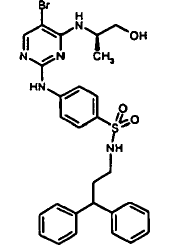 | 596,547 | 596/598 |
| 459 | 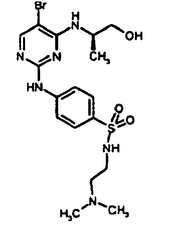 | 473,3935 | 473/475 |
| 460 | 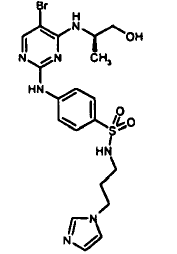 | 510,4146 | 510/512 |
| 461 | 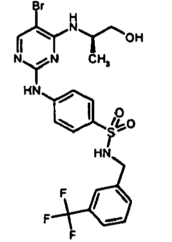 | 560,3929 | 560/562 |
| 462 | 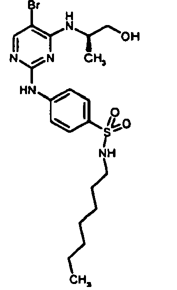 | 500,459 | 500/502 |
| 463 | 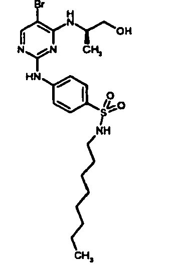 | 514,4858 | 514/516 |
| 464 | 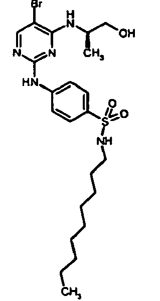 | 528,5126 | 528/530 |
| 465 | 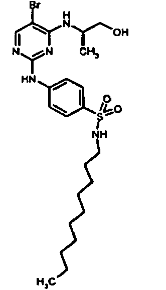 | 542,5394 | 542/544 |
| 466 | 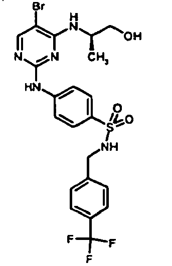 | 560,3929 | 560/562 |
| 467 | 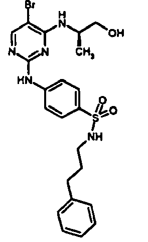 | 520,4494 | 520/522 |
| 468 | 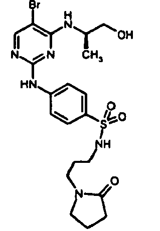 | 527,4413 | 527/529 |
| 469 | 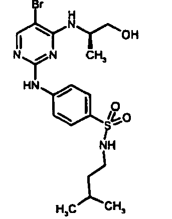 | 472,4054 | 472/474 |
| 470 | 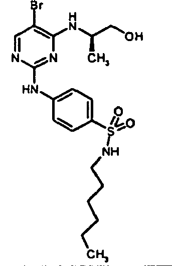 | 486,4322 | 486/488 |
| 471 | 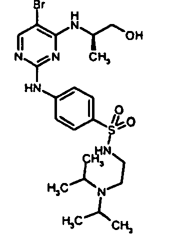 | 529,5007 | 529/531 |
| 472 | 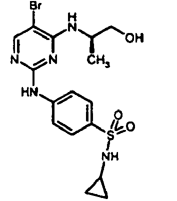 | 442,336 | 442/444 |
| 473 | 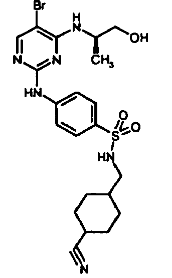 | 523,4532 | 523/525 |
| 474 | 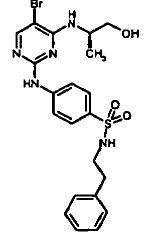 | 506,4226 | 506/508 |
| 475 | 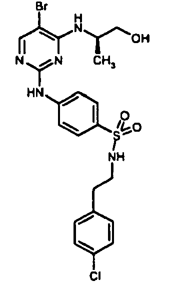 | 540,8677 | 540/542 |
| 476 | 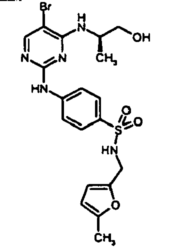 | 496,3838 | 496/498 |
| 477 | 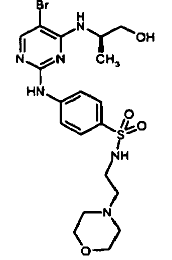 | 515,4303 | 515/517 |
| 478 | 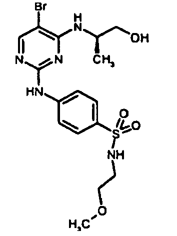 | 460,3508 | 460/462 |
| 479 | 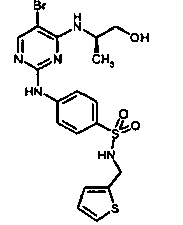 | 498,424 | 498/500 |
| 480 | 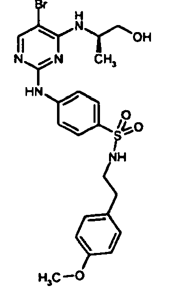 | 536,4484 | 536/538 |
| 481 | 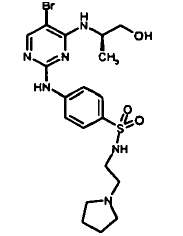 | 499,4313 | 499/501 |
| 482 | 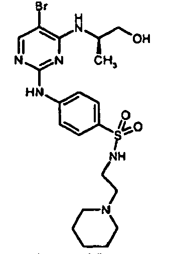 | 513,4581 | 513/515 |
| 483 | 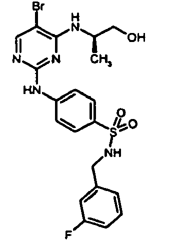 | 510,3859 | 510/512 |
| 484 | 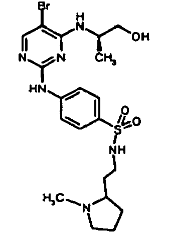 | 513,4581 | 513/515 |
| 485 | 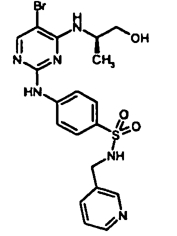 | 493,3839 | 493/495 |
| 486 | 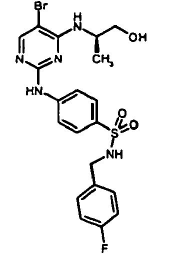 | 510,3859 | 510/512 |
| 487 | 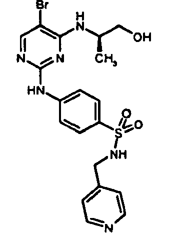 | 493,3839 | 493/495 |
| 488 | 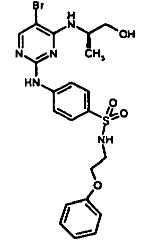 | 522,4216 | 522/524 |
| 489 | 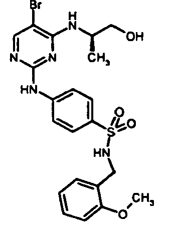 | 522,4216 | 522/524 |
| 490 | 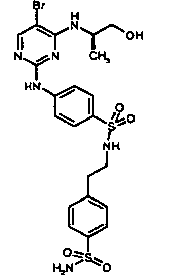 | 585,5015 | 585/587 |
| 491 | 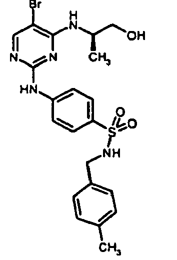 | 506,4226 | 506/508 |
| 492 | 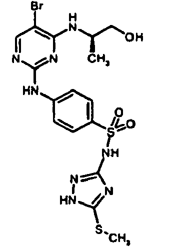 | 515,4151 | 515/517 |
| 493 | 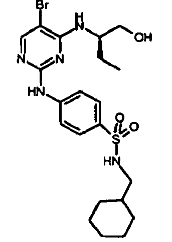 | 512,47 | |
| 494 | 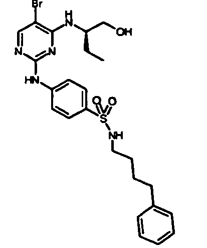 | 548,503 | |
| 495 | 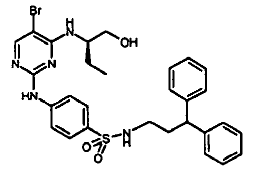 | 610,5738 | |
| 496 | 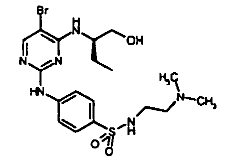 | 487,4203 | |
| 497 | 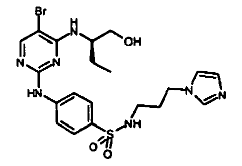 | 524,4414 | |
| 498 | 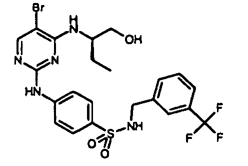 | 574,4197 | |
| 499 | 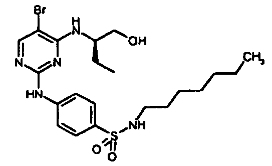 | 514,4858 | |
| 500 | 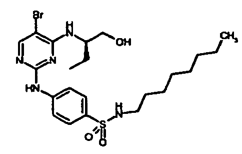 | 528,5126 | |
| 501 | 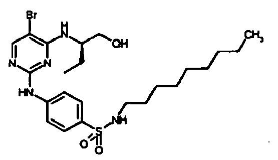 | 542,5394 | |
| 502 | 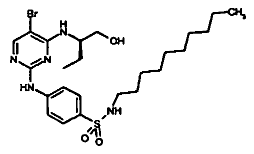 | 556,5662 | |
| 503 | 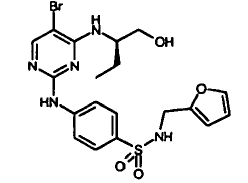 | 496,3838 | |
| 504 | 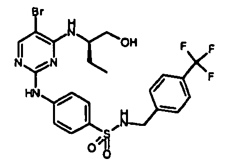 | 574,4197 | |
| 505 | 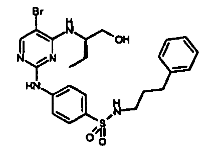 | 534,4762 | |
| 506 | 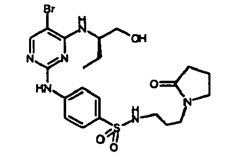 | 541,4681 | |
| 507 | 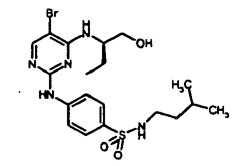 | 486,4322 | |
| 508 | 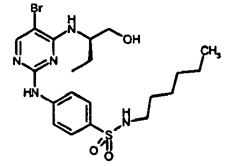 | 500,459 | |
| 509 | 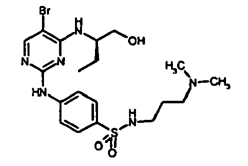 | 501,4471 | |
| 510 | 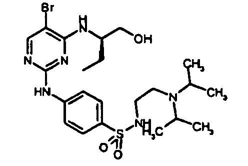 | 543,5275 | |
| 511 | 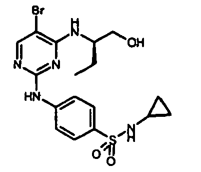 | 456,3628 | |
| 512 | 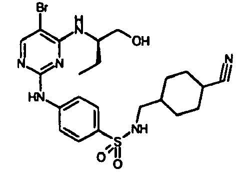 | 537,4801 | |
| 513 | 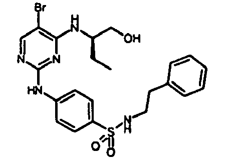 | 520,4494 | |
| 514 | 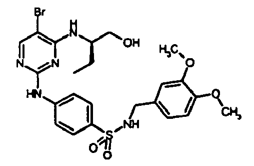 | 566,4742 | |
| 515 | 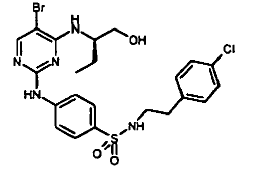 | 554,8945 | |
| 516 | 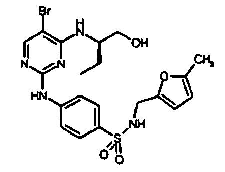 | 510,4106 | |
| 517 | 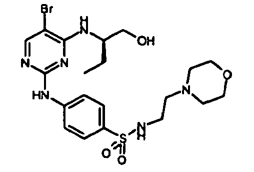 | 529,4571 | |
| 518 | 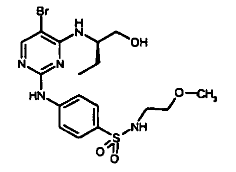 | 474,3776 | |
| 519 | 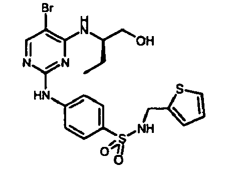 | 512,4508 | |
| 520 | 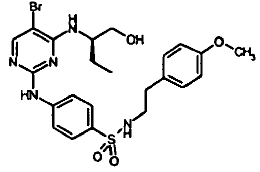 | 550,4752 | |
| 521 | 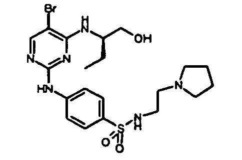 | 513,4581 | |
| 522 | 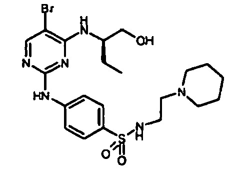 | 527,4849 | |
| 523 | 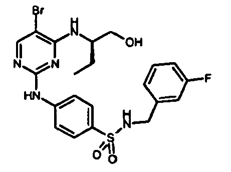 | 524,4127 | |
| 524 | 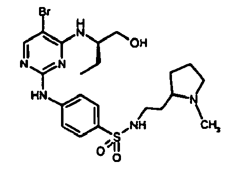 | 527,4849 | |
| 525 | 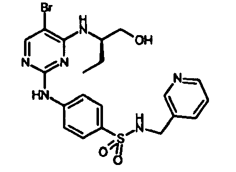 | 507,4107 | |
| 526 | 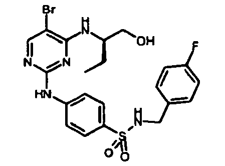 | 524,4127 | |
| 527 | 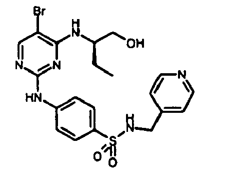 | 507,4107 | |
| 528 | 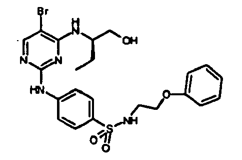 | 536,4484 | |
| 529 | 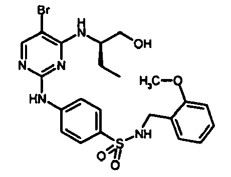 | 536,4484 | |
| 530 | 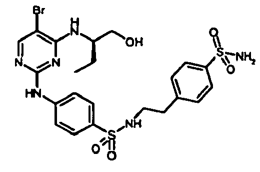 | 599,5283 | |
| 531 | 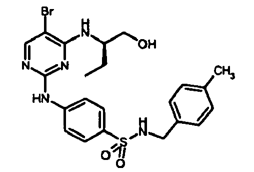 | 520,4494 | |
| 532 | 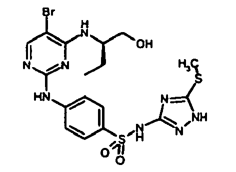 | 529,4419 | |

### Trennung von Diastereomerengemischen der erfindungsgemäßen Verbindungen

### Trennung am Beispiel des Diastereomerengemisches der Verbindung Nr. 274

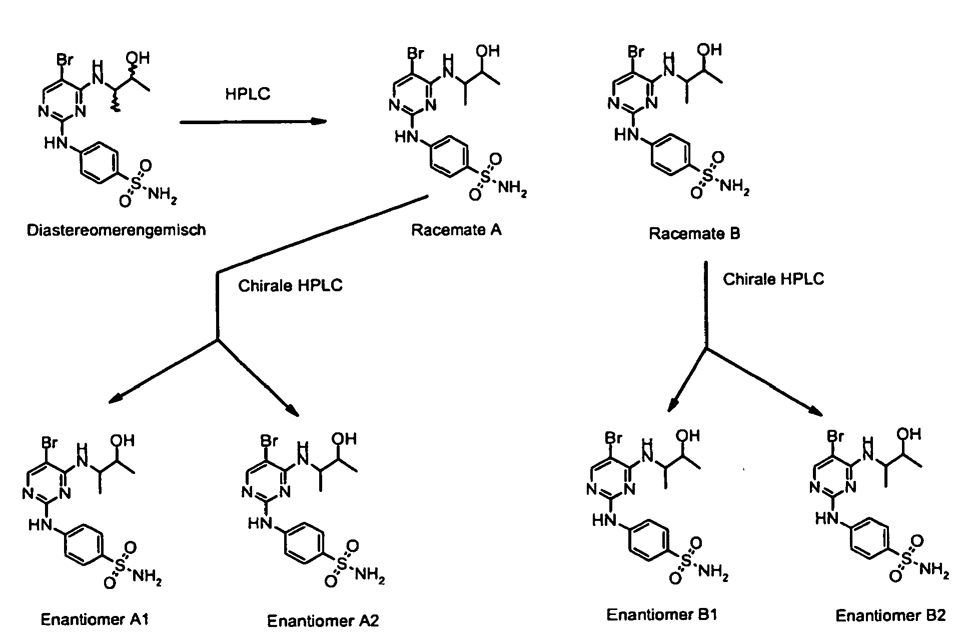

Das Diastereomerengemisch wurde in die beiden korrespondieren Racemate (A und B) mittels HPLC getrennt. Bedingungen:
- Säule:: Kromasil C18(5µm) 150x4,6mm
- Eluent:: 25% Acetonitril / Wasser mit 1 ml NH3/l;
- Fluß:: 1,0 ml/min
- Detektion:: PDA 300nm
- Retentionszeiten:: Racemate A - 11,6 min
Racemate B - 12,4 min

| | | |
|---|---|---|
| | 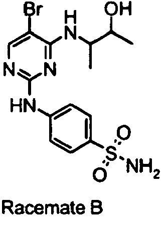 | 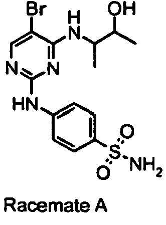 |

| NMR | DMSO-d6: | DMSO-d6: |
|---|---|---|
| | 9.68, s, 1 H | 9.68, s, 1 H |
| | 8.12, s, 1 H | 8.11, s, 1 H |
| | 7.87, d, 2 H | 7.85, d, 2 H |
| | 7.70, d, 2 H | 7.69, d, 2 H |
| | 7.14, s, 2 H | 7.16, s, 2 H |
| | 6.15, d, 1 H | 6.35, d, 1 H |
| | 5.01. d, 1 H | 4.90, d, 1 H |
| | 4.10, m, 1 H | 4.08, m, 1 H |
| | 3.80, m, 1 H | 3.80, m, 1 H |
| | 1.22, d, 3 H | 1.18, d, 3 H |
| | 1.1, d, 3 H | 1.12, d, 3 H |

Nachfolgend wurden die Racemate A und B jeweils mittels chiraler HPLC getrennt.
Bedingungen:
- Säule:: Chiralpak AD(10µm) 250x4,6mm
- Eluent:: Hexan/ Ethanol 80:20
- Fluß:: 1,0 ml/min
- Detektion:: PDA 300nm
- Retentionszeiten:: Enantiomer A1 - 16,6 min
Enantiomer A2 - 19,6 min
Enantiomer B1 - 16,0 min
Enantiomer B2 - 17,8 min

Herstellung der für die Synthese der erfindungsgemäßen Verbindungen der allgemeinen Formel I vorzugsweise verwendeten Zwischenstufen.

### Beispiel 1.0

### Herstellung von N-(2-Chlor-5-fluor-4-pyrimidinyl)-N-2-propynylamin

11,1g (66 mmol) 2,4-Dichlor-5-fluorpyrimidin werden in 60 ml Acetonitril gelöst, 10,2 ml (73 mmol) Triäthylamin und 6,0 ml (86 mmol) Propynylamin werden hinzugegeben. Das Reaktionsgemisch wird bei Raumtemperatur über Nacht gerührt und anschließend in Wasser gegossen. Die Mischung wird mittels Ethylacetat extrahiert, die kombinierten organischen Phasen werden über MgSO₂ getrocknet und das Lösemittel wird mittels Unterdruck evaporiert. Nach Umkristallisierung des verbleibenden Materials mit Diisopropylether / Hexan, beträgt die Ausbeute 10.6 g (87% der Theorie) des Produktes.

| | | | |
|---|---|---|---|
| 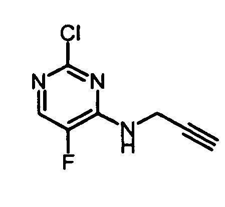 | 5-H | 8.18 (3.3Hz,1 H) | Lösemittel: DMSO |
| | 4CH | 4.14 (dd,2H) | Ausbeute: 87% |
| | | 3.20 (t, 1 H) | Schmelzpunkt: 96°C |
| | NH | 8.65 (tb, 1H) | |

Die nachfolgenden beschriebenen 4-(Diaminocyclohexyl)-Derivate werden über reduktive Aminierungen des beschriebenen Keto-Derivates unter Verwendung von Triacetoxyborhydrid (Abdel-Magid, Carson, Harris, Maryanoff, Sha, J. Org. Chem. 1996, 61, 3849) synthetisiert. Das Keto-Derivat wird durch TPAP-Oxidation (Griffith, Ley, Aldrichimica Acta 1990, 23, 13) des entsprechenden Alkohols erhalten.

In analoger Verfahrensweise werden auch folgende Zwischenverbindungen hergestellt.

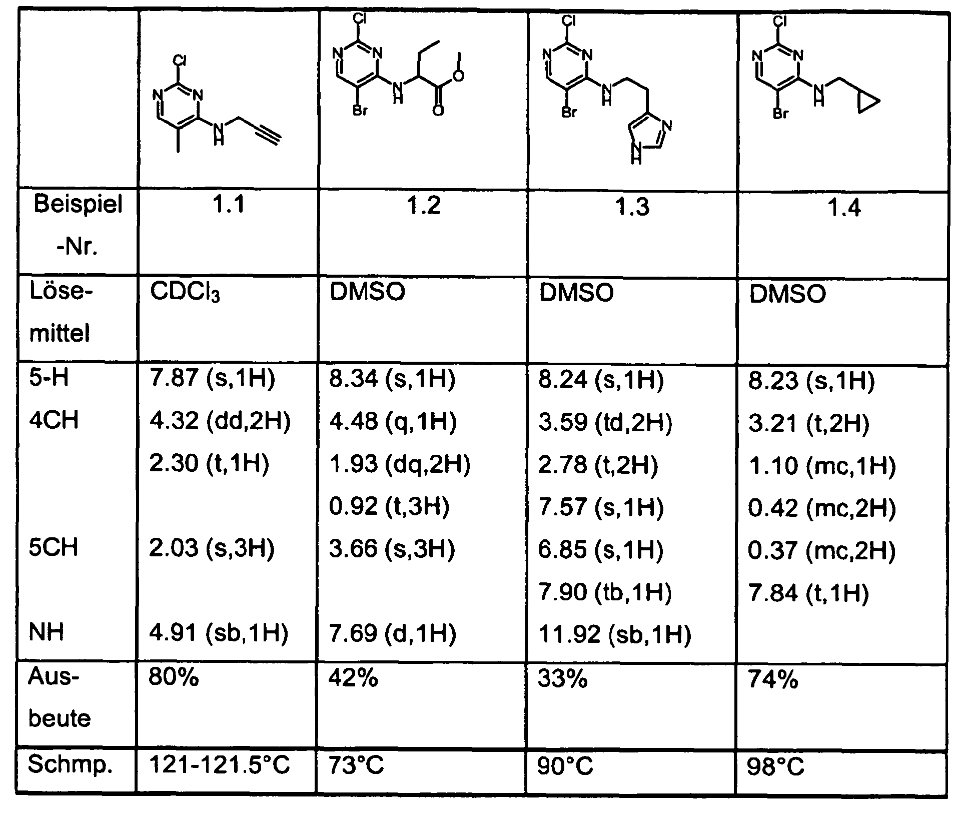

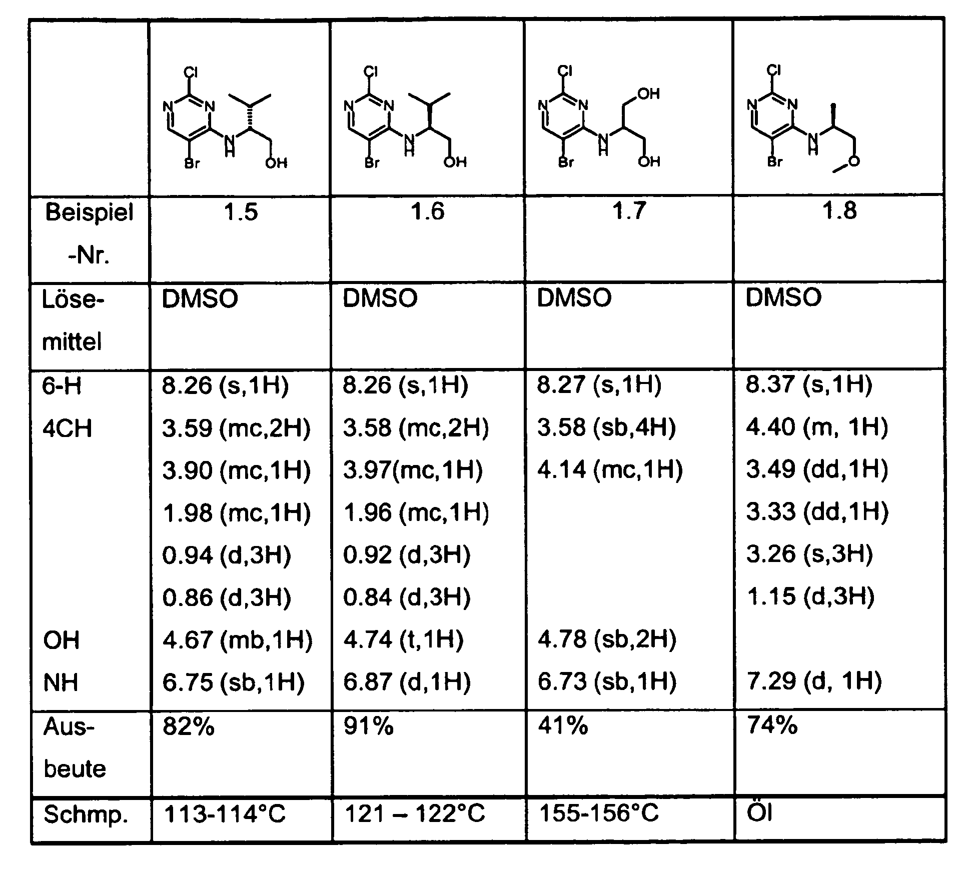

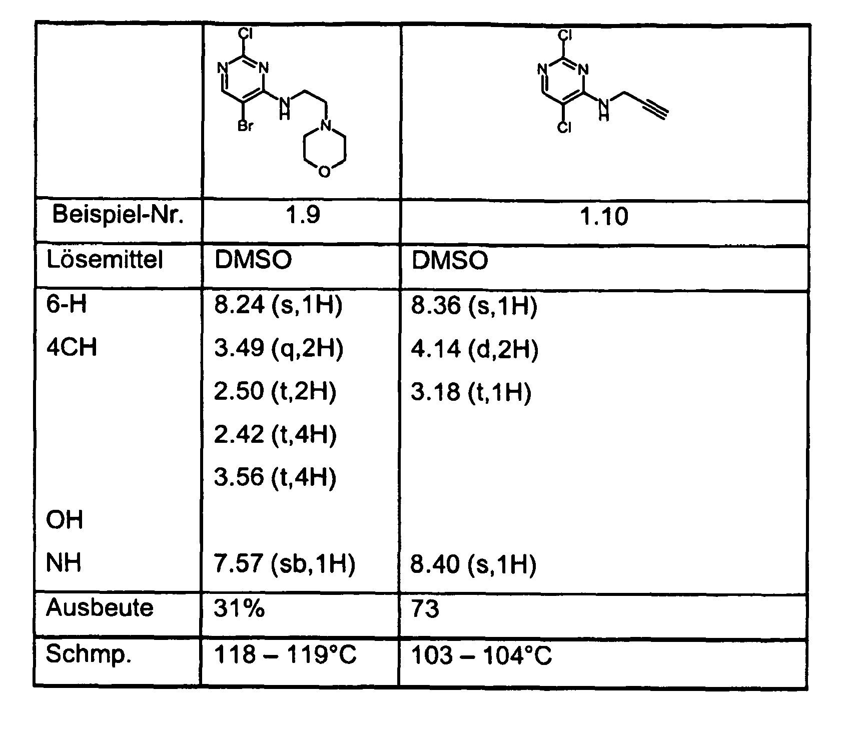

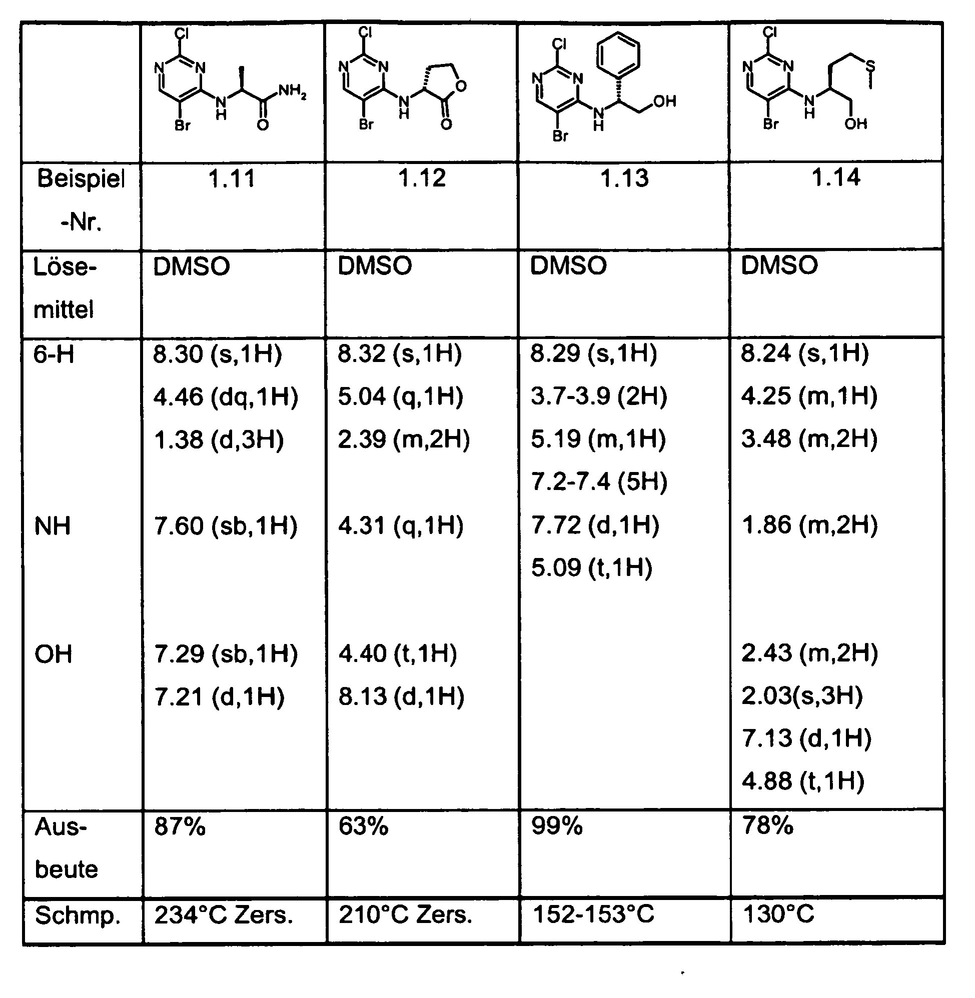

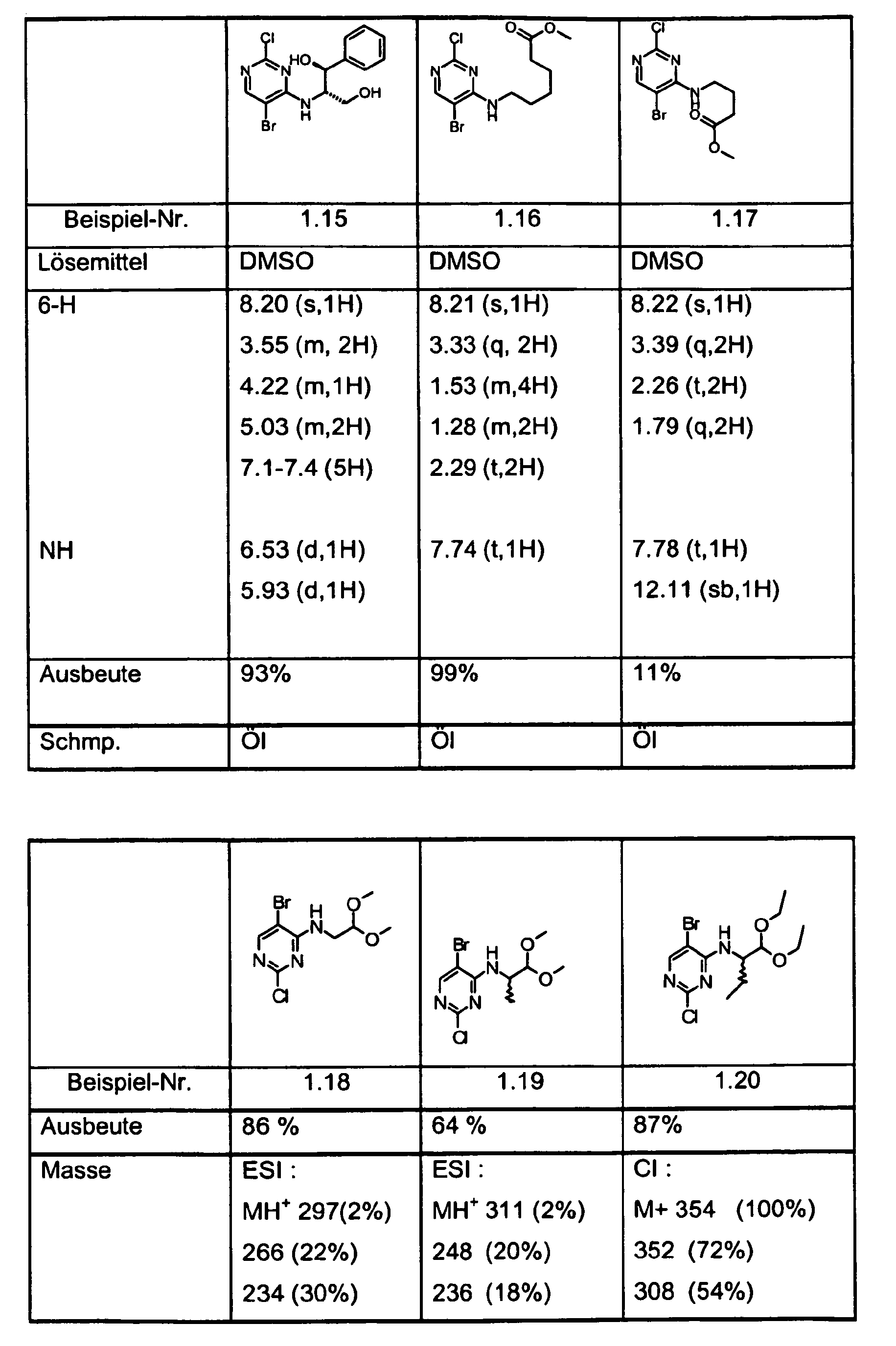

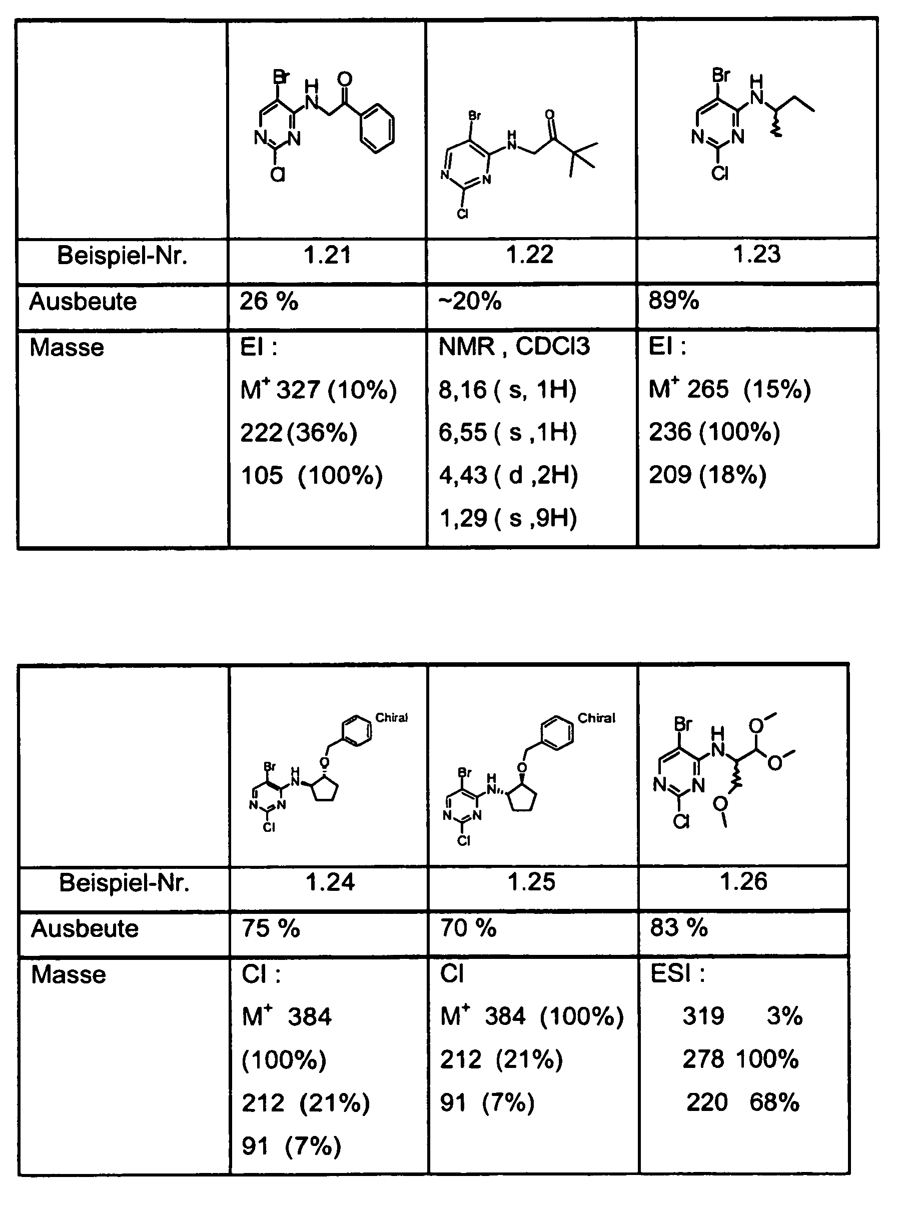

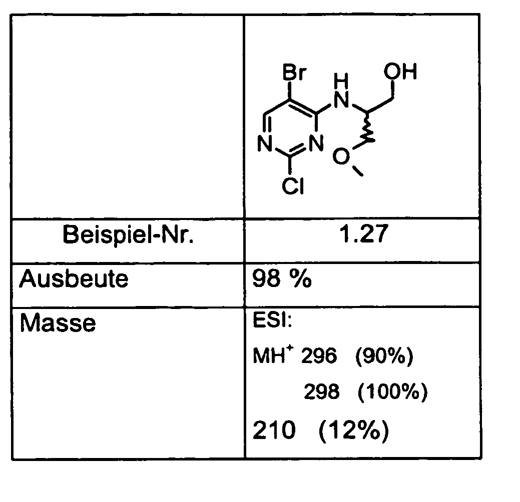

### Beispiel 2.0

### Herstellung von 5-Brom-2-chlor-4-(4,4,4-trifluorbutoxy)pyrimidin

3,19 g (14 mmol) 5-Brom-2,4-dichlorpyrimidin werden mit 8,06 g (63 mmol) 4,4,4-trifluorbutanol gemischt und 0,74 ml (8.4 mmol) Trifluormethansulfonsäure werden langsam dazu gegeben. Das Reaktionsgemisch wird bei Raumtemperatur über Nacht gerührt und anschließend in Wasser gegossen. Die Mischung wird mittels Ethylacetat extrahiert, die kombinierten organischen Phasen werden über MgSO₂ getrocknet und das Lösemittel wird mittels Unterdruck evaporiert. Das Produkt ist immer mit variierenden Mengen 2,4-Bisalkoxypyrimidin kontaminiert. Deshalb wird das verbleibende Material mittels Gradientenchromatographie mit Kieselgel als Trägermaterial (Eluent: Hexan und Hexan/ethlyacetat im Verhältnis 9:1) gereinigt. Dieses Verfahren führt zu einer Ausbeute von 1,70 g (38%) und liefert ebenfalls 1,93 g (34%) an 5-Brom-2,4-bis-(4,4,4-Trifluorbutoxy)pyrimidin (Ausgangsverbindung).

| | | | |
|---|---|---|---|
| 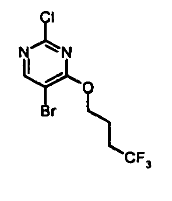 | 5-H | 8.74 (s,1 H) | Chromatographie: H bis H/EA 9:1 |
| | 4C | 4.48 (t,2H) | Ausbeute: 38% |
| | H | 2.00 (mc,2H) | Schmelzpunkt: 66.5 - 67.5°C |
| | | 2.44 (mc,2H) | |
| | 5C | | |
| | H | | |

In analoger Verfahrenweise werden auch die folgenden Verbindungen hergestellt:

| | | |
|---|---|---|
| | 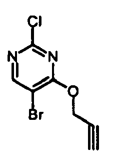 | 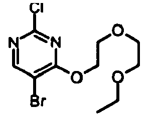 |

| Beispiel- Nr. | 2.1 | 2.2 |
|---|---|---|
| | CDCl₃ | DMSO |
| 5-H | 8.49 (s, 1H) | 8.75 (s, 1H) |
| 4CH | 5.10 (d,2H) | 4.05 (mc,2H) |
| | | 3.79 (mc,2H) |
| | | 3.60 (mc,2H) |
| 5CH | 2.59 (t, 1H) | 3.48 (mc,2H) |
| | | 3.40 (t,2H) |
| | | 1.07 (t,3H) |
| Chrom. | H to | DCM to DCM/ |
| | H/EA 4:1 | MeOH 95:5 |
| Ausbeute | 78% | 11% |
| Schmp. | 55°C | Öl |

Analog zu den Verfahrensbeispielen 1 und 2 werden auch folgende Zwischenprodukte hergestellt:

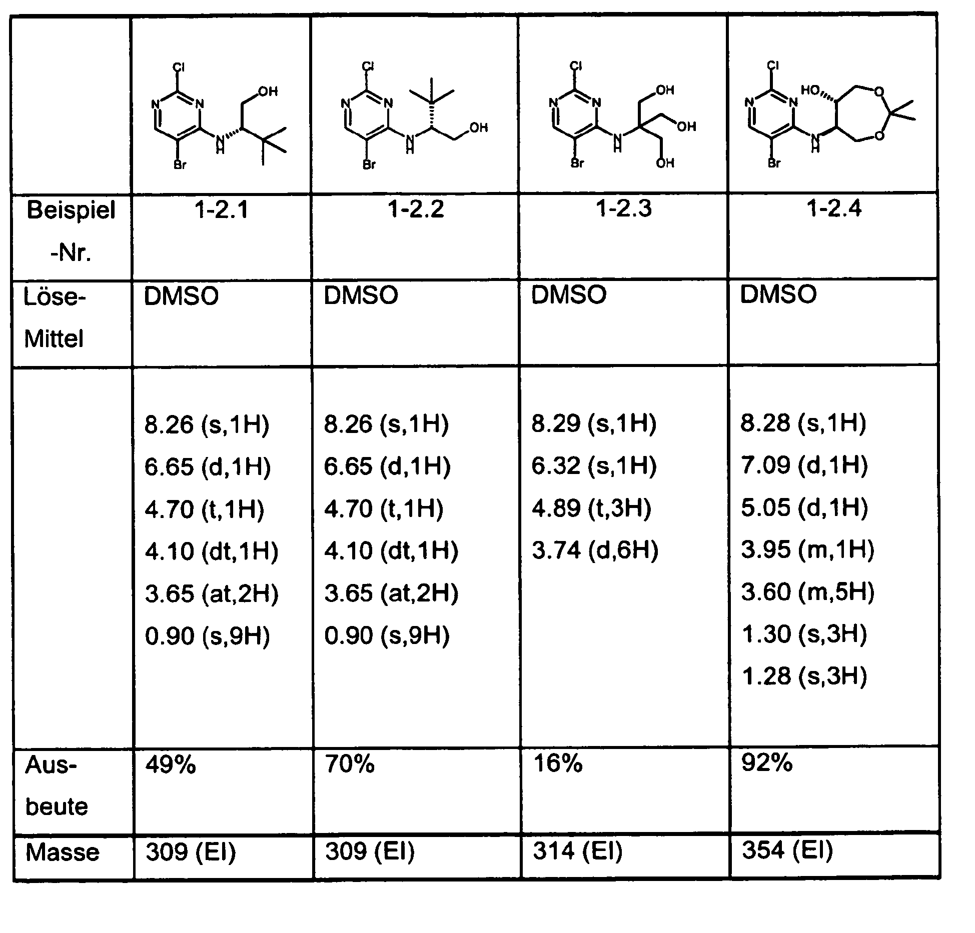

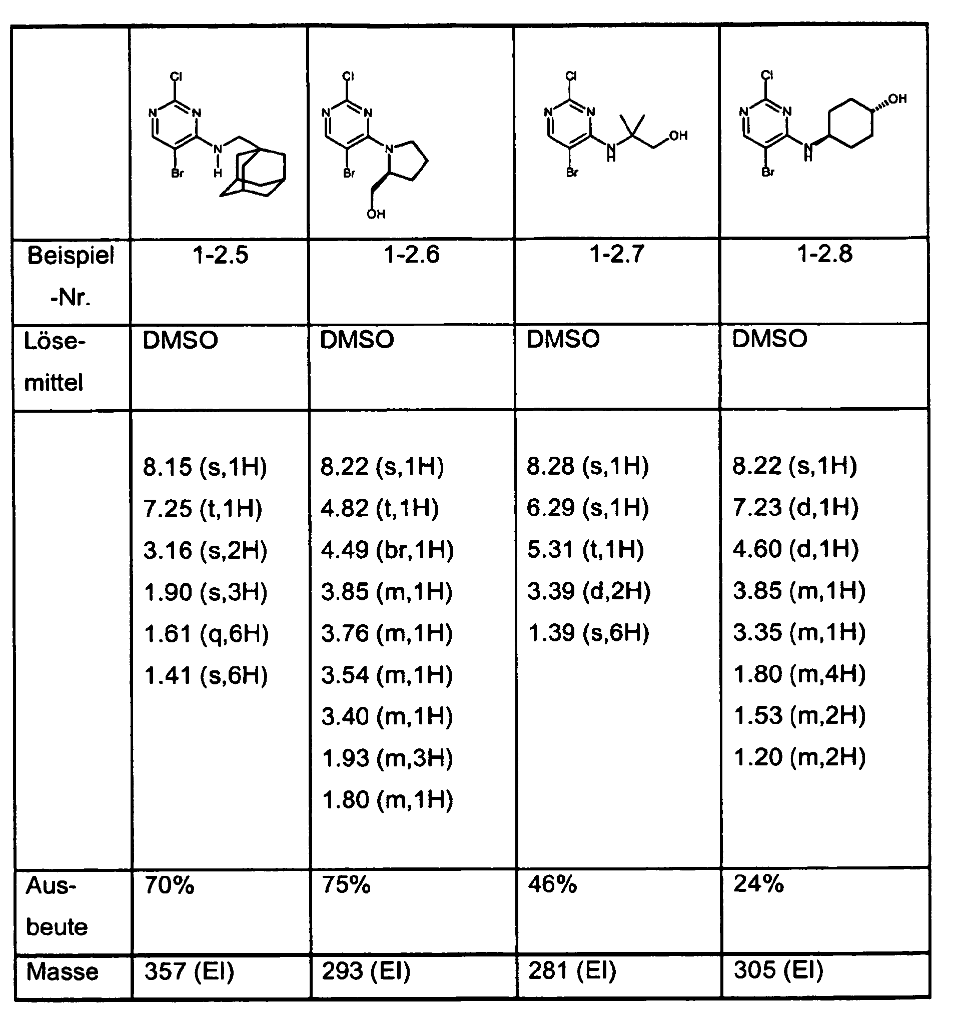

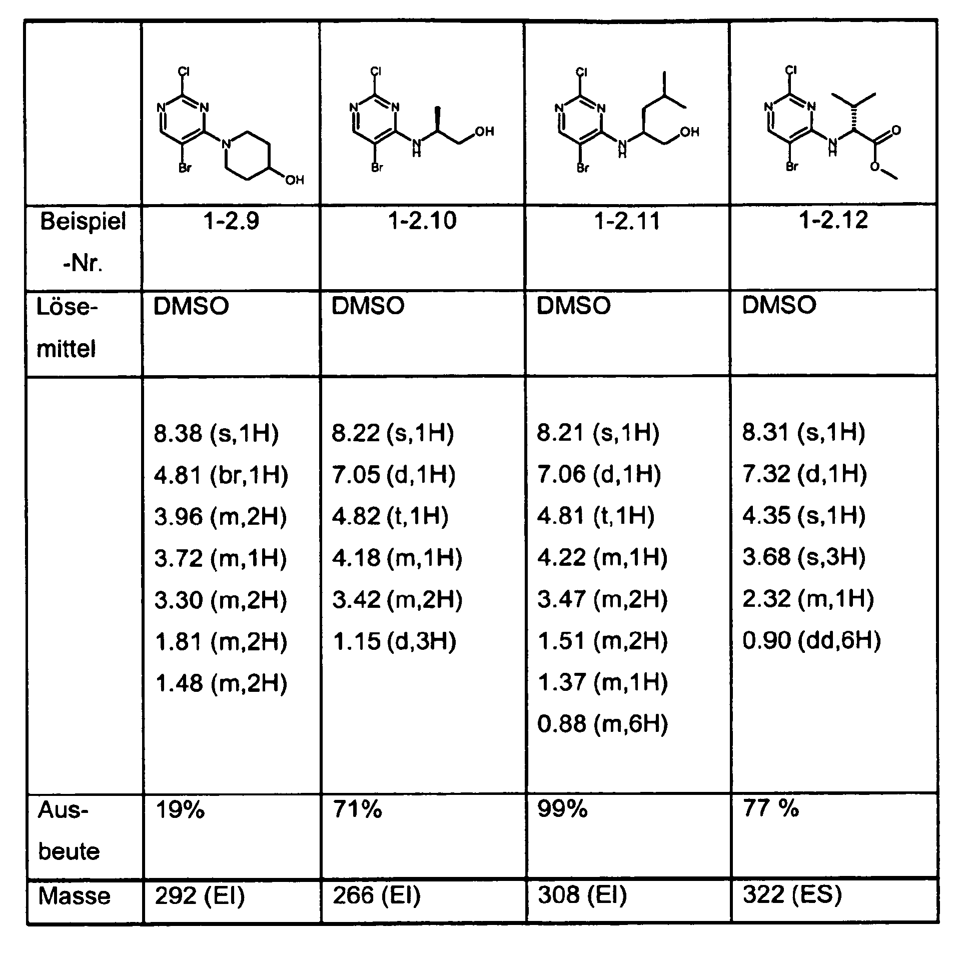

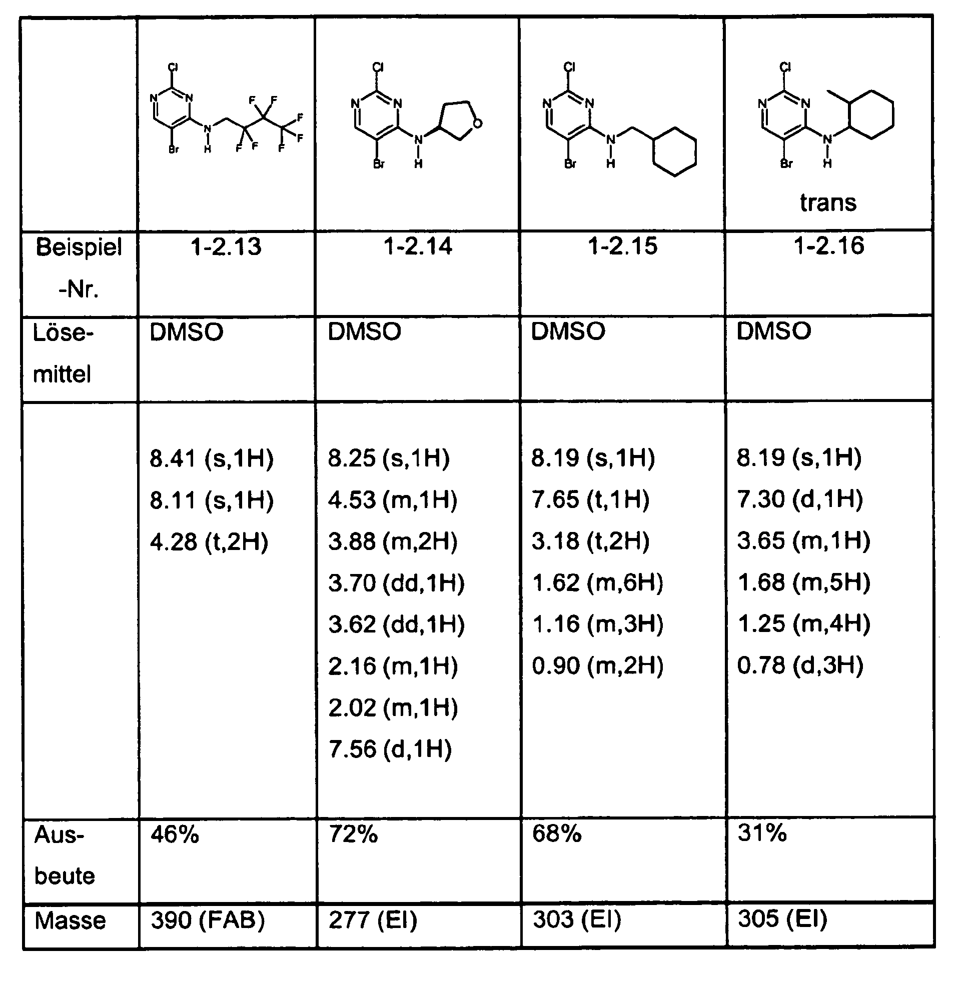

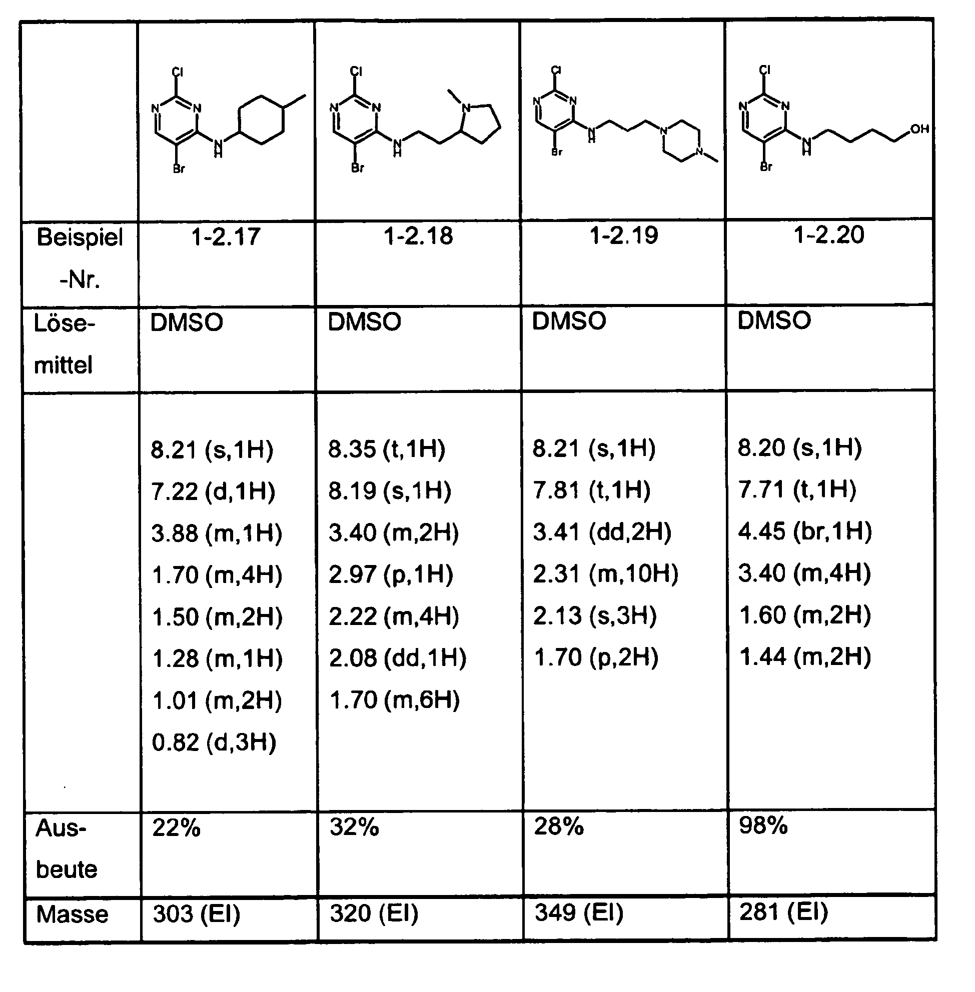

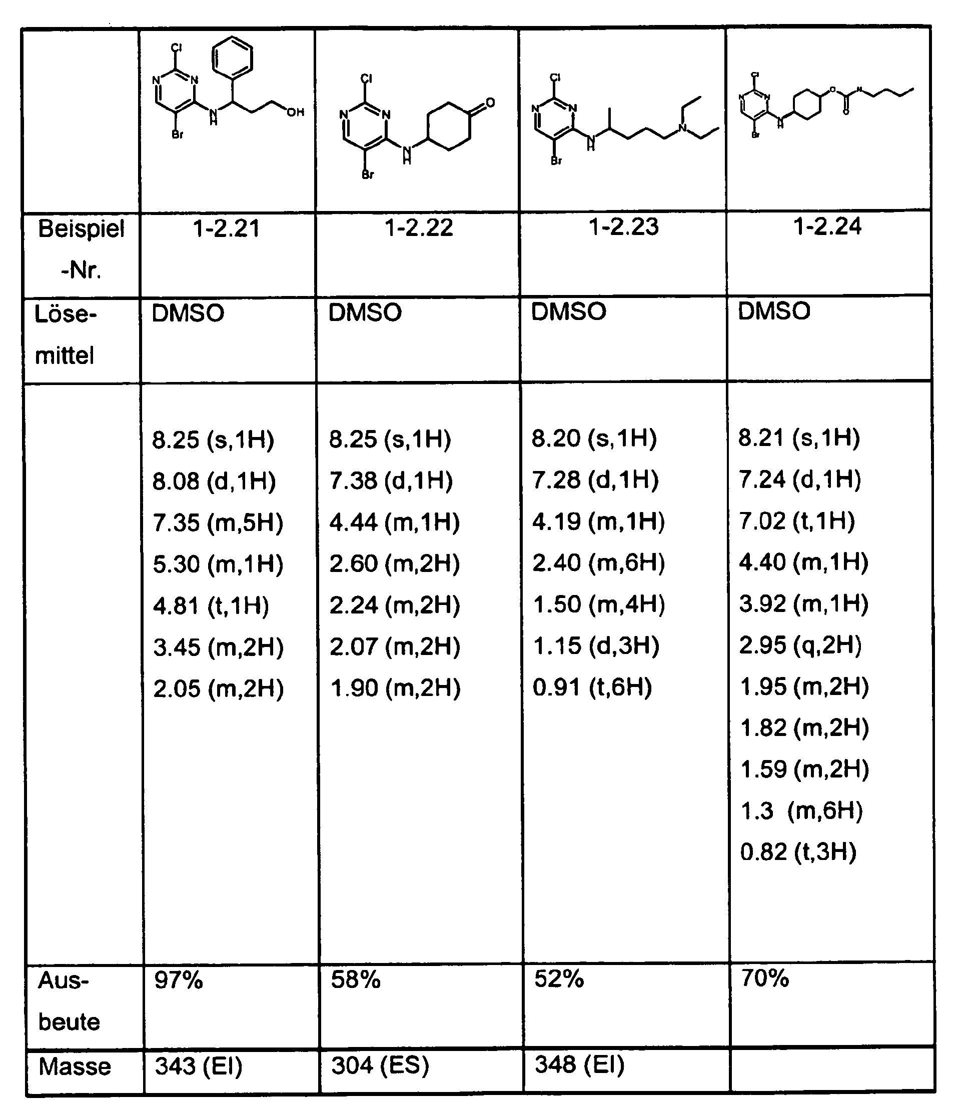

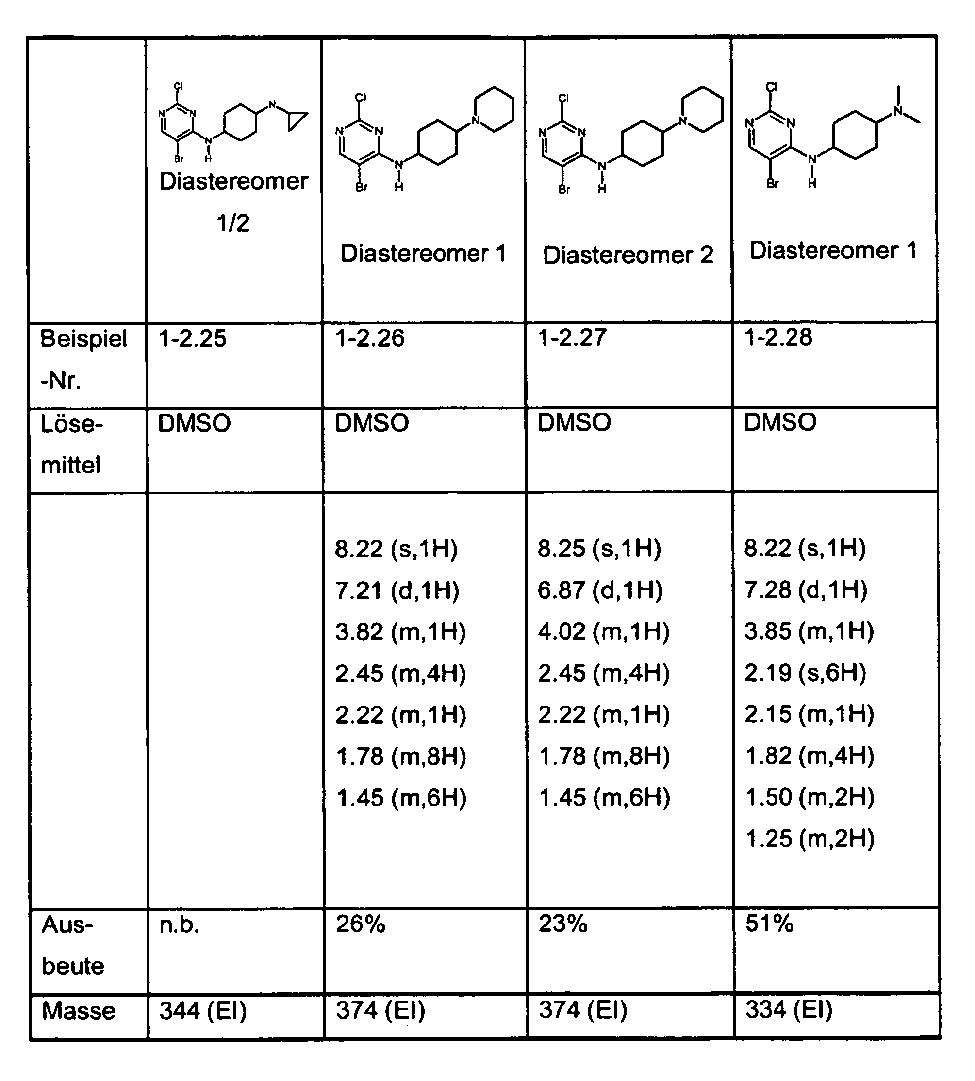

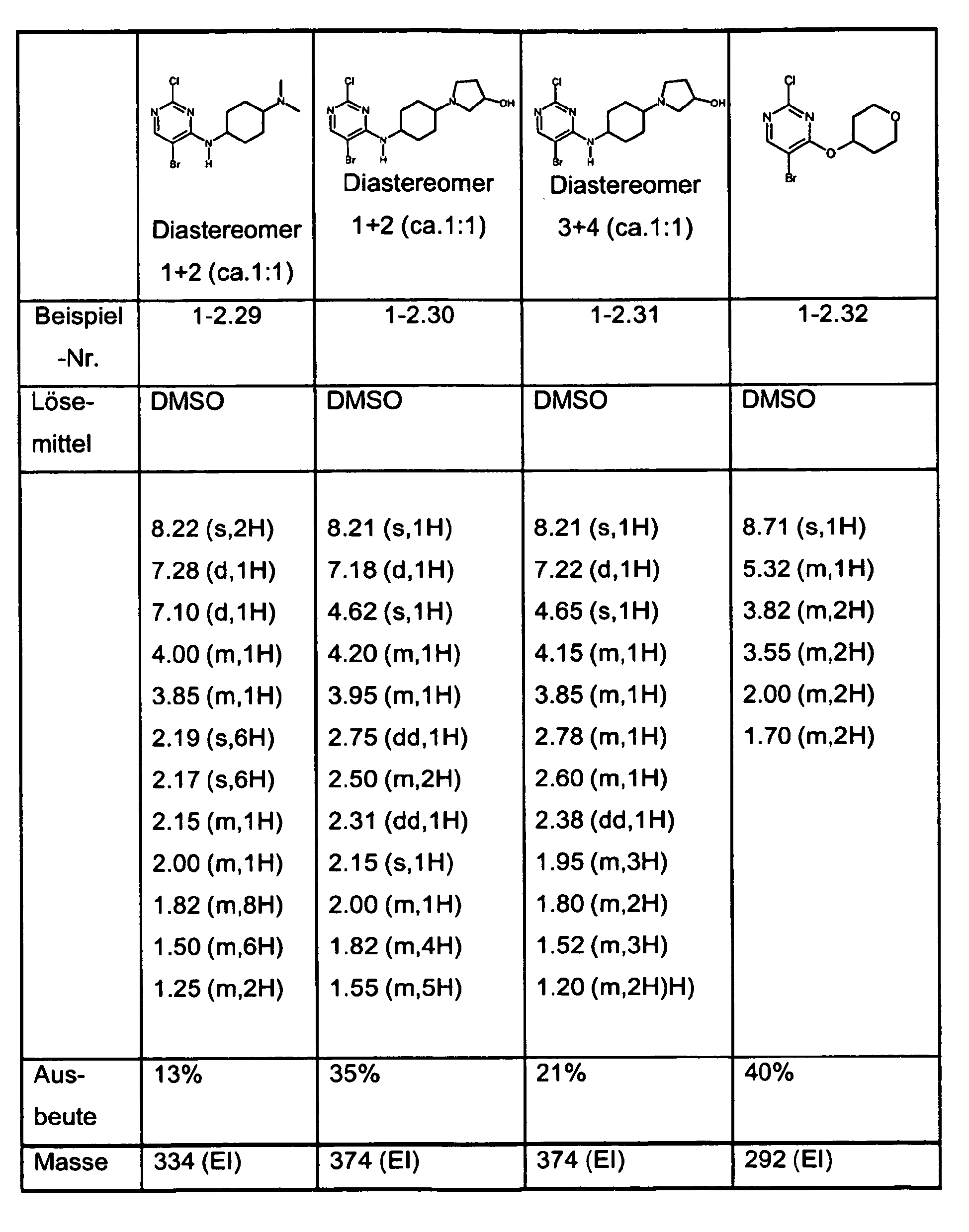

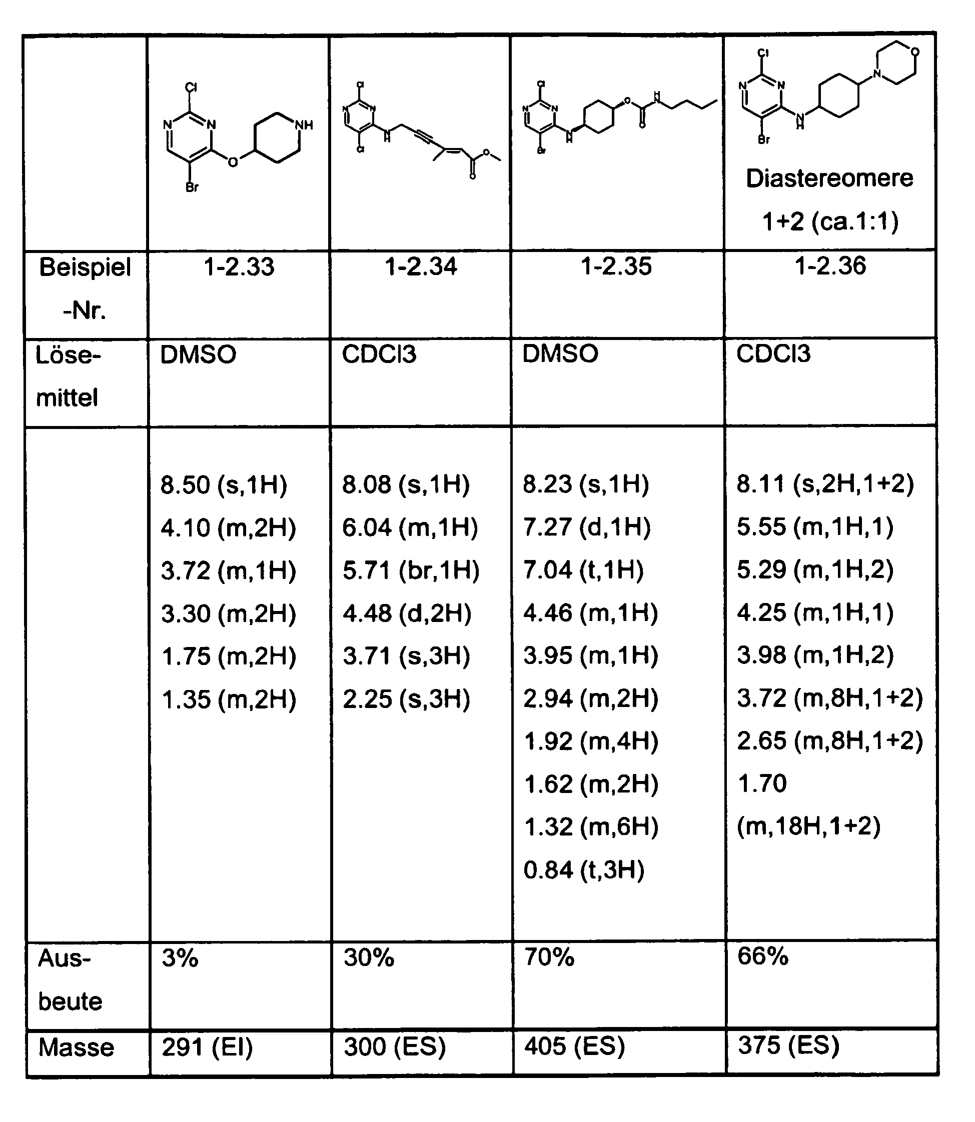

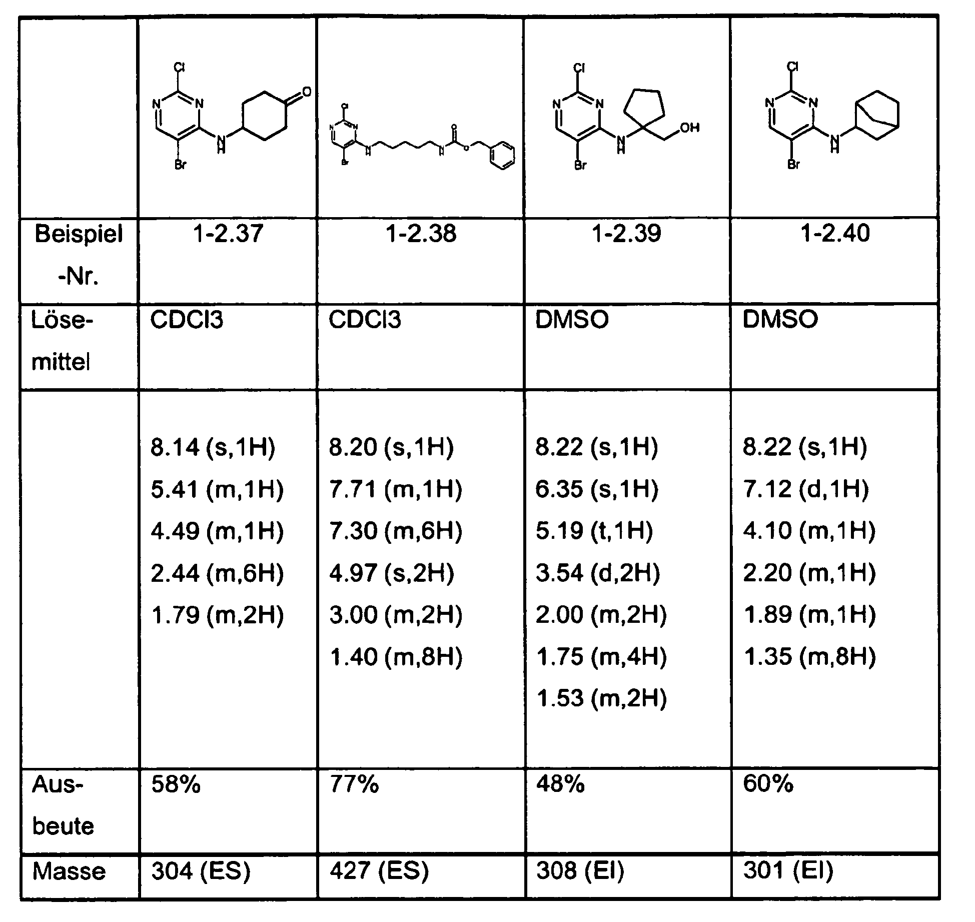

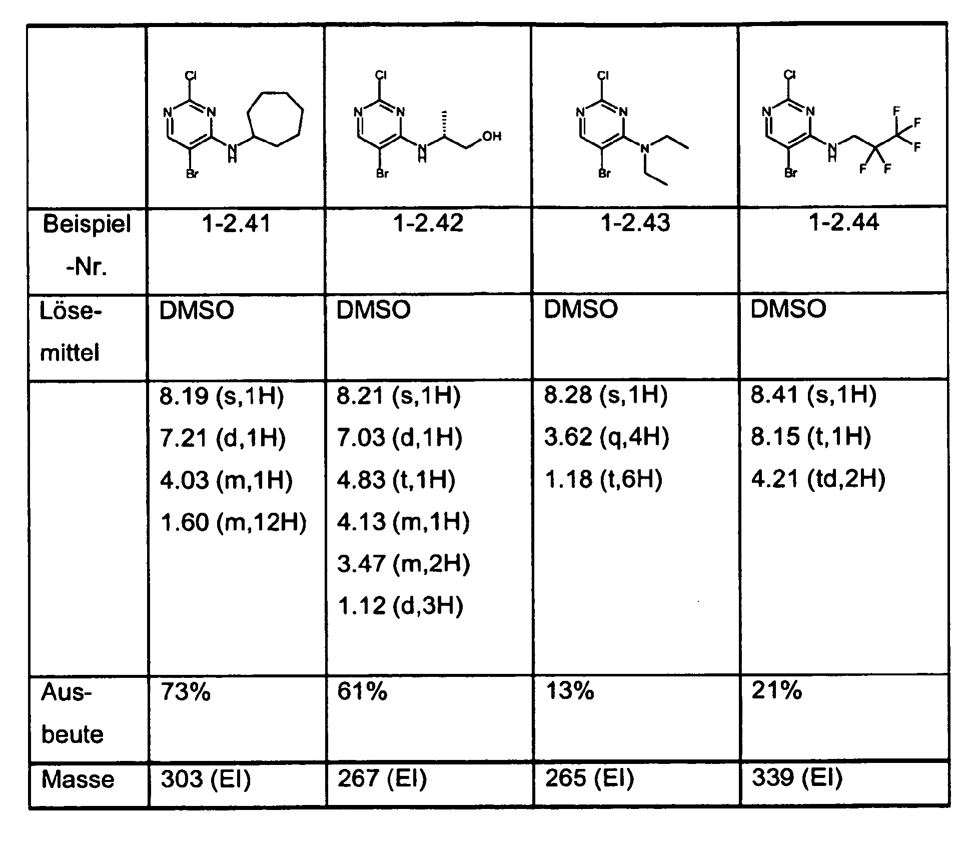

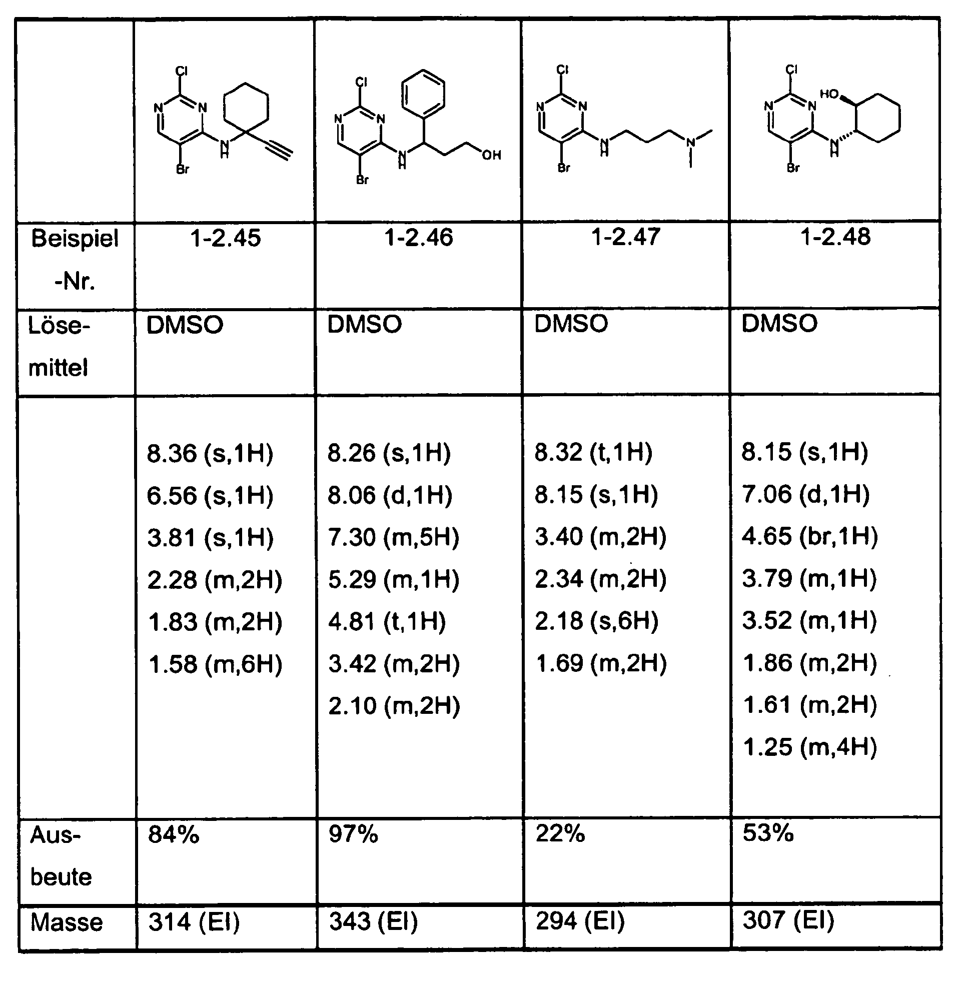

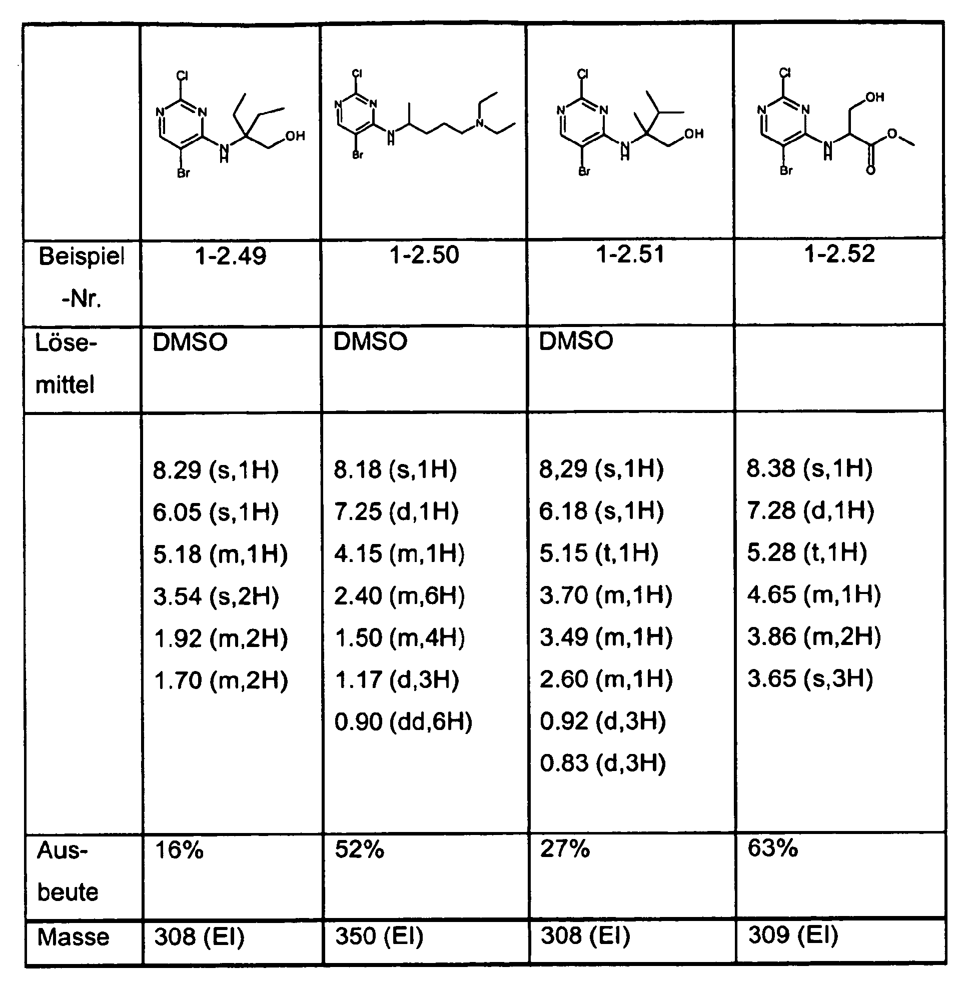

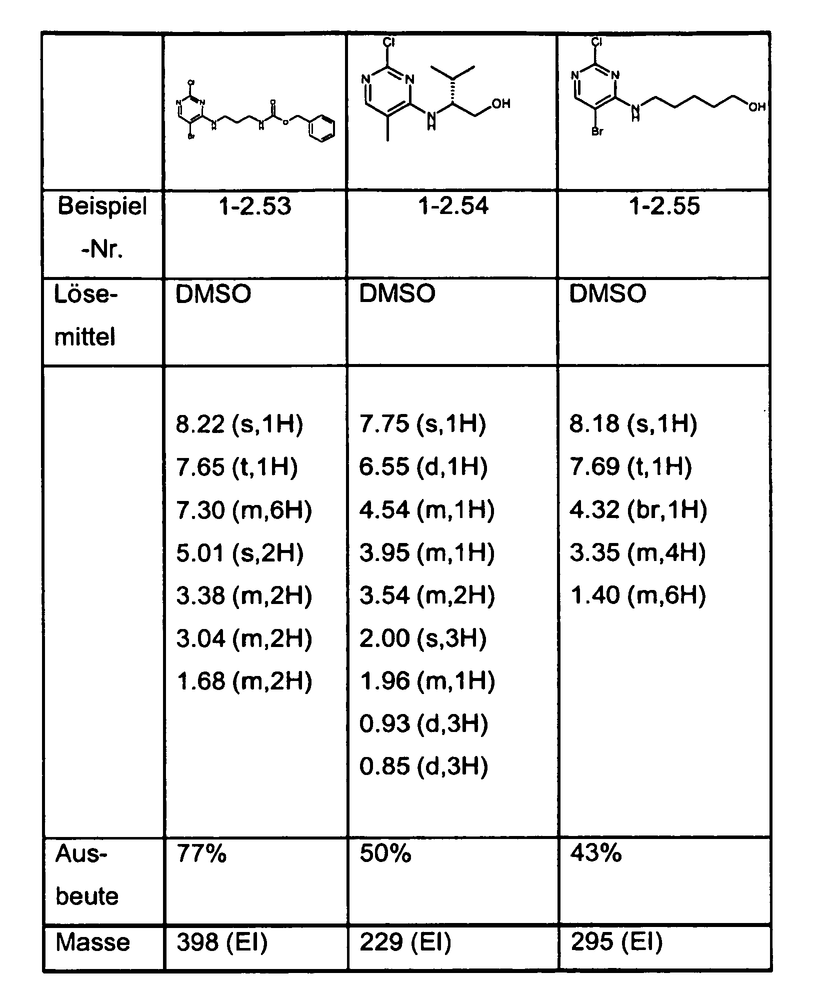

### Beispiel 3.0

### Herstellung der Amine

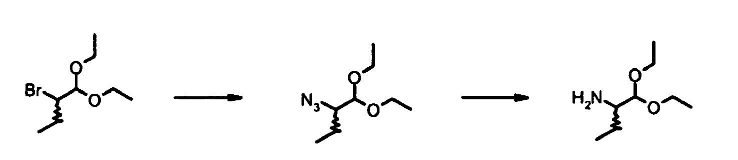

4,5 g (20 mmol) 2-Brombutyraldehyddiethylacetal (Fa. Pfaltz-Bauer) und 5,2 g (80 mmol) Natriumazid werden 5 Tage in 15 ml DMF bei 100°C gerührt. Dann wird auf kalte verdünnte Natriumhydrogencarbonatlösung gegossen, 3x mit Ether extrahiert, die org. Phase mit Magnesiumsulfat getrocknet und eingeengt: Rohausbeute 1,87 g (50% d.Th.).
936 mg des Rohproduktes werden in 50 ml Methanol gelöst, mit Palladium auf Kohle (10%ig) versetzt und 12 Stunden unter H₂-Atmossphäre gerührt. Nach Abfiltrieren des Katalysators und Einengen verbleiben 457mg (57% der Theorie) des gewünschten Amins.

| | | | | |
|---|---|---|---|---|
| | 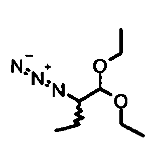 | 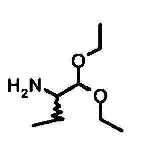 | 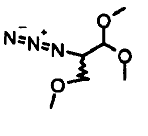 | 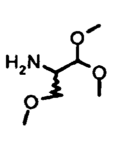 |

| Beispiel- Nr. | 3.0 | 3.1 | 3.2 | 3.3 |
|---|---|---|---|---|
| Ausbeute | 50% | 57% | 50 % | 71 % |
| NMR | 4,38 ( d, 1H ) | 4,19 ( d, 1H ) | 4,38 ( d, 1H) | 4,25 (d, 1H) |
| CDCl3 | 3,72 ( m , 2H ) | 3,68 ( m , 2H ) | 3,58 ( m , 2H) | 3,5 ( m , 1H) |
| | 3,6 ( m , 2H ) | 3,52 ( m, 2H ) | 3,5 ( m, 1H) | 3,42 (s, 3H) |
| | 3,25 ( m , 1H ) | 2,7 ( m , 1H ) | 3,49 ( s , 3H) | 3,41 ( s , 3H) |
| | 1,7 ( m , 1H ) | 1,60 ( m, 1H ) | 3,43 (s , 3H) | 3,40 ( m , 1H) |
| | 1,46 ( m , 1H ) | 1,25 ( m, 1H ) | 3,39 ( s, 3H) | 3,08 ( m, 1H) |
| | 1,25 ( trtr, 6H) | 1,2 ( trtr , 6H ) | | |
| | 1,0 ( tr , 3H ) | 0,95 ( tr , 3H ) | | |

### Beispiel 4.0

### Herstellung der freien Aldehyde

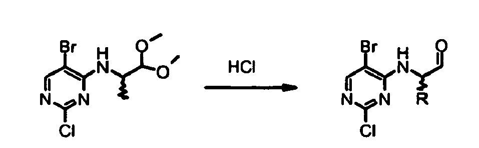

148 mg 0,5 mMol der Zwischenprodukt-Verbindung 1.18 werden in 1 ml Eisessig gelöst. Bei Raumtemperatur gibt man 0,5 ml 1N Salzsäure hinzu und rührt 12 Stunden. Zur Aufarbeitung wird auf Eiswasser gegossen und vorsichtig mit pulverisiertem Natriumhydrogencarbonat neutralisiert. Dann wird 3 mal mit

Essigester extrahiert, die org. Phase mit Magnesiumsulfat getrocknet und eingeengt. Rohprodukt 104 mg (83% der Theorie) des Aldehyds der Verbindung 4.0. Das Rohprodukt kann ohne weitere Reinigung eingesetzt werden.

| | | | | |
|---|---|---|---|---|
| | 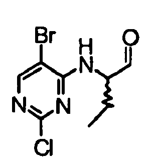 | 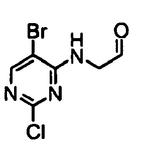 | 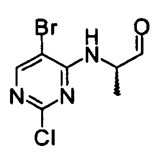 | 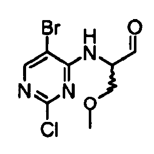 |

| Beispiel -Nr. | 4.1 | 4.0 | 4.2 | 4.3 |
|---|---|---|---|---|
| Ausbeute | 82 % | 83 % | 89 % | 79 % |
| Masse | ESI : | ESI : | ESI: | ESI: |
| | MH⁺ 278 | MH⁺ 250 | MH⁺ 266 | MH⁺ 294 |
| | (39%) | (9%) | (8%) | (10%) |
| | 210 (100%) | | | |

### Beispiel 5.0

### Herstellung der Ketone

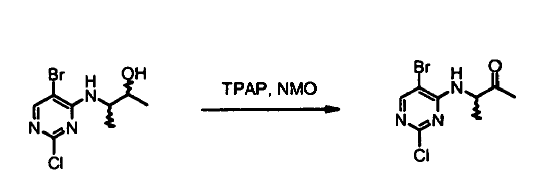

100 mg (0,356 mMol) der Verbindung 6.0 und126 mg N-Methylmorpholin-N-oxid werden in 5 ml Dichlormethan gelöst, und 10 min. mit pulverisiertem Molsieb (4 A) gerührt. Dann gibt man 6 mg Tetrapropylammoniumperruthenat hinzu und rührt 4 Stunden bei Raumtemperatur nach. Nach Einengen wird über Kieselgel chromatographiert (Hexan/Essigester 4:1 > 2:1). Ausbeute: 75 mg (76% derTheorie) des Ketons der Verbindung 5.0.

| | |
|---|---|
| | 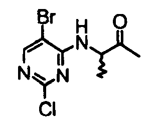 |

| Beispiel -Nr. | 5.0 |
|---|---|
| Ausbeute | 76% |
| Masse | ESI: |
| | MH⁺ 280 |
| | (100%) |
| | 200 (37%) |
| | 156 (30%) |

### Beispiel 6.0

### Herstellung der Alkohole

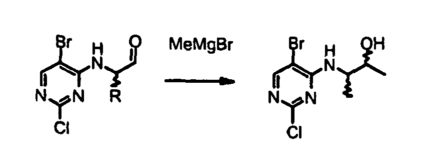

265 mg (1 mMol ) der Verbindung 4.2 werden in 20 ml Tetrahydrofuran gelöst. Unter Eisbadkühlung werden 5 Equivalente Methylmagnesiumbromid (3 molare Lösung in Ether) portionsweise hinzugegeben. Dann wird 3 Stunden bei Raumtemperatur nachgerührt und anschließend unter Kühlung mit Wasser gequencht. Dann wird mit Ammoniumchloridlösung versetzt, 3 mal mit Essigester extrahiert, die organische Phase mit Magnesiumsulfat getrocknet und einengt. Flashchromatographie (Hexan/Essigester 2:1) ergibt 213 mg (76% der Theorie) des Alkohols der Verbindung 6.0. 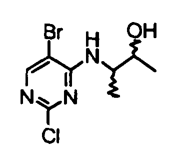 ESI : MH⁺ 282 (100%) 276 (5%)

In analoger Verfahrensweise werden auch folgende Zwischenprodukte hergestellt:

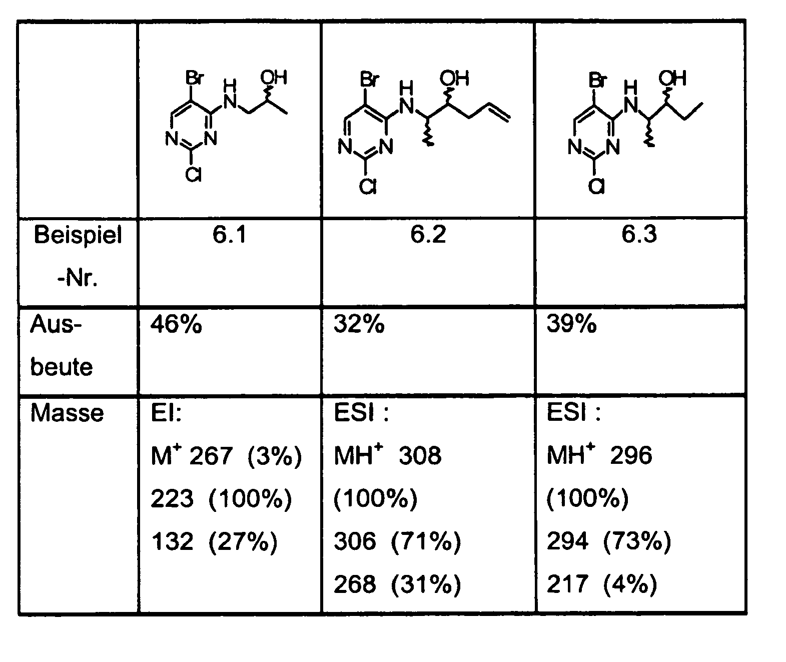

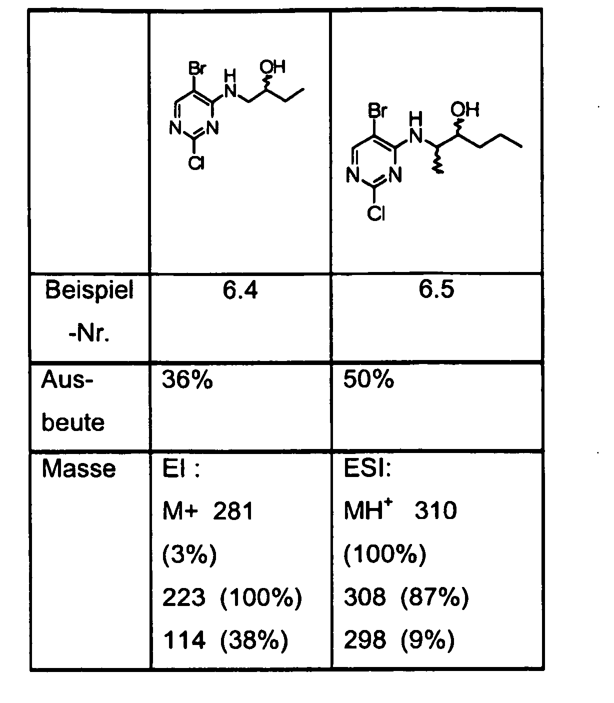

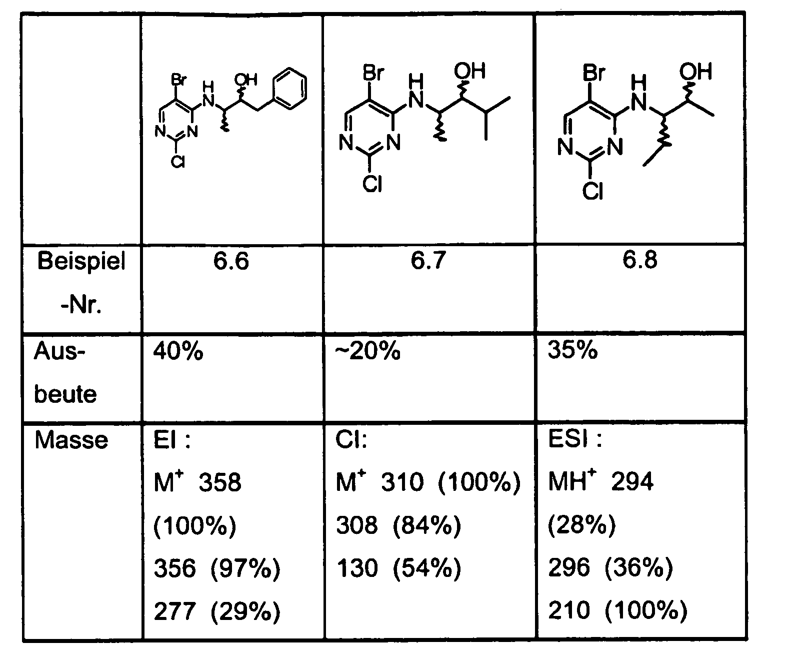

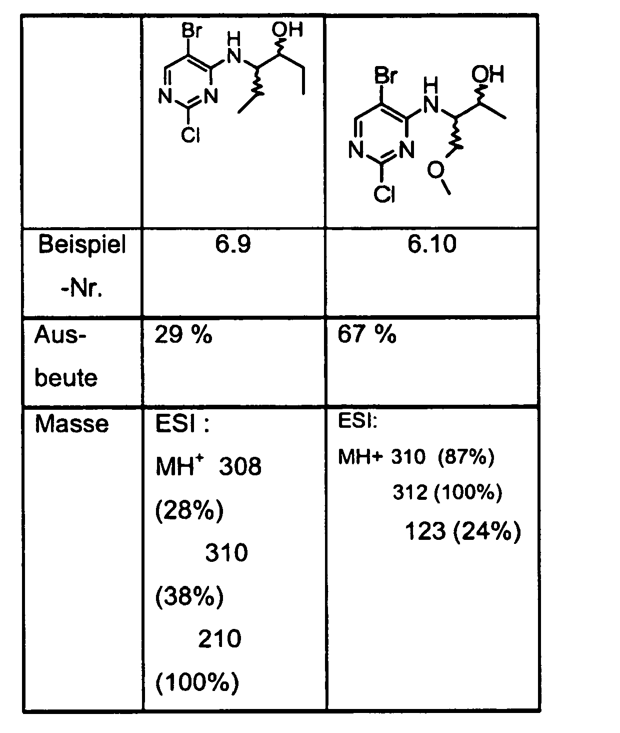

Gegenstand der vorliegenden Erfindung sind somit auch Verbindungen der allgemeinen Formel la 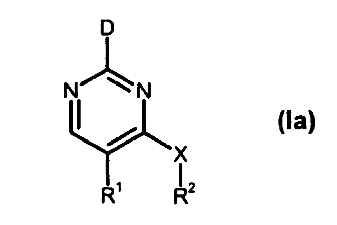 in der
D für Halogen, und X, R¹, und R² die in der allgemeinen Formel (I) angegebenen Bedeutungen haben.
Insbesonders wertvoll sind solche Zwischenprodukte der allgemeinen Formel la, in der D für Chlor steht und X, R¹ und R² die in der allgemeinen Formel angegebenen Bedeutungen haben.

Ein weiterer Gegenstand der vorliegenden Erfindung sind auch solche Verbindungen, die unter das Schutzrecht DE 4029650 fallen und deren Wirkung im fungiziden Bereich liegt, die jedoch nicht als CDK-Inhibitoren beschrieben sind, und auch ihre Verwendung zur Behandlung von Krebs wird nicht beschrieben wird.

| **Nr.** | **Struktur** | **Name** |
|---|---|---|
| 5 | 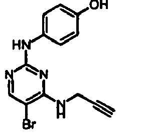 | 4-[[5-Brom-4-(2-propynylamino)-2-pyrimidinyl]amino]-phenol |
| 6 | 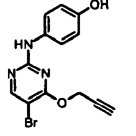 | 4-[[5-Brom-4-(2-propynyloxy)-2-pyrimidinyl]amino]-phenol |
| 16 | 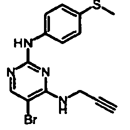 | 5-Brom-N2-(4-methylthiophenyl)-N4-2-propynyl-2,4-pyrimidindiamin |
| 22 | 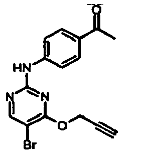 | 1-[4-[(5-Brom-4-(2-propynyloxy)-2-pyrimidinyl)amino]phenyl]-ethanon |
| 23 | 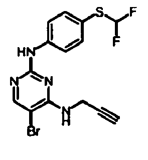 | 5-Brom-N2-(4-difluormethylthiophenyl)-N4-2-propynyl-2,4-pyrimidindiamin |
| 24 | 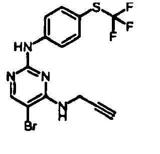 | 5-Brom-N4-2-propynyl-N2-(4-trifluormethylthiophenyl)-2,4-pyrimidinediamin |
| 35 | 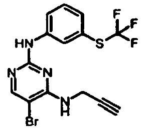 | 5-Brom-N4-2-propynyl-N2-(3-trifluormethylthiophenyl)-2,4-pyrimidindiamin |
| 37 | 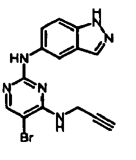 | N-[5-Brom-4-(2-propynylamino)-2-pyrimidinyl]-indazol-5-amin |
| 38 | 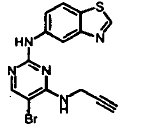 | N-[5-Brom-4-(2-propynylamino)-2-pyrimidinyl]-benzthiazol-5-amin |
| 42 | 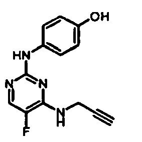 | 4-[[5-Fluor-4-(2-propynyloxy)-2-pyrimidinyl]amino]-phenol |
| 43 | 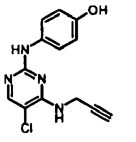 | 4-[[5-Chlor-4-(2-propynyloxy)-2-pyrimidinyl]amino]-phenol |
| 50 | 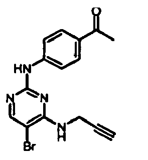 | 1-[4-[(5-Brom-4-(2-propynylamino)-2-pyrimidinyl)amino]phenyl]-ethanon |
| 54 | 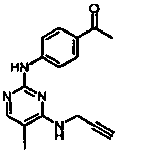 | 1-[4-[(5-lod-4-(2-propynylamino)-2-pyrimidinyl)amino]phenyl]-ethanon |
| 70 | 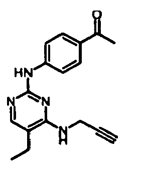 | 1-[4-[(5-Ethyl-4-(2-propynylamino)-2-pyrimidinyl)amino]phenyl]-ethanon |
| 81 | 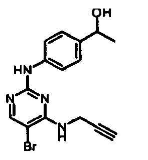 | 1-[4-[(5-Brom-4-(2-propynylamino)-2-pyrimidinyl)amino]phenyl]-ethanol |
| 82 | 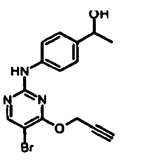 | 1-[4-[(5-Brom-4-(2-propynyloxy)-2-pyrimidinyl)amino]phenyl]-ethanol |

Die erfindungsgemäßen Mittel können ebenfalls zur Behandlung von Krebs, Autoimmunerkrankungen, kardiovaskulären Erkrankungen, Chemotherapeutika-induzierter Alopezie und Mukositis, infektiösen Erkrankungen, nephrologischen Erkrankungen, chronischen und akuten neurodegenerativen Erkrankungen und viralen Infektionen, wobei unter Krebs solide Tumoren und Leukämie, unter Autoimmunerkrankungen Psoriasis, Alopezie und Multiple Sklerose, unter kardiovaskulären Erkrankungen Stenosen, Arteriosklerosen und Restenosen, unter infektiösen Erkrankungen durch unizelluläre Parasiten hervorgerufene Erkrankungen, unter nephrologischen Erkrankungen Glomerulonephritis, unter chronisch neurodegenerativen Erkrankungen Huntington's Erkrankung, amyotrophe Lateralsklerose, Parkinsonsche Erkrankung, AIDS Dementia und Alzheimer'sche Erkrankung, unter akut neurodegenerativen Erkrankungen Ischämien des Gehirns und Neurotraumata, und unter viralen Infektionen Cytomegalus-Infektionen, Herpes, Hepatitis B oder C, und HIV Erkrankungen verwendet werden.

Die nachfolgenden Beispiele beschreiben die biologische Wirkung der erfindungsgemäßen Verbindungen ohne die Erfindung auf diese Beispiele zu beschränken.

### Beispiel 1

### CDK2/CycE Kinase Assay

Rekombinante CDK2- und CycE-GST-Fusionsproteine, gereinigt aus Bakulovirus-infizierten Insektenzellen (Sf9), wurden von Dr. Dieter Marmé, Klinik für Tumorbiologie Freiburg, erhalten. Histon IIIS, das als Kinase-Substrat verwendet wurde, wurde bei der Fa. Sigma gekauft.
CDK2/CycE (50 ng/Meßpunkt) wurde für 15 min bei 22°C in Anwesenheit verschiedener Konzentrationen an Testsubstanzen (0 µM, sowie innerhalb des Bereiches 0,01 - 100 µM) in Assaypuffer [50 mM Tris/HCl pH8,0, 10 mM MgCl₂, 0,1 mM Na ortho-Vanadat, 1,0 mM Dithiothreitol, 0,5 µM Adenosintrisphosphat (ATP), 10 µg/Meßpunkt Histon IIIS, 0,2 µCi/Meßpunkt ³³P-gamma ATP, 0,05% NP40, 12,5% Dimethylsulfoxid] inkubiert. Die Reaktion wurde durch Zugabe von EDTA-Lösung (250 mM, pH8,0, 14 µl/Meßpunkt) gestoppt.
Von jedem Reaktionsansatz wurden 10 µl auf P30 Filterstreifen (Fa. Wallac) aufgetragen, und nicht-eingebautes ³³P-ATP wurde durch dreimaliges Waschen der Filterstreifen für je 10 min in 0,5%iger Phosphorsäure entfernt. Nach dem Trocknen der Filterstreifen für 1 Stunde bei 70°C wurden die Filterstreifen mit Szintillator-Streifen (MeltiLex^{™} A, Fa. Wallac) bedeckt und für 1 Stunde bei 90°C eingebrannt. Die Menge an eingebautem ³³P (Substratphosphorylierung) wurde durch Szintillationsmessung in einem gamma-Strahlungsmeßgerät (Wallac) bestimmt.

### Beispiel 2

### Proliferationsassay

Kultivierte humane Tumorzellen (wie angegeben) wurden in einer Dichte von 5000 Zellen/Meßpunkt in einer 96-Loch Multititerplatte in 200 µl des entsprechenden Wachstumsmediums ausplattiert. Nach 24 Stunden wurden die Zellen einer Platte (Nullpunkt-Platte) mit Kristallviolett gefärbt (s.u.), während das Medium der anderen Platten durch frisches Kulturmedium (200 µl), dem die Testsubstanzen in verschiedenen Konzentrationen (0 µM, sowie im Bereich 0,01 - 30 µM; die finale Konzentration des Lösungsmittels Dimethylsulfoxid betrug 0,5%) zugesetzt waren, ersetzt. Die Zellen wurden für 4 Tage in Anwesenheit der Testsubstanzen inkubiert. Die Zellproliferation wurde durch Färbung der Zellen mit Kristallviolett bestimmt: Die Zellen wurden durch Zugabe von 20 µl/Meßpunkt einer 11 %igen Glutaraldehyd-Lösung 15 min bei Raumtemperatur fixiert. Nach dreimaligem Waschen der fixierten Zellen mit Wasser wurden die Platten bei Raumtemperatur getrocknet. Die Zellen wurden durch Zugabe von 100 µl/Meßpunkt einer 0,1%igen Kristallviolett-Lösung (pH durch Zugabe von Essigsäure auf pH3 eingestellt) gefärbt. Nach dreimaligem Waschen der gefärbten Zellen mit Wasser wurden die Platten bei Raumtemperatur getrocknet. Der Farbstoff wurde durch Zugabe von 100 µl/Meßpunkt einer 10%igen Essigsäure-Lösung gelöst. Die Extinktion wurde photometrisch bei einer Wellenlänge von 595 nm bestimmt. Die prozentuale Änderung des Zellwachstums wurde durch Normalisierung der Meßwerte auf die Extinktionwerte der Nullpunktplatte (=0%) und die Extinktion der unbehandelten (0 µM) Zellen (=100%) berechnet.

Die Ergebnisse aus Beispiel 1 und 2 sind in der folgenden Tabelle angegeben.

| **Beispiel Nummer** | **Inhibition IC₅₀ [nM]** | **Proliferation IC₅₀ [**µ**M]** | | | | **Sw** |
|---|---|---|---|---|---|---|
| | **CDK2/CycE** | **MCF7** | **H460** | **HCT116** | **DU145** | **(g/l)** |
| 22 | 40 | 1,2 | 1,5 | 1,5 | 1,5 | 0.003 |
| 37 | 70 | 4 | | | | 0.006 |
| 6 | 70 | 4 | 6 | | | 0.008 |
| 40 | 20 | 1 | 3 | 3 | 9 | 0.002 |
| 51 | 70 | 8 | | | | |
| 20 | 60 | 4 | | | | |
| 21 | 400 | 2 | | | | |
| 1 | 300 | 8 | | | | |
| 2 | 700 | | | | | |
| 16 | 300 | 3 | | | | |
| 24 | 400 | 5 | | | | |
| 26 | 300 | 3 | | | | |
| 35 | 120 | >10 | | | | |
| 23 | 180 | 3 | | | | |
| 11 | 6 | 0,2 | 0,5 | 0,3 | 0,2 | |
| 38 | 80 | >10 | | | | |
| 34 | 1800 | | | | | |
| 10 | 4 | 0,2 | 0,5 | 0,5 | 0,5 | |
| 12 | 400 | 4 | | | | |
| 25 | 70 | 1,2 | 1,5 | 1,1 | 1,2 | 0.017 |
| 9 | 7 | 0,9 | | 3 | 3 | |
| 7 | 6 | 0,7 | 1,5 | 1,2 | 0,5 | 0.028 |
| 31 | 800 | 7 | | | | 0.0023 |
| 14 | 200 | 3 | | | | 0.013 |
| 18 | 2000 | | | | | 0.039 |
| 3 | 200 | 8 | | | | 0.039 |
| 19 | 800 | >10 | | | | 0.041 |
| 13 | 2000 | >10 | | | | |
| 17 | 1000 | >10 | | | | 0.04 |
| 4 | 40 | 8 | | | | 0.042 |
| 15 | 300 | >10 | | | | 0.024 |
| 8 | <10 | 4 | | | | 0.007 |
| 43 | 200 | 6 | | | | 0.04 |
| 36 | 30 | 0,4 | 0,6 | 0,5 | 0,6 | 0.018 |
| 27 | >10000 | | | | | |
| 42 | 2000 | | | | | 0.043 |
| 39 | 300 | | | | | 0.0016 |
| 44 | 8 | 1,2 | 0,4 | 0,4 | 0,3 | 0.005 |
| 45 | 10 | 2 | 1,7 | 1,2 | 0,5 | 0.0094 |
| 50 | 150 | | | | | |
| 5 | 90 | 10 | | | | 0.043 |
| 46 | 7 | 2 | | | | 0.0069 |
| 52 | 200 | 0,2 | 1,6 | 1,2 | 2 | 0.0005 |
| 53 | 300 | 1,6 | | | | 0.026 |
| 54 | 100 | 1,1 | | | | 0.0015 |
| 47 | 12 | 0,7 | 1,8 | 1,3 | 0,9 | |
| 56 | 80 | 4 | | | | 0.023 |
| 49 | 50 | >10 | | | | 0.044 |
| 48 | 4 | 0,2 | 1 | 0,4 | 0,3 | 0.042 |
| 96 | 400 | | | | | 0.0005 |
| 98 | 2000 | | | | | |
| 85 | 2000 | | | | | 0.001 |
| 84 | 400 | | | | | 0.0005 |
| 86 | 3000 | | | | | |
| 87 | 250 | 0,8 | | | | 0.003 |
| 22 | 40 | 1,2 | 1,5 | 1,5 | 1,5 | 0.003 |
| 37 | 70 | 4 | | | | 0.006 |
| 6 | 70 | 4 | 6 | | | 0.008 |
| 16 | 300 | 3 | | | | |
| 24 | 400 | 5 | | | | |
| 35 | 120 | >10 | | | | |
| 23 | 180 | 3 | | | | |
| 38 | 80 | >10 | | | | |
| 43 | 200 | 6 | | | | 0.04 |
| 42 | 2000 | | | | | 0.043 |
| 50 | 150 | | | | | |
| 5 | 90 | 10 | | | | 0.043 |
| 54 | 100 | 1,1 | | | | 0.0015 |

### Überlegenheitsnachweis der erfindungsgemäßen Verbindungen gegenüber den bekannten Verbindungen

Zum Nachweis der Überlegenheit der erfindungsgemäßen Verbindungen gegenüber den bekannten Verbindungen wurden die erfindungsgemäßen Verbindungen mit bekannten Referenzverbindungen und strukturähnlichen bekannten Verbindungen im Enzymtest verglichen. Das Ergebnis ist in der folgenden Tabelle aufgeführt.

| **Beispiel-Nr.** | **R²** | **A** | **CDK2/ CycE IC₅₀ [nM]** | **MCF-7 IC₅₀ [**µ**M]** | **Löslichkeit (g/l)** |
|---|---|---|---|---|---|
| 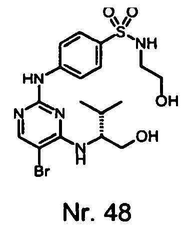 | CH(C₃H₇)-CH₂-OH- | -SO₂-N-(CH₂)₂-OH | 4 | 0.2 | 0,042 |
| 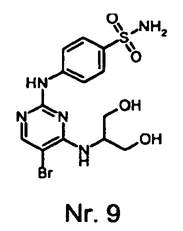 | CH(CH₂OH)₂ | SO₂NH₂ | 7 | 0,9 | 0,009 |
| 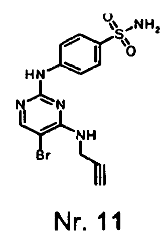 | Propargyl-NH- | SO₂NH₂ | 6 | 0,2 | |
| 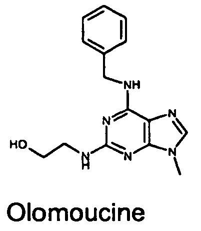 | | | 7000 | 30 | |
| 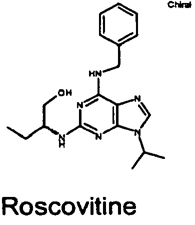 | | | 1500 | 8 | |
| 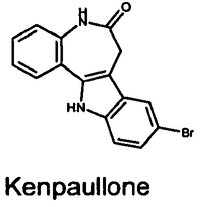 | | | 1800 | 6 | |
| 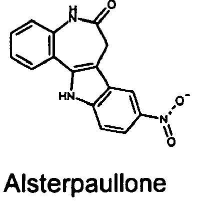 | | | 90 | 1.2 | |
| 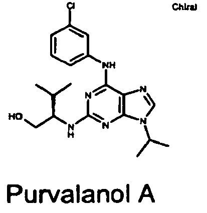 | | | 10 | 2 | |
| Beispiel 11 aus WO01/14375 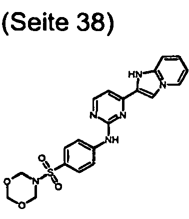 | | | 190 | | |

Aus den Ergebnissen der Tabelle ist zu erkennen, dass die erfindungsgemäßen Verbindungen sowohl im Enzym-Test, als auch im Zell-Test deutlich höhere Aktivitäten am Enzym und in MCF-7-Zellen als die aus dem Stand der Technik bekannten Verbindungen aufweisen. Damit sind die erfindungsgemäßen Verbindungen den bekannten Verbindungen weit überlegen.

## Patentansprüche

1. Verbindungen der allgemeinen Formel I 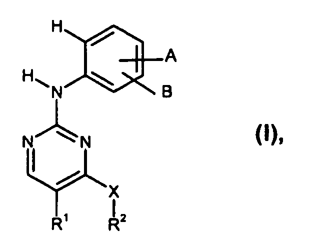 in der
R¹ Halogen
R² für -CH(CH₃)-(CH₂)ₙ-R⁵, -CH-(CH₂OH)₂, -(CH₂)ₙR⁷, -CH(C₃H₇)-(CH₂)ₙ-R⁵, -CH(C₂H₅)-(CH₂)ₙ-R⁵, -CH₂-CN, -CH(CH₃)COCH₃, -CH(CH₃)-C(OH)(CH₃)₂, -CH(CH(OH)CH₃)OCH₃, -CH(C₂H₅)CO-R⁵, C₂-C₄-Alkinyl, -(CH₂)ₙ-COR⁵, -(CH₂)n-CO-C₁-C₆-Alkyl, -(CH₂)ₙ-C(OH)(CH₃)-Phenyl, -CH(CH₃)-C(CH₃)-R⁵, -CH(CH₃)-C(CH₃)(C₂H₅)-R⁵,-CH(OCH₃)-CH₂-R⁵, -CH₂-CH(OH)-R⁵, -CH(OCH₃)-CHR⁵-CH₃, -CH(CH₃)-CH(OH)-CH₂-CH=CH₂, -CH(C₂H₅)-CH(OH)-(CH₂)ₙ-CH₃, -CH(CH₃)-CH(OH)-(CH₂)ₙ-CH₃, -CH(CH₃)-CH(OH)-CH(CH₃)₂, (CH₂OAC)₂, -(CH₂)ₙ-R⁶, -(CH₂)ₙ-(CF₂)ₙ-CF_{3,} -CH(CH₂)ₙ-R⁵)₂, -CH(CH₃)-CO-NH₂, -CH(CH₂OH)-Phenyl, -CH(CH₂OH)-CH(OH)-(CH₂)ₙR⁵, -CH(CH₂OH)-CH(OH)-Phenyl, -CH(CH₂OH)-C₂H₄-R⁵, -(CH₂)ₙ-C≡C-C(CH₃)=CH-COR⁵, -CH(Ph)-(CH₂)ₙ-R⁵, -(CH₂)ₙ-COR⁵,-(CH₂)ₙPO₃(R⁵)₂, -(CH₂)ₙ-COR⁵, -CH((CH₂)ₙOR⁵)CO-R⁵,-(CH₂)ₙCONHCH((CH₂)ₙR⁵)₂, -(CH₂)ₙNH-COR⁵,-CH(CH₂)ₙR⁵-(CH₂)ₙC₃-C₁₀-Cycloalkyl, -(CH₂)ₙ-C₃-C₁₀-Cycloalkyl, C₃-C₁₀-Cycloalkyl, gegebenenfalls ein- oder mehrfach, gleich oder verschieden mit Hydroxy, C₁-C₆-Alkyl oder der Gruppe -COONH(CH₂)ₙCH₃ oder -NR³R⁴ substituiertes C₁-C₆-Alkyl, C₃-C₁₀-Cycloalkyl, -(CH₂)ₙ-O-(CH₂)n-R⁵, -(CH₂)ₙ-NR³R⁴, -CH(C₃H₇)-(CH₂)ₙ-OC(O)-(CH₂)n-CH₃, -(CH₂)ₙ-R⁵, -C(CH₃)₂-(CH₂)ₙ-R⁵, -C(CH₂)ₙ(CH₃)-(CH₂)ₙR⁵, -C(CH₂)ₙ-(CH₂)ₙR⁵, -CH(t-Butyl)-(CH₂)ₙ-R⁵, -CCH₃(C₃H₇)-(CH₂)ₙR⁵, -CH(C₃H₇)-(CH₂)ₙ-R⁵, -CH(C₃H₇)-COR⁵, -CH(C₃H₇)-(CH₂)ₙ-OC(O)-NH-Ph, -CH((CH₂)ₙ(C₃H₇))-(CH₂)ₙR⁵, -CH(C₃H₇)-(CH₂)ₙ-OC(O)-NH-Ph(OR⁵)₃, -NR³R⁴, -NH-(CH₂)ₙ-NR³R⁴, R⁵-(CH₂)ₙ-C*H-CH(R⁵)-(CH₂)ₙ-R⁵, -(CH₂)ₙ-CO-NH-(CH₂)ₙ-CO-R⁵, -OC(O)NH-C₁-C₆-Alkyl oder -(CH₂)ₙ-CO-NH-(CH₂)ₙ-CH-((CH₂)ₙR⁵)₂,
oder für C₃-C₁₀-Cycloalkyl steht, welches mit der Gruppe 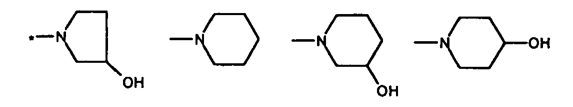 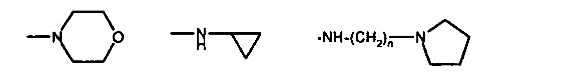 substituiert ist, oder für die Gruppe 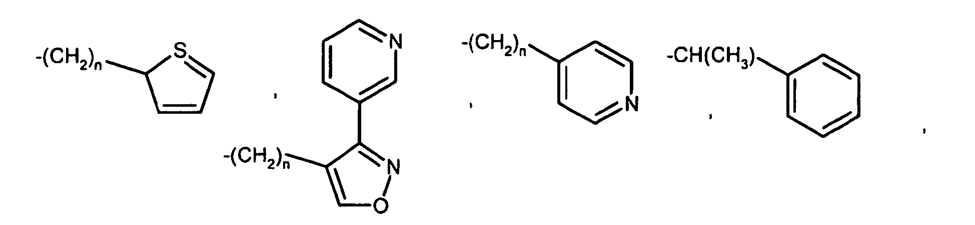 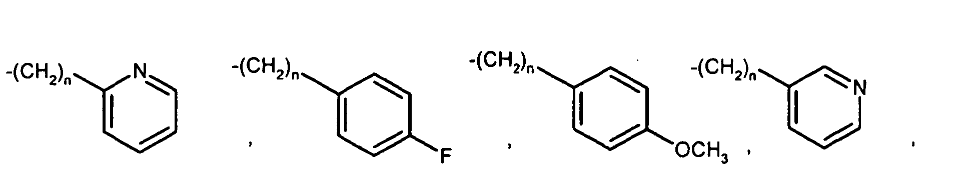 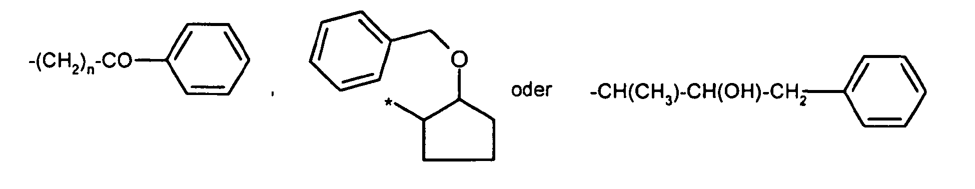 oder 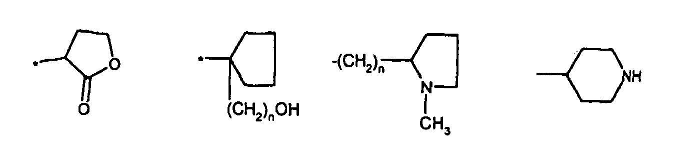 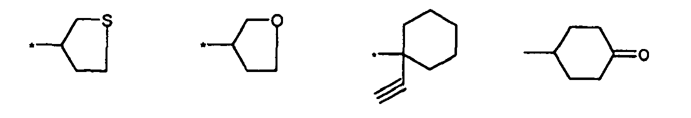 steht,
X für Sauerstoff oder für die Gruppe -NH- oder -N(C₁-C₃-Alkyl) steht, oder
R² für die Gruppe 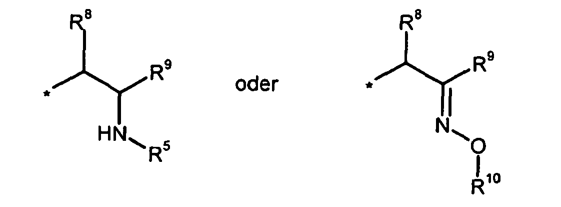 steht,
oder
X und R² gemeinsam eine Gruppe 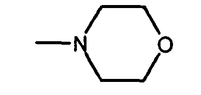 bilden,
A für die Gruppe -SO₂R⁷, SO₂-C₂H₄-OH, -SO₂CF₃ oder -SO₂-NR³R⁴ steht,
B für Wasserstoff, Hydroxy oder C₁-C₃-Alkyl steht
oder
A und B gemeinsam eine Gruppe 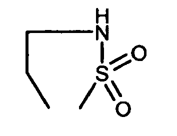 bilden können,
R³ und R⁴ jeweils unabhängig voneinander für Wasserstoff, Phenyl, Benzyloxy, C₁-C₁₂-Alkyl, C₁-C₆-Alkoxy, C₂-C₄-Alkenyloxy, C₃-C₆-Cycloalkyl, Hydroxy, Hydroxy-C₁-C₆-alkyl, Dihydroxy-C₁-C₆-alkyl, Heteroaryl, Heterocyclo-C₃-C₁₀-alkyl, Heteroaryl-C₁-C₃-alkyl,
gegebenenfalls mit Cyano substituiertes C₃-C₆-Cycloalkyl-C₁-C₃-alkyl, oder für
gegebenenfalls ein- oder mehrfach, gleich oder verschieden mit Phenyl, Pyridyl, Phenyloxy, C₃-C₆-Cycloalkyl, C₁-C₆-Alkyl oder C₁-C₆-Alkoxy substituiertes C₁-C₆-Alkyl steht, wobei das Phenyl selbst ein oder mehrfach, gleich oder verschieden mit Halogen, Trifluormethyl, C₁-C₆-Alkyl, C₁-C₆-Alkoxy oder mit der Gruppe -SO₂NR³R⁴ substituiert sein kann,
oder für die Gruppe -(CH₂)ₙNR³R⁴, -CNHNH₂ oder - NR³R⁴ oder für 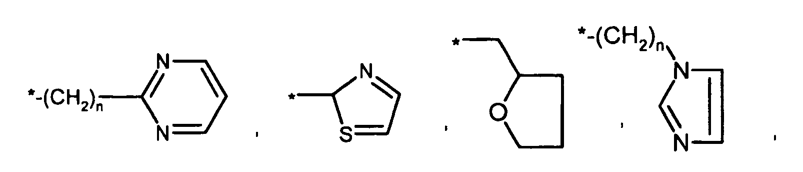 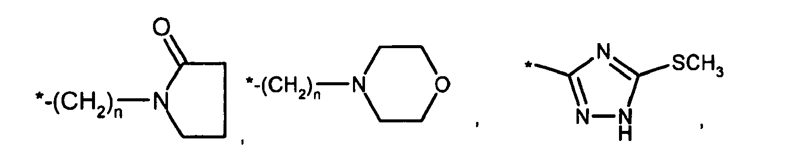 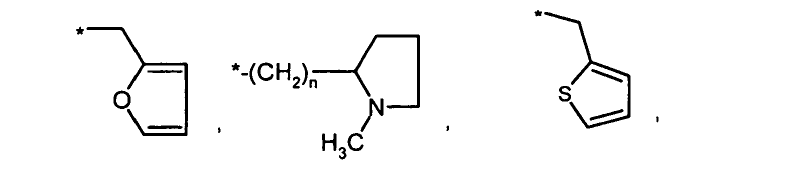 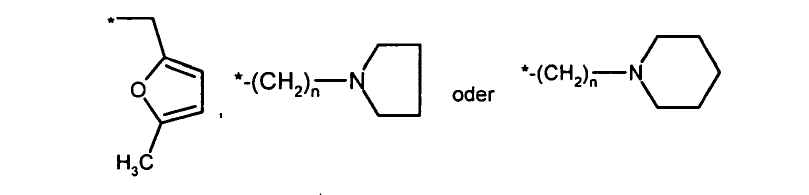 stehen, welche gegebenenfalls mit C₁-C₆-Alkyl substituiert sein können,
R⁵ für Hydroxy, Phenyl, C₁-C₆-Alkyl, C₃-C₆-Cycloalkyl, Benzoxy, C₁-C₆-Alkylthio oder C₁-C₆-Alkoxy steht,
R⁶ für die Gruppe 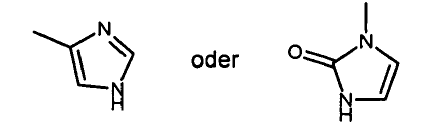 steht,
R⁷ für Halogen, Hydroxy, Phenyl, C₁-C₆-Alkyl, -(CH₂)ₙOH, -NR³R⁴ oder die Gruppe 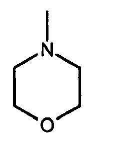 steht,
R⁸, R⁹ und R¹⁰ für Wasserstoff, Hydroxy, C₁-C₆-Alkyl oder für die Gruppe -(CH₂)ₙ-COOH stehen, und
n für 0 - 6 stehen, bedeuten,
sowie deren Diastereoisomeren, Enantiomeren und Salze.

2. Verbindung gemäß Anspruch 1 der folgenden Formeln:
5-Bromo-N2-[4-(2-diethylamino-ethanesulfonyl)-phenyl]-N4-prop-2-ynyl-pyrimidine-2,4-diamine,
N2-(3-Benzenesulfonyl-phenyl)-5-bromo-N4-prop-2ynyl-pyrimidine-2,4-diamine,
5-Bromo-N2-[4-(morpholine-4-sulfonyl)-phenyl]-N4-prop-2-ynyl-pyrimidine-2,4-diamine,
4-[5-Fluoro-4-((R)-1-hydroxymethyl-2-methyl-propylamino)-pyrimidin-2-ylamino]-benzenesulfonamide,
4-[4-((R)-1-Hydroxymethyl-2-methyl-propylamino)-5-iodo-pyrimidin-2-ylamino]-benzenesulfonamide,
4-(5-Fluoro-4-prop-2-ynylamino-pyrimidin-2-ylamino)-benzenesulfonamide,
4-{5-Bromo-4-[2-(2-oxo-2,3-dihydro-imidazol-1-yl)-ethylamino]-pyrimidin-2-ylamino}-benzenesulfonamide,
4-[5-Bromo-4-(2,2,3,3,3-pentafluoro-propoxy)-pyrimidin-2-ylamino]-benzenesulfonamide,
4-[[5-Bromo-4-(1,3-bisacetoxy-2-propyloxy)-2-pyrimidinyl]amino]-benzolsulfonamide,
4-[5-Bromo-4-(2-hydroxy-1-hydroxymethyl-ethoxy)-pyrimidin-2-ylamino]-benzenesulfonamide,
(S)-2-[5-Bromo-2-(4-sulfamoyl-phenylamino)-pyrimidin-4-ylamino]-propionamide,
5-Bromo-N4-prop-2-ynyl-N2-(3-trifluoromethanesulfonyl-phenyl)-pyrimidine-2,4-diamine,
3-(5-Bromo-4-prop-2-ynylamino-pyrimidin-2-ylamino)-benzenesulfonamide,
3-(5-Bromo-4-prop-2-ynylamino-pyrimidin-2-ylamino)-N-butyl-benzenesulfonamide,
2-[3-(5-Bromo-4-prop-2-ynylamino-pyrimidin-2-ylamino)-benzenesulfonyl]-ethanol,
4-(5-Bromo-4-prop-2-ynylamino-pyrimidin-2-ylamino)-benzenesulfonamide,
4-(5-Chloro-4-prop-2-ynylamino-pyrimidin-2-ylamino)-benzenesulfonamide,
2-[5-Bromo-2-(4-sulfamoyl-phenylamino)-pyrimidin-4-ylamino]-butyric acid methyl ester,
4-[5-Bromo-4-((R)-1-hydroxymethyl-2-methyl-propylamino)-pyrimidin-2-yl amino]-benzenesulfonamide,
2-{5-Bromo-2-[3-(2-hydroxy-ethanesulfonyl)-phenylamino]-pyrimidin-4-ylamino}-propane-1,3-diol,
4-[5-Bromo-4-(2-hydroxy-1-hydroxymethyl-ethylamino)-pyrimidin-2-ylamino]-benzenesulfonamide,
4-[5-Bromo-4-(cyclopropylmethyl-amino)-pyrimidin-2-ylamino]-benzenesulfonamide,
4-[5-Bromo-4-((R)-1-hydroxymethyl-2-methyl-propylamino)-pyrimidin-2-yl amino]-N-(2-hydroxy-ethyl)-benzenesulfonamide,
4-[5-Bromo-4-((S)-2-methoxy-1-methyl-ethylamino)-pyrimidin-2-ylamino]-benzenesulfonamide,
2-{3-[5-Bromo-4-((S)-2-methoxy-1-methyl-ethylamino)-pyrimidin-2-ylamino]-benzenesulfonyl}-ethanol,
2-{3-[5-Bromo-4-(2-morpholin-4-yl-ethylamino)-pyrimidin-2-ylamino]-benzenesulfonyl}-ethanol,
4-[5-Bromo-4-(2-morpholin-4-yl-ethylamino)-pyrimidin-2-ylamino]-benzenesulfonamide,
4-[5-Bromo-4-(4,4,4-trifluoro-butoxy)-pyrimidin-2-ylamino]-benzenesulfonamide,
4-[5-Bromo-4-(2-ethoxy-1-ethoxymethyl-ethoxy)-pyrimidin-2-ylamino]-benzenesulfonamide,
4-[5-Bromo-2-(4-sulfamoyl-phenylamino)-pyrimidin-4-ylamino]-butyric acid,
4-[5-Bromo-4-((R)-1-hydroxymethyl-2-methyl-propylamino)-pyrimidin-2-yl amino]-N-(2-hydroxy-ethyl)-benzenesulfonamide,
(R)-2-{5-Bromo-2-[3-(2-hydroxy-ethanesulfonyl)-phenylamino]-pyrimidin-4-ylamino}-3-methyl-butan-1-ol,
4-[5-Bromo-4-((S)-1-hydroxymethyl-2-methyl-propylamino)-pyrimidin-2-yl amino]-benzenesulfonamide,
4-[5-Bromo-4-((R)-2-hydroxy-1-phenyl-ethylamino)-pyrimidin-2-ylamino]-benzenesulfonamide,
4-[5-Bromo-4-((S)-2-methoxy-1-methyl-ethylamino)-pyrimidin-2-ylamino]-N-methyl-benzenesulfonamide,
4-[5-Bromo-4-((S)-2-methoxy-1-methyl-ethylamino)-pyrimidin-2-ylamino]-N, N-dimethyl-benzenesulfonamide,
5-[5-Bromo-2-(4-sulfamoyl-phenylamino)-pyrimidin-4-ylamino]-pentanoic acid benzyl ester,
6-[5-Bromo-2-(4-sulfamoyl-phenylamino)-pyrimidin-4-ylamino]-hexanoic acid methyl ester,
4-(5-lodo-4-prop-2-ynylamino-pyrimidin-2-ylamino)-benzenesulfonamide
4-[5-Bromo-4-((S)-1-hydroxymethyl-3-methylsulfanyl-propylamino)-pyrimidin-2-ylamino]-benzenesulfonamide,
4-[5-Bromo-4-((1S,2S)-2-hydroxy-1-hydroxymethyl-2-phenyl-ethylamino)-pyrimidin-2-ylamino]-benzenesulfonamide,
4-[5-Bromo-4-((R)-2-oxo-tetrahydro-furan-3-ylamino)-pyrimidin-2-ylamino]-benzenesulfonamide,
4-[5-Bromo-4-((S)-1-hydroxymethyl-2,2-dimethyl-propylamino)-pyrimidin-2-ylamino]-benzenesulfonamide,
4-{3-[5-Bromo-2-(4-sulfamoyl-phenylamino)-pyrimidin-4-ylamino]-propionylamino}-butyric acid methyl ester,
6-[5-Bromo-2-(4-sulfamoyl-phenylamino)-pyrimidin-4-ylamino]-hexanoic acid,
4-(5-Bromo-4-cyclohexylamino-pyrimidin-2-ylamino)-benzenesulfonamide,
4-{6-[5-Bromo-2-(4-sulfamoyl-phenylamino)-pyrimidin-4-ylamino]-hexanoylamino}-butyric acid methyl ester,
6-[5-Bromo-2-(4-sulfamoyl-phenylamino)-pyrimidin-4-ylamino]-hexanoic acid methyl ester,
4-[5-Bromo-4-((1R,2S)-2,3-dihydroxy-1-hydroxymethyl-propylamino)-pyrimidin-2-ylamino]-benzenesulfonamide,
4-[5-Bromo-4-((R)-2-cyclohexyl-1-hydroxymethyl-ethylamino)-pyrimidin-2-ylamino]-benzenesulfonamide,
6-[5-Bromo-2-(4-sulfamoyl-phenylamino)-pyrimidin-4-ylamino]-hexanoic acid (2-hydroxy-1-hydroxymethyl-ethyl)-amide,
4-[5-Bromo-4-((S)-2-hydroxy-1-methyl-ethylamino)-pyrimidin-2-ylamino]-benzenesulfonamide,
4-[5-Bromo-4-(4-hydroxy-cyclohexylamino)-pyrimidin-2-ylamino]-benzenesulfonamide,
4-{4-[(Adamantan-1-ylmethyl)-amino]-5-bromo-pyrimidin-2-ylamino}-benzenesulfonamide,
4-[5-Bromo-4-((R)-1-hydroxymethyl-2-methyl-propylamino)-pyrimidin-2-yl amino]-N-methyl-benzenesulfonamide,
(R)-2-[5-Bromo-2-(4-sulfamoyl-phenylamino)-pyrimidin-4-ylamino]-3-methyl-butyric acid,
4-(5-Bromo-4-cyclohexylamino-pyrimidin-2-ylamino)-N-(2-hydroxy-ethyl)-benzenesulfonamide,
4-[5-Bromo-4-(2,2,3,3,4,4,4-heptafluoro-butylamino)-pyrimidin-2-ylamino]-N-(2-hydroxy-ethyl)-benzenesulfonamide,
4-[5-Bromo-4-((S)-2-methoxy-1-methyl-ethylamino)-pyrimidin-2-ylamino]-N-(2-hydroxy-ethyl)-benzenesulfonamide,
Phenyl-carbamic acid (R)-2-[5-bromo-2-(4-sulfamoyl-phenylamino)-pyrimidin-4-ylamino]-3-methyl-butyl ester,
(R)-2-{5-Bromo-2-[4-(2-hydroxy-ethylsulfamoyl)-phenylamino]-pyrimidin-4-ylamino}-3-methyl-butyric acid,
(R)-2-{5-Bromo-2-[4-(2-hydroxy-ethylsulfamoyl)-phenylamino]-pyrimidin-4-ylamino}-3-methyl-butyric acid methyl ester,
4-(5-Chloro-4-prop-2-ynylamino-pyrimidin-2-ylamino)-N-(2-hydroxy-ethyl)-benzenesulfonamide,
4-[5-Bromo-4-(2-hydroxy-1, 1-dimethyl-ethylamino)-pyrimidin-2-ylamino]-benzenesulfonamide,
4-[5-Bromo-4-((S)-1-hydroxymethyl-3-methyl-butylamino)-pyrimidin-2-ylamino]-benzenesulfonamide,
4-[5-Bromo-4-(tetrahydro-pyran-4-yloxy)-pyrimidin-2-ylamino]-benzenesulfonamide,
4-[5-Bromo-4-((1S,2S)-2-hydroxy-cyclohexylamino)-pyrimidin-2-ylamino]-benzenesulfonamide,
Butyl-carbamic acid 4-[5-bromo-2-(4-sulfamoyl-phenylamino)-pyrimidin-4-ylamino]-cyclohexyl ester,
(R)-2-[5-Bromo-2-(4-methanesulfonyl-phenylamino)-pyrimidin-4-ylamino]-3-methyl-butan-1-ol,
(R)-2-{5-Bromo-2-[4-(morpholine-4-sulfonyl)-phenylamino]-pyrimidin-4-ylamino}-3-methyl-butan-1-ol,
4-[5-Bromo-4-(4-dimethylamino-cyclohexylamino)-pyrimidin-2-ylamino]-benzenesulfonamide,
4-[5-Bromo-4-(4-dimethylamino-cyclohexylamino)-pyrimidin-2-ylamino]-benzenesulfonamide,
4-[5-Bromo-4-(4-piperidin-1-yl-cyclohexylamino)-pyrimidin-2-ylamino]-benzenesulfonamide,
4-[5-Bromo-4-((R)-1-hydroxymethyl-2-methyl-propylamino)-pyrimidin-2-yl amino]-3-hydroxy-benzenesulfonamide,
4-[5-Bromo-4-(4-cyclopropylamino-cyclohexylamino)-pyrimidin-2-ylamino] -benzenesulfonamide,
(R)-2-[5-Bromo-2-(3-methanesulfonyl-phenylamino)-pyrimidin-4-ylamino]-3-methyl-butan-1-ol,
4-{5-Bromo-4-[2-(2-ethoxy-ethoxy)-ethoxy]-pyrimidin-2-ylamino}-benzenesulfonamide,
4-{5-Bromo-4-[4-(2-dimethylamino-ethylamino)-cyclohexylamino]-pyrimidin-2-ylamino}-benzenesulfonamide,
4-{5-Bromo-4-[4-(2-pyrrolidin-1-yl-ethylamino)-cyclohexylamino]-pyrimidin-2-ylamino}-benzenesulfonamide,
4-{5-Bromo-4-[4-(4-hydroxy-piperidin-1-yl)-cyclohexylamino]-pyrimidin-2-ylamino}-benzenesulfonamide,
4-{5-Bromo-2-[4-(2-hydroxy-ethylsulfamoyl)-phenylamino]-pyrimidin-4-ylamino}-butyric acid methyl ester,
4-{5-Bromo-4-[4-(3-hydroxy-pyrrolidin-1-yl)-cyclohexylamino]-pyrimidin-2-ylamino}-benzenesulfonamide,
4-{5-Bromo-4-[4-(3-hydroxy-pyrrolidin-1-yl)-cyclohexylamino]-pyrimidin-2-ylamino}-benzenesulfonamide,
4-[5-Bromo-4-((R)-1-hydroxymethyl-3-methyl-butylamino)-pyrimidin-2-ylamino]-benzenesulfonamide,
4-{5-Bromo-4-[2-(1-methyl-pyrrolidin-2-yl)-ethylamino]-pyrimidin-2-ylamino}-benzenesulfonamide,
4-[5-Bromo-4-(piperidin-4-yloxy)-pyrimidin-2-ylamino]-benzenesulfonamide,
4-[5-Bromo-4-(3-dimethylamino-propylamino)-pyrimidin-2-ylamino]-benzenesulfonamide,
Acetic acid (R)-2-[5-bromo-2-(4-sulfamoyl-phenylamino)-pyrimidin-4-ylamino]-3-methyl-butyl ester,
4-[5-Bromo-4-(4-hydroxy-cyclohexylamino)-pyrimidin-2-ylamino]-N-(2-hydroxy-ethyl)-benzenesulfonamide,
(3,4,5-Trimethoxy-phenyl)-carbamicacid (R)-2-[5-bromo-2-(4-sulfamoyl-phenylamino)-pyrimidin-4-ylamino]-3-methyl-butyl ester,
4-(5-Bromo-4-cycloheptylamino-pyrimidin-2-ylamino)-benzenesulfonamide,
4-[5-Bromo-4-((R)-1-hydroxymethyl-2,2-dimethyl-propylamino)-pyrimidin-2-ylamino]-benzenesulfonamide,
4-[5-Bromo-4-(4-oxo-cyclohexylamino)-pyrimidin-2-ylamino]-benzenesulfonamide,
(E)-6-{5-Chloro-2-[4-(2-hydroxy-ethylsulfamoyl)-phenylamino]-pyrimidin-4-ylamino}-3-methyl-hex-2-en-4-ynoic acid methyl ester,
4-[5-Bromo-4-(1-hydroxymethyl-cyclopentylamino)-pyrimidin-2-ylamino]-benzenesulfonamide,
4-[4-(Bicyclo[2.2.1]hept-2-ylamino)-5-bromo-pyrimidin-2-ylamino]-benzenesulfonamide,
4-[5-Bromo-4-(4-morpholin-4-yl-cyclohexylamino)-pyrimidin-2-ylamino]-benzenesulfonamide,
4-[5-Bromo-4-(4-morpholin-4-yl-cyclohexylamino)-pyrimidin-2-ylamino]-benzenesulfonamide,
4-[5-Bromo-4-((R)-2-hydroxy-1-methyl-ethylamino)-pyrimidin-2-ylamino]-benzenesulfonamide,
4-({5-Bromo-4-[(R)-(2-hydroxy-1-methylethyl)amino]pyrimidin-2-yl}amino)benzenesulfonamide,
4-[5-Bromo-4-(2,2,3,3,3-pentafluoro-propylamino)-pyrimidin-2-ylamino]-benzenesulfonamide,
4-[5-Bromo-4-(4-hydroxy-butylamino)-pyrimidin-2-ylamino]-benzenesulfonamide,
4-[5-Bromo-4-(1-ethynyl-cyclohexylamino)-pyrimidin-2-ylamino]-benzenesulfonamide,
4-[5-Bromo-4-(3-hydroxy-1-phenyl-propylamino)-pyrimidin-2-ylamino]-benzenesulfonamide,
(+)-4-[5-Bromo-4-(2-methyl-cyclohexylamino)-pyrimidin-2-ylamino]-benzenesulfonamide,
(-)-4-[5-Bromo-4-(2-methyl-cyclohexylamino)-pyrimidin-2-ylamino]-benzenesulfonamide,
4-{5-Bromo-4-[4-(2-hydroxy-1-hydroxymethyl-ethylamino)-cyclohexylamino]-pyrimidin-2-ylamino}-benzenesulfonamide,
4-{5-Bromo-4-[4-(2-hydroxy-1-hydroxymethyl-ethylamino)-cyclohexylamino]-pyrimidin-2-ylamino}-benzenesulfonamide,
(R)-2-[5-Bromo-2-(4-trifluoromethanesulfonyl-phenylamino)-pyrimidin-4-ylamino]-3-methyl-butan-1-ol,
4-[5-Bromo-4-(4-cyclopropylamino-cyclohexylamino)-pyrimidin-2-ylamino] -benzenesulfonamide,
4-[5-Bromo-4-(4-cyclopropylamino-cyclohexylamino)-pyrimidin-2-ylamino] -benzenesulfonamide,
4-[5-Bromo-4-(tetrahydro-thiophen-3-ylamino)-pyrimidin-2-ylamino]-benzenesulfonamide,
2-[5-Bromo-2-(4-sulfamoyl-phenylamino)-pyrimidin-4-ylamino]-3-hydroxy-propionic acid methyl ester,
4-[5-Bromo-4-((R)-1-hydroxymethyl-2-methyl-propylamino)-pyrimidin-2-yl amino]-N-pyrimidin-2-yl-benzenesulfonamide,
4-[5-Bromo-4-((R)-1-hydroxymethyl-2-methyl-propylamino)-pyrimidin-2-yl amino]-N-thiazol-2-yl-benzenesulfonamide,
4-[5-Bromo-4-(4-hydroxy-butylamino)-pyrimidin-2-ylamino]-N-methyl-benzenesulfonamide,
4-{5-Bromo-4-[2-(3H-imidazol-4-yl)-ethylamino]-pyrimidin-2-ylamino}-benzenesulfonamide,
4-[5-Bromo-4-(1-ethyl-1-hydroxymethyl-propylamino)-pyrimidin-2-ylamino]-benzenesulfonamide,
4-[5-Bromo-4-(1-hydroxymethyl-1,2-dimethyl-propylamino)-pyrimidin-2-ylamino]-benzenesulfonamide,
4-[5-Bromo-4-(4-methyl-cyclohexylamino)-pyrimidin-2-ylamino]-benzenesulfonamide,
4-{5-Bromo-4-[(S)-(tetrahydro-furan-3-yl)amino]-pyrimidin-2-ylamino}-benzenesulfonamide,
4-[5-Bromo-4-(cyclohexylmethyl-amino)-pyrimidin-2-ylamino]-benzenesulfonamide,
3-[5-Bromo-2-(4-sulfamoyl-phenylamino)-pyrimidin-4-ylamino]-propionic acid,
4-[5-Bromo-2-(4-sulfamoyl-phenylamino)-pyrimidin-4-ylamino]-butyric acid ethyl ester,
{3-[5-Bromo-2-(4-sulfamoyl-phenylamino)-pyrimidin-4-ylamino]-propyl}-phosphonic acid diethyl ester,
4-{5-Bromo-4-[(3-pyridin-3-yl-isoxazol-4-ylmethyl)-amino]-pyrimidin-2-ylamino}-benzenesulfonamide,
{[5-Bromo-2-(4-sulfamoyl-phenylamino)-pyrimidin-4-ylamino]-methyl}-phosphonic acid dimethyl ester,
{[5-Bromo-2-(4-sulfamoyl-phenylamino)-pyrimidin-4-ylamino]-methyl}-phosphonic acid monomethyl ester,
4-{5-Bromo-4-[(pyridin-4-ylmethyl)-amino]-pyrimidin-2-ylamino}-benzenesulfonamide,
4-{5-Bromo-4-[(thiophen-2-ylmethyl)-amino]-pyrimidin-2-ylamino}-benzenesulfonamide,
4-{5-Bromo-4-[(pyridin-2-ylmethyl)-amino]-pyrimidin-2-ylamino}-benzenesulfonamide,
4-[5-Bromo-4-(4-fluoro-benzylamino)-pyrimidin-2-ylamino]-benzenesulfonamide,
4-[5-Bromo-4-(4-methoxy-benzylamino)-pyrimidin-2-ylamino]-benzenesulfonamide,
{3-[5-Bromo-2-(4-sulfamoyl-phenylamino)-pyrimidin-4-ylamino]-propyl}-phosphonic acid,
4-[5-Bromo-4-(2-hydroxy-ethylamino)-pyrimidin-2-ylamino]-benzenesulfonamide,
4-[5-Bromo-4-(1-phenyl-ethylamino)-pyrimidin-2-ylamino]-benzenesulfonamide,
N-Allyloxy-4-[5-bromo-4-((R)-1-hydroxymethyl-2-methyl-propylamino)-pyrimidin-2-ylamino]-benzenesulfonamide,
3-[5-Bromo-4-((R)-1-hydroxymethyl-2-methyl-propylamino)-pyrimidin-2-yl amino]-N-(5-hydroxy-pentyl)-benzenesulfonamide,
3-[5-Bromo-4-((R)-1-hydroxymethyl-2-methyl-propylamino)-pyrimidin-2-yl amino]-N-(2-hydroxy-1-hydroxymethyl-ethyl)-benzenesulfonamide,
3-[5-Bromo-4-((R)-1-hydroxymethyl-2-methyl-propylamino)-pyrimidin-2-ylamino]-N-(3-hydroxy-propyl)-benzenesulfonamide,
N-Benzyloxy-4-[5-bromo-4-((R)-1-hydroxymethyl-2-methyl-propylamino)-pyrimidin-2-ylamino]-benzenesulfonamide,
4-[5-Bromo-4-((R)-1-hydroxymethyl-2-methyl-propylamino)-pyrimidin-2-ylamino]-N-(2-hydroxy-butyl)-benzenesulfonamide,
4-[5-Bromo-4-((R)-1-hydroxymethyl-2-methyl-propylamino)-pyrimidin-2-yl amino]-N-cyclopropylmethyl-benzenesulfonamide,
4-[5-Bromo-4-((R)-1-hydroxymethyl-2-methyl-propylamino)-pyrimidin-2-ylamino]-N-(4-cyano-cyclohexylmethyl)-benzenesulfonamide,
4-[5-Bromo-4-((R)-1-hydroxymethyl-2-methyl-propylamino)-pyrimidin-2-ylamino]-N-(5-hydroxy-pentyl)-benzenesulfonamide,
4-[5-Bromo-4-((R)-1-hydroxymethyl-2-methyl-propylamino)-pyrimidin-2-ylamino]-N-(tetrahydro-furan-2-ylmethyl)-benzenesulfonamide,
4-[5-Bromo-4-((S)-1-hydroxymethyl-2-methyl-propylamino)-pyrimidin-2-ylamino]-N-hydroxy-benzenesulfonamide,
4-[5-Bromo-4-((S)-1-hydroxymethyl-propylamino)-pyrimidin-2-ylamino]-benzenesulfonamide,
4-[5-Bromo-4-((R)-1-hydroxymethyl-2-methyl-propylamino)-pyrimidin-2-ylamino]-N-(2-hydroxy-1-hydroxymethyl-ethyl)-benzenesulfonamide,
4-[5-Bromo-4-((R)-1-hydroxymethyl-2-methyl-propylamino)-pyrimidin-2-ylamino]-N-(3-hydroxy-propyl)-benzenesulfonamide,
4-[5-Bromo-4-((R)-1-hydroxymethyl-2-methyl-propylamino)-pyrimidin-2-ylamino]-N-hydroxy-benzenesulfonamide,
5-[5-Bromo-4-((R)-1-hydroxymethyl-2-methyl-propylamino)-pyrimidin-2-ylamino]-2-methyl-benzenesulfonamide,
3-[5-Bromo-4-((R)-1-hydroxymethyl-2-methyl-propylamino)-pyrimidin-2-ylamino]-N-ethoxy-benzenesulfonamide,
N-Allyloxy-3-[5-bromo-4-((R)-1-hydroxymethyl-2-methyl-propylamino)-pyrimidin-2-ylamino]-benzenesulfonamide,
4-(5-Bromo-4-morpholin-4-yl-pyrimidin-2-ylamino)-benzenesulfonamide,
7-[(5-Bromo-4-{[(R)-2-hydroxy-1-methylethyl]amino}pyrimidin-2-yl)amino]-3,4-dihydro-2H-1,2-benzothiazine 1,1-dioxide ,
4-[5-Bromo-4-(2-hydroxy-2-phenyl-propylamino)-pyrimidin-2-ylamino]-benzenesulfonamide,
4-[5-Bromo-4-(2-hydroxy-1,2-dimethyl-propylamino)-pyrimidin-2-ylamino]-benzenesulfonamide,
4-[5-Bromo-4-(2-hydroxy-1,2-dimethyl-butylamino)-pyrimidin-2-ylamino]-benzenesulfonamide,
4-[5-Bromo-4-(2-oxo-2-phenyl-ethylamino)-pyrimidin-2-ylamino]-benzenesulfonamide,
4-[5-Bromo-4-(2-hydroxy-propylamino)-pyrimidin-2-ylamino]-benzenesulfonamide,
4-[5-Bromo-4-(2-hydroxy-1-methyl-propylamino)-pyrimidin-2-ylamino]-benzenesulfonamide,
4-(5-Bromo-4-sec-butylamino-pyrimidin-2-ylamino)-benzenesulfonamide,
4-[5-Bromo-4-(2-hydroxy-1-methyl-butylamino)-pyrimidin-2-ylamino]-benzenesulfonamide,
4-[5-Bromo-4-(3,3-dimethyl-2-oxo-butylamino)-pyrimidin-2-ylamino]-benzenesulfonamide,
4-[5-Bromo-4-(2-hydroxy-1-methyl-3-phenyl-propylamino)-pyrimidin-2-ylamino]-benzenesulfonamide,
4-[5-Bromo-4-(2-hydroxy-butylamino)-pyrimidin-2-ylamino]-benzenesulfonamide,
4-[5-Bromo-4-(2-hydroxy-1-methyl-pent-4-enylamino)-pyrimidin-2-ylamino]-benzenesulfonamide,
4-[5-Bromo-4-(2-hydroxy-1-methyl-pentylamino)-pyrimidin-2-ylamino]-benzenesulfonamide,
4-[5-Bromo-4-(1-methyl-2-oxo-propylamino)-pyrimidin-2-ylamino]-benzenesulfonamide,
4-[5-Bromo-4-(2-hydroxy-1,3-dimethyl-butylamino)-pyrimidin-2-ylamino]-benzenesulfonamide,
4-[4-((1R,2R)-2-Benzyloxy-cyclopentylamino)-5-bromo-pyrimidin-2-ylamino]-benzenesulfonamide,
4-[4-((1S,2S)-2-Benzyloxy-cyclopentylamino)-5-bromo-pyrimidin-2-ylamino]-benzenesulfonamide,
4-[5-Bromo-4-(1-ethyl-2-hydroxy-propylamino)-pyrimidin-2-ylamino]-benzenesulfonamide,
4-[5-Bromo-4-(1-ethyl-2-hydroxy-butylamino)-pyrimidin-2-ylamino]-benzenesulfonamide,
4-[5-Bromo-4-(2-hydroxy-2-phenyl-ethylamino)-pyrimidin-2-ylamino]-benzenesulfonamide,
4-[5-Bromo-4-(2-hydroxy-3,3-dimethyl-butylamino)-pyrimidin-2-ylamino]-benzenesulfonamide,
4-[5-Bromo-4-(2-hydroxyimino-1-methyl-propylamino)-pyrimidin-2-ylamino]-benzenesulfonamide,
4-(5-Bromo-4-{2-[(E)-methoxyimino]-1-methyl-propylamino}-pyrimidin-2-ylamino)-benzenesulfonamide,
4-(5-Bromo-4-{2-[(E)-tert-butoxyimino]-1-methyl-propylamino}-pyrimidin-2-ylamino)-benzenesulfonamide,
[2-[5-Bromo-2-(4-sulfamoyl-phenylamino)-pyrimidin-4-ylamino]-1-methyl-prop-(E)-ylideneaminooxy]-acetic acid,
4-[5-Bromo-4-(2-cyclopropylamino-1-methyl-propylamino)-pyrimidin-2-ylamino]-benzenesulfonamide,
4-[5-Bromo-4-(2-hydroxy-1-methoxymethyl-ethylamino)-pyrimidin-2-ylamino]-benzenesulfonamide,
4-[5-Bromo-4-(2-hydroxy-1-methoxymethyl-propylamino)-pyrimidin-2-ylamino]-benzenesulfonamide,
5-[5-Bromo-4-((R)-1-hydroxymethyl-propylamino)-pyrimid in-2-ylamino]-N-cyclohexylmethyl-2-methyl-benzenesulfonamide,
5-[5-Bromo-4-((R)-1-hydroxymethyl-propylamino)-pyrimidin-2-ylamino]-2-methyl-N-(4-phenyl-butyl)-benzenesulfonamide,
5-[5-Bromo-4-((R)-1-hydroxymethyl-propylamino)-pyrimidin-2-ylamino]-N-(3,3-diphenyl-propyl)-2-methyl-benzenesulfonamide,
5-[5-Bromo-4-((R)-1-hydroxymethyl-propylamino)-pyrimidin-2-ylamino]-N-(2-dimethylamino-ethyl)-2-methyl-benzenesulfonamide,
5-[5-Bromo-4-((R)-1-hydroxymethyl-propylamino)-pyrimidin-2-ylamino]-N-3-imidazol-1-yl-propyl)-2-methyl-benzenesulfonamide,
5-[5-Bromo-4-((R)-1-hydroxymethyl-propylamino)-pyrimidin-2-ylamino]-2-methyl-N-(3-trifluoromethyl-benzyl)-benzenesulfonamide,
5-[5-Bromo-4-((R)-1-hydroxymethyl-propylamino)-pyrimidin-2-ylamino]-N-heptyl-2-methyl-benzenesulfonamide,
5-[5-Bromo-4-((R)-1-hydroxymethyl-propylamino)-pyrimidin-2-ylamino]-2-methyl-N-octyl-benzenesulfonamide,
5-[5-Bromo-4-((R)-1-hydroxymethyl-propylamino)-pyrimidin-2-ylamino]-2-methyl-N-nonyl-benzenesulfonamide,
5-[5-Bromo-4-((R)-1-hydroxymethyl-propylamino)-pyrimidin-2-ylamino]-N-decyl-2-methyl-benzenesulfonamide,
5-[5-Bromo-4-((R)-1-hydroxymethyl-propylamino)-pyrimidin-2-ylamino]-N-furan-2-ylmethyl-2-methyl-benzenesulfonamide,
5-[5-Bromo-4-((R)-1-hydroxymethyl-propylamino)-pyrimidin-2-ylamino]-2-methyl-N-(4-trifluoromethyl-benzyl)-benzenesulfonamide,
5-[5-Bromo-4-((R)-1-hydroxymethyl-propylamino)-pyrimidin-2-ylamino]-2-methyl-N-(3-phenyl-propyl)-benzenesulfonamide,
5-[5-Bromo-4-((R)-1-hydroxymethyl-propylamino)-pyrimidin-2-ylamino]-2-methyl-N-[3-(2-oxo-pyrrolidin-1-yl)-propyl]-benzenesulfonamide,
5-[5-Bromo-4-((R)-1-hydroxymethyl-propylamino)-pyrimidin-2-ylamino]-2-methyl-N-(3-methyl-butyl)-benzenesulfonamide,
5-[5-Bromo-4-((R)-1-hydroxymethyl-propylamino)-pyrimidin-2-ylamino]-N-hexyl-2-methyl-benzenesulfonamide,
5-[5-Bromo-4-((R)-1-hydroxymethyl-propylamino)-pyrimidin-2-ylamino]-N-(3-dimethylamino-propyl)-2-methyl-benzenesulfonamide,
5-[5-Bromo-4-((R)-1-hydroxymethyl-propylamino)-pyrimidin-2-ylamino]-N-(2-diisopropylamino-ethyl)-2-methyl-benzenesulfonamide,
5-[5-Bromo-4-((R)-1-hydroxymethyl-propylamino)-pyrimidin-2-ylamino]-N-cyclopropyl-2-methyl-benzenesulfonamide,
5-[5-Bromo-4-((R)-1-hydroxymethyl-propylamino)-pyrimidin-2-ylamino]-N-(4-cyano-cyclohexylmethyl)-2-methyl-benzenesulfonamide,
5-[5-Bromo-4-((R)-1-hydroxymethyl-propylamino)-pyrimidin-2-ylamino]-2-methyl-N-phenethyl-benzenesulfonamide,
5-[5-Bromo-4-((R)-1-hydroxymethyl-propylamino)-pyrimidin-2-ylamino]-N-[2-(4-chloro-phenyl)-ethyl]-2-methyl-benzenesulfonamide,
5-[5-Bromo-4-((R)-1-hydroxymethyl-propylamino)-pyrimidin-2-ylamino]-2-methyl-N-(5-methyl-furan-2-ylmethyl)-benzenesulfonamide,
5-[5-Bromo-4-((R)-1-hydroxymethyl-propylamino)-pyrimidin-2-ylamino]-2-methyl-N-(2-morpholin-4-yl-ethyl)-benzenesulfonamide,
5-[5-Bromo-4-((R)-1-hydroxymethyl-propylamino)-pyrimidin-2-ylamino]-N-(2-methoxy-ethyl)-2-methyl-benzenesulfonamide,
5-[5-Bromo-4-((R)-1-hydroxymethyl-propylamino)-pyrimidin-2-ylamino]-2-methyl-N-thiophen-2-ylmethyl-benzenesulfonamide,
5-[5-Bromo-4-((R)-1-hydroxymethyl-propylamino)-pyrimidin-2-ylamino]-N-[2-(4-methoxy-phenyl)-ethyl]-2-methyl-benzenesulfonamide,
5-[5-Bromo-4-((R)-1-hydroxymethyl-propylamino)-pyrimidin-2-ylamino]-2-methyl-N-(2-pyrrolidin-1-yl-ethyl)-benzenesulfonamide,
5-[5-Bromo-4-((R)-1-hydroxymethyl-propylamino)-pyrimidin-2-ylamino]-2-methyl-N-(2-piperidin-1-yl-ethyl)-benzenesulfonamide,
5-[5-Bromo-4-((R)-1-hydroxymethyl-propylamino)-pyrimidin-2-ylamino]-N-(3-fluoro-benzyl)-2-methyl-benzenesulfonamide,
5-[5-Bromo-4-((R)-1-hydroxymethyl-propylamino)-pyrimidin-2-ylamino]-2-methyl-N-[2-(1-methyl-pyrrolidin-2-yl)-ethyl]-benzenesulfonamide,
5-[5-Bromo-4-((R)-1-hydroxymethyl-propylamino)-pyrimidin-2-ylamino]-2-methyl-N-pyridin-3-ylmethyl-benzenesulfonamide,
5-[5-Bromo-4-((R)-1-hydroxymethyl-propylamino)-pyrimidin-2-ylamino]-N-(4-fluoro-benzyl)-2-methyl-benzenesulfonamide,
5-[5-Bromo-4-((R)-1-hydroxymethyl-propylamino)-pyrimidin-2-ylamino]-2-methyl-N-pyridin-4-ylmethyl-benzenesulfonamide,
5-[5-Bromo-4-((R)-1-hydroxymethyl-propylamino)-pyrimidin-2-ylamino]-2-methyl-N-(2-phenoxy-ethyl)-benzenesulfonamide,
5-[5-Bromo-4-((R)-1-hydroxymethyl-propylamino)-pyrimidin-2-ylamino]-N-(2-methoxy-benzyl)-2-methyl-benzenesulfonamide,
5-[5-Bromo-4-((R)-1-hydroxymethyl-propylamino)-pyrimidin-2-ylamino]-2-methyl-N-[2-(4-sulfamoyl-phenyl)-ethyl]-benzenesulfonamide,
5-[5-Bromo-4-((R)-1-hydroxymethyl-propylamino)-pyrimidin-2-ylamino]-2-methyl-N-(4-methyl-benzyl)-benzenesulfonamide,
5-[5-Bromo-4-((R)-2-hydroxy-1-methyl-ethylamino)-pyrimidin-2-ylamino]-N-cyclohexylmethyl-2-methyl-benzenesulfonamide,
5-[5-Bromo-4-((R)-2-hydroxy-1-methyl-ethylamino)-pyrimidin-2-ylamino]-2-methyl-N-(4-phenyl-butyl)-benzenesulfonamide,
5-[5-Bromo-4-((R)-2-hydroxy-1-methyl-ethylamino)-pyrimidin-2-ylamino]-N-(2-dimethylamino-ethyl)-2-methyl-benzenesulfonamide,
5-[5-Bromo-4-((R)-2-hydroxy-1-methyl-ethylamino)-pyrimidin-2-ylamino]-N-(3-imidazol-1-yl-propyl)-2-methyl-benzenesulfonamide,
5-[5-Bromo-4-((R)-2-hydroxy-1-methyl-ethylamino)-pyrimidin-2-ylamino]-2-methyl-N-(3-trifluoromethyl-benzyl)-benzenesulfonamide,
5-[5-Bromo-4-((R)-2-hydroxy-1-methyl-ethylamino)-pyrimidin-2-ylamino]-N-furan-2-ylmethyl-2-methyl-benzenesulfonamide,
5-[5-Bromo-4-((R)-2-hydroxy-1-methyl-ethylamino)-pyrimidin-2-ylamino]-2-methyl-N-(4-trifluoromethyl-benzyl)-benzenesulfonamide,
5-[5-Bromo-4-((R)-2-hydroxy-1-methyl-ethylamino)-pyrimidin-2-ylamino]-2-methyl-N-(3-phenyl-propyl)-benzenesulfonamide,
5-[5-Bromo-4-((R)-2-hydroxy-1-methyl-ethylamino)-pyrimidin-2-ylamino]-2-methyl-N-[3-(2-oxo-pyrrolidin-1-yl)-propyl]-benzenesulfonamide,
5-[5-Bromo-4-((R)-2-hydroxy-1-methyl-ethylamino)-pyrimidin-2-ylamino]-2-methyl-N-(3-methyl-butyl)-benzenesulfonamide,
5-[5-Bromo-4-((R)-2-hydroxy-1-methyl-ethylamino)-pyrimidin-2-ylamino]-N-hexyl-2-methyl-benzenesulfonamide,
5-[5-Bromo-4-((R)-2-hydroxy-1-methyl-ethylamino)-pyrimidin-2-ylamino]-N-(3-dimethylamino-propyl)-2-methyl-benzenesulfonamide,
5-[5-Bromo-4-((R)-2-hydroxy-1-methyl-ethylamino)-pyrimidin-2-ylamino]-N-(2-diisopropylamino-ethyl)-2-methyl-benzenesulfonamide,
5-[5-Bromo-4-((R)-2-hydroxy-1-methyl-ethylamino)-pyrimidin-2-ylamino]-N-cyclopropyl-2-methyl-benzenesulfonamide,
5-[5-Bromo-4-((R)-2-hydroxy-1-methyl-ethylamino)-pyrimidin-2-ylamino]-N-(4-cyano-cyclohexylmethyl)-2-methyl-benzenesulfonamide,
5-[5-Bromo-4-((R)-2-hydroxy-1-methyl-ethylamino)-pyrimidin-2-ylamino]-2-methyl-N-phenethyl-benzenesulfonamide,
5-[5-Bromo-4-((R)-2-hydroxy-1-methyl-ethylamino)-pyrimidin-2-ylamino]-N-[2-(4-chloro-phenyl)-ethyl]-2-methyl-benzenesulfonamide,
5-[5-Bromo-4-((R)-2-hydroxy-1-methyl-ethylamino)-pyrimidin-2-ylamino]-2-methyl-N-(5-methyl-furan-2-ylmethyl)-benzenesulfonamide,
5-[5-Bromo-4-((R)-2-hydroxy-1-methyl-ethylamino)-pyrimidin-2-ylamino]-2-methyl-N-(2-morpholin-4-yl-ethyl)-benzenesulfonamide,
5-[5-Bromo-4-((R)-2-hydroxy-1-methyl-ethylamino)-pyrimidin-2-ylamino]-N-(2-methoxy-ethyl)-2-methyl-benzenesulfonamide,
5-[5-Bromo-4-((R)-2-hydroxy-1-methyl-ethylamino)-pyrimidin-2-ylamino]-2-methyl-N-thiophen-2-ylmethyl-benzenesulfonamide,
5-[5-Bromo-4-((R)-2-hydroxy-1-methyl-ethylamino)-pyrimidin-2-ylamino]-N-[2-(4-methoxy-phenyl)-ethyl]-2-methyl-benzenesulfonamide,
5-[5-Bromo-4-((R)-2-hydroxy-1-methyl-ethylamino)-pyrimidin-2-ylamino]-2-methyl-N-(2-pyrrolidin-1-yl-ethyl)-benzenesulfonamide,
5-[5-Bromo-4-((R)-2-hydroxy-1-methyl-ethylamino)-pyrimidin-2-ylamino]-2-methyl-N-(2-piperidin-1-yl-ethyl)-benzenesulfonamide,
5-[5-Bromo-4-((R)-2-hydroxy-1-methyl-ethylamino)-pyrimidin-2-ylamino]-N-(3-fluoro-benzyl)-2-methyl-benzenesulfonamide,
5-[5-Bromo-4-((R)-2-hydroxy-1-methyl-ethylamino)-pyrimidin-2-ylamino]-2-methyl-N-[2-(1-methyl-pyrrolidin-2-yl)-ethyl]-benzenesulfonamide,
5-[5-Bromo-4-((R)-2-hydroxy-1-methyl-ethylamino)-pyrimidin-2-ylamino]-2-methyl-N-pyridin-3-ylmethyl-benzenesulfonamide,
5-[5-Bromo-4-((R)-2-hydroxy-1-methyl-ethylamino)-pyrimidin-2-ylamino]-N-(4-fluoro-benzyl)-2-methyl-benzenesulfonamide,
5-[5-Bromo-4-((R)-2-hydroxy-1-methyl-ethylamino)-pyrimidin-2-ylamino]-2-methyl-N-pyridin-4-ylmethyl-benzenesulfonamide,
5-[5-Bromo-4-((R)-2-hydroxy-1-methyl-ethylamino)-pyrimidin-2-ylamino]-2-methyl-N-(2-phenoxy-ethyl)-benzenesulfonamide,
5-[5-Bromo-4-((R)-2-hydroxy-1-methyl-ethylamino)-pyrimidin-2-ylamino]-N-(2-methoxy-benzyl)-2-methyl-benzenesulfonamide,
5-[5-Bromo-4-((R)-2-hydroxy-1-methyl-ethylamino)-pyrimidin-2-ylamino]-2-methyl-N-[2-(4-sulfamoyl-phenyl)-ethyl]-benzenesulfonamide,
5-[5-Bromo-4-((R)-2-hydroxy-1-methyl-ethylamino)-pyrimidin-2-ylamino]-2-methyl-N-(4-methyl-benzyl)-benzenesulfonamide,
3-[5-Bromo-4-((R)-1-hydroxymethyl-propylamino)-pyrimidin-2-ylamino]-N-cyclohexylmethyl-benzenesulfonamide,
3-[5-Bromo-4-((R)-1-hydroxymethyl-propylamino)-pyrimidin-2-ylamino]-N-(3-imidazol-1-yl-propyl)-benzenesulfonamide,
3-[5-Bromo-4-((R)-1-hydroxymethyl-propylamino)-pyrimidin-2-ylamino]-N-(3-trifluoromethyl-benzyl)-benzenesulfonamide,
3-[5-Bromo-4-((R)-1-hydroxymethyl-propylamino)-pyrimidin-2-ylamino]-N-furan-2-ylmethyl-benzenesulfonamide,
3-[5-Bromo-4-((R)-1-hydroxymethyl-propylamino)-pyrimidin-2-ylamino]-N-(4-trifluoromethyl-benzyl)-benzenesulfonamide,
3-[5-Bromo-4-((R)-1-hydroxymethyl-propylamino)-pyrimidin-2-ylamino]-N-(3-phenyl-propyl)-benzenesulfonamide,
3-[5-Bromo-4-((R)-1-hydroxymethyl-propylamino)-pyrimidin-2-ylamino]-N-[3-(2-oxo-pyrrolidin-1-yl)-propyl]-benzenesulfonamide,
3-[5-Bromo-4-((R)-1-hydroxymethyl-propylamino)-pyrimidin-2-ylamino]-N-(3-methyl-butyl)-benzenesulfonamide,
3-[5-Bromo-4-((R)-1-hydroxymethyl-propylamino)-pyrimidin-2-ylamino]-N-(3-dimethylamino-propyl)-benzenesulfonamide,
3-[5-Bromo-4-((R)-1-hydroxymethyl-propylamino)-pyrimidin-2-ylamino]-N-(2-diisopropylamino-ethyl)-benzenesulfonamide,
3-[5-Bromo-4-((R)-1-hydroxymethyl-propylamino)-pyrimidin-2-ylamino]-N-(4-cyano-cyclohexylmethyl)-benzenesulfonamide,
3-[5-Bromo-4-((R)-1-hydroxymethyl-propylamino)-pyrimidin-2-ylamino]-N-phenethyl-benzenesulfonamide,
3-[5-Bromo-4-((R)-1-hydroxymethyl-propylamino)-pyrimidin-2-ylamino]-N-[2-(4-chloro-phenyl)-ethyl]-benzenesulfonamide,
3-[5-Bromo-4-((R)-1-hydroxymethyl-propylamino)-pyrimidin-2-ylamino]-N-(5-methyl-furan-2-ylmethyl)-benzenesulfonamide,
3-[5-Bromo-4-((R)-1-hydroxymethyl-propylamino)-pyrimidin-2-ylamino]-N-(2-morpholin-4-yl-ethyl)-benzenesulfonamide,
3-[5-Bromo-4-((R)-1-hydroxymethyl-propylamino)-pyrimidin-2-ylamino]-N-(2-methoxy-ethyl)-benzenesulfonamide,
3-[5-Bromo-4-((R)-1-hydroxymethyl-propylamino)-pyrimidin-2-ylamino]-N-thiophen-2-ylmethyl-benzenesulfonamide,
3-[5-Bromo-4-((R)-1-hydroxymethyl-propylamino)-pyrimidin-2-ylamino]-N-(2-pyrrolidin-1-yl-ethyl)-benzenesulfonamide,
3-[5-Bromo-4-((R)-1-hydroxymethyl-propylamino)-pyrimidin-2-ylamino]-N-(2-piperidin-1-yl-ethyl)-benzenesulfonamide,
3-[5-Bromo-4-((R)-1-hydroxymethyl-propylamino)-pyrimidin-2-ylamino]-N-(3-fluoro-benzyl)-benzenesulfonamide,
3-[5-Bromo-4-((R)-1-hydroxymethyl-propylamino)-pyrimidin-2-ylamino]-N-[2-(1-methyl-pyrrolidin-2-yl)-ethyl]-benzenesulfonamide,
3-[5-Bromo-4-((R)-1-hydroxymethyl-propylamino)-pyrimidin-2-ylamino]-N-pyridin-3-ylmethyl-benzenesulfonamide,
3-[5-Bromo-4-((R)-1-hydroxymethyl-propylamino)-pyrimidin-2-ylamino]-N-(4-fluoro-benzyl)-benzenesulfonamide,
3-[5-Bromo-4-((R)-1-hydroxymethyl-propylamino)-pyrimidin-2-ylamino]-N-pyridin-4-ylmethyl-benzenesulfonamide,
3-[5-Bromo-4-((R)-1-hydroxymethyl-propylamino)-pyrimidin-2-ylamino]-N-(2-phenoxy-ethyl)-benzenesulfonamide,
3-[5-Bromo-4-((R)-1-hydroxymethyl-propylamino)-pyrimidin-2-ylamino]-N-(2-methoxy-benzyl)-benzenesulfonamide,
3-[5-Bromo-4-((R)-1-hydroxymethyl-propylamino)-pyrimidin-2-ylamino]-N-(4-methyl-benzyl)-benzenesulfonamide,
3-[5-Bromo-4-((R)-1-hydroxymethyl-propylamino)-pyrimidin-2-ylamino]-N-(5-methylsulfanyl-1H-[1,2,4]triazol-3-yl)-benzenesulfonamide,
3-[5-Bromo-4-((R)-2-hydroxy-1-methyl-ethylamino)-pyrimidin-2-ylamino]-N-(2-dimethylamino-ethyl)-benzenesulfonamide,
3-[5-Bromo-4-((R)-2-hydroxy-1-methyl-ethylamino)-pyrimidin-2-ylamino]-N-(3-imidazol-1-yl-propyl)-benzenesulfonamide,
3-[5-Bromo-4-((R)-2-hydroxy-1-methyl-ethylamino)-pyrimidin-2-ylamino]-N-(3-trifluoromethyl-benzyl)-benzenesulfonamide,
3-[5-Bromo-4-((R)-2-hydroxy-1-methyl-ethylamino)-pyrimidin-2-ylamino]-N-furan-2-ylmethyl-benzenesulfonamide,
3-[5-Bromo-4-((R)-2-hydroxy-1-methyl-ethylamino)-pyrimidin-2-ylamino]-N-(4-trifluoromethyl-benzyl)-benzenesulfonamide,
3-[5-Bromo-4-((R)-2-hydroxy-1-methyl-ethylamino)-pyrimidin-2-ylamino]-N-[3-(2-oxo-pyrrolidin-1-yl)-propyl]-benzenesulfonamide,
3-[5-Bromo-4-((R)-2-hydroxy-1-methyl-ethylamino)-pyrimidin-2-ylamino]-N-(3-methyl-butyl)-benzenesulfonamide,
3-[5-Bromo-4-((R)-2-hydroxy-1-methyl-ethylamino)-pyrimidin-2-ylamino]-N-(3-dimethylamino-propyl)-benzenesulfonamide,
3-[5-Bromo-4-((R)-2-hydroxy-1-methyl-ethylamino)-pyrimidin-2-ylamino]-N-(2-diisopropylamino-ethyl)-benzenesulfonamide,
3-[5-Bromo-4-((R)-2-hydroxy-1-methyl-ethylamino)-pyrimidin-2-ylamino]-N-(4-cyano-cyclohexylmethyl)-benzenesulfonamide,
3-[5-Bromo-4-((R)-2-hydroxy-1-methyl-ethylamino)-pyrimidin-2-ylamino]-N-phenethyl-benzenesulfonamide,
3-[5-Bromo-4-((R)-2-hydroxy-1-methyl-ethylamino)-pyrimidin-2-ylamino]-N-[2-(4-chloro-phenyl)-ethyl]-benzenesulfonamide,
3-[5-Bromo-4-((R)-2-hydroxy-1-methyl-ethylamino)-pyrimidin-2-ylamino]-N-(5-methyl-furan-2-ylmethyl)-benzenesulfonamide,
3-[5-Bromo-4-((R)-2-hydroxy-1-methyl-ethylamino)-pyrimidin-2-ylamino]-N-(2-morpholin-4-yl-ethyl)-benzenesulfonamide,
3-[5-Bromo-4-((R)-2-hydroxy-1-methyl-ethylamino)-pyrimidin-2-ylamino]-N-(2-methoxy-ethyl)-benzenesulfonamide,
3-[5-Bromo-4-((R)-2-hydroxy-1-methyl-ethylamino)-pyrimidin-2-ylamino]-N-thiophen-2-ylmethyl-benzenesulfonamide,
3-[5-Bromo-4-((R)-2-hydroxy-1-methyl-ethylamino)-pyrimidin-2-ylamino]-N-(2-pyrrolidin-1-yl-ethyl)-benzenesulfonamide,
3-[5-Bromo-4-((R)-2-hydroxy-1-methyl-ethylamino)-pyrimidin-2-ylamino]-N-(2-piperidin-1-yl-ethyl)-benzenesulfonamide,
3-[5-Bromo-4-((R)-2-hydroxy-1-methyl-ethylamino)-pyrimidin-2-ylamino]-N-(3-fluoro-benzyl)-benzenesulfonamide,
3-[5-Bromo-4-((R)-2-hydroxy-1-methyl-ethylamino)-pyrimidin-2-ylamino]-N-[2-(1-methyl-pyrrolidin-2-yl)-ethyl]-benzenesulfonamide,
3-[5-Bromo-4-((R)-2-hydroxy-1-methyl-ethylamino)-pyrimidin-2-ylamino]-N-pyridin-3-ylmethyl-benzenesulfonamide,
3-[5-Bromo-4-((R)-2-hydroxy-1-methyl-ethylamino)-pyrimidin-2-ylamino]-N-(4-fluoro-benzyl)-benzenesulfonamide,
3-[5-Bromo-4-((R)-2-hydroxy-1-methyl-ethylamino)-pyrimidin-2-ylamino]-N-pyridin-4-ylmethyl-benzenesulfonamide,
3-[5-Bromo-4-((R)-2-hydroxy-1-methyl-ethylamino)-pyrimidin-2-ylamino]-N-(2-phenoxy-ethyl)-benzenesulfonamide,
3-[5-Bromo-4-((R)-2-hydroxy-1-methyl-ethylamino)-pyrimidin-2-ylamino]-N-(2-methoxy-benzyl)-benzenesulfonamide,
3-[5-Bromo-4-((R)-2-hydroxy-1-methyl-ethylamino)-pyrimidin-2-ylamino]-N-(4-methyl-benzyl)-benzenesulfonamide,
3-[5-Bromo-4-((R)-2-hydroxy-1-methyl-ethylamino)-pyrimidin-2-ylamino]-N-(5-methylsulfanyl-1 H-[1,2,4]triazol-3-yl)-benzenesulfonamide,
4-[5-Bromo-4-((R)-1-hydroxymethyl-propylamino)-pyrimidin-2-ylamino]-benzenesulfonamide,
3-[5-Bromo-4-((R)-2-hydroxy-1-methyl-ethylamino)-pyrimidin-2-ylamino]-benzenesulfonyl fluoride,
5-[5-Bromo-4-((R)-2-hydroxy-1-methyl-ethylamino)-pyrimidin-2-ylamino]-2-methyl-benzenesulfonyl fluoride,
3-[5-Bromo-4-((R)-1-hydroxymethyl-propylamino)-pyrimidin-2-ylamino]-benzenesulfonyl fluoride,
5-[5-Bromo-4-((R)-1-hydroxymethyl-propylamino)-pyrimidin-2-ylamino]-2-methyl-benzenesulfonyl fluoride,
3-[5-Bromo-4-((S)-1-hydroxymethyl-2-methyl-propylamino)-pyrimidin-2-ylamino]-benzenesulfonyl fluoride,
3-[5-Bromo-4-((S)-1-hydroxymethyl-2-methyl-propylamino)-pyrimidin-2-ylamino]-benzenesulfonyl fluoride,
4-[5-Bromo-4-((R)-2-hydroxy-1-methyl-ethylamino)-pyrimidin-2-ylamino]-benzenesulfonyl fluoride,
4-[5-Bromo-4-((R)-1-hydroxymethyl-propylamino)-pyrimidin-2-ylamino]-benzenesulfonyl fluoride,
4-[5-Bromo-4-((S)-1-hydroxymethyl-2-methyl-propylamino)-pyrimidin-2-ylamino]-benzenesulfonyl fluoride,
4-[5-Bromo-4-((R)-2-hydroxy-1-methyl-ethylamino)-pyrimidin-2-ylamino]-N-cyclohexylmethyl-benzenesulfonamide,
4-[5-Bromo-4-((R)-2-hydroxy-1-methyl-ethylamino)-pyrimidin-2-ylamino]-N-(4-phenyl-butyl)-benzenesulfonamide,
4-[5-Bromo-4-((R)-2-hydroxy-1-methyl-ethylamino)-pyrimidin-2-ylamino]-N-(2-dimethylamino-ethyl)-benzenesulfonamide,
4-[5-Bromo-4-((R)-2-hydroxy-1-methyl-ethylamino)-pyrimidin-2-ylamino]-N-(3-imidazol-1-yl-propyl)-benzenesulfonamide,
4-[5-Bromo-4-((R)-2-hydroxy-1-methyl-ethylamino)-pyrimidin-2-ylamino]-N-(3-trifluoromethyl-benzyl)-benzenesulfonamide,
4-[5-Bromo-4-((R)-2-hydroxy-1-methyl-ethylamino)-pyrimidin-2-ylamino]-N-(4-trifluoromethyl-benzyl)-benzenesulfonamide,
4-[5-Bromo-4-((R)-2-hydroxy-1-methyl-ethylamino)-pyrimidin-2-ylamino]-N-(3-phenyl-propyl)-benzenesulfonamide,
4-[5-Bromo-4-((R)-2-hydroxy-1-methyl-ethylamino)-pyrimidin-2-ylamino]-N-[3-(2-oxo-pyrrolidin-1-yl)-propyl]-benzenesulfonamide,
4-[5-Bromo-4-((R)-2-hydroxy-1-methyl-ethylamino)-pyrimidin-2-ylamino]-N-(3-methyl-butyl)-benzenesulfonamide,
4-[5-Bromo-4-((R)-2-hydroxy-1-methyl-ethylamino)-pyrimidin-2-ylamino]-N-(2-diisopropylamino-ethyl)-benzenesulfonamide,
4-[5-Bromo-4-((R)-2-hydroxy-1-methyl-ethylamino)-pyrimidin-2-ylamino]-N-cyclopropyl-benzenesulfonamide,
4-[5-Bromo-4-((R)-2-hydroxy-1-methyl-ethylamino)-pyrimidin-2-ylamino]-N-(4-cyano-cyclohexylmethyl)-benzenesulfonamide,
4-[5-Bromo-4-((R)-2-hydroxy-1-methyl-ethylamino)-pyrimidin-2-ylamino]-N-phenethyl-benzenesulfonamide,
4-[5-Bromo-4-((R)-2-hydroxy-1-methyl-ethylamino)-pyrimidin-2-ylamino]-N-[2-(4-chloro-phenyl)-ethyl]-benzenesulfonamide,
4-[5-Bromo-4-((R)-2-hydroxy-1-methyl-ethylamino)-pyrimidin-2-ylamino]-N-(5-methyl-furan-2-ylmethyl)-benzenesulfonamide,
4-[5-Bromo-4-((R)-2-hydroxy-1-methyl-ethylamino)-pyrimidin-2-ylamino]-N-(2-morpholin-4-yl-ethyl)-benzenesulfonamide,
4-[5-Bromo-4-((R)-2-hydroxy-1-methyl-ethylamino)-pyrimidin-2-ylamino]-N-(2-methoxy-ethyl)-benzenesulfonamide,
4-[5-Bromo-4-((R)-2-hydroxy-1-methyl-ethylamino)-pyrimidin-2-ylamino]-N-thiophen-2-ylmethyl-benzenesulfonamide,
4-[5-Bromo-4-((R)-2-hydroxy-1-methyl-ethylamino)-pyrimidin-2-ylamino]-N-[2-(4-methoxy-phenyl)-ethyl]-benzenesulfonamide,
4-[5-Bromo-4-((R)-2-hydroxy-1-methyl-ethylamino)-pyrimidin-2-ylamino]-N-(2-pyrrolidin-1-yl-ethyl)-benzenesulfonamide,
4-[5-Bromo-4-((R)-2-hydroxy-1-methyl-ethylamino)-pyrimidin-2-ylamino]-N-(2-piperidin-1-yl-ethyl)-benzenesulfonamide,
4-[5-Bromo-4-((R)-2-hydroxy-1-methyl-ethylamino)pyrimidin-2-ylamino]-N-(3-fluoro-benzyl)-benzenesulfonamide,
4-[5-Bromo-4-((R)-2-hydroxy-1-methyl-ethylamino)-pyrimidin-2-ylamino]-N-[2-(1-methyl-pyrrolidin-2-yl)-ethyl]-benzenesulfonamide,
4-[5-Bromo-4-((R)-2-hydroxy-1-methyl-ethylamino)-pyrimidin-2-ylamino]-N-pyridin-3-ylmethyl-benzenesulfonamide,
4-[5-Bromo-4-((R)-2-hydroxy-1-methyl-ethylamino)-pyrimidin-2-ylamino]-N-(4-fluoro-benzyl)-benzenesulfonamide,
4-[5-Bromo-4-((R)-2-hydroxy-1-methyl-ethylamino)-pyrimidin-2-ylamino]-N-pyridin-4-ylmethyl-benzenesulfonamide,
4-[5-Bromo-4-((R)-2-hydroxy-1-methyl-ethylamino)-pyrimidin-2-ylamino]-N-(2-phenoxy-ethyl)-benzenesulfonamide,
4-[5-Bromo-4-((R)-2-hydroxy-1-methyl-ethylamino)-pyrimidin-2-ylamino]-N-(2-methoxy-benzyl)-benzenesulfonamide,
4-[5-Bromo-4-((R)-2-hydroxy-1-methyl-ethylamino)-pyrimidin-2-ylamino]-N-(4-methyl-benzyl)-benzenesulfonamide,
4-[5-Bromo-4-((R)-2-hydroxy-1-methyl-ethylamino)-pyrimidin-2-ylamino]-N-(5-methylsulfanyl-1H-[1,2,4]triazol-3-yl)-benzenesulfonamide,
4-[5-Bromo-4-((R)-1-hydroxymethyl-propylamino)-pyrimidin-2-ylamino]-N-cyclohexylmethyl-benzenesulfonamide,
4-[5-Bromo-4-((R)-1-hydroxymethyl-propylamino)-pyrimidin-2-ylamino]-N-(2-dimethylamino-ethyl)-benzenesulfonamide,
4-[5-Bromo-4-((R)-1-hydroxymethyl-propylamino)-pyrimidin-2-ylamino]-N-(3-imidazol-1-yl-propyl)-benzenesulfonamide,
4-[5-Bromo-4-((R)-1-hydroxymethyl-propylamino)-pyrimidin-2-ylamino]-N-(3-trifluoromethyl-benzyl)-benzenesulfonamide,
4-[5-Bromo-4-((R)-1-hydroxymethyl-propylamino)-pyrimidin-2-ylamino]-N-furan-2-ylmethyl-benzenesulfonamide,
4-[5-Bromo-4-((R)-1-hydroxymethyl-propylamino)-pyrimidin-2-ylamino]-N-(4-trifluoromethyl-benzyl)-benzenesulfonamide,
4-[5-Bromo-4-((R)-1-hydroxymethyl-propylamino)-pyrimidin-2-ylamino]-N-(3-phenyl-propyl)-benzenesulfonamide,
4-[5-Bromo-4-((R)-1-hydroxymethyl-propylamino)-pyrimidin-2-ylamino]-N-[3-(2-oxo-pyrrolidin-1-yl)-propyl]-benzenesulfonamide,
4-[5-Bromo-4-((R)-1-hydroxymethyl-propylamino)-pyrimidin-2-ylamino]-N-(3-methyl-butyl)-benzenesulfonamide,
4-[5-Bromo-4-((R)-1-hydroxymethyl-propylamino)-pyrimidin-2-ylamino]-N-(3-dimethylamino-propyl)-benzenesulfonamide,
4-[5-Bromo-4-((R)-1-hydroxymethyl-propylamino)-pyrimidin-2-ylamino]-N-(2-diisopropylamino-ethyl)-benzenesulfonamide,
4-[5-Bromo-4-((R)-1-hydroxymethyl-propylamino)-pyrimidin-2-ylamino]-N-cyclopropyl-benzenesulfonamide,
4-[5-Bromo-4-((R)-1-hydroxymethyl-propylamino)-pyrimidin-2-ylamino]-N-(4-cyano-cyclohexylmethyl)-benzenesulfonamide,
4-[5-Bromo-4-((R)-1-hydroxymethyl-propylamino)-pyrimidin-2-ylamino]-N-phenethyl-benzenesulfonamide,
4-[5-Bromo-4-((R)-1-hydroxymethyl-propylamino)-pyrimidin-2-ylamino]-N-(3,4-dimethoxy-benzyl)-benzenesulfonamide,
4-[5-Bromo-4-((R)-1-hydroxymethyl-propylamino)-pyrimidin-2-ylamino]-N-[2-(4-chloro-phenyl)-ethyl]-benzenesulfonamide,
4-[5-Bromo-4-((R)-1-hydroxymethyl-propylamino)-pyrimidin-2-ylamino]-N-(5-methyl-furan-2-ylmethyl)-benzenesulfonamide,
4-[5-Bromo-4-((R)-1-hydroxymethyl-propylamino)-pyrimidin-2-ylamino]-N-(2-morpholin-4-yl-ethyl)-benzenesulfonamide,
4-[5-Bromo-4-((R)-1-hydroxymethyl-propylamino)-pyrimidin-2-ylamino]-N-(2-methoxy-ethyl)-benzenesulfonamide,
4-[5-Bromo-4-((R)-1-hydroxymethyl-propylamino)-pyrimidin-2-ylamino]-N-thiophen-2-ylmethyl-benzenesulfonamide,
4-[5-Bromo-4-((R)-1-hydroxymethyl-propylamino)-pyrimidin-2-ylamino]-N-[2-(4-methoxy-phenyl)-ethyl]-benzenesulfonamide,
4-[5-Bromo-4-((R)-1-hydroxymethyl-propylamino)-pyrimidin-2-ylamino]-N-(2-pyrrolidin-1-yl-ethyl)-benzenesulfonamide,
4-[5-Bromo-4-((R)-1-hydroxymethyl-propylamino)-pyrimidin-2-ylamino]-N-(2-piperidin-1-yl-ethyl)-benzenesulfonamide,
4-[5-Bromo-4-((R)-1-hydroxymethyl-propylamino)-pyrimidin-2-ylamino]-N-(3-fluoro-benzyl)-benzenesulfonamide,
4-[5-Bromo-4-((R)-1-hydroxymethyl-propylamino)-pyrimidin-2-ylamino]-N-[2-(1-methyl-pyrrolidin-2-yl)-ethyl]-benzenesulfonamide,
4-[5-Bromo-4-((R)-1-hydroxymethyl-propylamino)-pyrimidin-2-ylamino]-N-pyridin-3-ylmethyl-benzenesulfonamide,
4-[5-Bromo-4-((R)-1-hydroxymethyl-propylamino)-pyrimidin-2-ylamino]-N-(4-fluoro-benzyl)-benzenesulfonamide,
4-[5-Bromo-4-((R)-1-hydroxymethyl-propylamino)-pyrimidin-2-ylamino]-N-pyridin-4-ylmethyl-benzenesulfonamide,
4-[5-Bromo-4-((R)-1-hydroxymethyl-propylamino)-pyrimidin-2-ylamino]-N-(2-phenoxy-ethyl)-benzenesulfonamide,
4-[5-Bromo-4-((R)-1-hydroxymethyl-propylamino)-pyrimidin-2-ylamino]-N-(2-methoxy-benzyl)-benzenesulfonamide,
4-[5-Bromo-4-((R)-1-hydroxymethyl-propylamino)-pyrimidin-2-ylamino]-N-(4-methyl-benzyl)-benzenesulfonamide or
4-[5-Bromo-4-((R)-1-hydroxymethyl-propylamino)-pyrimidin-2-ylamino]-N-(5-methylsulfanyl-1H-[1,2,4]triazol-3-yl)-benzenesulfonamide.

3. Verwendung der Verbindung der allgemeinen Formel (la) 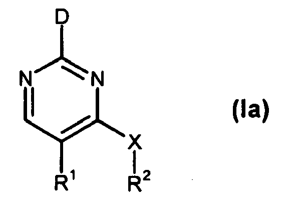 in der D für Cl steht, und X für -NH- oder -O- steht, R¹, und R² die in der allgemeinen Formel (I) angegebenen Bedeutungen haben, als Zwischenprodukte zur Herstellung der Verbindung der allgemeinen Formel (I) durch Umsetzung in MeCN/HCl mit Ar-NH₂.

4. Verwendung der Verbindungen der allgemeinen Formel (I), gemäß den Ansprüchen 1 oder 2, zur Herstellung eines Arzneimittels zur Behandlung von Krebs, Autoimmunerkrankungen, Chemotherapeutika-induzierter Alopezie und Mukositis, kardiovaskulären Erkrankungen, infektiösen Erkrankungen, nephrologischen Erkrankungen, chronisch und akut neurodegenerativen Erkrankungen und viralen Infektionen.

5. Verwendung gemäß Anspruch 4, **dadurch gekennzeichnet, daß** unter Krebs solide Tumoren und Leukämie, unter Autoimmunerkrankungen Psoriasis, Alopezie und Multiple Sklerose, unter kardiovaskulären Erkrankungen Stenosen, Arteriosklerosen und Restenosen, unter infektiösen Erkrankungen durch unizelluläre Parasiten hervorgerufene Erkrankungen, unter nephrologischen Erkrankungen Glomerulonephritis, unter chronisch neurodegenerativen Erkrankungen Huntington's Erkrankung, amyotrophe Lateralsklerose, Parkinsonsche Erkrankung, AIDS Dementia und Alzheimer'sche Erkrankung, unter akut neurodegenerativen Erkrankungen Ischämien des Gehirns und Neurotraumata, und unter viralen Infektionen Cytomegalus-Infektionen, Herpes, Hepatitis B und C und HIV Erkrankungen zu verstehen sind.

6. Arzneimittel, die mindestens eine Verbindung gemäß Anspruch 1 oder 2 enthalten.

7. Arzneimittel gemäß Anspruch 6, zur Behandlung von Krebs, Autoimmunerkrankungen, kardiovaskulären Erkrankungen, infektiöse Erkrankungen, nephrologische Erkrankungen, neurodegenerative Erkrankungen und virale Infektionen.

8. Pharmazeutisches Präparat, enthaltend Verbindungen gemäß Anspruch 1 oder 2 mit geeigneten Formulierungs-und Trägerstoffen.

## Claims

1. Compounds of the general formula I 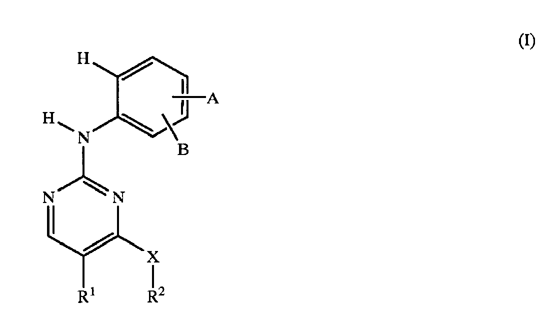 in which
R¹ stands for halogen,
R² stands for -CH(CH₃)-(CH₂)ₙ-R⁵, -CH-(CH₂OH)₂, -(CH₂)ₙR⁷, -CH(C₃H₇)-(CH₂)ₙ-R⁵, -CH(C₂H₅)-(CH₂)ₙ-R⁵, -CH₂-CN, -CH(CH₃) COCH₃, -CH(CH₃)-C(OH)(CH₃)₂,-CH(CH(OH)CH₃)OCH₃, -CH(C₂H₅)CO-R⁵, C₂-C₄-alkinyl, -(CH₂)ₙ-COR⁵, -(CH₂)ₙ-CO-C₁-C₆-alkyl, -(CH₂)ₙ-C(OH)(CH₃)-phenyl, -CH(CH₃)-C(CH₃)-R⁵, -CH(CH₃)-C(CH₃)(C₂H₅)-R⁵, -CH(OCH₃)-CH₂-R⁵, -CH₂-CH(OH)-R⁵, -CH(OCH₃)-CHR⁵-CH₃, -CH(CH₃)-CH(OH)-CH₂-CH=CH_{2,} -CH(C₂H₅)-CH(OH)-(CH₂)ₙ-CH₃, -CH(CH₃)-CH(OH)-(CH₂)ₙ-CH₃, -CH(CH₃)-CH(OH)-CH(CH₃)₂, (CH₂OAC)₂, -(CH₂)ₙ-R⁶, -(CH₂)ₙ-(CF₂)ₙ-CF₃, -CH (CH₂)ₙ-R⁵)₂, -CH(CH₃)-CO-NH₂, -CH(CH₂OH)-phenyl, -CH(CH₂OH)-CH(OH)-(CH₂)ₙR⁵-, -CH(CH₂OH)-CH(OH)-phenyl , -CH(CH₂OH)-C₂H₄-R⁵, -(CH₂)ₙ-C≡C-C(CH₃)=CH-COR⁵, -CH(Ph)-(CH₂)ₙR⁵, -(CH₂)ₙ-COR⁵, -(CH₂)ₙPO₃(R⁵)₂, -(CH₂)ₙ-COR⁵, -CH((CH₂)ₙOR⁵)CO-R⁵, -(CH₂)ₙCONHCH((CH₂)ₙR⁵)₂, -(CH₂)ₙNH-COR⁵, -CH(CH₂)ₙR⁵-(CH₂)ₙC₃-C₁₀-cycloalkyl, -(CH₂)ₙ-C₃-C₁₀-cycloalkyl, C₃-C₁₀-cycloalkyl; C₁-C₆-alkyl, C₃-C₁₀-cycloalkyl, -(CH₂)ₙ-O-(CH₂)ₙ-R⁵, -(CH₂)ₙ-NR³R⁴ that is optionally substituted in one or more places in the same way or differently with hydroxy, C₁-C₆-alkyl or the group -COONH(CH₂)ₙCH₃ or -NR³R⁴, -CH(C₃H₇)-(CH₂)ₙ-OC(O)-(CH₂)ₙ-CH₃-, -(CH₂)ₙ-R⁵, -C(CH₃)₂-(CH₂)ₙ-R⁵, -C(CH₂)ₙ(CH₃)-(CH₂)ₙR⁵, -C(CH₂)ₙ-(CH₂)ₙR⁵, -CH(t-butyl)-(CH₂)ₙ-R⁵, -CCH₃(C₃H₇)-(CH₂)ₙR⁵, -CH(C₃H₇)-(CH₂)ₙ-R⁵, -CH(C₃H₇)-COR⁵, -CH(C₃H₇)-(CH₂)ₙ-OC(O)-NH-Ph, -CH((CH₂)ₙ(C₃H₇))-(CH₂)ₙR⁵, -CH(C₃H₇)-(CH₂)ₙ-OC(O)-NH-Ph(OR⁵)₃, -NR³R⁴, -NH-(CH₂)ₙ-NR³R⁴, R⁵-(CH₂)ₙ-C*H-CH(R⁵)-(CH₂)ₙ-R⁵, -(CH₂)ₙ-CO-NH-(CH₂)ₙ-CO-R⁵, -OC(O)NH-C₁-C₆-alkyl or -(CH₂)ₙ-CO-NH-(CH₂)ₙ-CH-((CH₂)ₙR⁵)₂, or for C₃-C₁₀-cycloalkyl, which is substituted with the group 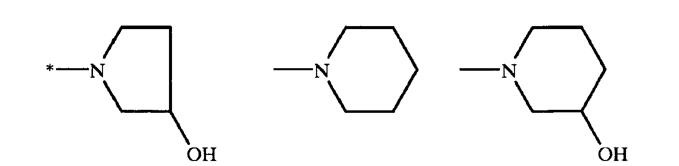 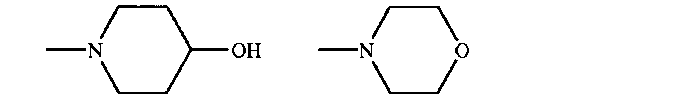 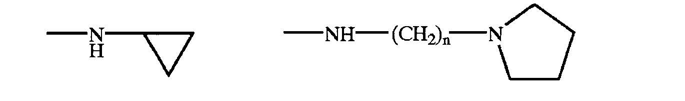 or for the group 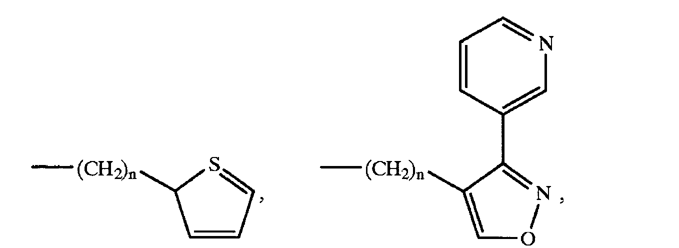 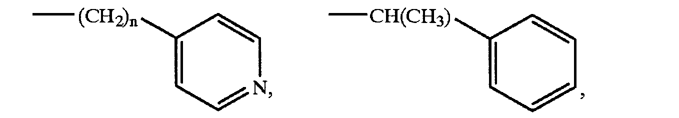 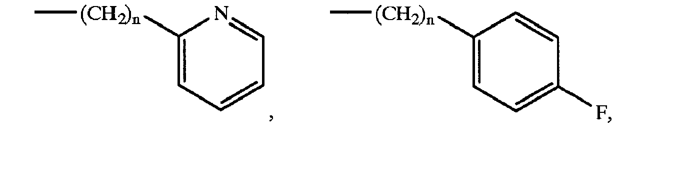 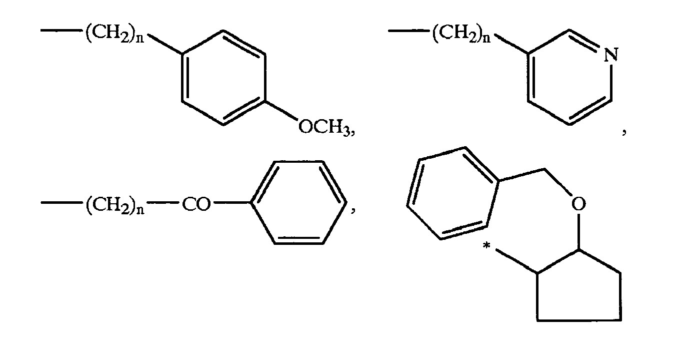 or 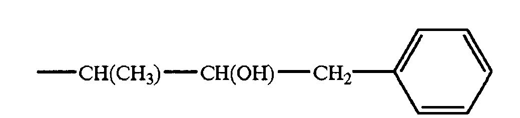 or for 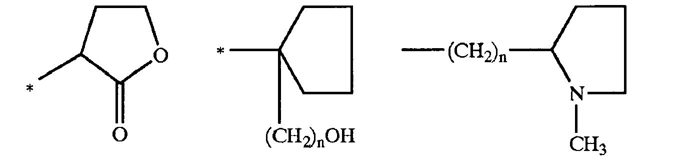 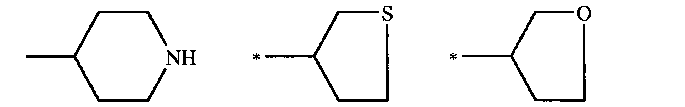 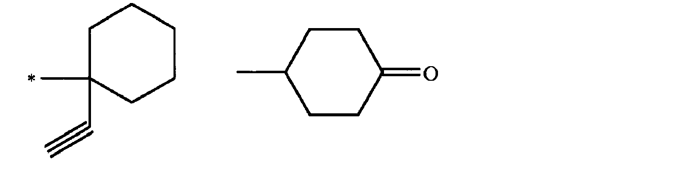 X stands for oxygen or for the group -NH-, -N(C₁-C₃-alkyl) or
R² stands for the group 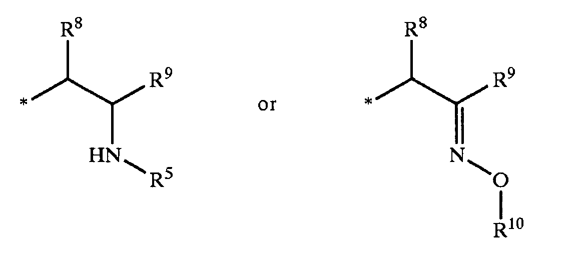 X and R² together form a group 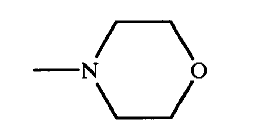 A stands for the group -SO₂R⁷, -SO₂-C₂H₄-OH, -SO₂CF₃ or -SO₂-NR³R⁴,
B stands for hydrogen, hydroxy or C₁-C₃-alkyl,
or
A and B together can form a group 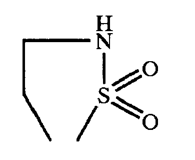 R³ and R⁴, in each case independently of one another, stand for hydrogen, phenyl, benzyloxy, C₁-C₁₂-alkyl, C₁-C₆-alkoxy, C₂-C₄-alkenyloxy, C₃-C₆-cycloalkyl, hydroxy, hydroxy-C₁-C₆-alkyl, dihydroxy-C₁-C₆-alkyl, heteroaryl, heterocyclo-C₃-C₁₀-alkyl, heteroaryl-C₁-C₃-alkyl, C₃-C₆-cycloalkyl-C₁-C₃-alkyl that is optionally substituted with cyano, or for
C₁-C₆-alkyl that is optionally substituted in one or more places in the same way or differently with phenyl, pyridyl, phenyloxy, C₃-C₆-cycloalkyl, C₁-C₆-alkyl or C₁-C₆-alkoxy, whereby the phenyl itself can be substituted in one or more places in the same way or differently with halogen, trifluoromethyl, C₁-C₆-alkyl, C₁-C₆-alkoxy or with the group -SO₂NR³R⁴,
or for the group -(CH₂)ₙNR³R⁴, -CNHNH₂ or -NR³R⁴ or for 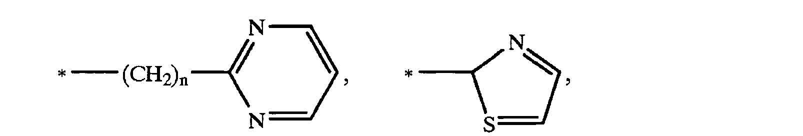 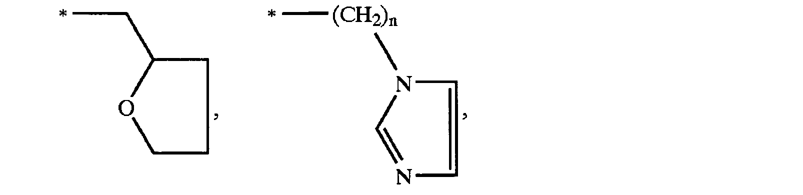 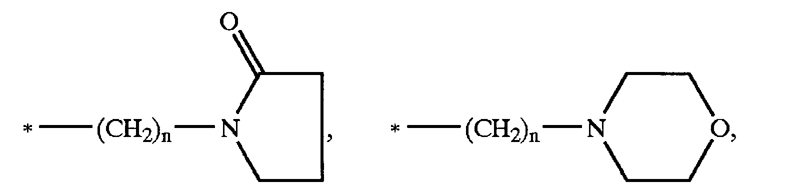 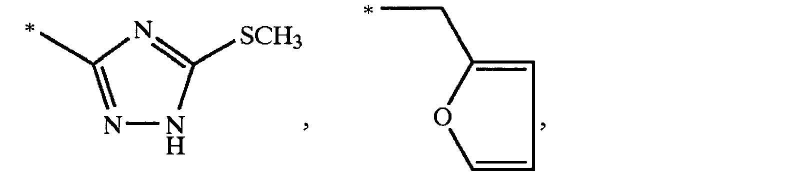 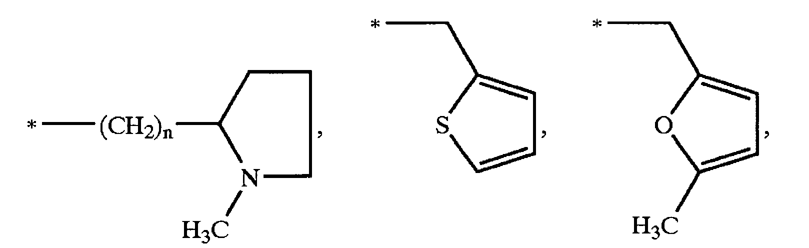 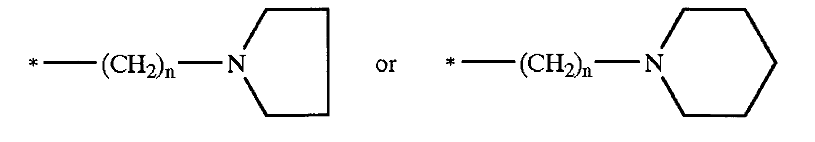 which optionally can be substituted with C₁-C₆-alkyl,
R⁵ stands for hydroxy, phenyl, C₁-C₆-alkyl, C₃-C₆-cycloalkyl, benzoxy, C₁-C₆-alkylthio or C₁-C₆-alkoxy,
R⁶ stands for the group 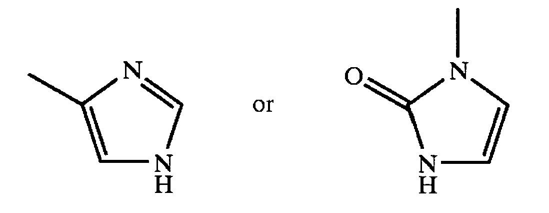 R⁷ stands for halogen, hydroxy, phenyl, C₁-C₆-alkyl, -(CH₂)ₙOH, -NR³R⁴ or the group 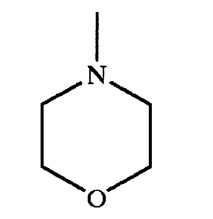 R⁸, R⁹ and
R¹⁰ stand for hydrogen, hydroxy, C₁-C₆-alkyl or for the group -(CH₂)ₙ-COOH, and
n stands for 0-6,
as well as diastereomers, enantiomers and salts thereof.

2. Compound according to Claim 1 of the following formulae:
5-bromo-N2-[4-(2-diethylamino-ethanesulfonyl)-phenyl]-N4-prop-2-ynyl-pyrimidine-2,4-diamine,
N2-(3-benzenesulfonyl-phenyl)-5-bromo-N4-prop-2-ynyl-pyrimidine-2,4-diamine,
5-bromo-N2-[4-(morpholine-4-sulfonyl)-phenyl]-N4-prop-2-ynyl-pyrimidine-2,4-diamine,
4-[5-fluoro-4-((R)-1-hydroxymethyl-2-methyl-propylamino)-pyrimidin-2-ylamino]-benzenesulfonamide,
4-[4-((R)-1-hydroxymethyl-2-methyl-propylamino)-5-iodo-pyrimidin-2-ylamino]-benzenesulfonamide,
4-(5-fluoro-4-prop-2-ynylamino-pyrimidin-2-ylamino)-benzenesulfonamide,
4-{5-bromo-4-[2-(2-oxo-2,3-dihydro-imidazol-1-yl)-ethylamino]-pyrimidin-2-ylamino}-benzenesulfonamide,
4-[5-bromo-4-(2,2,3,3,3-pentafluoro-propoxy)-pyrimidin-2-ylamino]-benzenesulfonamide,
4-[[5-bromo-4-(1,3-bisacetoxy-2-propyloxy)-2-pyrimidinyl]amino]-benzenesulfonamide,
4-[5-bromo-4-(2-hydroxy-1-hydroxymethyl-ethoxy)-pyrimidin-2-ylamino]-benzenesulfonamide,
(S)-2-[5-bromo-2-(4-sulfamoyl-phenylamino)-pyrimidin-4-ylamino]-propionamide,
5-bromo-N4-prop-2-ynyl-N2-(3-trifluoromethanesulfonyl-phenyl)-pyrimidine-2,4-diamine,
3-(5-bromo-4-prop-2-ynylamino-pyrimidin-2-ylamino)-benzenesulfonamide,
3-(5-bromo-4-prop-2-ynylamino-pyrimidin-2-ylamino)-N-butyl-benzenesulfonamide,
2-[3-(5-bromo-4-prop-2-ynytamino-pyrimidin-2-ylamino)-benzenesulfonyl]-ethanol,
4-(5-bromo-4-prop-2-ynylamino-pyrimidin-2-ylamino)-benzenesulfonamide,
4-(5-chloro-4-prop-2-ynylamino-pyrimidin-2-ylamino)-benzenesulfonamide,
2-[5-bromo-2-(4-sulfamoyl-phenylamino)-pyrimidin-4-ylamino]-butyric acid methyl ester,
4-[5-bromo-4-((R)-1-hydroxymethyl-2-methyl-propylamino)-pyrimidin-2-yl amino]-benzenesulfonamide,
2-{5-bromo-2-[3-(2-hydroxy-ethanesulfonyl)-phenylamino]-pyrimidin-4-ylamino}-propane-1,3-diol,
4-[5-bromo-4-(2-hydroxy-1-hydroxymethyl-ethylamino)-pyrimidin-2-ylamino]-benzenesulfonamide,
4-[5-bromo-4-(cyclopropylmethyl-amino)-pyrimidin-2-ylamino]-benzenesulfonamide,
4-[5-bromo-4-((R)-1-hydroxymethyl-2-methyl-propylamino)-pyrimidin-2-yl amino]-N-(2-hydroxy-ethyl)-benzenesulfonamide,
4-[5-bromo-4-((S)-2-methoxy-1-methyl-ethylamino)-pyrimidin-2-ylamino]-benzenesulfonamide,
2-{3-[5-bromo-4-((S)-2-methoxy-1-methyl-ethylamino)-pyrimidin-2-ylamino]-benzenesulfonyl}-ethanol,
2-{3-[5-bromo-4-(2-morpholin-4-yl-ethylamino)-pyrimidin-2-ylamino]-benzenesulfonyl}-ethanol,
4-[5-bromo-4-(2-morpholin-4-yl-ethylamino)-pyrimidin-2-ylamino]-benzenesulfonamide,
4-[5-bromo-4-(4,4,4-trifluoro-butoxy)-pyrimidin-2-ylamino]-benzenesulfonamide,
4-[5-bromo-4-(2-ethoxy-1-ethoxymethyl-ethoxy)-pyrimidin-2-ylamino]-benzenesulfonamide,
4-[5-bromo-2-(4-sulfamoyl-phenylamino)-pyrimidin-4-ylamino]-butyric acid,
4-[5-bromo-4-((R)-1-hydroxymethyl-2-methyl-propylamino)-pyrimidin-2-yl amino]-N-(2-hydroxy-ethyl)-benzenesulfonamide,
(R)-2-{5-bromo-2-[3-(2-hydroxy-ethanesulfonyl)-phenylamino]-pyrimldin-4-ylamino}-3-methyl-butan-1-ol,
4-[5-bromo-4-((S)-1-hydroxymethyl-2-methyl-propylamino)-pyrimidin-2-yl amino]-benzenesulfonamide,
4-[5-bromo-4-((R)-2-hydroxy-1-phenyl-ethylamino)-pyrimidin-2-ylamino]-benzenesulfonamide,
4-[5-bromo-4-((S)-2-methoxy-1-methyl-ethylamino)-pyrimidin-2-ylamino]-N-methyl-benzenesulfonamide,
4-[5-bromo-4-((S)-2-methoxy-1-methyl-ethylamino)-pyrimidin-2-ylamino]-N,N-dimethyl-benzenesulfonamide,
5-[5-bromo-2-(4-sulfamoyl-phenylaminorpyrimidin-4-ylamino]-pentanoic acid benzyl ester,
6-[5-bromo-2-(4-sulfamoyl-phenylamino)-pyrimidin-4-ylamino]-hexanoic acid methyl ester,
4-(5-iodo-4-prop-2-ynylamino-pyrimidin-2-ylamino)-benzenesulfonamide
4-[5-bromo-4-((S)-1-hydroxymethyl-3-methylsulfanyl-propylamino)-pyrimidin-2-ylamino]-benzenesulfonamide,
4-[5-bromo-4-((1S,2S)-2-hydroxy-1-hydroxymethyl-2-phenyl-ethylamino)-pyrimidin-2-ylamino]-benzenesulfonamide,
4-[5-bromo-4-((R)-2-oxo-tetrahydro-furan-3-ylamino)-pyrimidin-2-ylamino]-benzenesulfonamide,
4-[5-bromo-4-((S)-1-hydroxymethyl-2,2-dimethyl-propylamino)-pyrimidin-2-ylamino]-benzenesulfonamide,
4-{3-[5-bromo-2-(4-sulfamoyl-phenylamino)-pyrimidin-4-ylamino]-propionylamino}-butyric acid methyl ester,
6-[5-bromo-2-(4-sulfamoyl-phenylamino)-pyrimidin-4-ylamino]-hexanoic acid,
4-(5-bromo-4-cyclohexylamino-pyrimidin-2-ylamino)-benzenesulfonamide,
4-{6-[5-bromo-2-(4-sulfamoyl-phenylamino)-pyrimidin-4-ylamino]-hexanoylamino}-butyric acid methyl ester,
6-[5-bromo-2-(4-sulfamoyl-phenylaminorpyrimidin-4-ylamino]-hexanoic acid methyl ester,
4-[5-bromo-4-((1R,2S)-2,3-dihydroxy-1-hydroxymethyl-propylamino)-pyrimidin-2-ylamino]-benzenesulfonamide,
4-[5-bromo-4-((R)-2-cyclohexyl-1-hydroxymethyl-ethylamino)-pyrimidin-2-ylamino]-benzenesulfonamide,
6-[5-bromo-2-(4-sulfamoyl-phenylamino)-pyrimidin-4-ylamino]-hexanoic acid (2-hydroxy-1-hydroxymethyl-ethyl)-amide,
4-[5-bromo-4-((S)-2-hydroxy-1-methyl-ethylamino)-pyrimidin-2-ylamino]-benzenesulfonamide,
4-[5-bromo-4-(4-hydroxy-cyclohexylamino)-pyrimidin-2-ylamino]-benzenesulfonamide,
4-{4-[(adamantan-1-ylmethyl)-amino]-5-bromo-pyrimidin-2-ylamino}-benzenesulfonamide,
4-[5-bromo-4-((R)-1-hydroxymethyl-2-methyl-propylamino)-pyrimidin-2-yl amino]-N-methyl-benzenesulfonamide,
(R)-2-[5-bromo-2-(4-sulfamoyl-phenylamino)-pyrimidin-4-ylamino]-3-methyl-butyric acid,
4-(5-bromo-4-cyclohexylamino-pyrimidin-2-ylamino)-N-(2-hydroxy-ethyl)-benzenesulfonamide,
4-[5-bromo-4-(2,2,3,3,4,4,4-heptafluoro-butylamino)-pyrimidin-2-ylamino]-N-(2-hydroxy-ethyl)-benzenesulfonamide,
4-[5-bromo-4-((S)-2-methoxy-1-methyl-ethylamino)-pyrimidin-2-ylamino]-N-(2-hydroxy-ethyl)-benzenesulfonamide,
phenyl-carbamic acid (R)-2-[5-bromo-2-(4-sulfamoyl-phenylamino)-pyrimidin-4-ylamino]-3-methyl-butyl ester,
(R)-2-{5-bromo-2-[4-(2-hydroxy-ethylsulfamoyl)-phenylamino]-pyrimidin-4-ylamino}-3-methyl-butyric acid,
(R)-2-{5-bromo-2-[4-(2-hydroxy-ethylsulfamoyl)-phenylamino]-pyrimidin-4-ylamino}-3-methyl-butyric acid methyl ester,
4-(5-chloro-4-prop-2-ynylamino-pyrimidin-2-ylamino)-N-(2-hydroxy-ethyl)-benzenesulfonamide,
4-[5-bromo-4-(2-hydroxy-1,1-dimethyl-ethylamino)-pyrimidin-2-ylamino]-benzenesulfonamide,
4-[5-bromo-4-((S)-1-hydroxymethyl-3-methyl-butylamino)-pyrimidin-2-ylamino]-benzenesulfonamide,
4-[5-bromo-4-(tetrahydro-pyran-4-yloxy)-pyrimidin-2-ylamino]-benzenesulfonamide,
4-[5-bromo-4-((1S,2S)-2-hydroxy-cyclohexylamino)-pyrimidin-2-ylamino]-benzenesulfonamide,
butyl-carbamic acid 4-[5-bromo-2-(4-sulfamoyl-phenylamino)-pyrimidin-4-ylamino]-cyclohexyl ester,
(R)-2-[5-bromo-2-(4-methanesulfonyl-phenylamino)-pyrimidin-4-ylamino]-3-methyl-butan-1-ol,
(R)-2-{5-bromo-2-[4-(morpholine-4-sulfonyl)-phenylamino]-pyrimidin-4-ylamino}-3-methyl-butan-1-ol,
4-[5-bromo-4-(4-dimethylamino-cyclohexylamino)-pyrimidin-2-ylamino]-benzenesulfonamide,
4-[5-bromo-4-(4-dimethylamino-cyclohexylamino)-pyrimidin-2-ylamino]-benzenesulfonamide,
4-[5-bromo-4-(4-piperidin-1-yl-cyclohexylamino)-pyrimidin-2-ylamino]-benzenesulfonamide,
4-[5-bromo-4-((R)-1-hydroxymethyl-2-methyl-propylamino)-pyrimidin-2-yl amino]-3-hydroxy-benzenesulfonamide,
4-[5-bromo-4-(4-cyclopropylamino-cyclohexylamino)-pyrimidin-2-ylamino] -benzenesulfonamide,
(R)-2-[5-bromo-2-(3-methanesulfonyl-phenylamino)-pyrimidin-4-ylamino]-3-methyl-butan-1-ol,
4-{5-bromo-4-[2-(2-ethoxy-ethoxy)-ethoxy]-pyrimidin-2-ylamino}-benzenesulfonamide,
4-{5-bromo-4-[4-(2-dimethylamino-ethylamino)-cyclohexylamino]-pyrimidin-2-ylamino}-benzenesulfonamide,
4-{5-bromo-4-[4-(2-pyrrolidin-1-yl-ethylamino)-cyclohexylamino]-pyrimidin-2-ylamino}-benzenesulfonamide,
4-{5-bromo-4-[4-(4-hydroxy-piperidin-1-yl)-cyclohexylamino]-pyrimidin-2-ylamino}-benzenesulfonamide,
4-{5-bromo-2-[4-(2-hydroxy-ethylsulfamoyl)-phenylamino]-pyrimidin-4-ylamino}-butyric acid methyl ester,
4-{5-bromo-4-[4-(3-hydroxy-pyrrolidin-1-yl)-cyclohexylamino]-pyrimidin-2-ylamino}-benzenesulfonamide,
4-{5-bromo-4-[4-(3-hydroxy-pyrrolidin-1-yl)-cyclohexylamino]-pyrimidin-2-ylamino}-benzenesulfonamide,
4-[5-bromo-4-((R)-1-hydroxymethyl-3-methyl-butylamino)-pyrimidin-2-ylamino]-benzenesulfonamide,
4-{5-bromo-4-[2-(1-methyl-pyrrolidin-2-yl)-ethylamino]-pyrimidin-2-ylamino}-benzenesulfonamide,
4-[5-bromo-4-(piperidin-4-yloxy)-pyrimidin-2-ylamino]-benzenesulfonamide,
4-[5-bromo-4-(3-dimethylamino-propylamino)-pyrimidin-2-ylamino]-benzenesulfonamide,
acetic acid (R)-2-[5-bromo-2-(4-sulfamoyl-phenylamino)-pyrimidin-4-ylamino]-3-methyl-butyl ester,
4-[5-bromo-4-(4-hydroxy-cyclohexylamino)-pyrimidin-2-ylamino]-N-(2-hydroxyethyl)-benzenesulfonamide,
(3,4,5- trimethoxy-phenyl)-carbamicacid (R)-2-[5-bromo-2-(4-sulfamoyl-phenylamino)-pyrimidin-4-ylamino]-3-methyl-butyl ester,
4-(5-bromo-4-cycloheptylamino-pyrimidin-2-ylamino)-benzenesulfonamide,
4-[5-bromo-4-((R)-1-hydroxymethyl-2,2-dimethyl-propylamino)-pyrimidin-2-ylamino]-benzenesulfonamide,
4-[5-bromo-4-(4-oxo-cyclohexylamino)-pyrimidin-2-ylamino]-benzenesulfonamide,
(E)-6-{5-chloro-2-[4-(2-hydroxy-ethylsulfamoyl)-phenylamino]-pyrimidin-4-ylamino}-3-methyl-hex-2-en-4-ynoic acid methyl ester,
4-[5-bromo-4-(1-hydroxymethyl-cyclopentylamino)-pyrimidin-2-ylamino]-benzenesulfonamide,
4-[4-(bicyclo[2.2.1]hept-2-ylamino)-5-bromo-pyrimidin-2-ylamino]-benzenesulfonamide,
4-[5-bromo-4-(4-morpholin-4-yl-cyclohexylamino)-pyrimidin-2-ylamino]-benzenesulfonamide,
4-[5-bromo-4-(4-morpholin-4-yl-cyclohexylamino)-pyrimidin-2-ylamino]-benzenesulfonamide,
4-[5-bromo-4-((R)-2-hydroxy-1-methyl-ethylamino)-pyrimidin-2-ylamino]-benzenesulfonamide,
4-({5-bromo-4-[(R)-(2-hydroxy-1-methylethyl)amino]pyrimidin-2-yl}amino)benzenesulfonamide,
4-[5-bromo-4-(2,2,3,3,3-pentafluoro-propylamino)-pyrimidin-2-ylamino]-benzenesulfonamide,
4-[5-bromo-4-(4-hydroxy-butylamino)-pyrimidin-2-ylamino]-benzenesulfonamide,
4-[5-bromo-4-(1-ethynyl-cyclohexylamino)-pyrimidin-2-ylamino]-benzenesulfonamide,
4-[5-bromo-4-(3-hydroxy-1-phenyl-propylamino)-pyrimidin-2-ylamino]-benzenesulfonamide,
(+)-4-[5-bromo-4-(2-methyl-cyclohexylamino)-pyrimidin-2-ylamino]-benzenesulfonamide,
(-)-4-[5-bromo-4-(2-methyl-cyclohexylamino)-pyrimidin-2-ylamino]-benzenesulfonamide,
4-{5-bromo-4-[4-(2-hydroxy-1-hydroxymethyl-ethylamino)-cyclohexylamino]-pyrimidin-2-ylamino}-benzenesulfonamide,
4-{5-bromo-4-[4-(2-hydroxy-1-hydroxymethyl-ethylamino)-cyclohexylamino]-pyrimidin-2-ylamino}-benzenesulfonamide,
(R)-2-[5-bromo-2-(4-trifluoromethanesulfonyl-phenylamino)-pyrimidin-4-ylamino]-3-methyl-butan-1-ol,
4-[5-bromo-4-(4-cyclopropylamino-cyclohexylamino)-pyrimidin-2-ylamino] -benzenesulfonamide,
4-[5-bromo-4-(4-cyclopropylamino-cyclohexylamino)-pyrimidin-2-ylamino] -benzenesulfonamide,
4-[5-bromo-4-(tetrahydro-thiophen-3-ylamino)-pyrimidin-2-ylamino]-benzenesulfonamide,
2-[5-bromo-2-(4-sulfamoyl-phenylamino)-pyrimidin-4-ylamino]-3-hydroxypropionic acid methyl ester,
4-[5-bromo-4-((R)-1-hydroxymethyl-2-methyl-propylamino)-pyrimidin-2-yl amino]-N-pyrimidin-2-yl-benzenesulfonamide,
4-[5-bromo-4-((R)-1-hydroxymethyl-2-methyl-propylamino)-pyrimidin-2-yl amino]-N-thiazol-2-yl-benzenesulfonamide,
4-[5-bromo-4-(4-hydroxy-butylamino)-pyrimidin-2-ylamino]-N-methyl-benzenesulfonamide,
4-{5-bromo-4-[2-(3H-imidazol-4-yl)-ethylamino]-pyrimidin-2-ylamino}-benzenesulfonamide,
4-[5-bromo-4-(1-ethyl-1-hydroxymethyl-propylamino)-pyrimidin-2-ylamino]-benzenesulfonamide,
4-[5-bromo-4-(1-hydroxymethyl-1,2-dimethyl-propylamino)-pyrimidin-2-ylamino]-benzenesulfonamide,
4-[5-bromo-4-(4-methyl-cyclohexylamino)-pyrimidin-2-ylamino]-benzenesulfonamide,
4-{5-bromo-4-[(S)-(tetrahydro-furan-3-yl)amino]-pyrimidin-2-ylamino}-benzenesulfonamide,
4-[5-bromo-4-(cyclohexylmethyl-amino)-pyrimidin-2-ylamino]-benzenesulfonamide,
3-[5-bromo-2-(4-sulfamoyl-phenylamino)-pyrimidin-4-ylamino]-propionic acid,
4-[5-bromo-2-(4-sulfamoyl-phenylamino)-pyrimidin-4-ylamino]-butyric acid ethyl ester,
{3-[5-bromo-2-(4-sulfamoyl-phenylamino)-pyrimidin-4-ylamino]-propyl}-phosphonic acid diethyl ester,
4-{5-bromo-4-[(3-pyridin-3-yl-isoxazol-4-ylmethyl)-amino]-pyrimidin-2-ylamino}-benzenesulfonamide,
{[5-bromo-2-(4-sulfamoyl-phenylamino)-pyrimidin-4-ylamino]-methyl}-phosphonic acid dimethyl ester,
{[5-bromo-2-(4-sulfamoyl-phenylamino)-pyrimidin-4-ylamino]-methyl}-phosphonic acid monomethyl ester,
4-{5-bromo-4-[(pyridin-4-ylmethyl)-amino]-pyrimidin-2-ylamino}-benzenesulfonamide,
4-{5-bromo-4-[(thiophen-2-ylmethyl)-amino]-pyrimidin-2-ylamino}-benzenesulfonamide,
4-{5-bromo-4-[(pyridin-2-ylmethyl)-amino]-pyrimidin-2-ylamino}-benzenesulfonamide,
4-[5-bromo-4-(4-fluoro-benzylamino)-pyrimidin-2-ylamino]-benzenesulfonamide,
4-[5-bromo-4-(4-methoxy-benzylamino)-pyrimidin-2-ylamino]-benzenesulfonamide,
{3-[5-bromo-2-(4-sulfamoyl-phenylamino)-pyrimidin-4-ylamino]-propyl}-phosphonic acid,
4-[5-bromo-4-(2-hydroxy-ethylamino)-pyrimidin-2-ylamino]-benzenesulfonamide,
4-[5-bromo-4-(1-phenyl-ethylamino)-pyrimidin-2-ylamino]-benzenesulfonamide,
N-allyloxy-4-[5-bromo-4-((R)-1-hydroxymethyl-2-methyl-propylamino)-pyrimidin-2-ylamino]-benzenesulfonamide,
3-[5-bromo-4-((R)-1-hydroxymethyl-2-methyl-propylamino)-pyrimidin-2-yl amino]-N-(5-hydroxy-pentyl)-benzenesulfonamide,
3-[5-bromo-4-((R)-1-hydroxymethyl-2-methyl-propylamino)-pyrimidin-2-yl amino]-N-(2-hydroxy-1-hydroxymethyl-ethyl)-benzenesulfonamide,
3-[5-bromo-4-((R)-1-hydroxymethyl-2-methyl-propylamino)-pyrimidin-2-ylamino]-N-(3-hydroxy-propyl)-benzenesulfonamide,
N-benzyloxy-4-[5-bromo-4-((R)-1-hydroxymethyl-2-methyl-propylamino)-pyrimidin-2-ylamino]-benzenesulfonamide,
4-[5-bromo-4-((R)-1-hydroxymethyl-2-methyl-propylamino)-pyrimidin-2-ylamino]-N-(2-hydroxy-butyl)-benzenesulfonamide,
4-[5-bromo-4-((R)-1-hydroxymethyl-2-methyl-propylamino)-pyrimidin-2-yl amino]-N-cyclopropylmethyl-benzenesulfonamide,
4-[5-bromo-4-((R)-1-hydroxymethyl-2-methyl-propylamino)-pyrimidin-2-ylamino]-N-(4-cyano-cyclohexylmethyl)-benzenesulfonamide,
4-[5-bromo-4-((R)-1-hydroxymethyl-2-methyl-propylamino)-pyrimidin-2-ylamino]-N-(5-hydroxy-pentyl)-benzenesulfonamide,
4-[5-bromo-4-((R)-1-hydroxymethyl-2-methyl-propylamino)-pyrimidin-2-ylamino]-N-(tetrahydro-furan-2-ylmethyl)-benzenesulfonamide,
4-[5-bromo-4-((S)-1-hydroxymethyl-2-methyl-propylamino)-pyrimidin-2-ylamino]-N-hydroxy-benzenesulfonamide,
4-[5-bromo-4-((S)-1-hydroxymethyl-propylamino)-pyrimidin-2-ylamino]-benzenesulfonamide,
4-[5-bromo-4-((R)-1-hydroxymethyl-2-methyl-propylamino)-pyrimidin-2-ylamino]-N-(2-hydroxy-1-hydroxymethyl-ethyl)-benzenesulfonamide,
4-[5-bromo-4-((R)-1-hydroxymethyl-2-methyl-propylamino)-pyrimidin-2-ylamino]-N-(3-hydroxy-propyl)-benzenesulfonamide,
4-[5-bromo-4-((R)-1-hydroxymethyl-2-methyl-propylamino)-pyrimidin-2-ylamino]-N-hydroxy-benzenesulfonamide,
5-[5-bromo-4-((R)-1-hydroxymethyl-2-methyl-propylamino)-pyrimidin-2-ylamino]-2-methyl-benzenesulfonamlde,
3-[5-bromo-4-((R)-1-hydroxymethyl-2-methyl-propylamino)-pyrimidin-2-ylamino]-N-ethoxy-benzenesulfonamide,
N-allyloxy-3-[5-bromo-4-((R)-1-hydroxymethyl-2-methyl-propylamino)-pyrimidin-2-ylamino]-benzenesulfonamide,
4-(5-bromo-4-morpholin-4-yl-pyrimidin-2-ylamino)-benzenesulfonamide,
7-[(5-bromo-4-{[(R)-2-hydroxy-1-methylethyl]amino}pyrimidin-2-yl)amino]-3,4-dihydro-2H-1,2-benzothiazine 1,1-dioxide ,
4-[5-bromo-4-(2-hydroxy-2-phenyl-propylamino)-pyrimidin-2-ylamino]-benzenesulfonamide,
4-[5-bromo-4-(2-hydroxy-1,2-dimethyl-propylamino)-pyrimidin-2-ylamino]-benzenesulfonamide,
4-[5-bromo-4-(2-hydroxy-1,2-dimethyl-butylaminorpyrimidin-2-ylamino]-benzenesulfonamide,
4-[5-bromo-4-(2-oxo-2-phenyl-ethylamino)-pyrimidin-2-ylamino]-benzenesulfonamide,
4-[5-bromo-4-(2-hydroxy-propylamino)-pyrimidin-2-ylamino]-benzenesulfonamide,
4-[5-bromo-4-(2-hydroxy-1-methyl-propylamino)-pyrimidin-2-ylamino]-benzenesulfonamide,
4-(5-bromo-4-sec-butylamino-pyrimidin-2-ylamino)-benzenesulfonamide,
4-[5-bromo-4-(2-hydroxy-1-methyl-butylamino)-pyrimidin-2-ylamino]-benzenesulfonamide,
4-[5-bromo-4-(3,3-dimethyl-2-oxo-butylamino)-pyrimidin-2-ylamino]-benzenesulfonamide,
4-[5-bromo-4-(2-hydroxy-1-methyl-3-phenyl-propylamino)-pyrimidin-2-ylamino]-benzenesulfonamide,
4-[5-bromo-4-(2-hydroxy-butylamino)-pyrimidin-2-ylamino]-benzenesulfonamide,
4-[5-bromo-4-(2-hydroxy-1-methyl-pent-4-enylamino)-pyrimidin-2-ylamino]-benzenesulfonamide,
4-[5-bromo-4-(2-hydroxy-1-methyl-pentylamino)-pyrimidin-2-ylamino]-benzenesulfonamide,
4-[5-bromo-4-(1-methyl-2-oxo-propylamino)-pyrimidin-2-ylamino]-benzenesulfonamide,
4-[5-bromo-4-(2-hydroxy-1,3-dimethyl-butylamino)-pyrimidin-2-ylamino]-benzenesulfonamide,
4-[4-((1R,2R)-2-benzyloxy-cyclopentylamino)-5-bromo-pyrimidin-2-ylamino]-benzenesulfonamide,
4-[4-((1S,2S)-2-benzyloxy-cyclopentylamino)-5-bromo-pyrimidin-2-ylamino]-benzenesulfonamide,
4-[5-bromo-4-(1-ethyl-2-hydroxy-propylamino)-pyrimidin-2-ylamino]-benzenesulfonamide,
4-[5-bromo-4-(1-ethyl-2-hydroxy-butylamino)-pyrimidin-2-ylamino]-benzenesulfonamide,
4-[5-bromo-4-(2-hydroxy-2-phenyl-ethylamino)-pyrimidin-2-ylamino]-benzenesulfonamide,
4-[5-bromo-4-(2-hydroxy-3,3-dimethyl-butylamino)-pyrimidin-2-ylamino]-benzenesulfonamide,
4-[5-bromo-4-(2-hydroxyimino-1-methyl-propylamino)-pyrimidin-2-ylamino]-benzenesulfonamide,
4-(5-bromo-4-{2-[(E)-methoxyimino]-1-methyl-propylamino}-pyrimidin-2-ylamino)-benzenesulfonamide,
4-(5-bromo-4-{2-[(E)-tert-butoxyimino]-1-methyl-propylamino}-pyrimidin-2-ylamino)-benzenesulfonamide,
[2-[5-bromo-2-(4-sulfamoyl-phenylamino)-pyrimidin-4-ylamino]-1-methyl-prop-(E)-ylideneaminooxy]-acetic acid,
4-[5-bromo-4-(2-cyclopropylamino-1-methyl-propylamino)-pyrimidin-2-ylamino]-benzenesulfonamide,
4-[5-bromo-4-(2-hydroxy-1-methoxymethyl-ethylamino)-pyrimidin-2-ylamino]-benzenesulfonamide,
4-[5-bromo-4-(2-hydroxy-1-methoxymethyl-propylamino)-pyrimidin-2-ylamino]-benzenesulfonamide,
5-[5-bromo-4-((R)-1-hydroxymethyl-propylamino)-pyrimidin-2-ylamino]-N-cyclohexylmethyl-2-methyl-benzenesulfonamide,
5-[5-bromo-4-((R)-1-hydroxymethyl-propylamino)-pyrimidin-2-ylamino]-2-methyl-N-(4-phenyl-butyl)-benzenesulfonamide,
5-[5-bromo-4-((R)-1-hydroxymethyl-propylamino)-pyrimidin-2-ylamino]-N-(3,3-diphenyl-propyl)-2-methyl-benzenesulfonamide,
5-[5-bromo-4-((R)-1-hydroxymethyl-propylamino)-pyrimidin-2-ylamino]-N-(2-dimethylamino-ethyl)-2-methyl-benzenesulfonamide,
5-[5-bromo-4-((R)-1-hydroxymethyl-propylamino)-pyrimidin-2-ylamino]-N-3-imidazol-1-yl-propyl)-2-methyl-benzenesulfonamide,
5-[5-bromo-4-((R)-1-hydroxymethyl-propylamino)-pyrimidin-2-ylamino]-2-methyl-N-(3-trifluoromethyl-benzyl)-benzenesulfonamide,
5-[5-bromo-4-((R)-1-hydroxymethyl-propylamino)-pyrimidin-2-ylamino]-N-heptyl-2-methyl-benzenesulfonamide,
5-[5-bromo-4-((R)-1-hydroxymethyl-propylamino)-pyrimidin-2-ylamino]-2-methyl-N-octyl-benzenesulfonamide,
5-[5-bromo-4-((R)-1-hydroxymethyl-propylamino)-pyrimidin-2-ylamino]-2-methyl-N-nonyl-benzenesulfonamide,
5-[5-bromo-4-((R)-1-hydroxymethyl-propylamino)-pyrimidin-2-ylamino]-N-decyl-2-methyl-benzenesulfonamide,
5-[5-bromo-4-((R)-1-hydroxymethyl-propylamino)-pyrimidin-2-ylamino]-N-furan-2-ylmethyl-2-methyl-benzenesulfonamide,
5-[5-bromo-4-((R)-1-hydroxymethyl-propylamino)-pyrimidin-2-ylamino]-2-methyl-N-(4-trifluoromethyl-benzyl)-benzenesulfonamide,
5-[5-bromo-4-((R)-1-hydroxymethyl-propylamino)-pyrimidin-2-ylamino]-2-methyl-N-(3-phenyl-propyl)-benzenesulfonamide,
5-[5-bromo-4-((R)-1-hydroxymethyl-propylamino)-pyrimidin-2-ylamino]-2-methyl-N-[3-(2-oxo-pyrrolidin-1-yl)-propyl]-benzenesulfonamide,
5-[5-bromo-4-((R)-1-hydroxymethyl-propylamino)-pyrimidin-2-ylamino]-2-methyl-N-(3-methyl-butyl)-benzenesulfonamide,
5-[5-bromo-4-((R)-1-hydroxymethyl-propylamino)-pyrimidin-2-ylamino]-N-hexyl-2-methyl-benzenesulfonamide,
5-[5-bromo-4-((R)-1-hydroxymethyl-propylamino)-pyrimidin-2-ylamino]-N-(3-dimethylamino-propyl)-2-methyl-benzenesulfonamide,
5-[5-bromo-4-((R)-1-hydroxymethyl-propylaminorpyrimidin-2-ylamino]-N-(2-diisopropylamino-ethyl)-2-methyl-benzenesulfonamide,
5-[5-bromo-4-((R)-1-hydroxymethyl-propylamino)-pyrimidin-2-ylamino]-N-cyclopropyl-2-methyl-benzenesulfonamide,
5-[5-bromo-4-((R)-1-hydroxymethyl-propylamino)-pyrimidin-2-ylamino]-N-(4-cyano-cyclohexylmethyl)-2-methyl-benzenesulfonamide,
5-[5-bromo-4-((R)-1-hydroxymethyl-propylamino)-pyrimidin-2-ylamino]-2-methyl-N-phenethyl-benzenesulfonamide,
5-[5-bromo-4-((R)-1-hydroxymethyl-propylamino)-pyrimidin-2-ylamino]-N-[2-(4-chloro-phenyl)-ethyl]-2-methyl-benzenesulfonamide,
5-[5-bromo-4-((R)-1-hydroxymethyl-propylamino)-pyrimidin-2-ylamino]-2-methyl-N-(5-methyl-furan-2-ylmethyl)-benzenesulfonamide,
5-[5-bromo-4-((R)-1-hydroxymethyl-propylamino)-pyrimidin-2-ylamino]-2-methyl-N-(2-morpholin-4-yl-ethyl)-benzenesulfonamide,
5-[5-bromo-4-((R)-1-hydroxymethyl-propylamino)-pyrimidin-2-ylamino]-N-(2-methoxy-ethyl)-2-methyl-benzenesulfonamide,
5-[5-bromo-4-((R)-1-hydroxymethyl-propylamino)-pyrimidin-2-ylamino]-2-methyl-N-thiophen-2-ylmethyl-benzenesulfonamide,
5-[5-bromo-4-((R)-1-hydroxymethyl-propylamino)-pyrimidin-2-ylamino]-N-[2-(4-methoxy-phenyl)-ethyl]-2-methyl-benzenesulfonamide,
5-[5-bromo-4-((R)-1-hydroxymethyl-propylamino)-pyrimidin-2-ylamino]-2-methyl-N-(2-pyrrolidin-1-yl-ethyl)-benzenesulfonamide,
5-[5-bromo-4-((R)-1-hydroxymethyl-propylamino)-pyrimidin-2-ylamino]-2-methyl-N-(2-piperidin-1-yl-ethyl)-benzenesulfonamide,
5-[5-bromo-4-((R)-1-hydroxymethyl-propylamino)-pyrimidin-2-ylamino]-N-(3-fluoro-benzyl)-2-methyl-benzenesulfonamide,
5-[5-bromo-4-((R)-1-hydroxymethyl-propylamino)-pyrimidin-2-ylamino]-2-methyl-N-[2-(1-methyl-pyrrolidin-2-yl)-ethyl]-benzenesulfonamide,
5-[5-bromo-4-((R)-1-hydroxymethyl-propylamino)-pyrimidin-2-ylamino]-2-methyl-N-pyridin-3-ylmethyl-benzenesulfonamide,
5-[5-bromo-4-((R)-1-hydroxymethyl-propylamino)-pyrimidin-2-ylamino]-N-(4-fluoro-benzyl)-2-methyl-benzenesulfonamide,
5-[5-bromo-4-((R)-1-hydroxymethyl-propylamino)-pyrimidin-2-ylamino]-2-methyl-N-pyridin-4-ylmethyl-benzenesulfonamide,
5-[5-bromo-4-((R)-1-hydroxymethyl-propylamino)-pyrimidin-2-ylamino]-2-methyl-N-(2-phenoxy-ethyl)-benzenesulfonamide,
5-[5-bromo-4-((R)-1-hydroxymethyl-propylamino)-pyrimidin-2-ylamino]-N-(2-methoxy-benzyl)-2-methyl-benzenesulfonamide,
5-[5-bromo-4-((R)-1-hydroxymethyl-propylamino)-pyrimidin-2-ylamino]-2-methyl-N-[2-(4-sulfamoyl-phenyl)-ethyl]-benzenesulfonamide,
5-[5-bromo-4-((R)-1-hydroxymethyl-propylamino)-pyrimidin-2-ylamino]-2-methyl-N-(4-methyl-benzyl)-benzenesulfonamide,
5-[5-bromo-4-((R)-2-hydroxy-1-methyl-ethylamino)-pyrimidin-2-ylamino]-N-cyclohexylmethyl-2-methyl-benzenesulfonamide,
5-[5-bromo-4-((R)-2-hydroxy-1-methyl-ethylamino)-pyrimidin-2-ylamino]-2-methyl-N-(4-phenyl-butyl)-benzenesulfonamide,
5-[5-bromo-4-((R)-2-hydroxy-1-methyl-ethylamino)-pyrimidin-2-ylamino]-N-(2-dimethylamino-ethyl)-2-methyl-benzenesulfonamide,
5-[5-bromo-4-((R)-2-hydroxy-1-methyl-ethylamino)-pyrimidin-2-ylamino]-N-(3-imidazol-1-yl-propyl)-2-methyl-benzenesulfonamide,
5-[5-bromo-4-((R)-2-hydroxy-1-methyl-ethylamino)-pyrimidin-2-ylamino]-2-methyl-N-(3-trifluoromethyl-benzyl)-benzenesulfonamide,
5-[5-bromo-4-((R)-2-hydroxy-1-methyl-ethylamino)-pyrimidin-2-ylamino]-N-furan-2-ylmethyl-2-methyl-benzenesulfonamide,
5-[5-bromo-4-((R)-2-hydroxy-1-methyl-ethylamino)-pyrimidin-2-ylamino]-2-methyl-N-(4-trifluoromethyl-benzyl)-benzenesulfonamide,
5-[5-bromo-4-((R)-2-hydroxy-1-methyl-ethylamino)-pyrimidin-2-ylamino]-2-methyl-N-(3-phenyl-propyl)-benzenesulfonamide,
5-[5-bromo-4-((R)-2-hydroxy-1-methyl-ethylamino)-pyrimidin-2-ylamino]-2-methyl-N-[3-(2-oxo-pyrrolidin-1-yl)-propyl]-benzenesulfonamide,
5-[5-bromo-4-((R)-2-hydroxy-1-methyl-ethylamino)-pyrimidin-2-ylamino]-2-methyl-N-(3-methyl-butyl)-benzenesulfonamide,
5-[5-bromo-4-((R)-2-hydroxy-1-methyl-ethylamino)-pyrimidin-2-ylamino]-N-hexyl-2-methyl-benzenesulfonamide,
5-[5-bromo-4-((R)-2-hydroxy-1-methyl-ethylamino)-pyrimidin-2-ylamino]-N-(3-dimethylamino-propyl)-2-methyl-benzenesulfonamide,
5-[5-bromo-4-((R)-2-hydroxy-1-methyl-ethylamino)-pyrimidin-2-ylamino]-N-(2-diisopropylamino-ethyl)-2-methyl-benzenesulfonamide,
5-[5-bromo-4-((R)-2-hydroxy-1-methyl-ethylamino)-pyrimidin-2-ylamino]-N-cyclopropyl-2-methyl-benzenesulfonamide,
5-[5-bromo-4-((R)-2-hydroxy-1-methyl-ethylamino)-pyrimidin-2-ylamino]-N-(4-cyano-cyclohexylmethyl)-2-methyl-benzenesulfonamide,
5-[5-bromo-4-((R)-2-hydroxy-1-methyl-ethylamino)-pyrimidin-2-ylamino]-2-methyl-N-phenethyl-benzenesulfonamide,
5-[5-bromo-4-((R)-2-hydroxy-1-methyl-ethylamino)-pyrimidin-2-ylamino]-N-[2-(4-chloro-phenyl)-ethyl]-2-methyl-benzenesulfonamide,
5-[5-bromo-4-((R)-2-hydroxy-1-methyl-ethylamino)-pyrimidin-2-ylamino]-2-methyl-N-(5-methyl-furan-2-ylmethyl)-benzenesulfonamide,
5-[5-bromo-4-((R)-2-hydroxy-1-methyl-ethylamino)-pyrimidin-2-ylamino]-2-methyl-N-(2-morpholin-4-yl-ethyl)-benzenesulfonamide,
5-[5-bromo-4-((R)-2-hydroxy-1-methyl-ethylamino)-pyrimidin-2-ylamino]-N-(2-methoxy-ethyl)-2-methyl-benzenesulfonamide,
5-[5-bromo-4-((R)-2-hydroxy-1-methyl-ethylamino)-pyrimidin-2-ylamino]-2-methyl-N-thiophen-2-ylmethyl-benzenesulfonamide,
5-[5-bromo-4-((R)-2-hydroxy-1-methyl-ethylamino)-pyrimidin-2-ylamino]-N-[2-(4-methoxy-phenyl)-ethyl]-2-methyl-benzenesulfonamide,
5-[5-bromo-4-((R)-2-hydroxy-1-methyl-ethylamino)-pyrimidin-2-ylamino]-2-methyl-N-(2-pyrrolidin-1-yl-ethyl)-benzenesulfonamide,
5-[5-bromo-4-((R)-2-hydroxy-1-methyl-ethylamino)-pyrimidin-2-ylamino]-2-methyl-N-(2-piperidin-1-yl-ethyl)-benzenesulfonamide,
5-[5-bromo-4-((R)-2-hydroxy-1-methyl-ethylamino)-pyrimidin-2-ylamino]-N-(3-fluoro-benzyl)-2-methyl-benzenesulfonamide,
5-[5-bromo-4-((R)-2-hydroxy-1-methyl-ethylamino)-pyrimidin-2-ylamino]-2-methyl-N-[2-(1-methyl-pyrrolidin-2-yl)-ethyl]-benzenesulfonamide,
5-[5-bromo-4-((R)-2-hydroxy-1-methyl-ethylamino)-pyrimidin-2-ylamino]-2-methyl-N-pyridin-3-ylmethyl-benzenesulfonamide,
5-[5-bromo-4-((R)-2-hydroxy-1-methyl-ethylamino)-pyrimidin-2-ylamino]-N-(4-fluoro-benzyl)-2-methyl-benzenesulfonamide,
5-[5-bromo-4-((R)-2-hydroxy-1-methyl-ethylamino)-pyrimidin-2-ylamino]-2-methyl-N-pyridin-4-ylmethyl-benzenesulfonamide,
5-[5-bromo-4-((R)-2-hydroxy-1-methyl-ethylamino)-pyrimidin-2-ylamino]-2-methyl-N-(2-phenoxy-ethyl)-benzenesulfonamide,
5-[5-bromo-4-((R)-2-hydroxy-1-methyl-ethylamino)-pyrimidin-2-ylamino]-N-(2-methoxy-benzyl)-2-methyl-benzenesulfonamide,
5-[5-bromo-4-((R)-2-hydroxy-1-methyl-ethylamino)-pyrimidin-2-ylamino]-2-methyl-N-[2-(4-sulfamoyl-phenyl)-ethyl]-benzenesulfonamide,
5-[5-bromo-4-((R)-2-hydroxy-1-methyl-ethylamino)-pyrimidin-2-ylamino]-2-methyl-N-(4-methyl-benzyl)-benzenesulfonamide,
3-[5-bromo-4-((R)-1-hydroxymethyl-propylamino)-pyrimidin-2-ylamino]-N-cyclohexylmethyl-benzenesulfonamide,
3-[5-bromo-4-((R)-1-hydroxymethyl-propylamino)-pyrimidin-2-ylamino]-N-(3-imidazol-1-yl-propyl)-benzenesulfonamide,
3-[5-bromo-4-((R)-1-hydroxymethyl-propylamino)-pyrimidin-2-ylamino]-N-(3-trifluoromethyl-benzyl)-benzenesulfonamide,
3-[5-bromo-4-((R)-1-hydroxymethyl-propylamino)-pyrimidin-2-ylamino]-N-furan-2-ylmethyl-benzenesulfonamide,
3-[5-bromo-4-((R)-1-hydroxymethyl-propylamino)-pyrimidin-2-ylamino]-N-(4-trifluoromethyl-benzyl)-benzenesulfonamide,
3-[5-bromo-4-((R)-1-hydroxymethyl-propylamino)-pyrimidin-2-ylamino]-N-(3-phenyl-propyl)-benzenesulfonamide,
3-[5-bromo-4-((R)-1-hydroxymethyl-propylamino)-pyrimidin-2-ylamino]-N-[3-(2-oxo-pyrrolidin-1-yl)-propyl]-benzenesulfonamide,
3-[5-bromo-4-((R)-1-hydroxymethyl-propylamino)-pyrimidin-2-ylamino]-N-(3-methyl-butyl)-benzenesulfonamide,
3-[5-bromo-4-((R)-1-hydroxymethyl-propylamino)-pyrimidin-2-ylamino]-N-(3-dimethylamino-propyl)-benzenesulfonamide,
3-[5-bromo-4-((R)-1-hydroxymethyl-propylamino)-pyrimidin-2-ylamino]-N-(2-diisopropylamino-ethyl)-benzenesulfonamide,
3-[5-bromo-4-((R)-1-hydroxymethyl-propylamino)-pyrimidin-2-ylamino]-N-(4-cyano-cyclohexylmethyl)-benzenesulfonamide,
3-[5-bromo-4-((R)-1-hydroxymethyl-propylamino)-pyrimidin-2-ylamino]-N-phenethyl-benzenesulfonamide,
3-[5-bromo-4-((R)-1-hydroxymethyl-propylamino)-pyrimidin-2-ylamino]-N-[2-(4-chloro-phenyl)-ethyl]-benzenesulfonamide,
3-[5-bromo-4-((R)-1-hydroxymethyl-propylamino)-pyrimidin-2-ylamino]-N-(5-methyl-furan-2-ylmethyl)-benzenesulfonamide,
3-[5-bromo-4-((R)-1-hydroxymethyl-propylamino)-pyrimidin-2-ylamino]-N-(2-morpholin-4-yl-ethyl)-benzenesulfonamide,
3-[5-bromo-4-((R)-1-hydroxymethyl-propylamino)-pyrimidin-2-ylamino]-N-(2-methoxy-ethyl)-benzenesulfonamide,
3-[5-bromo-4-((R)-1-hydroxymethyl-propylamino)-pyrimidin-2-ylamino]-N-thiophen-2-ylmethyl-benzenesulfonamide,
3-[5-bromo-4-((R)-1-hydroxymethyl-propylamino)-pyrimidin-2-ylamino]-N-(2-pyrrolidin-1-yl-ethyl)-benzenesulfonamide,
3-[5-bromo-4-((R)-1-hydroxymethyl-propylamino)-pyrimidin-2-ylamino]-N-(2-piperidin-1-yl-ethyl)-benzenesulfonamide,
3-[5-bromo-4-((R)-1-hydroxymethyl-propylamino)-pyrimidin-2-ylamino]-N-(3-fluoro-benzyl)-benzenesulfonamide,
3-[5-bromo-4-((R)-1-hydroxymethyl-propylamino)-pyrimidin-2-ylamino]-N-[2-(1-methyl-pyrrolidin-2-yl)-ethyl]-benzenesulfonamide,
3-[5-bromo-4-((R)-1-hydroxymethyl-propylamino)-pyrimidin-2-ylamino]-N-pyridin-3-ylmethyl-benzenesulfonamide,
3-[5-bromo-4-((R)-1-hydroxymethyl-propylamino)-pyrimidin-2-ylamino]-N-(4-fluoro-benzyl)-benzenesulfonamide,
3-[5-bromo-4-((R)-1-hydroxymethyl-propylamino)-pyrimidin-2-ylamino]-N-pyridin-4-ylmethyl-benzenesulfonamide,
3-[5-bromo-4-((R)-1-hydroxymethyl-propylamino)-pyrimidin-2-ylamino]-N-(2-phenoxy-ethyl)-benzenesulfonamide,
3-[5-bromo-4-((R)-1-hydroxymethyl-propylamino)-pyrimidin-2-ylamino]-N-(2-methoxy-benzyl)-benzenesulfonamide,
3-[5-bromo-4-((R)-1-hydroxymethyl-propylamino)-pyrimidin-2-ylamino]-N-(4-methyl-benzyl)-benzenesulfonamide,
3-[5-bromo-4-((R)-1-hydroxymethyl-propylamino)-pyrimidin-2-ylamino]-N-(5-methylsulfanyl-1H-[1,2,4]triazol-3-yl)-benzenesulfonamide,
3-[5-bromo-4-((R)-2-hydroxy-1-methyl-ethylamino)-pyrimidin-2-ylamino]-N-(2-dimethylamino-ethyl)-benzenesulfonamide,
3-[5-bromo-4-((R)-2-hydroxy-1-methyl-ethylamino)-pyrimidin-2-ylamino]-N-(3-imidazol-1-yl-propyl)-benzenesulfonamide,
3-[5-bromo-4-((R)-2-hydroxy-1-methyl-ethylamino)-pyrimidin-2-ylamino]-N-(3-trifluoromethyl-benzyl)-benzenesulfonamide,
3-[5-bromo-4-((R)-2-hydroxy-1-methyl-ethylamino)-pyrimidin-2-ylamino]-N-furan-2-ylmethyl-benzenesulfonamide,
3-[5-bromo-4-((R)-2-hydroxy-1-methyl-ethylamino)-pyrimidin-2-ylamino]-N-(4-trifluoromethyl-benzyl)-benzenesulfonamide,
3-[5-bromo-4-((R)-2-hydroxy-1-methyl-ethylamino)-pyrimidin-2-ylamino]-N-[3-(2-oxo-pyrrolidin-1-yl)-propyl]-benzenesulfonamide,
3-[5-bromo-4-((R)-2-hydroxy-1-methyl-ethylamino)-pyrimidin-2-ylamino]-N-(3-methyl-butyl)-benzenesulfonamide,
3-[5-bromo-4-((R)-2-hydroxy-1-methyl-ethylamino)-pyrimidin-2-ylamino]-N-(3-dimethylamino-propyl)-benzenesulfonamide,
3-[5-bromo-4-((R)-2-hydroxy-1-methyl-ethylaminorpyrimidin-2-ylamino]-N-(2-diisopropylamino-ethyl)-benzenesulfonamide,
3-[5-bromo-4-((R)-2-hydroxy-1-methyl-ethylamino)-pyrimidin-2-ylamino]-N-(4-cyano-cyclohexylmethyl)-benzenesulfonamide,
3-[5-bromo-4-((R)-2-hydroxy-1-methyl-ethylamino)-pyrimidin-2-ylamino]-N-phenethyl-benzenesulfonamide,
3-[5-bromo-4-((R)-2-hydroxy-1-methyl-ethylamino)-pyrimidin-2-ylamino]-N-[2-(4-chloro-phenyl)-ethyl]-benzenesulfonamide,
3-[5-bromo-4-((R)-2-hydroxy-1-methyl-ethylamino)-pyrimidin-2-ylamino]-N-(5-methyl-furan-2-ylmethyl)-benzenesulfonamide,
3-[5-bromo-4-((R)-2-hydroxy-1-methyl-ethylamino)-pyrimidin-2-ylamino]-N-(2-morpholin-4-yl-ethyl)-benzenesulfonamide,
3-[5-bromo-4-((R)-2-hydroxy-1-methyl-ethylamino)-pyrimidin-2-ylamino]-N-(2-methoxy-ethyl)-benzenesulfonamide,
3-[5-bromo-4-((R)-2-hydroxy-1-methyl-ethylamino-pyrimidin-2-ylamino]-N-thiophen-2-ylmethyl-benzenesulfonamide,
3-[5-bromo-4-((R)-2-hydroxy-1-methyl-ethylamino)-pyrimidin-2-ylamino]-N-(2-pyrrolidin-1-yl-ethyl)-benzenesulfonamide,
3-[5-bromo-4-((R)-2-hydroxy-1-methyl-ethylamino)-pyrimidin-2-ylamino]-N-(2-piperidin-1-yl-ethyl)-benzenesulfonamide,
3-[5-bromo-4-((R)-2-hydroxy-1-methyl-ethylamino)-pyrimidin-2-ylamino]-N-(3-fluoro-benzyl)-benzenesulfonamide,
3-[5-bromo-4-((R)-2-hydroxy-1-methyl-ethylamino)-pyrimidin-2-ylamino]-N-[2-(1-methyl-pyrrolidin-2-yl)-ethyl]-benzenesulfonamide,
3-[5-bromo-4-((R)-2-hydroxy-1-methyl-ethylamino)-pyrimidin-2-ylamino]-N-pyridin-3-ylmethyl-benzenesulfonamide,
3-[5-bromo-4-((R)-2-hydroxy-1-methyl-ethylamino)-pyrimidin-2-ylamino]-N-(4-fluoro-benzyl)-benzenesulfonamide,
3-[5-bromo-4-((R)-2-hydroxy-1-methyl-ethylamino)-pyrimidin-2-ylamino]-N-pyridin-4-ylmethyl-benzenesulfonamide,
3-[5-bromo-4-((R)-2-hydroxy-1-methyl-ethylamino)-pyrimidin-2-ylamino]-N-(2-phenoxy-ethyl)-benzenesulfonamide,
3-[5-bromo-4-((R)-2-hydroxy-1-methyl-ethylaminorpyrimidin-2-ylamino]-N-(2-methoxy-benzyl)-benzenesulfonamide,
3-[5-bromo-4-((R)-2-hydroxy-1-methyl-ethylamino)-pyrimidin-2-ylamino]-N-(4-methyl-benzyl)-benzenesulfonamide,
3-[5-bromo-4-((R)-2-hydroxy-1-methyl-ethylamino)-pyrimidin-2-ylamino]-N-(5-methylsulfanyl-1H-[1,2,4]triazol-3-yl)-benzenesulfonamide,
4-[5-bromo-4-((R)-1-hydroxymethyl-propylamino)-pyrimidin-2-ylamino]-benzenesulfonamide,
3-[5-bromo-4-((R)-2-hydroxy-1-methyl-ethylamino)-pyrimidin-2-ylamino]-benzenesulfonyl fluoride,
5-[5-bromo-4-((R)-2-hydroxy-1-methyl-ethylamino)-pyrimidin-2-ylamino]-2-methyl-benzenesulfonyl fluoride,
3-[5-bromo-4-((R)-1-hydroxymethyl-propylamino)-pyrimidin-2-ylamino]-benzenesulfonyl fluoride,
5-[5-bromo-4-((R)-1-hydroxymethyl-propylamino)-pyrimidin-2-ylamino]-2-methyl-benzenesulfonyl fluoride,
3-[5-bromo-4-((S)-1-hydroxymethyl-2-methyl-propylamino)-pyrimidin-2-ylamino]-benzenesulfonyl fluoride,
3-[5-bromo-4-((S)-1-hydroxymethyl-2-methyl-propylamino)-pyrimidin-2-ylamino]-benzenesulfonyl fluoride,
4-[5-bromo-4-((R)-2-hydroxy-1-methyl-ethylamino)-pyrimidin-2-ylamino]-benzenesulfonyl fluoride,
4-[5-bromo-4-((R)-1-hydroxymethyl-propylamino)-pyrimidin-2-ylamino]-benzenesulfonyl fluoride,
4-[5-bromo-4-((S)-1-hydroxymethyl-2-methyl-propylamino)-pyrimidin-2-ylamino]-benzenesulfonyl fluoride,
4-[5-bromo-4-((R)-2-hydroxy-1-methyl-ethylamino)-pyrimidin-2-ylaminol-N-cyclohexylmethyl-benzenesulfonamide,
4-[5-bromo-4-((R)-2-hydroxy-1-methyl-ethylamino)-pyrimidin-2-ylamino]-N-(4-phenyl-butyl)-benzenesulfonamide,
4-[5-bromo-4-((R)-2-hydroxy-1-methyl-ethylamino)-pyrimidin-2-ylamino]-N-(2-dimethylamino-ethyl)-benzenesulfonamide,
4-[5-bromo-4-((R)-2-hydroxy-1-methyl-ethylamino)-pyrimidin-2-ylamino]-N-(3-imidazol-1-yl-propyl)-benzenesulfonamide,
4-[5-bromo-4-((R)-2-hydroxy-1-methyl-ethylamino)-pyrimidin-2-ylamino]-N-(3-trifluoromethyl-benzyl)-benzenesulfonamide,
4-[5-bromo-4-((R)-2-hydroxy-1-methyl-ethylamino)-pyrimidin-2-ylamino]-N-(4-trifluoromethyl-benzyl)-benzenesulfonamide,
4-[5-bromo-4-((R)-2-hydroxy-1-methyl-ethylamino)-pyrimidin-2-ylamino]-N-(3-phenyl-propyl)-benzenesulfonamide,
4-[5-bromo-4-((R)-2-hydroxy-1-methyl-ethylamino)-pyrimidin-2-ylamino]-N-[3-(2-oxo-pyrrolidin-1-yl)-propyl]-benzenesulfonamide,
4-[5-bromo-4-((R)-2-hydroxy-1-methyl-ethylamino)-pyrimidin-2-ylamino]-N-(3-methyl-butyl)-benzenesulfonamide,
4-[5-bromo-4-((R)-2-hydroxy-1-methyl-ethylamino)-pyrimidin-2-ylamino]-N-(2-diisopropylamino-ethyl)-benzenesulfonamide,
4-[5-bromo-4-((R)-2-hydroxy-1-methyl-ethylamino)-pyrimidin-2-ylamino]-N-cyclopropyl-benzenesulfonamide,
4-[5-bromo-4-((R)-2-hydroxy-1-methyl-ethylamino)-pyrimidin-2-ylamino]-N-(4-cyano-cyclohexylmethyl)-benzenesulfonamide,
4-[5-bromo-4-((R)-2-hydroxy-1-methyl-ethylamino)-pyrimidin-2-ylamino]-N-phenethyl-benzenesulfonamide,
4-[5-bromo-4-((R)-2-hydroxy-1-methyl-ethylamino)-pyrimidin-2-ylamino]-N-[2-(4-chloro-phenyl)-ethyl]-benzenesulfonamide,
4-[5-bromo-4-((R)-2-hydroxy-1-methyl-ethylamino)-pyrimid in-2-ylamino]-N-(5-methyl-furan-2-ylmethyl)-benzenesulfonamide,
4-[5-bromo-4-((R)-2-hydroxy-1-methyl-ethylamino)-pyrimidin-2-ylamino]-N-(2-morpholin-4-yl-ethyl)-benzenesulfonamide,
4-[5-bromo-4-((R)-2-hydroxy-1-methyl-ethylamino)-pyrimidin-2-ylamino]-N-(2-methoxy-ethyl)-benzenesulfonamide,
4-[5-bromo-4-((R)-2-hydroxy-1-methyl-ethylamino)-pyrimidin-2-ylamino]-N-thiophen-2-ylmethyl-benzenesulfonamide,
4-[5-bromo-4-((R)-2-hydroxy-1-methyl-ethylamino)-pyrimidin-2-ylamino]-N-[2-(4-methoxy-phenyl)-ethyl]-benzenesulfonamide,
4-[5-bromo-4-((R)-2-hydroxy-1-methyl-ethylamino)-pyrimidin-2-ylamino]-N-(2-pyrrolidin-1-yl-ethyl)-benzenesulfonamide,
4-[5-bromo-4-((R)-2-hydroxy-1-methyl-ethylamino)-pyrimidin-2-ylamino]-N-(2-piperidin-1-yl-ethyl)-benzenesulfonamide,
4-[5-bromo-4-((R)-2-hydroxy-1-methyl-ethylamino)pyrimidin-2-ylamino]-N-(3-fluoro-benzyl)-benzenesulfonamide,
4-[5-bromo-4-((R)-2-hydroxy-1-methyl-ethylamino)-pyrimidin-2-ylamino]-N-[2-(1-methyl-pyrrolidin-2-yl)-ethyl]-benzenesulfonamide,
4-[5-bromo-4-((R)-2-hydroxy-1-methyl-ethylamino)-pyrimidin-2-ylamino]-N-pyridin-3-ylmethyl-benzenesulfonamide,
4-[5-bromo-4-((R)-2-hydroxy-1-methyl-ethylamino)-pyrimidin-2-ylamino]-N-(4-fluoro-benzyl)-benzenesulfonamide,
4-[5-bromo-4-((R)-2-hydroxy-1-methyl-ethylamino)-pyrimidin-2-ylamino]-N-pyridin-4-ylmethyl-benzenesulfonamide,
4-[5-bromo-4-((R)-2-hydroxy-1-methyl-ethylamino)-pyrimidin-2-ylamino]-N-(2-phenoxy-ethyl)-benzenesulfonamide,
4-[5-bromo-4-((R)-2-hydroxy-1-methyl-ethylamino)-pyrimidin-2-ylamino]-N-(2-methoxy-benzyl)-benzenesulfonamide,
4-[5-bromo-4-((R)-2-hydroxy-1-methyl-ethylamino)-pyrimidin-2-ylamino]-N-(4-methyl-benzyl)-benzenesulfonamide,
4-[5-bromo-4-((R)-2-hydroxy-1-methyl-ethylamino)-pyrimidin-2-ylamino]-N-(5-methylsulfanyl-1H-[1,2,4]triazol-3-yl)-benzenesulfonamide,
4-[5-bromo-4-((R)-1-hydroxymethyl-propylamino)-pyrimidin-2-ylamino]-N-cyclohexylmethyl-benzenesulfonamide,
4-[5-bromo-4-((R)-1-hydroxymethyl-propylamino)-pyrimidin-2-ylamino]-N-(2-dimethylamino-ethyl)-benzenesulfonamide,
4-[5-bromo-4-((R)-1-hydroxymethyl-propylamino)-pyrimidin-2-ylaminol-N-(3-imidazol-1-yl-propyl)-benzenesulfonamide,
4-[5-bromo-4-((R)-1-hydroxymethyl-propylamino)-pyrimidin-2-ylamino]-N-(3-trifluoromethyl-benzyl)-benzenesulfonamide,
4-[5-bromo-4-((R)-1-hydroxymethyl-propylaminoypyrimidin-2-ylamino]-N-furan-2-ylmethyl-benzenesulfonamide,
4-[5-bromo-4-((R)-1-hydroxymethyl-propylamino)-pyrimidin-2-ylamino]-N-(4-trifluoromethyl-benzyl)-benzenesulfonamide,
4-[5-bromo-4-((R)-1-hydroxymethyl-propylamino)-pyrimidin-2-ylamino]-N-(3-phenyl-propyl)-benzenesulfonamide,
4-[5-bromo-4-((R)-1-hydroxymethyl-propylamino)-pyrimidin-2-ylamino]-N-[3-(2-oxo-pyrrolidin-1-yl)-propyl]-benzenesulfonamide,
4-[5-bromo-4-((R)-1-hydroxymethyl-propylamino)-pyrimidin-2-ylamino]-N-(3-methyl-butyl)-benzenesulfonamide,
4-[5-bromo-4-((R)-1-hydroxymethyl-propylamino)-pyrimidin-2-ylamino]-N-(3-dimethylamino-propyl)-benzenesulfonamide,
4-[5-bromo-4-((R)-1-hydroxymethyl-propylamino)-pyrimidin-2-ylamino]-N-(2-diisopropylamino-ethyl)-benzenesulfonamide,
4-[5-bromo-4-((R)-1-hydroxymethyl-propylamino)-pyrimidin-2-ylamino]-N-cyclopropyl-benzenesulfonamide,
4-[5-bromo-4-((R)-1-hydroxymethyl-propylamino)-pyrimidin-2-ylamino]-N-(4-cyano-cyclohexylmethyl)-benzenesulfonamide,
4-[5-bromo-4-((R)-1-hydroxymethyl-propylamino)-pyrimidin-2-ylamino]-N-phenethyl-benzenesulfonamide,
4-[5-bromo-4-((R)-1-hydroxymethyl-propylamino)-pyrimidin-2-ylamino]-N-(3,4-dimethoxy-benzyl)-benzenesulfonamide,
4-[5-bromo-4-((R)-1-hydroxymethyl-propylamino)-pyrimidin-2-ylamino]-N-[2-(4-chloro-phenyl)-ethyl]-benzenesulfonamide,
4-[5-bromo-4-((R)-1-hydroxymethyl-propylamino)-pyrimidin-2-ylamino]-N-(5-methyl-furan-2-ylmethyl)-benzenesulfonamide,
4-[5-bromo-4-((R)-1-hydroxymethyl-propylamino)-pyrimidin-2-ylamino]-N-(2-morpholin-4-yl-ethyl)-benzenesulfonamide,
4-[5-bromo-4-((R)-1-hydroxymethyl-propylamino)-pyrimidin-2-ylamino]-N-(2-methoxy-ethyl)-benzenesulfonamide,
4-[5-bromo-4-((R)-1-hydroxymethyl-propylamino)-pyrimidin-2-ylamino]-N-thiophen-2-ylmethyl-benzenesulfonamide,
4-[5-bromo-4-((R)-1-hydroxymethyl-propylamino)-pyrimidin-2-ylaminol-N-[2-(4-methoxy-phenyl)-ethyl]-benzenesulfonamide,
4-[5-bromo-4-((R)-1-hydroxymethyl-propylamino)-pyrimidin-2-ylamino]-N-(2-pyrrolidin-1-yl-ethyl)-benzenesulfonamide,
4-[5-bromo-4-((R)-1-hydroxymethyl-propylamino)-pyrimidin-2-ylamino]-N-(2-piperidin-1-yl-ethyl)-benzenesulfonamide,
4-[5-bromo-4-((R)-1-hydroxymethyl-propylamino)-pyrimidin-2-ylamino]-N-(3-fluoro-benzyl)-benzenesulfonamide,
4-[5-bromo-4-((R)-1-hydroxymethyl-propylamino)-pyrimidin-2-ylamino]-N-[2-(1-methyl-pyrrolidin-2-yl)-ethyl]-benzenesulfonamide,
4-[5-bromo-4-((R)-1-hydroxymethyl-propylamino)-pyrimidin-2-ylamino]-N-pyridin-3-ylmethyl-benzenesulfonamide,
4-[5-bromo-4-((R)-1-hydroxymethyl-propylamino)-pyrimidin-2-ylamino]-N-(4-fluoro-benzyl)-benzenesulfonamide,
4-[5-bromo-4-((R)-1-hydroxymethyl-propylamino)-pyrimidin-2-ylamino]-N-pyridin-4-ylmethyl-benzenesulfonamide,
4-[5-bromo-4-((R)-1-hydroxymethyl-propylamino)-pyrimidin-2-ylamino]-N-(2-phenoxy-ethyl)-benzenesulfonamide,
4-[5-bromo-4-((R)-1-hydroxymethyl-propylamino)-pyrimidin-2-ylamino]-N-(2-methoxy-benzyl)-benzenesulfonamide,
4-[5-bromo-4-((R)-1-hydroxymethyl-propylamino)-pyrimidin-2-ylamino]-N-(4-methyl-benzyl)-benzenesulfonamide or
4-[5-bromo-4-((R)-1-hydroxymethyl-propylamino)-pyrimidin-2-ylamino]-N-(5-methylsulfanyl-1H-[1,2,4]triazol-3-yl)-benzenesulfonamide

3. Use of the compound of the general formula (Ia) 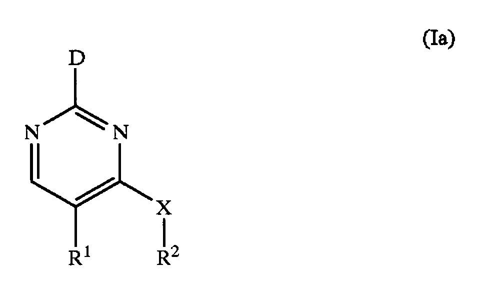 in which D stands for Cl and X for -NH- or -O-, R¹ and R² have the meanings that are indicated in the general formula (I), as intermediate products for the production of the compound of the general formula (I) by reaction in MeCN/HCl with Ar-NH₂.

4. Use of the compounds of the general formula (I) according to Claims 1 or 2 for the production of a pharmaceutical agent for treating cancer, auto-immune diseases, chemotherapy agent-induced alopecia and mucositis, cardiovascular diseases, infectious diseases, nephrological diseases, chronic and acute neurodegenerative diseases and viral infections.

5. Use according to Claim 4, **characterized in that** cancer is defined as solid tumours and leukaemia, auto-immune diseases are defined as psoriasis, alopecia and multiple sclerosis, cardiovascular diseases are defined as stenoses, arterioscleroses and restenoses, infectious diseases are defined as diseases that are caused by unicellular parasites, nephrological diseases are defined as glomerulonephritis, chronic neurodegenerative diseases are defined as Huntington's disease, amyotrophic lateral sclerosis, Parkinson's disease, AIDS, dementia and Alzheimer's disease, acute neurodegenerative diseases are defined as ischaemias of the brain and neurotraumas, and viral infections are defined as cytomegalic infections, herpes, hepatitis B and C and HIV diseases.

6. Pharmaceutical agent that contains at least one compound according to Claim 1 or 2.

7. Pharmaceutical agent according to Claim 6 for treating cancer, auto-immune diseases, cardiovascular diseases, infectious diseases, nephrological diseases, neurodegenerative diseases and viral infections.

8. Pharmaceutical preparation containing compounds according to Claim 1 or 2 with suitable formulation and vehicle substances.

## Revendications

1. Composés de formule générale I 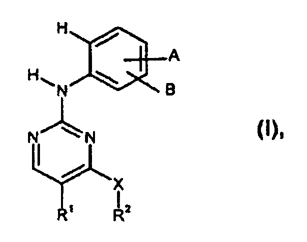 dans laquelle
R¹ représente un atome d'halogène,
R² représente un groupe -CH(CH₃)-(CH₂)ₙ-R⁵, un groupe -CH-(CH₂OH)₂, un groupe -(CH₂)ₙR⁷, un groupe -CH(C₃H₇)-(CH₂)ₙ-R⁵, un groupe -CH(C₂H₅)-(CH₂)ₙ-R⁵, un groupe -CH₂-CN, un groupe -CH(CH₃)COCH₃, un groupe -CH(CH₃)-C(OH)(CH₃)₂, un groupe -CH(CH(OH)CH₃)OCH₃, un groupe -CH(C₂H₅)CO-R⁵, un groupe alcynyle en C₂-C₄, un groupe -(CH₂)ₙ-COR⁵, un groupe -(CH₂)ₙ-CO-C₁-C₆-alkyle, un groupe -(CH₂)ₙ-C(OH)(CH₃)-phényle, un groupe -CH(CH₃)-C(CH₃)-R⁵, un groupe -CH(CH₃)-C (CH₃)(C₂H₅)-R⁵, un groupe -CH(OCH₃)-CH₂-R⁵, un groupe -CH₂-CH(OH)-R⁵, un groupe CH(OCH₃)-CHR⁵-CH₃, un groupe -CH(CH₃)-CH(OH)-CH₂-CH=CH₂, un groupe -CH(C₂H₅)-CH(OH)-(CH₂)ₙ-CH₃, un groupe -CH(CH₃)-CH(OH)-(CH₂)ₙ-CH₃, un groupe -CH(CH₃)-CH(OH)-CH(CH₃)₂, un groupe (CH₂OAC)₂, un groupe -(CH₂)ₙ-R⁶, un groupe -(CH₂)ₙ-(CF₂)ₙ-CF₃, un groupe -CH(CH₂)ₙ-R⁵)₂, un groupe -CH(CH₃)-CO-NH₂, un groupe -CH(CH₂OH)-phényle, un groupe -CH(CH₂OH)-CH(OH)-(CH₂)ₙR⁵, un groupe -CH(CH₂OH)-CH(OH)-phényle, un groupe -CH(CH₂OH)-C₂H₄-R⁵, un groupe -(CH₂)ₙ-C≡C-C(CH₃)=CH-COR⁵, un groupe -CH(Ph)-(CH₂)ₙ-R⁵, un groupe -(CH₂)ₙ-COR⁵, un groupe -(CH₂)ₙ-PO₃(R⁵)₂, un groupe -(CH₂)ₙ-COR⁵, un groupe -CH((CH₂)ₙ-OR⁵)CO-R⁵, un groupe -(CH₂)ₙ-CONHCH((CH₂)ₙR⁵)₂, un groupe -(CH₂)ₙNH-COR⁵, un groupe -CH(CH₂)ₙR⁵-(CH₂)ₙC₃-C₁₀-cycloalkyle, un groupe -(CH₂)ₙ-C₃-C₁₀-cycloalkyle, un groupe cycloalkyle en C₃-C₁₀, un groupe alkyle en C₁-C₆ ou un groupe cycloalkyle en C₃-C₁₀ éventuellement mono- ou polysubstitué, de façon identique ou différente, par un groupe hydroxy, un groupe alkyle en C₁-C₆ ou le groupe -COONH(CH₂)ₙCH₃ ou -NR³R⁴, un groupe -(CH₂)ₙ-O-(CH₂)ₙ-R⁵, un groupe -(CH₂)ₙ-NR³R⁴, un groupe -CH(C₃H₇)-(CH₂)ₙ-OC(O)-(CH₂)ₙ-CH₃, un groupe -(CH₂)ₙR⁵, un groupe -C(CH₃)₂-(CH₂)ₙ-R⁵, un groupe -C(CH₂)ₙ(CH₃)-(CH₂)ₙR⁵, un groupe-C(CH₂)ₙ-(CH₂)ₙR⁵, un groupe -CH(t-butyl)-(CH₂)ₙ-R⁵, un groupe -CCH₃(C₃H₇)-(CH₂)ₙR⁵, un groupe -CH(C₃H₇)-(CH₂)ₙ-R⁵, un groupe -CH(C₃H₇)-COR⁵, un groupe -CH(C₃H₇)-(CH₂)ₙ-OC(O)-NH-Ph, un groupe -CH((CH₂)ₙ(C₃H₇))-(CH₂)ₙR⁵, un groupe -CH(C₃H₇)-(CH₂)ₙ-OC(O)-NH-Ph(OR⁵)₃, un groupe -NR³R⁴, un groupe -NH(CH₂)ₙ-NR³R⁴, un groupe R⁵-(CH₂)ₙ-C*H-CH(R⁵)-(CH₂)ₙ-R⁵, un groupe -(CH₂)ₙ-CO-NH-(CH₂)ₙ-CO-R⁵, un groupe -OC(O)NH-C₁-C₆-al kyle ou un groupe -(CH₂)ₙ-CO-NH-(CH₂)ₙ-CH((CH₂)ₙ-P⁵)₂,
ou représente un groupe cycloalkyle en C₃-C₁₀ qui est substitué par le groupe 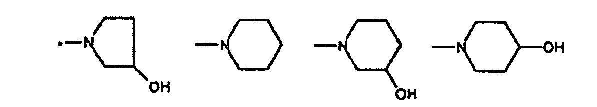 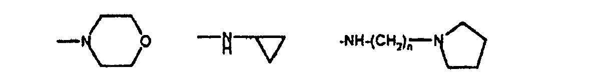 ou représente le groupe 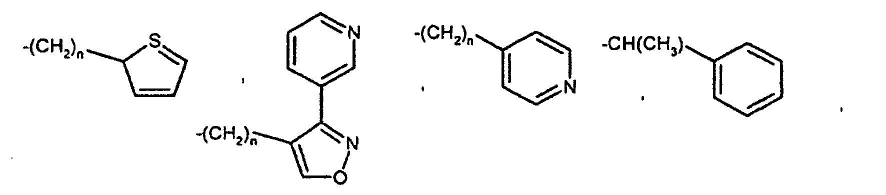 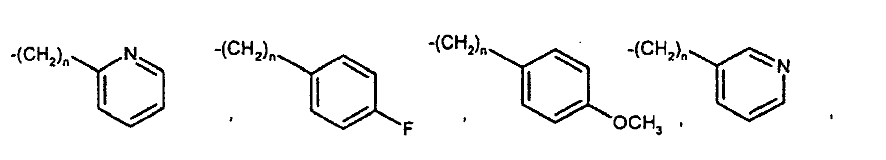 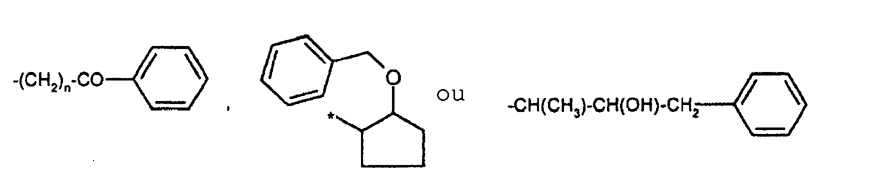 ou 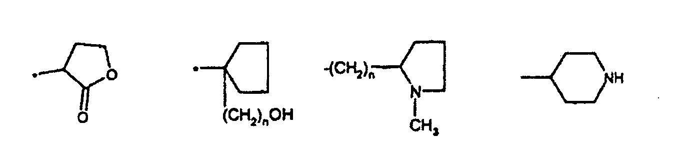 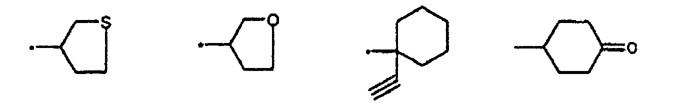
X représente un atome d'oxygène ou le groupe -NH- ou -N(C₁-C₃-alkyle), ou
R² représente le groupe 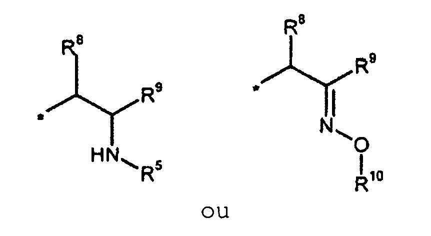
X et R² forment conjointement un groupe 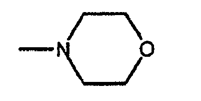
A représente le groupe -SO₂R⁷, SO₂-C₂H₄-OH, -SO₂CF₃ ou -SO₂-NR³R⁴,
B représente un atome d'hydrogène, un groupe hydroxy ou un groupe alkyle en C₁-C₃,
ou
A et B peuvent former conjointement un groupe 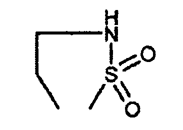
R³ et R⁴ représentent chacun, indépendamment l'un de l'autre, un atome d'hydrogène, un groupe phényle, un groupe benzyloxy, un groupe alkyle en C₁-C₁₂, un groupe alcoxy en C₁-C₆, un groupe alcényloxy en C₂-C₄, un groupe cycloalkyle en C₃-C₆, un groupe hydroxy, un groupe hydroxy-C₁-C₆-alkyle, un groupe dihydroxy-C₁-C₆-alkyle, un groupe hétéroaryle, un groupe hétérocyclo-C₃-C₁₀-alkyle, un groupe hétéroaryl-C₁-C₃-alkyle, un groupe C₃-C₆-cycloalkyl-C₁-C₃-alkyle éventuellement substitué par un groupe cyano,
ou
un groupe alkyle en C₁-C₆ éventuellement mono-ou polysubstitué, de façon identique ou différente, par un groupe phényle, un groupe pyridyle, un groupe phényloxy, un groupe cycloalkyle en C₃-C₆, un groupe alkyle en C₁-C₆ ou un groupe alcoxy en C₁-C₆, où le groupe phényle peut être lui-même mono- ou polysubstitué, de façon identique ou différente, par un atome d'halogène, un groupe trifluorométhyle, un groupe alkyle en C₁-C₆, un groupe alcoxy en C₁-C₆ ou par le groupe -SO₂NR³R⁴,
ou le groupe -(CH₂)ₙNR³R⁴, -CNHNH₂ ou -NR³R⁴
ou 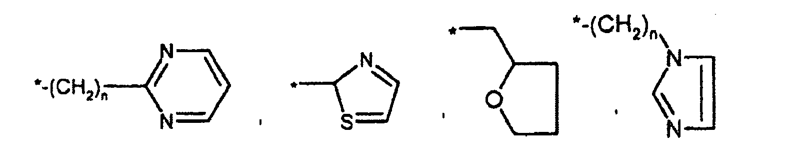 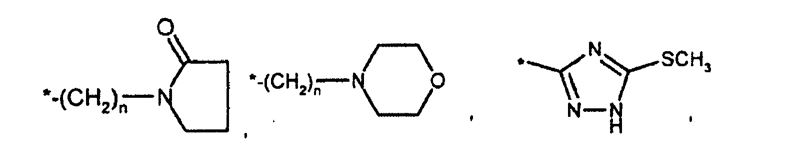 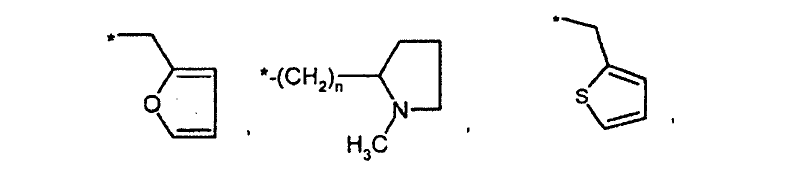 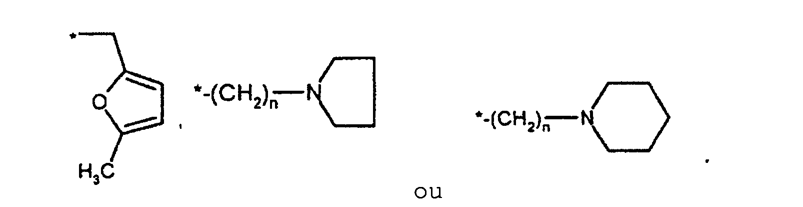 qui peuvent être éventuellement substitués par un groupe alkyle en C₁-C₆,
R⁵ représente un groupe hydroxy, un groupe phényle, un groupe alkyle en C₁-C₆, un groupe cycloalkyle en C₃-C₆, un groupe benzoxy, un groupe alkylthio en C₁-C₆ ou un groupe alcoxy en C₁-C₆,
R⁶ représente le groupe 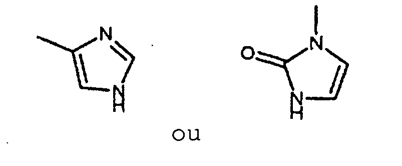
R⁷ représente un atome d'halogène, un groupe hydroxy, un groupe phényle, un groupe alkyle en C₁-C₆, un groupe -(CH₂)ₙOH, un groupe -NR³R⁴ ou le groupe 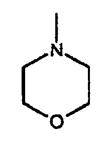
R⁸, R⁹ et R¹⁰ représentent un atome d'hydrogène, un groupe hydroxy, un groupe alkyle en C₁-C₆ ou le groupe -(CH₂)ₙ-COOH, et
n vaut de 0 à 6,
ainsi que leurs diastéréoisomères, leurs énantiomères et leurs sels.

2. Composé selon la revendication 1 de formules suivantes :
la 5-bromo-N2-[4-(2-diéthylamino-éthanesulfonyl)-phényl]-N4-prop-2-ynyl-pyrimidine-2,4-diamine,
la N2-(3-benzènesulfonyl-phényl)-5-bromo-N4-prop-2-ynyl-pyrimidine-2,4-diamine,
la 5-bromo-N2-[4-(morpholine-4-sulfonyl)-phényl]-N4-prop-2-ynyl-pyrimidine-2,4-diamine,
le 4-[5-fluoro-4-((R)-1-hydroxyméthyl-2-méthyl-propylamino)-pyrimidin-2-ylamino]-benzènesulfonamide,
le 4-[4-((R)-1-hydroxyméthyl-2-méthyl-propylamino)-5-iodo-pyrimidin-2-ylamino]-benzènesulfonamide,
le 4-(5-fluoro-4-prop-2-ynylamino-pyrimidin-2-ylamino)-benzènesulfonamide,
le 4-{5-bromo-4-[2-(2-oxo-2,3-dihydro-imidazol-1-yl)-éthylamino]-pyrimidin-2-ylamino}-benzènesulfonamide,
le 4-[5-bromo-4-(2,2,3,3,3-pentafluoro-propoxy)-pyrimidin-2-ylamino]-benzènesulfonamide,
le 4-[[5-bromo-4-(1,3-bisacétoxy-2-propyloxy)-2-pyrimidinyl]amino]-benzolsulfonamide,
le 4-[5-bromo-4-(2-hydroxy-1-hydroxyméthyl-éthoxy)-pyrimidin-2-ylamino]-benzènesulfonamide,
le (S)-2-[5-bromo-2-(4-sulfamoyl-phénylamino)-pyrimidin-4-ylamino]-propionamide,
la 5-bromo-N4-prop-2-ynyl-N2-(3-trifluorométhanesulfonyl-phényl)-pyrimidine-2,4-diamine,
le 3-(5-bromo-4-prop-2-ynylamino-pyrimidin-2-ylamino)-benzènesulfonamide,
le 3-(5-bromo-4-prop-2-ynylamino-pyrimidin-2-ylamino)-N-butyl-benzènesulfonamide,
le 2-[3-(5-bromo-4-prop-2-ynylamino-pyrimidin-2-ylamino)-benzènesulfonyl]-éthanol,
le 4-(5-bromo-4-prop-2-ynylamino-pyrimidin-2-ylamino)-benzènesulfonamide,
le 4-(5-chloro-4-prop-2-ynylamino-pyrimidin-2-ylamino)-benzènesulfonamide,
l'ester méthylique de l'acide 2-[5-bromo-2-(4-sulfamoyl-phénylamino)-pyrimidin-4-ylamino]-butyrique,
le 4-[5-bromo-4-((R)-1-hydroxyméthyl-2-méthyl-propylamino)-pyrimidin-2-ylamino]-benzènesulfonamide,
le 2-{5-bromo-2-[3-(2-hydroxy-éthanesulfonyl)-phénylamino]-pyrimidin-4-ylamino}-propane-1,3-diol,
le 4-[5-bromo-4-(2-hydroxy-1-hydoxyméthyl-éthylamino)-pyrimidin-2-ylamino]-benzènesulfonamide,
le 4-[5-bromo-4-(cyclopropylméthyl-amino)-pyrimidin-2-ylamino]-benzènesulfonamide,
le 4-[5-bromo-4-((R)-1-hydroxyméthyl-2-méthyl-propylamino)-pyrimidin-2-ylamino]-N-(2-hydroxy-éthyl)-benzènesulfonamide,
le 4-[5-bromo-4-((S)-2-méthoxy-1-méthyl-éthylamino)-pyrimidin-2-ylamino]-benzènesulfonamide,
le 2-{3-[5-bromo-4-((S)-2-méthoxy-1-méthyl-éthylamino)-pyrimidin-2-ylamino]-benzènesulfonyl}-éthanol,
le 2-{3-[5-bromo-4-(2-morpholin-4-yl-éthylamino)-pyrimidin-2-ylamino]-benzènesulfonyl}-éthanol,
le 4-[5-bromo-4-(2-morpholin-4-yl-éthylamino)-pyrimidin-2-ylamino]-benzènesulfonamide,
le 4-[5-bromo-4-(4,4,4-trifluoro-butoxy)-pyrimidin-2-ylamino]-benzènesulfonamide,
le 4-[5-bromo-4-(2-éthoxy-1-éthoxyméthyl-éthoxy)-pyrimidin-2-ylamino]-benzènesulfonamide,
l'acide 4-[5-bromo-2-(4-sulfamoyl-phénylamino)-pyrimidin-4-ylamino]-butyrique,
le 4-[5-bromo-4-((R)-1-hydroxyméthyl-2-méthyl-propylamino)-pyrimidin-2-ylamino]-N-(2-hydroxy-éthyl)-benzènesulfonamide,
le (R)-2-{5-bromo-2-[3-(2-hydroxy-éthanesulfonyl)-phénylamino]-pyrimidin-4-ylamino}-3-méthyl-butan-1-ol,
le 4-[5-bromo-4-((S)-1-hydroxyméthyl-2-méthyl-propylamino)-pyrimidin-2-ylamino]-benzènesulfonamide,
le 4-[5-bromo-4-((R)-2-hydroxy-1-phényl-éthylamino)-pyrimidin-2-ylamino]-benzènesulfonamide,
le 4-[5-bromo-4-((S)-2-méthoxy-1-méthyl-éthylamino)-pyrimidin-2-ylamino]-N-méthyl-benzènesulfonamide,
le 4-[5-bromo-4-((S)-2-méthoxy-1-méthyl-éthylamino)-pyrimidin-2-ylamino]-N,N-diméthyl-benzènesulfonamide,
l'ester benzylique de l'acide 5-[5-bromo-2-(4-sulfamoyl-phénylamino)-pyrimidin-4-ylamino]-pentanoïque,
l'ester méthylique de l'acide 6-[5-bromo-2-(4-sulfamoyl-phénylamino)-pyrimidin-4-ylamino)-hexanoïque,
le 4-(5-iodo-4-prop-2-ynylamino-pyrimidin-2-ylamino)-benzènesulfonamide,
le 4-[5-bromo-4-((S)-1-hydroxyméthyl-3-méthylsulfanyl-propylamino)-pyrimidin-2-ylamino]-benzènesulfonamide,
le 4-[5-bromo-4-((1S,2S)-2-hydroxy-1-hydroxyméthyl-2-phényl-éthylamino)-pyrimidin-2-ylamino]-benzènesulfonamide,
le 4-[5-bromo-4-((R)-2-oxo-tétrahydro-furan-3-ylamino)-pyrimidin-2-ylamino]-benzènesulfonamide,
le 4-[5-bromo-4-((S)-1-hydroxyméthyl-2,2-diméthyl-propylamino)-pyrimidin-2-ylamino]-benzènesulfonamide,
l'ester méthylique de l'acide 4-{3-[5-bromo-2-(4-sulfamoyl-phénylamino)-pyrimidin-4-ylamino]-propionylamino}-butyrique,
l'acide 6-[5-bromo-2-(4-sulfamoyl-phénylamino)-pyrimidin-4-ylammino]-hexanoïque,
le 4-(5-bromo-4-cyclohexylamino-pyrimidin-2-ylamino)-benzènesulfonamide,
l'ester méthylique de l'acide 4-{6-[5-bromo-2-(4-sulfamoyl-phénylamino)-pyrimidin-4-ylamino]-hexanoylamino}-butyrique,
l'ester méthylique de l'acide 6-[5-bromo-2-(4-sulfamoyl-phénylamino)-pyrimidin-4-ylamino]-hexanoïque,
le 4-[5-bromo-4-((1R,2S)-2,3-dihydroxy-1-hydroxyméthyl-propylamino)-pyrimidin-2-ylamino]-benzènesulfonamide,
le 4-[5-bromo-4-((R)-2-cyclohexyl-1-hydroxyméthyl-éthylamino)-pyrimidin-2-ylamino]-benzènesulfonamide,
le (2-hydroxy-1-hydroxyméthyl-éthyl)-amide de l'acide 6-[5-bromo-2-(4-sulfamoyl-phénylamino)-pyrimidin-4-ylamino]-hexanoïque,
le 4-[5-bromo-4-((S)-2-hydroxy-1-méthyl-éthylamino)-pyrimidin-2-ylamino]-benzènesulfonamide,
le 4-[5-bromo-4-(4-hydroxy-cyclohexylamino)-pyrimidin-2-ylamino]-benzènesulfonamide,
le 4-{4-[(adamantan-1-ylméthyl)-amino]-5-bromo-pyrimidin-2-ylamino}-benzènesulfonamide,
le 4-[5-bromo-4-((R)-1-hydroxyméthyl-2-méthyl-propylamino)-pyrimidin-2-ylamino]-N-méthyl-benzènesulfonamide,
l'acide (R)-2-[5-bromo-2-(4-sulfamoyl-phénylamino)-pyrimidin-4-ylamino]-3-méthyl-butyrique,
le 4-(5-bromo-4-cyclohexylamino-pyrimidin-2-ylamino)-N-(2-hydroxy-éthyl)-benzènesulfonamide,
le 4-[5-bromo-4-(2,2,3,3,4,4,4-heptafluoro-butylamino)-pyrimidin-2-ylamino]-N-(2-hydroxy-éthyl)-benzènesulfonamide,
le 4-[5-bromo-4-((S)-2-méthoxy-1-méthyl-éthylamino)-pyrimidin-2-ylamino]-N-(2-hydroxy-éthyl)-benzènesulfonamide,
l'ester (R)-2-[5-bromo-2-(4-sulfamoyl-phénylamino)-pyrimidin-4-ylamino]-3-méthyl-butylique de l'acide phényl-carbamique,
l'acide (R)-2-{5-bromo-2-[4-(2-hydroxy-éthylsulfamoyl)-phénylamino]-pyrimidin-4-ylamino}-3-méthyl-butyrique,
l'ester méthylique de l'acide (R)-2-{5-bromo-2-[4-(2-hydroxy-éthylsulfamoyl)-phénylamino]-pyrimidin-4-ylamino}-3-méthyl-butyrique,
le 4-(5-chloro-4-prop-2-ynylamino-pyrimidin-2-ylamino)-N-(2-hydroxy-éthyl)-benzènesulfonamide,
le 4-[5-bromo-4-(2-hydroxy-1,1-diméthyl-éthylamino)-pyrimidin-2-ylamino]-benzènesulfonamide,
le 4-[5-bromo-4-((S)-1-hydroxyméthyl-3-méthyl-butylamino)-pyrimidin-2-ylamino]-benzènesulfonamide,
le 4-[5-bromo-4-(tétrahydro-pyran-4-yloxy)-pyrimidin-2-ylamino]-benzènesulfonamide,
le 4-[5-bromo-4-((1S,2S)-2-hydroxy-cyclohexylamino)-pyrimidin-2-ylamino]-benzènesulfonamide,
l'ester 4-[5-bromo-2-(4-sulfamoyl-phénylamino)-pyrimidin-4-ylamino]-cyclohexylique de l'acide butyl-carbamique,
le (R)-2-[5-bromo-2-(4-méthanesulfonyl-phénylamino)-pyrimidin-4-ylamino]-3-méthyl-butan-1-ol,
le (R)-2-{5-bromo-2-[4-(morpholine-4-sulfonyl)-phénylamino]-pyrimidin-4-ylamino}-3-méthyl-butan-1-ol,
le 4-[5-bromo-4-(4-diméthylamino-cyclohexylamino)-pyrimidin-2-ylamino]-benzènesulfonamide,
le 4-[5-bromo-4-(4-diméthylamino-cyclohexylamino)-pyrimidin-2-ylamino]-benzènesulfonamide,
le 4-[5-bromo-4-(4-pipéridin-1-yl-cyclohexylamino)-pyrimidin-2-ylamino]-benzènesulfonamide,
le 4-[5-bromo-4-((R)-1-hydroxyméthyl-2-méthyl-propylamino)-pyrimidin-2-ylamino]-3-hydroxy-benzènesulfonamide,
le 4-[5-bromo-4-(4-cyclopropylamino-cyclohexylamino)-pyrimidin-2-ylamino]-benzènesulfonamide,
le (R)-2-[5-bromo-2-(3-méthanesulfonyl-phénylamino)-pyrimidin-4-ylamino]-3-méthyl-butan-1-ol,
le 4-{5-bromo-4-[2-(2-éthoxy-éthoxy)-éthoxy]-pyrimidin-2-ylamino}-benzènesulfonamide,
le 4-{5-bromo-4-[4-(2-diméthylamino-éthylamino)-cyclohexylamino]-pyrimidin-2-ylamino}-benzènesulfonamide,
le 4-{5-bromo-4-[4-(2-pyrrolidin-1-yl-éthylamino)-cyclohexylamino]-pyrimidin-2-ylamino}-benzènesulfonamide,
le 4-{5-bromo-4-[4-(4-hydroxy-pipéridin-1-yl)-cyclohexylamino]-pyrimidin-2-ylamino}-benzènesulfonamide,
l'ester méthylique de l'acide 4-{5-bromo-2-[4-(2-hydroxy-éthylsulfamoyl)-phénylamino]-pyrimidin-4-ylamino}-butyrique,
le 4-{5-bromo-4-[4-(3-hydroxy-pyrrolidin-1-yl)-cyclohexylamino]-pyrimidin-2-ylamino}-benzènesulfonamide,
le 4-{5-bromo-4-[4-(3-hydroxy-pyrrolidin-1-yl)-cyclohexylamino]-pyrimidin-2-ylamino}-benzènesulfonamide,
le 4-[5-bromo-4-((R)-1-hydroxyméthyl-3-méthyl-butylamino)-pyrimidin-2-ylamino]-benzènesulfonamide,
le 4-{5-bromo-4-[2-(1-méthyl-pyrrolidin-2-yl)-éthylamino]-pyrimidin-2-ylamino}-benzènesulfonamide,
le 4-[5-bromo-4-(pipéridin-4-yloxy)-pyrimidin-2-ylamino]-benzènesulfonamide,
le 4-[5-bromo-4-(3-diméthylamino-propylamino)-pyrimidin-2-ylamino]-benzènesulfonamide,
l'ester (R)-2-[5-bromo-2-(4-sulfamoyl-phénylamino)-pyrimidin-4-ylamino]-3-méthyl-butylique de l'acide acétique,
le 4-[5-bromo-4-(4-hydroxy-cyclohexylamino)-pyrimidin-2-ylamino]-N-(2-hydroxy-éthyl)-benzènesulfonamide,
l'ester (R)-2-[5-bromo-2-(4-sulfamoyl-phénylamino)-pyrimidin-4-ylamino]-3-méthyl-butylique de l'acide (3,4,5-triméthoxy-phényl)-carbamique,
le 4-(5-bromo-4-cycloheptylamino-pyrimidin-2-ylamino)-benzènesulfonamide,
le 4-[5-bromo-4-((R)-1-hydroxyméthyl-2,2-diméthyl-propylamino)-pyrimidin-2-ylamino]-benzènesulfonamide,
le 4-[5-bromo-4-(4-oxo-cyclohexylamino)-pyrimidin-2-ylamino]-benzènesulfonamide,
l'ester méthylique de l'acide (E)-6-{5-chloro-2-[4-(2-hydroxy-éthylsulfamoyl)-phénylamino]-pyrimidin-4-ylamino}-3-méthyl-hex-2-én-4-ynoïque,
le 4-[5-bromo-4-(1-hydroxyméthyl-cyclopentylamino)-pyrimidin-2-ylamino]-benzènesulfonamide,
le 4-[4-(bicyclo[2.2.1]hept-2-ylamino)-5-bromo-pyrimidin-2-ylamino]-benzènesulfonamide,
le 4-[5-bromo-4-(4-morpholin-4-yl-cyclohexylamino)-pyrimidin-2-ylamino]-benzènesulfonamide,
le 4-[5-bromo-4-(4-morpholin-4-yl-cyclohexylamino)-pyrimidin-2-ylamino]-benzènesulfonamide,
le 4-[5-bromo-4((R)-2-hydroxy-1-méthyl-éthylamino)-pyrimidin-2-ylamino]-benzènesulfonamide,
le 4-({5-bromo-4-[(R)-(2-hydroxy-1-méthyléthyl)amino]pyrimidin-2-yl}amino)benzènesulfonamide,
le 4-[5-bromo-4-(2,2,3,3,3-pentafluoro-propylamino)-pyrimidin-2-ylamino]-benzènesulfonamide,
le 4-[5-bromo-4-(4-hydroxy-butylamino)-pyrimidin-2-ylamino]-benzènesulfonamide,
le 4-[5-bromo-4-(1-éthynyl-cyclohexylamino)-pyrimidin-2-ylamino]-benzènesulfonamide,
le 4-[5-bromo-4-(3-hydroxy-1-phényl-propylamino)-pyrimidin-2-ylamino]-benzènesulfonamide,
le (+)-4-[5-bromo-4-(2-méthyl-cyclohexylamino)-pyrimidin-2-ylamino]-benzènesulfonamide,
le (-)-4-[5-bromo-4-(2-méthyl-cyclohexylamino)-pyrimidin-2-ylamino]-benzènesulfonamide,
le 4-{5-bromo-4-[4-(2-hydroxy-1-hydroxyméthyl-éthylamino)-cyclohexylamino]-pyrimidin-2-ylamino}-benzènesulfonamide,
le 4-{5-bromo-4-[4-(2-hydroxy-1-hydroxyméthyl-éthylamino)-cyclohexylamino]-pyrimidin-2-ylamino)-benzènesulfonamide,
le (R)-2-[5-bromo-2-(4-trifluorométhanesulfonyl-phénylamino)-pyrimidin-4-ylamino]-3-méthyl-butan-1-ol,
le 4-[5-bromo-4-(4-cyclopropylamino-cyclohexylamino)-pyrimidin-2-ylamino]-benzènesulfonamide,
le 4-[5-bromo-4-(4-cyclopropylamino-cyclohexylamino)pyrimidin-2-ylamino]-benzènesulfonamide,
le 4-[5-bromo-4-(tétrahydro-thiophén-3-ylamino)-pyrimidin-2-ylamino]-benzènesulfonamide,
l'ester méthylique de l'acide 2-[5-bromo-2-(4-sulfamoyl-phénylamino)-pyrimidin-4-ylamino]-3-hydroxy-propionique,
le 4-[5-bromo-4-((R)-1-hydroxyméthyl-2-méthyl-propylamino)-pyrimidin-2-ylamino]-N-pyrimidin-2-yl-benzènesulfonamide,
le 4-[5-bromo-4-((R)-1-hydroxyméthyl-2-méthyl-propylamino)-pyrimidin-2-ylamino]-N-thiazol-2-yl-benzènesulfonamide,
le 4-[5-bromo-4-(4-hydroxy-butylamino)-pyrimidin-2-ylamino]-N-méthyl-benzènesulfonamide,
le 4-{5-bromo-4-[2-(3H-imidazol-4-yl)-éthylamino]-pyrimidin-2-ylamino}-benzènesulfonamide,
le 4-[5-bromo-4-(1-éthyl-1-hydroxyméthyl-propylamino)-pyrimidin-2-ylamino]-benzènesulfonamide,
le 4-[5-bromo-4-(1-hydroxyméthyl-1,2-diméthyl-propylamino)-pyrimidin-2-ylamimno]-benzènesulfonamide,
le 4-[5-bromo-4-(4-méthyl-cyclohexylamino)-pyrimidin-2-ylamino]-benzènesulfonamide,
le 4-{5-bromo-4-[(S)-(tétrahydro-furan-3-yl)amino]-pyrimidin-2-ylamino}-benzènesulfonamide,
le 4-[5-bromo-4-(cyclohexylméthyl-amino)-pyrimidin-2-ylamino]-benzènesulfonamide,
l'acide 3-[5-bromo-2-(4-sulfamoyl-phénylamino)-pyrimidin-4-ylamino]-propionique,
l'ester éthylique de l'acide 4-[5-bromo-2-(4-sulfamoyl-phénylamino)-pyrimidin-4-ylamino]-butyrique,
l'ester diéthylique de l'acide {3-[5-bromo-2-(4-sulfamoyl-phénylamino)-pyrimidin-4-ylamino]-propyl}-phosphonique,
le 4-{5-bromo-4-[(3-pyridin-3-yl-isoxazol-4-ylméthyl)-amino]-pyrimidin-2-ylamino}-benzènesulfonamide,
l'ester diméthylique de l'acide {[5-bromo-2-(4-sulfamoyl-phénylamino)-pyrimidin-4-ylamino]-méthyl}-phosphonique,
l'ester monométhylique de l'acide {[5-bromo-2-(4-sulfamoyl-phénylamino)-pyrimidin-4-ylamino]-méthyl}-phosphonique,
le 4-{5-bromo-4-[(pyridin-4-ylméthyl)-amino]-pyrimidin-2-ylamino}-benzènesulfonamide,
le 4-{5-bromo-4-[(thiophén-2-ylméthyl)-amino]-pyrimidin-2-ylamino}-benzènesulfonamide,
le 4-{5-bromo-4-[(pyridin-2-ylméthyl)-amino]-pyrimidin-2-ylamino}-benzènesulfonamide,
le 4-[5-bromo-4-(4-fluoro-benzylamino)-pyrimidin-2-ylamino]-benzènesulfonamide,
le 4-[5-bromo-4-(4-méthoxy-benzylamino)-pyrimidin-2-ylamino]-benzènesulfonamide,
l'acide {3-[5-bromo-2-(4-sulfamoyl-phénylamino)-pyrimidin-4-ylamino]-propyl}-phosphonique,
le 4-[5-bromo-4-(2-hydroxy-éthylamino)-pyrimidin-2-ylamino]-benzènesulfonamide,
le 4-[5-bromo-4-(1-phényl-éthylamino)-pyrimidin-2-ylamino]-benzènesulfonamide,
le N-allyloxy-4-[5-bromo-4-((R)-1-hydroxyméthyl-2-méthyl-propylamino)-pyrimidin-2-ylamino]-benzènesulfonamide,
le 3-[5-bromo-4-((R)-1-hydroxyméthyl-2-méthyl-propylamino)-pyrimidin-2-ylamino]-N-(5-hydroxy-pentyl)-benzènesulfonamide,
le 3-[5-bromo-4-((R)-1-hydroxyméthyl-2-méthyl-propylamino)-pyrimidin-2-ylamino]-N-(2-hydroxy-1-hydroxyméthyl-éthyl)-benzènesulfonamide,
le 3-[5-bromo-4-((R)-1-hydroxyméthyl-2-méthyl-propylamino)-pyrimidin-2-ylamino]-N-(3-hydroxy-propyl)-benzènesulfonamide,
le N-benzyloxy-4-[5-bromo-4-((R)-1-hydroxyméthyl-2-méthyl-propylamino)-pyrimidin-2-ylamino]-benzènesulfonamide,
le 4-[5-bromo-4-((R)-1-hydroxyméthyl-2-méthyl-propylamino)-pyrimidin-2-ylamino]-N-(2-hydroxy-butyl)-benzènesulfonamide,
le 4-[5-bromo-4-((R)-1-hydroxyméthyl-2-méthyl-propylamino)-pyrimidin-2-ylamino]-N-cyclopropylméthyl-benzènesulfonamide,
le 4-[5-bromo-4-((R)-1-hydroxyméthyl-2-méthyl-propylamino)-pyrimidin-2-ylamino]-N-(4-cyano-cyclohexylméthyl)-benzènesulfonamide,
le 4-[5-bromo-4-((R)-1-hydroxyméthyl-2-méthyl-propylamino)-pyrimidin-2-ylamino]-N-(5-hydroxy-pentyl)-benzènesulfonamide,
le 4-[5-bromo-4-((R)-1-hydroxyméthyl-2-méthyl-propylamino)-pyrimidin-2-ylamino]-N-(tétrahydro-furan-2-ylméthyl)-benzènesulfonamide,
le 4-[5-bromo-4-((S)-1-hydroxyméthyl-2-méthyl-propylamino)-pyrimidin-2-ylamino]-N-hydroxy-benzènesulfonamide,
le 4-[5-bromo-4-((S)-1-hydroxyméthyl-propylamino)-pyrimidin-2-ylamino]-benzènesulfonamide,
le 4-[5-bromo-4-((R)-1-hydroxyméthyl-2-méthyl-propylamino)-pyrimidin-2-ylamino]-N-(2-hydroxy-1-hydroxyméthyl-éthyl)-benzènesulfonamide,
le 4-[5-bromo-4-((R)-1-hydroxyméthyl-2-méthyl-propylamino)-pyrimidin-2-ylamino]-N-(3-hydroxy-propyl)-benzènesulfonamide,
le 4-[5-bromo-4-((R)-1-hydroxyméthyl-2-méthyl-propylamino)-pyrimidin-2-ylamino]-N-hydroxy-benzènesulfonamide,
le 5-[5-bromo-4-((R)-1-hydroxyméthyl-2-méthyl-propylamino)-pyrimidin-2-ylamino]-2-méthyl-benzènesulfonamide,
le 3-[5-bromo-4-((R)-1-hydroxyméthyl-2-méthyl-propylamino)-pyrimidin-2-ylamino]-N-éthoxy-benzènesulfonamide,
le N-allyloxy-3-[5-bromo-4-((R)-1-hydroxyméthyl-2-méthyl-propylamino)-pyrimidin-2-ylamino]-benzènesulfonamide,
le 4-(5-bromo-4-morpholin-4-yl-pyrimidin-2-ylamino)-benzènesulfonamide,
le 7-[(5-bromo-4-{[(R)-2-hydroxy-1-méthyléthyl]amino}pyrimidin-2-yl)amino]-3,4-dihydro-2H-1,2-benzothiazine-1,1-dioxyde,
le 4-[5-bromo-4-(2-hydroxy-2-phényl-propylamino)-pyrimidin-2-ylamino]-benzènesulfonamide,
le 4-[5-bromo-4-(2-hydroxy-1,2-diméthyl-propylamino)-pyrimidin-2-ylamino]-benzènesulfonamide,
le 4-[5-bromo-4-(2-hydroxy-1,2-diméthyl-butylamino)-pyrimidin-2-ylamino]-benzènesulfonamide,
le 4-[5-bromo-4-(2-oxo-2-phényl-éthylamino)-pyrimidin-2-ylamino]-benzènesulfonamide,
le 4-[5-bromo-4-(2-hydroxy-propylamino)-pyrimidin-2-ylamino]-benzènesulfonamide,
le 4-[5-bromo-4-(2-hydroxy-1-méthyl-propylamino)-pyrimidin-2-ylamino]-benzènesulfonamide,
le 4-(5-bromo-4-sec-butylamino-pyrimidin-2-ylamino)-benzènesulfonamide,
le 4-[5-bromo-4-(2-hydroxy-1-méthyl-butylamino)-pyrimidin-2-ylamino]-benzènesulfonamide,
le 4-[5-bromo-4-(3,3-diméthyl-2-oxo-butylamino)-pyrimidin-2-ylamino]-benzènesulfonamide,
le 4-[5-bromo-4-(2-hydroxy-1-méthyl-3-phényl-propylamino)-pyrimidin-2-ylamino]-benzènesulfonamide,
le 4-[5-bromo-4-(2-hydroxy-butylamino)-pyrimidin-2-ylamino]-benzènesulfonamide,
le 4-[5-bromo-4-(2-hydroxy-1-méthyl-pent-4-énylamino)-pyrimidin-2-ylamino]-benzènesulfonamide,
le 4-[5-bromo-4-(2-hydroxy-1-méthyl-pentylamino)-pyrimidin-2-ylamino]-benzènesulfonamide,
le 4-[5-bromo-4-(1-méthyl-2-oxo-propylamino)-pyrimidin-2-ylamino]-benzènesulfonamide,
le 4-[5-bromo-4-(2-hydroxy-1,3-diméthyl-butylamino)-pyrimidin-2-ylamino]-benzènesulfonamide,
le 4-[4-((1R,2R)-2-benzyloxy-cyclopentylamino)-5-bromo-pyrimidin-2-ylamino]-benzènesulfonamide,
le 4-[4-((1S,2S)-2-benzyloxy-cyclopentylamino)-5-bromo-pyrimidin-2-ylamino]-benzènesulfonamide,
le 4-[5-bromo-4-(1-éthyl-2-hydroxy-propylamino)-pyrimidin-2-ylamino]-benzènesulfonamide,
le 4-[5-bromo-4-(1-éthyl-2-hydroxy-butylamino)-pyrimidin-2-ylamino]-benzènesulfonamide,
le 4-[5-bromo-4-(2-hydroxy-2-phényl-éthylamino)-pyrimidin-2-ylamino]-benzènesulfonamide,
le 4-[5-bromo-4-(2-hydroxy-3,3-diméthyl-butylamino)-pyrimidin-2-ylamino]-benzènesulfonamide,
le 4-[5-bromo-4-(2-hydroxyimino-1-méthyl-propylamino)-pyrimidin-2-ylamino]-benzènesulfonamide,
le 4-(5-bromo-4-{2-[(E)-méthoxyimino]-1-méthyl-propylamino}-pyrimidin-2-ylamino)-benzènesulfonamide,
le 4-(5-bromo-4-{2-[(E)-tert-butoxyimino]-1-méthyl-propylamino}-pyrimidin-2-ylamino)-benzènesulfonamide,
l'acide [2-[5-bromo-2-(4-sulfamoyl-phénylamino)-pyrimidin-4-ylamino]-1-méthyl-prop-(E)-ylidénaminooxy]-acétique,
le 4-[5-bromo-4-(2-cyclopropylamino-1-méthyl-propylamino)-pyrimidin-2-ylamino]-benzènesulfonamide,
le 4-[5-bromo-4-(2-hydroxy-1-méthoxyméthyl-éthylamino)-pyrimidin-2-ylamino]-benzènesulfonamide,
le 4-[5-bromo-4-(2-hydroxy-1-méthoxyméthyl-propylamino)-pyrimidin-2-ylamino]-benzènesulfonamide,
le 5-[5-bromo-4-((R)-1-hydroxyméthyl-propylamino)-pyrimidin-2-ylamino]-N-cyclohexylméthyl-2-méthyl-benzènesulfonamide,
le 5-[5-bromo-4-((R)-1-hydroxyméthyl-propylamino)-pyrimidin-2-ylamino]-2-méthyl-N-(4-phényl-butyl)-benzènesulfonamide,
le 5-[5-bromo-4-((R)-1-hydroxyméthyl-propylamino)-pyrimidin-2-ylamino]-N-(3,3-diphényl-propyl)-2-méthyl-benzènesulfonamide,
le 5-[5-bromo-4-((R)-1-hydroxyméthyl-propylamino)-pyrimidin-2-ylamino]-N-(2-diméthylamino-éthyl)-2-méthyl-benzènesulfonamide,
le 5-[5-bromo-4-((R)-1-hydroxyméthyl-propylamino)-pyrimidin-2-ylamino]-N-3-imidazol-1-yl-propyl)-2-méthyl-benzènesulfonamide,
le 5-[5-bromo-4-((R)-1-hydroxyméthyl-propylamino)-pyrimidin-2-ylamino]-2-méthyl-N-(3-trifluorométhyl-benzyl)-benzènesulfonamide,
le 5-[5-bromo-4-((R)-1-hydroxyméthyl-propylamino)-pyrimidin-2-ylamino]-N-heptyl-2-méthyl-benzènesulfonamide,
le 5-[5-bromo-4-((R)-1-hydroxyméthyl-propylamino)-pyrimidin-2-ylamino]-2-méthyl-N-octyl-benzènesulfonamide,
le 5-[5-bromo-4-((R)-1-hydroxyméthyl-propylamino)-pyrimidin-2-ylamino]-2-méthyl-N-nonyl-benzènesulfonamide,
le 5-[5-bromo-4-((R)-1-hydroxyméthyl-propylamino)-pyrimidin-2-ylamino]-N-décyl-2-méthyl-benzènesulfonamide,
le 5-[5-bromo-4-((R)-1-hydroxyméthyl-propylamino)-pyrimidin-2-ylamino]-N-furan-2-ylméthyl-2-méthyl-benzènesulfonamide,
le 5-[5-bromo-4-((R)-1-hydroxyméthyl-propylamino)-pyrimidin-2-ylamino]-2-méthyl-N-(4-trifluorométhyl-benzyl)-benzènesulfonamide,
le 5-[5-bromo-4-((R)-1-hydroxyméthyl-propylamino)-pyrimidin-2-ylamino]-2-méthyl-N-(3-phényl-propyl)-benzènesulfonamide,
le 5-[5-bromo-4-((R)-1-hydroxyméthyl-propylamino)-pyrimidin-2-ylamino]-2-méthyl-N-[3-(2-oxo-pyrrolidin-1-yl)-propyl]-benzènesulfonamide,
le 5-[5-bromo-4-((R)-1-hydroxyméthyl-propylamino)-pyrimidin-2-ylamino]-2-méthyl-N-(3-méthyl-butyl)-benzènesulfonamide,
le 5-[5-bromo-4-((R)-1-hydroxyméthyl-propylamino)-pyrimidin-2-ylamino]-N-hexyl-2-méthyl-benzènesulfonamide,
le 5-[5-bromo-4-((R)-1-hydroxyméthyl-propylamino)-pyrimidin-2-ylamino]-N-(3-diméthylamino-propyl)-2-méthyl-benzènesulfonamide,
le 5-[5-bromo-4-((R)-1-hydroxyméthyl-propylamino)-pyrimidin-2-ylamino]-N-(2-diisopropylamino-éthyl)-2-méthyl-benzènesulfonamide,
le 5-[5-bromo-4-((R)-1-hydroxyméthyl-propylamino)-pyrimidin-2-ylamino]-N-cyclopropyl-2-méthyl-benzènesulfonamide,
le 5-[5-bromo-4-((R)-1-hydroxyméthyl-propylamino)-pyrimidin-2-ylamino]-N-(4-cyano-cyclohexylméthyl)-2-méthyl-benzènesulfonamide,
le 5-[5-bromo-4-((R)-1-hydroxyméthyl-propylamino)-pyrimidin-2-ylamino]-2-méthyl-N-phénéthyl-benzènesulfonamide,
le 5-[5-bromo-4-((R)-1-hydroxyméthyl-propylamino)-pyrimidin-2-ylamino]-N-[2-(4-chloro-phényl)-éthyl]-2-méthyl-benzènesulfonamide,
le 5-[5-bromo-4-((R)-1-hydroxyméthyl-propylamino)-pyrimidin-2-ylamino]-2-méthyl-N-(5-méthyl-furan-2-ylméthyl)-benzènesulfonamide,
le 5-[5-bromo-4-((R)-1-hydroxyméthyl-propylamino)-pyrimidin-2-ylamino]-2-méthyl-N-(2-morpholin-4-yl-éthyl)-benzènesulfonamide,
le 5-[5-bromo-4-((R)-1-hydroxyméthyl-propylamino)-pyrimidin-2-ylamino]-N-(2-méthoxy-éthyl)-2-méthyl-benzènesulfonamide,
le 5-[5-bromo-4-((R)-1-hydroxyméthyl-propylamino)-pyrimidin-2-ylamino]-2-méthyl-N-thiophén-2-ylméthyl-benzènesulfonamide,
le 5-[5-bromo-4-((R)-1-hydroxyméthyl-propylamino)-pyrimidin-2-ylamino]-N-[2-(4-méthoxy-phényl)-éthy]-2-méthyl-benzènesulfonamide,
le 5-[5-bromo-4-((R)-1-hydroxyméthyl-propylamino)-pyrimidin-2-ylamino]-2-méthyl-N-(2-pyrrolidin-1-yl-éthyl)-benzènesulfonamide,
le 5-[5-bromo-4-((R)-1-hydroxyméthyl-propylamino)-pyrimidin-2-ylamino]-2-méthyl-N-(2-pipéridin-1-yl-éthyl)-benzènesulfonamide,
le 5-[5-bromo-4-((R)-1-hydroxyméthyl-propylamino)-pyrimidin-2-ylamino]-N-(3-fluoro-benzyl)-2-méthyl-benzènesulfonamide,
le 5-[5-bromo-4-((R)-1-hydroxyméthyl-propylamino)-pyrimidin-2-ylamino]-2-méthyl-N-[2-(1-méthyl-pyrrolidin-2-yl)-éthyl]-benzènesulfonamide,
le 5-[5-bromo-4-((R)-1-hydroxyméthyl-propylamino)-pyrimidin-2-ylamino]-2-méthyl-N-pyridin-3-ylméthyl-benzènesulfonamide,
le 5-[5-bromo-4-((R)-1-hydroxyméthyl-propylamino)-pyrimidin-2-ylamino]-N-(4-fluoro-benzyl)-2-méthyl-benzènesulfonamide,
le 5-[5-bromo-4-((R)-1-hydroxyméthyl-propylamino)-pyrimidin-2-ylamino]-2-méthyl-N-pyridin-4-ylméthyl-benzènesulfonamide,
le 5-[5-bromo-4-((R)-1-hydroxyméthyl-propylamino)-pyrimidin-2-ylamino]-2-méthyl-N-(2-phénoxy-éthyl)-benzènesulfonamide,
le 5-[5-bromo-4-((R)-1-hydroxyméthyl-propylamino)-pyrimidin-2-ylamino]-N-(2-méthoxy-benzyl)-2-méthyl-benzènesulfonamide,
le 5-[5-bromo-4-((R)-1-hydroxyméthyl-propylamino)-pyrimidin-2-ylamino]-2-méthyl-N-[2-(4-sulfamoyl-phényl)-éthyl)-benzènesulfonamide,
le 5-[5-bromo-4-((R)-1-hydroxyméthyl-propylamino)-pyrimidin-2-ylamino]-2-méthyl-N-(4-méthyl-benzyl)-benzènesulfonamide,
le 5-[5-bromo-4-((R)-2-hydroxy-1-méthyl-éthylamino)-pyrimidin-2-ylamino]-N-cyclohexylméthyl-2-méthyl-benzènesulfonamide,
le 5-[5-bromo-4-((R)-2-hydroxy-1-méthyl-éthylamino)-pyrimidin-2-ylamino]-2-méthyl-N-(4-phényl-butyl)-benzènesulfonamide,
le 5-[5-bromo-4-((R)-2-hydroxy-1-méthyl-éthylamino)-pyrimidin-2-ylamino]-N-(2-diméthylamino-éthyl)-2-méthyl-benzènesulfonamide,
le 5-[5-bromo-4-((R)-2-hydroxy-1-méthyl-éthylamino)-pyrimidin-2-ylamino]-N-(3-imidazol-1-yl-propyl)-2-méthyl-benzènesulfonamide,
le 5-[5-bromo-4-((R)-2-hydroxy-1-méthyl-éthylamino)-pyrimidin-2-ylamino]-2-méthyl-N-(3-trifluorométhyl-benzyl)-benzènesulfonamide,
le 5-[5-bromo-4-((R)-2-hydroxy-1-méthyl-éthylamino)-pyrimidin-2-ylamino]-N-furan-2-ylméthyl-2-méthyl-benzènesulfonamide,
le 5-[5-bromo-4-((R)-2-hydroxy-1-méthyl-éthylamino)-pyrimidin-2-ylamino]-2-méthyl-N-(4-trifluorométhyl-benzyl)-benzènesulfonamide,
le 5-[5-bromo-4-((R)-2-hydroxy-1-méthyl-éthylamino)-pyrimidin-2-ylamino]-2-méthyl-N-(3-phényl-propyl)-benzènesulfonamide,
le 5-[5-bromo-4-((R)-2-hydroxy-1-méthyl-éthylamino)-pyrimidin-2-ylamino]-2-méthyl-N-[3-(2-oxo-pyrrolidin-1-yl)-propyl]-benzènesulfonamide,
le 5-[5-bromo-4-((R)-2-hydroxy-1-méthyl-éthylamino)-pyrimidin-2-ylamino]-2-méthyl-N-(3-méthyl-butyl)-benzènesulfonamide,
le 5-[5-bromo-4-((R)-2-hydroxy-1-méthyl-éthylamino)-pyrimidin-2-ylamino]-N-hexyl-2-méthyl-benzènesulfonamide,
le 5-[5-bromo-4-((R)-2-hydroxy-1-méthyl-éthylamino)-pyrimidin-2-ylamino]-N-(3-diméthylamino-propyl)-2-méthyl-benzènesulfonamide,
le 5-[5-bromo-4-((R)-2-hydroxy-1-méthyl-éthylamino)-pyrimidin-2-ylamino]-N-(2-diisopropylamino-éthyl)-2-méthyl-benzènesulfonamide,
le 5-[5-bromo-4-((R)-2-hydroxy-1-méthyl-éthylamino)-pyrimidin-2-ylamino]-N-cyclopropyl-2-méthyl-benzènesulfonamide,
le 5-[5-bromo-4-((R)-2-hydroxy-1-méthyl-éthylamino)-pyrimidin-2-ylamino]-N-(4-cyano-cyclohexylméthyl)-2-méthyl-benzènesulfonamide,
le 5-[5-bromo-4-((R)-2-hydroxy-1-méthyl-éthylamino)-pyrimidin-2-ylamino]-2-méthyl-N-phénétyl-benzènesulfonamide,
le 5-[5-bromo-4-((R)-2-hydroxy-1-méthyl-éthylamino)-pyrimidin-2-ylamino]-N-[2-(4-chloro-phényl)-éthyl]-2-méthyl-benzènesulfonamide,
le 5-[5-bromo-4-((R)-2-hydroxy-1-méthyl-éthylamino)-pyrimidin-2-ylamino]-2-méthyl-N-(5-méthyl-furan-2-ylméthyl)-benzènesulfonamide,
le 5-[5-bromo-4-((R)-2-hydroxy-1-méthyl-éthylamino)-pyrimidin-2-ylamino]-2-méthyl-N-(2-morpholin-4-yl-éthyl)-benzènesulfonamide,
le 5-[5-bromo-4-((R)-2-hydroxy-1-méthyl-éthylamino)-pyrimidin-2-ylamino]-N-(2-méthoxy-éthyl)-2-méthyl-benzènesulfonamide,
le 5-[5-bromo-4-((R)-2-hydroxy-1-méthyl-éthylamino)-pyrimidin-2-ylamino]-2-méthyl-N-thiophén-2-ylméthyl-benzènesulfonamide,
le 5-[5-bromo-4-((R)-2-hydroxy-1-méthyl-éthylamino)-pyrimidin-2-ylamino]-N-[2-(4-méthoxy-phényl)-éthyl]-2-méthyl-benzènesulfonamide,
le 5-[5-bromo-4-((R)-2-hydroxy-1-méthyl-éthylamino)-pyrimidin-2-ylamino]-2-méthyl-N-(2-pyrrolidin-1-yl-éthyl)-benzènesulfonamide,
le 5-[5-bromo-4-((R)-2-hydroxy-1-méthyl-éthylamino)-pyrimidin-2-ylamino]-2-méthyl-N-(2-pipéridin-1-yl-éthyl)-benzènesulfonamide,
le 5-[5-bromo-4-((R)-2-hydroxy-1-méthyl-éthylamino)-pyrimidin-2-ylamino]-N-(3-fluoro-benzyl)-2-méthyl-benzènesulfonamide,
le 5-[5-bromo-4-((R)-2-hydroxy-1-méthyl-éthylamino)-pyrimidin-2-ylamino]-2-méthyl-N-[2-(1-méthyl-pyrrolidin-2-yl)-éthyl-benzènesulfonamide,
le 5-[5-bromo-4-((R)-2-hydroxy-1-méthyl-éthylamino)-pyrimidin-2-ylamino]-2-méthyl-N-pyridin-3-ylméthyl-benzènesulfonamide,
le 5-[5-bromo-4-((R)-2-hydroxy-1-méthyl-éthylamino)-pyrimidin-2-ylamino]-N-(4-fluoro-benzyl)-2-méthyl-benzènesulfonamide,
le 5-[5-bromo-4-((R)-2-hydroxy-1-méthyl-éthylamino)-pyrimidin-2-ylamino]-2-méthyl-N-pyridin-4-ylméthyl-benzènesulfonamide,
le 5-[5-bromo-4-((R)-2-hydroxy-1-méthyl-éthylamino)-pyrimidin-2-ylamino]-2-méthyl-N-(2-phénoxy-éthyl)-benzènesulfonamide,
le 5-[5-bromo-4-((R)-2-hydroxy-1-méthyl-éthylamino)-pyrimidin-2-ylamino]-N-(2-méthoxy-benzyl)-2-méthyl-benzènesulfonamide,
le 5-[5-bromo-4-((R)-2-hydroxy-1-méthyl-éthylamino)-pyrimidin-2-ylamino]-2-méthyl-N-[2-(4-sulfamoyl-phényl)-éthyl]-benzènesulfonamide,
le 5-[5-bromo-4-((R)-2-hydroxy-1-méthyl-éthylamino)-pyrimidin-2-ylamino]-2-méthyl-N-(4-méthyl-benzyl)-benzènesulfonamide,
le 3-[5-bromo-4-((R)-1-hydroxyméthyl-propylamino)-pyrimidin-2-ylamino]-N-cyclohexylméthyl-benzènesulfonamide,
le 3-[5-bromo-4-((R)-1-hydroxyméthyl-propylamino)-pyrimidin-2-ylamino]-N-(3-imidazol-1-yl-propyl)-benzènesulfonamide,
le 3-[5-bromo-4-((R)-1-hydroxyméthyl-propylamino)-pyrimidin-2-ylamino]-N-(3-trifluorométhyl-benzyl)-benzènesulfonamide,
le 3-[5-bromo-4-((R)-1-hydroxyméthyl-propylamino)-pyrimidin-2-ylamino]-N-furan-2-ylméthyl-benzènesulfonamide,
le 3-[5-bromo-4-((R)-1-hydroxyméthyl-propylamino)-pyrimidin-2-ylamino]-N-(4-trifluorométhyl-benzyl)-benzènesulfonamide,
le 3-[5-bromo-4-((R)-1-hydroxyméthyl-propylamino)-pyrimidin-2-ylamino]-N-(3-phényl-propyl)-benzènesulfonamide,
le 3-[5-bromo-4-((R)-1-hydroxyméthyl-propylamino)-pyrimidin-2-ylamino]-N-[3-(2-oxo-pyrrolidin-1-yl)-propyl]-benzènesulfonamide,
le 3-[5-bromo-4-((R)-1-hydroxyméthyl-propylamino)-pyrimidin-2-ylamino]-N-(3-méthyl-butyl)-benzènesulfonamide,
le 3-[5-bromo-4-((R)-1-hydroxyméthyl-propylamino)-pyrimidin-2-ylamino]-N-(3-diméthylamino-propyl)-benzènesulfonamide,
le 3-[5-bromo-4-((R)-1-hydroxyméthyl-propylamino)-pyrimidin-2-ylamino]-N-(2-diisopropylamino-éthyl)-benzènesulfonamide,
le 3-[5-bromo-4-((R)-1-hydroxyméthyl-propylamino)-pyrimidin-2-ylamino]-N-(4-cyano-cyclohexylméthyl)-benzènesulfonamide,
le 3-[5-bromo-4-((R)-1-hydroxyméthyl-propylamino)-pyrimidin-2-ylamino]-N-phénéthyl-benzènesulfonamide,
le 3-[5-bromo-4-((R)-1-hydroxyméthyl-propylamino)-pyrimidin-2-ylamino]-N-[2-(4-chloro-phényl)-éthyl]-benzènesulfonamide,
le 3-[5-bromo-4-((R)-1-hydroxyméthyl-propylamino)-pyrimidin-2-ylamino]-N-(5-méthyl-furan-2-ylméthyl)-benzènesulfonamide,
le 3-[5-bromo-4-((R)-1-hydroxyméthyl-propylamino)-pyrimidin-2-ylamino]-N-(2-morpholin-4-yl-éthyl)-benzènesulfonamide,
le 3-[5-bromo-4-((R)-1-hydroxyméthyl-propylamino)-pyrimidin-2-ylamino]-N-(2-méthoxy-éthyl)-benzènesulfonamide,
le 3-[5-bromo-4-((R)-1-hydroxyméthyl-propylamino)-pyrimidin-2-ylamino]-N-thiophén-2-ylméthyl-benzènesulfonamide,
le 3-[5-bromo-4-((R)-1-hydroxyméthyl-propylamino)-pyrimidin-2-ylamino]-N-(2-pyrrolidin-1-yl-éthyl)-benzènesulfonamide,
le 3-[5-bromo-4-((R)-1-hydroxyméthyl-propylamino)-pyrimidin-2-ylamino]-N-(2-pipéridin-1-yl-éthyl)-benzènesulfonamide,
le 3-[5-bromo-4-((R)-1-hydroxyméthyl-propylamino)-pyrimidin-2-ylamino]-N-(3-fluoro-benzyl)-benzènesulfonamide,
le 3-[5-bromo-4-((R)-1-hydroxyméthyl-propylamino)-pyrimidin-2-ylamino]-N-[2-(1-méthyl-pyrrolidin-2-yl)-éthyl]-benzènesulfonamide,
le 3-[5-bromo-4-((R)-1-hydroxyméthyl-propylamino)-pyrimidin-2-ylamino]-N-pyridin-3-ylméthyl-benzènesulfonamide,
le 3-[5-bromo-4-((R)-1-hydroxyméthyl-propylamino)-pyrimidin-2-ylamino]-N-(4-fluoro-benzyl)-benzènesulfonamide,
le 3-[5-bromo-4-((R)-1-hydroxyméthyl-propylamino)-pyrimidin-2-ylamino]-N-pyridin-4-ylméthyl-benzènesulfonamide,
le 3-[5-bromo-4-((R)-1-hydroxyméthyl-propylamino)-pyrimidin-2-ylamino]-N-(2-phénoxy-éthyl)-benzènesulfonamide,
le 3-[5-bromo-4-((R)-1-hydroxyméthyl-propylamino)-pyrimidin-2-ylamino]-N-(2-méthoxy-brenzyl)-benzènesulfonamide,
le 3-[5-bromo-4-((R)-1-hydroxyméthyl-propylamino)-pyrimidin-2-ylamino]-N-(4-méthyl-benzyl)-benzènesulfonamide,
le 3-[5-bromo-4-((R)-1-hydroxyméthyl-propylamino)-pyrimidin-2-ylamino]-N-(5-méthylsulfanyl-1H-[1,2,4]triazol-3-yl)-benzènesulfonamide,
le 3-[5-bromo-4-((R)-2-hydroxy-1-méthyl-éthylamino)-pyrimidin-2-ylamino]-N-(2-diméthylamino-éthyl)-benzènesulfonamide,
le 3-[5-bromo-4-((R)-2-hydroxy-1-méthyl-éthylamino)-pyrimidin-2-ylamino]-N-(3-imidazol-1-yl-propyl)-benzènesulfonamide,
le 3-[5-bromo-4-((R)-2-hydroxy-1-méthyl-éthylamino)-pyrimidin-2-ylamino]-N-(3-trifluorométhyl-benzyl)-benzènesulfonamide,
le 3-[5-bromo-4-((R)-2-hydroxy-1-méthyl-éthylamino)-pyrimidin-2-ylamino]-N-furan-2-ylméthyl-benzènesulfonamide,
le 3-[5-bromo-4-((R)-2-hydroxy-1-méthyl-éthylamino)-pyrimidin-2-ylamino]-N-(4-trifluorométhyl-benzyl)-benzènesulfonamide,
le 3-[5-bromo-4-((R)-2-hydroxy-1-méthyl-éthylamino)-pyrimidin-2-ylamino]-N-[3-(2-oxo-pyrrolidin-1-yl)-propyl]-benzènesulfonamide,
le 3-[5-bromo-4-((R)-2-hydroxy-1-méthyl-éthylamino)-pyrimidin-2-ylamino]-N-(3-méthyl-butyl)-benzènesulfonamide,
le 3-[5-bromo-4-((R)-2-hydroxy-1-méthyl-éthylamino)-pyrimidin-2-ylamino]-N-(3-diméthylamino-propyl)-benzènesulfonamide,
le 3-[5-bromo-4-((R)-2-hydroxy-1-méthyl-éthylamino)-pyrimidin-2-ylamino]-N-(2-diisopropylamino-éthyl)-benzènesulfonamide,
le 3-[5-bromo-4-((R)-2-hydroxy-1-méthyl-éthylamino)-pyrimidin-2-ylamino]-N-(4-cyano-cyclohexylméthyl)-benzènesulfonamide,
le 3-[5-bromo-4-((R)-2-hydroxy-1-méthyl-éthylamino)-pyrimidin-2-ylamino]-N-phénétyl-benzènesulfonamide,
le 3-[5-bromo-4-((R)-2-hydroxy-1-méthyl-éthylamino)-pyrimidin-2-ylamino]-N-[2-(4-chloro-phényl)-éthyl]-benzènesulfonamide,
le 3-[5-bromo-4-((R)-2-hydroxy-1-méthyl-éthylamino)-pyrimidin-2-ylamino]-N-(5-méthyl-furan-2-ylméthyl)-benzènesulfonamide,
le 3-[5-bromo-4-((R)-2-hydroxy-1-méthyl-éthylamino)-pyrimidin-2-ylamino]-N-(2-morpholin-4-yl-éthyl)-benzènesulfonamide,
le 3-[5-bromo-4-((R)-2-hydroxy-1-méthyl-éthylamino)-pyrimidin-2-ylamino]-N-(2-méthoxy-éthyl)-benzènesulfonamide,
le 3-[5-bromo-4-((R)-2-hydroxy-1-méthyl-éthylamino)-pyrimidin-2-ylamino]-N-thiophén-2-ylméthyl-benzènesulfonamide,
le 3-[5-bromo-4-((R)-2-hydroxy-1-méthyl-éthylamino)-pyrimidin-2-ylamino]-N-(2-pyrrolidin-1-yl-éthyl)-benzènesulfonamide,
le 3-[5-bromo-4-((R)-2-hydroxy-1-méthyl-éthylamino)-pyrimidin-2-ylamino]-N-(2-pipéridin-1-yl-éthyl)-benzènesulfonamide,
le 3-[5-bromo-4-((R)-2-hydroxy-1-méthyl-éthylamino)-pyrimidin-2-ylamino]-N-(3-fluoro-benzyl)-benzènesulfonamide,
le 3-[5-bromo-4-((R)-2-hydroxy-1-méthyl-éthylamino)-pyrimidin-2-ylamino]-N-[2-(1-méthyl-pyrrolidin-2-yl)-éthyl]-benzènesulfonamide,
le 3-[5-bromo-4-((R)-2-hydroxy-1-méthyl-éthylamino)-pyrimidin-2-ylamino]-N-pyridin-3-ylméthyl-benzènesulfonamide,
le 3-[5-bromo-4-((R)-2-hydroxy-1-méthyl-éthylamino)-pyrimidin-2-ylamino]-N-(4-fluoro-benzyl)-benzènesulfonamide,
le 3-[5-bromo-4-((R)-2-hydroxy-1-méthyl-éthylamino)-pyrimidin-2-ylamino]-N-pyridin-4-ylméthyl-benzènesulfonamide,
le 3-[5-bromo-4-((R)-2-hydroxy-1-méthyl-éthylamino)-pyrimidin-2-ylamino]-N-(2-phénoxy-éthyl)-benzènesulfonamide,
le 3-[5-bromo-4-((R)-2-hydroxy-1-méthyl-éthylamino)-pyrimidin-2-ylamino]-N-(2-méthoxy-benzyl)-benzènesulfonamide,
le 3-[5-bromo-4-((R)-2-hydroxy-1-méthyl-éthylamino)-pyrimidin-2-ylamino]-N-(4-méthyl-benzyl)-benzènesulfonamide,
le 3-[5-bromo-4-((R)-2-hydroxy-1-méthyl-éthylamino)-pyrimidin-2-ylamino]-N-(5-méthylsulfanyl-1H-[1,2,4]triazol-3-yl)-benzènesulfonamide,
le 4-[5-bromo-4-((R)-1-hydroxyméthyl-propylamino)-pyrimidin-2-ylamino]-benzènesulfonamide,
le fluorure de 3-[5-bromo-4-((R)-2-hydroxy-1-méthyl-éthylamino)-pyrimidin-2-ylamino]-benzènesulfonyle,
le fluorure de 5-[5-bromo-4-((R)-2-hydroxy-1-méthyl-éthylamino)-pyrimidin-2-ylamino]-2-méthyl-benzènesulfonyle,
le fluorure de 3-[5-bromo-4-((R)-1-hydroxyméthyl-propylamino)-pyrimidin-2-ylamino]-benzènesulfonyle,
le fluorure de 5-[5-bromo-4-((R)-1-hydroxyméthyl-propylamino)-pyrimidin-2-ylamino]-2-méthyl-benzènesulfonyle,
le fluorure de 3-[5-bromo-4-((S)-1-hydroxyméthyl-2-méthyl-propylamino)-pyrimidin-2-ylamino]-benzènesulfonyle,
le fluorure de 3-[5-bromo-4-((S)-1-hydroxyméthyl-2-méthyl-propylamino)-pyrimidin-2-ylamino]-benzènesulfonyle,
le fluorure de 4-[5-bromo-4-((R)-2-hydroxy-1-méthyl-éthylamino)-pyrimidin-2-ylamino]-benzènesulfonyle,
le fluorure de 4-[5-bromo-4-((R)-1-hydroxyméthyl-propylamino)-pyrimidin-2-ylamino]-benzènesulfonyle,
le fluorure de 4-[5-bromo-4-((S)-1-hydroxyméthyl-2-méthyl-propylamino)-pyrimidin-2-ylamino]-benzènesulfonyle,
le 4-[5-bromo-4-((R)-2-hydroxy-1-méthyl-éthylamino)-pyrimidin-2-ylamino]-N-cyclohexylméthyl-benzènesulfonamide,
le 4-[5-bromo-4-((R)-2-hydroxy-1-méthyl-éthylamino)-pyrimidin-2-ylamino]-N-(4-phényl-butyl)-benzènesulfonamide,
le 4-[5-bromo-4-((R)-2-hydroxy-1-méthyl-éthylamino)-pyrimidin-2-ylamino]-N-(2-diméthylamino-éthyl)-benzènesulfonamide,
le 4-[5-bromo-4-((R)-2-hydroxy-1-méthyl-éthylamino)-pyrimidin-2-ylamino]-N-(3-imidazol-1-yl-propyl)-benzènesulfonamide,
le 4-[5-bromo-4-((R)-2-hydroxy-1-méthyl-éthylamino)-pyrimidin-2-ylamino]-N-(3-trifluorométhyl-benzyl)-benzènesulfonamide,
le 4-[5-bromo-4-((R)-2-hydroxy-1-méthyl-éthylamino)-pyrimidin-2-ylamino]-N-(4-trifluorométhyl-benzyl)-benzènesulfonamide,
le 4-[5-bromo-4-((R)-2-hydroxy-1-méthyl-éthylamino)-pyrimidin-2-ylamino]-N-(3-phényl-propyl)-benzènesulfonamide,
le 4-[5-bromo-4-((R)-2-hydroxy-1-méthyl-éthylamino)-pyrimidin-2-ylamino]-N-[3-(2-oxo-pyrrolidin-1-yl)-propyl]-benzènesulfonamide,
le 4-[5-bromo-4-((R)-2-hydroxy-1-méthyl-éthylamino)-pyrimidin-2-ylamino]-N-(3-méthyl-butyl)-benzènesulfonamide,
le 4-[5-bromo-4-((R)-2-hydroxy-1-méthyl-éthylamino)-pyrimidin-2-ylamino]-N-(2-diisopropylamino-éthyl)-benzènesulfonamide,
le 4-[5-bromo-4-((R)-2-hydroxy-1-méthyl-éthylamino)-pyrimidin-2-ylamino]-N-cyclopropyl-benzènesulfonamide,
le 4-[5-bromo-4-((R)-2-hydroxy-1-méthyl-éthylamino)-pyrimidin-2-ylamino]-N-(4-cyano-cyclohexylméthyl)-benzènesulfonamide,
le 4-[5-bromo-4-((R)-2-hydroxy-1-méthyl-éthylamino)-pyrimidin-2-ylamino]-N-phénéthyl-benzènesulfonamide,
le 4-[5-bromo-4-((R)-2-hydroxy-1-méthyl-éthylamino)-pyrimidin-2-ylamino]-N-[2-(4-chloro-phényl)-éthyl]-benzènesulfonamide,
le 4-[5-bromo-4-((R)-2-hydroxy-1-méthyl-éthylamino)-pyrimidin-2-ylamino]-N-(5-méthyl-furan-2-ylméthyl)-benzènesulfonamide,
le 4-[5-bromo-4-((R)-2-hydroxy-1-méthyl-éthylamino)-pyrimidin-2-ylamino]-N-(2-morpholin-4-yl-éthyl)-benzènesulfonamide,
le 4-[5-bromo-4-((R)-2-hydroxy-1-méthyl-éthylamino)-pyrimidin-2-ylamino]-N-(2-méthoxy-éthyl)-benzènesulfonamide,
le 4-[5-bromo-4-((R)-2-hydroxy-1-méthyl-éthylamino)-pyrimidin-2-ylamino]-N-thiophén-2-ylméthyl-benzènesulfonamide,
le 4-[5-bromo-4-((R)-2-hydroxy-1-méthyl-éthylamino)-pyrimidin-2-ylamino]-N-[2-(4-méthoxy-phényl)-éthyl]-benzènesulfonamide,
le 4-[5-bromo-4-((R)-2-hydroxy-1-méthyl-éthylamino)-pyrimidin-2-ylamino]-N-(2-pyrrolidin-1-yl-éthyl)-benzènesulfonamide,
le 4-[5-bromo-4-((R)-2-hydroxy-1-méthyl-éthylamino)-pyrimidin-2-ylamino]-N-(2-pipéridin-1-yl-éthyl)-benzènesulfonamide,
le 4-[5-bromo-4-((R)-2-hydroxy-1-méthyl-éthylamino)-pyrimidin-2-ylamino]-N-(3-fluoro-benzyl)-benzènesulfonamide,
le 4-[5-bromo-4-((R)-2-hydroxy-1-méthyl-éthylamino)-pyrimidin-2-ylamino]-N-[2-(1-méthyl-pyrrolidin-2-yl)-éthyl]-benzènesulfonamide,
le 4-[5-bromo-4-((R)-2-hydroxy-1-méthyl-éthylamino)-pyrimidin-2-ylamino]-N-pyridin-3-ylméthyl-benzènesulfonamide,
le 4-[5-bromo-4-((R)-2-hydroxy-1-méthyl-éthylamino)-pyrimidin-2-ylamino]-N-(4-fluoro-benzyl)-benzènesulfonamide,
le 4-[5-bromo-4-((R)-2-hydroxy-1-méthyl-éthylamino)-pyrimidin-2-ylamino]-N-pyridin-4-ylméthyl-benzènesulfonamide,
le 4-[5-bromo-4-((R)-2-hydroxy-1-méthyl-éthylamino)-pyrimidin-2-ylamino]-N-(2-phénoxy-éthyl)-benzènesulfonamide,
le 4-[5-bromo-4-((R)-2-hydroxy-1-méthyl-éthylamino)-pyrimidin-2-ylamino]-N-(2-méthoxy-benzyl)-benzènesulfonamide,
le 4-[5-bromo-4-((R)-2-hydroxy-1-méthyl-éthylamino)-pyrimidin-2-ylamino]-N-(4-méthyl-benzyl)-benzènesulfonamide,
le 4-[5-bromo-4-((R)-2-hydroxy-1-méthyl-éthylamino)-pyrimidin-2-ylamino]-N-(5-méthylsulfanyl-1H-[1,2,4]triazol-3-yl)-benzènesulfonamide,
le 4-[5-bromo-4-((R)-1-hydroxyméthyl-propylamino)-pyrimidin-2-ylamino]-N-cyclohexylméthyl-benzènesulfonamide,
le 4-[5-bromo-4-((R)-1-hydroxyméthyl-propylamino)-pyrimidin-2-ylamino]-N-(2-diméthylamino-éthyl)-benzènesulfonamide,
le 4-[5-bromo-4-((R)-1-hydroxyméthyl-propylamino)-pyrimidin-2-ylamino]-N-(3-imidazol-1-yl-propyl)-benzènesulfonamide,
le 4-[5-bromo-4-((R)-1-hydroxyméthyl-propylamino)-pyrimidin-2-ylamino]-N-(3-trifluorométhyl-benzyl)-benzènesulfonamide,
-le 4-[5-bromo-4-((R)-1-hydroxyméthyl-propylamino)-pyrimidin-2-ylamino]-N-furan-2-ylméthyl-benzènesulfonamide,
le 4-[5-bromo-4-((R)-1-hydroxyméthyl-propylamino)-pyrimidin-2-ylamino]-N-(4-trifluorométhyl-benzyl)-benzènesulfonamide,
le 4-[5-bromo-4-((R)-1-hydroxyméthyl-propylamino)-pyrimidin-2-ylamino]-N-(3-phényl-propyl)-benzènesulfonamide,
le 4-[5-bromo-4-((R)-1-hydroxyméthyl-propylamino)-pyrimidin-2-ylamino]-N-[3-(2-oxo-pyrrolidin-1-yl)-propyl]-benzènesulfonamide,
le 4-[5-bromo-4-((R)-1-hydroxyméthyl-propylamino)-pyrimidin-2-ylamino]-N-(3-méthyl-butyl)-benzènesulfonamide,
le 4-[5-bromo-4-((R)-1-hydroxyméthyl-propylamino)-pyrimidin-2-ylamino]-N-(3-diméthylamino-propyl)-benzènesulfonamide,
le 4-[5-bromo-4-((R)-1-hydroxyméthyl-propylamino)-pyrimidin-2-ylamino]-N-(2-diisopropylamino-éthyl)-benzènesulfonamide,
le 4-[5-bromo-4-((R)-1-hydroxyméthyl-propylamino)-pyrimidin-2-ylamino]-N-cyclopropyl-benzènesulfonamide,
le 4-[5-bromo-4-((R)-1-hydroxyméthyl-propylamino)-pyrimidin-2-ylamino]-N-(4-cyano-cyclohexylméthyl)-benzènesulfonamide,
le 4-[5-bromo-4-((R)-1-hydroxyméthyl-propylamino)-pyrimidin-2-ylamino]-N-phénéthyl-benzènesulfonamide,
le 4-[5-bromo-4-((R)-1-hydroxyméthyl-propylamino)-pyrimidin-2-ylamino]-N-(3,4-diméthoxy-benzyl)-benzènesulfonamide,
le 4-[5-bromo-4-((R)-1-hydroxyméthyl-propylamino)-pyrimidin-2-ylamino]-N-[2-(4-chloro-phényl)-éthyl]-benzènesulfonamide,
le 4-[5-bromo-4-((R)-1-hydroxyméthyl-propylamino)-pyrimidin-2-ylamino]-N-(5-méthyl-furan-2-ylméthyl)-benzènesulfonamide,
le 4-[5-bromo-4-((R)-1-hydroxyméthyl-propylamino)-pyrimidin-2-ylamino]-N-(2-morpholin-4-yl-éthyl)-benzènesulfonamide,
le 4-[5-bromo-4-((R)-1-hydroxyméthyl-propylamino)-pyrimidin-2-ylamino]-N-(2-méthoxy-éthyl)-benzènesulfonamide,
le 4-[5-bromo-4-((R)-1-hydroxyméthyl-propylamino)-pyrimidin-2-ylamino]-N-thiophén-2-ylméthyl-benzènesulfonamide,
le 4-[5-bromo-4-((R)-1-hydroxyméthyl-propylamino)-pyrimidin-2-ylamino]-N-[2-(4-méthoxy-phényl)-éthyl]-benzènesulfonamide,
le 4-[5-bromo-4-((R)-1-hydroxyméthyl-propylamino)-pyrimidin-2-ylamino]-N-(2-pyrrolidin-1-yl-éthyl)-benzènesulfonamide,
le 4-[5-bromo-4-((R)-1-hydroxyméthyl-propylamino)-pyrimidin-2-ylamino]-N-(2-pipéridin-1-yl-éthyl)-benzènesulfonamide,
le 4-[5-bromo-4-((R)-1-hydroxyméthyl-propylamino)-pyrimidin-2-ylamino]-N-(3-fluoro-benzyl)-benzènesulfonamide,
le 4-[5-bromo-4-((R)-1-hydroxyméthyl-propylamino)-pyrimidin-2-ylamino]-N-[2-(1-méthyl-pyrrolidin-2-yl)-éthyl]-benzènesulfonamide,
le 4-[5-bromo-4-((R)-1-hydroxyméthyl-propylamino)-pyrimidin-2-ylamino]-N-pyridin-3-ylméthyl-benzènesulfonamide,
le 4-[5-bromo-4-((R)-1-hydroxyméthyl-propylamino)-pyrimidin-2-ylamino]-N-(4-fluoro-benzyl)-benzènesulfonamide,
le 4-[5-bromo-4-((R)-1-hydroxyméthyl-propylamino)-pyrimidin-2-ylamino]-N-pyridin-4-ylméthyl-benzènesulfonamide,
le 4-[5-bromo-4-((R)-1-hydroxyméthyl-propylamino)-pyrimidin-2-ylamino]-N-(2-phénoxy-éthyl)-benzènesulfonamide,
le 4-[5-bromo-4-((R)-1-hydroxyméthyl-propylamino)-pyrimidin-2-ylamino]-N-(2-méthoxy-benzyl)-benzènesulfonamide,
le 4-[5-bromo-4-((R)-1-hydroxyméthyl-propylamino)-pyrimidin-2-ylamino]-N-(4-méthyl-benzyl)-benzènesulfonamide, ou
le 4-[5-bromo-4-((R)-1-hydroxyméthyl-propylamino)-pyrimidin-2-ylamino]-N-(5-méthylsulfanyl-1H-[1,2,4]triazol-3-yl)-benzènesulfonamide.

3. Composé de formule générale (Ia) 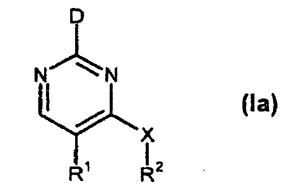 dans laquelle D représente un atome de chlore, et X représente un groupe -NH- ou un groupe -O-, R¹ et R² présentent les significations indiquées dans la formule générale (I), en tant qu'intermédiaires pour la préparation du composé de formule générale (I) par réaction dans du MeCN/HCl avec de l'Ar-NH₂.

4. Utilisation des composés de formule générale (I) selon les revendications 1 ou 2, pour la production d'un médicament destiné au traitement du cancer, de maladies auto-immunes, d'une inflammation de muqueuse et de l'alopécie induite par des agents chimiothérapeutiques, de maladies cardiovasculaires, de maladies infectieuses, de maladies néphrologiques, de maladies neurodégénératives chroniques et aiguës et d'infections virales.

5. Utilisation selon la revendication 4, **caractérisée en ce que**, par cancer, on entend des tumeurs solides et la leucémie, par maladies auto-immunes, on entend le psoriasis, l'alopécie et la sclérose en plaques, par maladies cardiovasculaires, on entend des sténoses, des artérioscléroses et des resténoses, par maladies infectieuses, on entend des maladies provoquées par des parasites unicellulaires, par maladies néphrologiques, on entend la glomérulonéphrite, par maladies neurodégénératives chroniques, on entend la maladie de Huntington, la sclérose latérale amyotrophique, la maladie de Parkinson, la démence due au SIDA et la maladie d'Alzheimer, par maladies neurodégénératives aiguës, on entend des ischémies du cerveau et des neurotraumatismes, et par infections virales, on entend des infections par cytomégalovirus, l'herpès, l'hépatite B et C et des maladies dues au VIH.

6. Médicaments qui contiennent au moins un composé selon la revendication 1 ou 2.

7. Médicaments selon la revendication 6, destinés au traitement du cancer, de maladies auto-immunes, de maladies cardiovasculaires, de maladies infectieuses, de maladies néphrologiques, de maladies neurodégénératives et d'infections virales.

8. Préparation pharmaceutique contenant des composés selon la revendication 1 ou 2 avec des substances de formulation et de support appropriées.
